# EUROPEAN PATENT SPECIFICATION

(11) **EP 2 567 970 B1**
(45) Date of publication and mention of the grant of the patent: **17.06.2015**
(21) Application number: 12171823.3
(22) Date of filing: 03.10.2006
(51) Int. Cl.: C07K 14/435

(54) **Novel nucleotide and amino acid sequences, and assays and methods of use thereof for diagnosis**
Neuartiges Nukleotid und neuartige Aminosäuresequenzen sowie Assays und Verfahren zu deren Verwendung zur Diagnose
Nouvelles séquences de nucléotides et d'acides aminés, leurs dosages et procédés d'utilisation pour le diagnostic

(30) Priority: 03.10.2005 US 722400 P; 14.11.2005 US 735825 P; 30.11.2005 IL 17229705; 06.12.2005 US 742929 P
(43) Date of publication of application: 13.03.2013
(62) Divisional of application: 06796147.4
(73) Proprietor: Compugen Ltd., 69512 Tel Aviv (IL)
(72) Inventor: Sella-Tavor, Osnat, 19330 Kfar Kish (IL); Pollock, Sarah, 63506 Tel-Aviv (IL); Cojocaru, Gad, S., 47248 Ramat-Hasharon (IL); Novik, Amit, 40600 Tel-Mond (IL); Bazak, Lily, 53461 Givatayim (IL); Tsypkin, Elena, 64074 Tel-Aviv (IL); Wallach, Shira, 45265 Hod Hasharon (IL); Sameah-Greenwald, Shirley, 44419 Kfar Saba (IL)
(74) Representative: Becker Kurig Straus

(56) References cited:
- WO-A2-01/64835
- WO-A2-02/060950
- WO-A2-2005/072340
- US-A- 5 952 199

## Description

### FIELD OF THE INVENTION

The present invention is related to methods for detecting the presence or severity of an inflammatory or artherosclerotic disease, disorder or condition.

### BACKGROUND OF THE INVENTION

Diagnostic markers are important for early diagnosis of many diseases, as well as predicting response to treatment, monitoring treatment and determining prognosis of such diseases.

Serum markers are examples of such diagnostic markers and are used for diagnosis of many different diseases. Such serum markers typically encompass secreted proteins and/or peptides; however, some serum markers may be released to the blood upon tissue lysis, such as from myocardial infarction (for example Troponin-I). Serum markers can also be used as risk factors for disease (for example base-line levels of CRP, as a predictor of cardiovascular disease), to monitor disease activity and progression (for example, determination of CRP levels to monitor acute phase inflammatory response) and to predict and monitor drug response (for example, as shedded fragments of the protein Erb-B2).

Immunohistochemistry (IHC) is the study of distribution of an antigen of choice in a sample based on specific antibody-antigen binding, typically on tissue slices. The antibody features a label which can be detected, for example as a stain which is detectable under a microscope. The tissue slices are prepared by being fixed. IHC is therefore particularly suitable for antibody-antigen reactions that are not disturbed or destroyed by the process of tissue fixation.

IHC permits determining the localization of binding, and hence mapping of the presence of the antigen within the tissue and even within different compartments in the cell. Such mapping can provide useful diagnostic information, including:
1) the histological type of the tissue sample
2) the presence of specific cell types within the sample
3) information on the physiological and/or pathological state of cells (e.g. which phase of the cell-cycle they are in)
4) the presence of disease related changes within the sample
5) differentiation between different specific disease subtypes where it is already known the tissue is of disease state (for example, the differentiation between different tumor types when it is already known the sample was taken from cancerous tissue).

IHC information is valuable for more than diagnosis. It can also be used to determine prognosis and therapy treatment (as in the case of HER-2 in breast cancer) and monitor disease.

IHC protein markers could be from any cellular location. Most often these markers are membrane proteins but secreted proteins or intracellular proteins (including intranuclear) can be used as an IHC marker too.

IHC has at least two major disadvantages. It is performed on tissue samples and therefore a tissue sample has to be collected from the patient, which most often requires invasive procedures like biopsy associated with pain, discomfort, hospitalization and risk of infection. In addition, the interpretation of the result is observer dependant and therefore subjective. There is no measured value but rather only an estimation (on a scale of 1-4) of how prevalent the antigen on target is.

US 5,952,199 discloses_chimeric vascular endothelial growth factor (VEGF) receptor proteins comprising amino acid sequences derived from the VEGF receptors flt-1, FLT4, and KDR and the murine homologue to the human KDR receptor FLK-1. The chimeric VEGF receptor proteins bind to VEGF and antagonize the endothelial cell proliferative and angiogenic activity thereof.

WO 2005/072340_discloses specific polypeptides, polynucleotides encoding the same, as well as methods and pharmaceutical compositions which can be used to treat various disorders including cancer, immunological-related disorders, blood-related disorders and skin-related disorders. Methods and kits for diagnosing, determining predisposition and/or prognosis of various disorders using as diagnostic markers the polypeptides and polynucleotides are also disclosed.

WO 01/64835_provides polynucleotides and proteins encoded by such polynucleotides, along with uses for these polynucleotides and proteins, for example in therapeutic, diagnostic and research methods.

WO 02/060950_discloses purified Flt4 receptor tyrosine kinase polypeptides and fragments thereof, polynucleotides encoding such polypeptides, antibodies that specifically bind such polypeptides, and uses thereof for imaging lymphatic tissues particularly during cancer treatment. Monitoring lymphatic vessels for inflammation, infection, growth and traumas is highlighted as part of the anti-cancer treatment regimen.

### SUMMARY OF THE INVENTION

The present invention provides means and methods for detecting the presence or severity of an inflammatory or atherosclerotic disease, disorder or condition.

The present invention relates, in some embodiments, to diagnostic assays for disease detection, which in some embodiments, utilizes a biological sample taken from a subject (patient), which for example may comprise a body fluid or secretion including but not limited to seminal plasma, blood, serum, urine, prostatic fluid, seminal fluid, semen, the external secretions of the skin, respiratory, intestinal, and genitourinary tracts, tears, cerebrospinal fluid, sputum, saliva, milk, peritoneal fluid, pleural fluid, cyst fluid, secretions of the breast ductal system (and/or lavage thereof), broncho alveolar lavage, lavage of the reproductive system, lavage of any other part of the body or system in the body, stool or a tissue sample, or any combination thereof. In some embodiments, the term encompasses samples of in vivo cell culture constituents. The sample can optionally be diluted with a suitable eluant before contacting the sample to an antibody and/or performing any other diagnostic assay.

This invention discloses edge portions of an isolated polypeptide having an amino acid sequence corresponding to that set forth in SEQ ID NOs: 18.

In one embodiment the edge portion this invention consists of an isolated polypeptide consisting of an amino acid sequence being having the sequence ELYTSTSPSSSSSSPLSSSSSSSSSSSS (SEQ ID NO: 462) of HSFLT_P10 (SEQ ID NO:18).

In another embodiment, the edge portion consist of an amino acid sequence having the sequence DQEAPYLLRNLSDHTVAISSSTTLDCHANGVPEPQITWFKNNHKIQQEPELYTSTS-PSSSSSSPLSSSSSSSSSSSS (SEQ ID NO: 463) of HSFLT_P10 (SEQ ID NO:18).

In one embodiment the method of the present invention comprises detecting the expression of a unique edge portion of a polypeptide having the amnino acid sequence as set forth in SEQ ID NO:18, wherein said unique edge portion consists of the amino acid sequence set forth in any one of SEQ ID NOs: 463 and 462.

In some embodiemtns the inflammatory disease is a chronic inflammatory disease, preferably preeclampsia.

Methods for preparing, isolating, deriving, etc., the polypeptides of this invention are well known are well known in the art. In some embodiments, the polypeptides may be obtained through known protein evolution techniques available in the art. In some embodiments, the polypeptides of this invention may be obtained via rational design, based on a particular native polypeptide sequence.

In some embodiments, this invention provides for antibodies or antibody fragments specifically interacting with or recognizing a polypeptide of this invention.

In one embodiment, the antibody recognizes one or more epitopes (antigen determinants) contained within the polypeptides of this invention. In some embodiments, reference to the antibody property of "specific interaction" or "recognition" is to be understood as including covalent and noncovalent associations, and with a variance of affinity over several orders of magnitude. Such terms are to be understood as relative, with respect to an index molecule, for which the antibody is though to have little to no specific interaction or recognition.

In one embodiment, the antibodies will specifically interact or recognize a particular antigen determinant. In some embodiments, the antibodies or antibody fragments of this invention will recognize or interact with a polypeptide or protein of the invention, and will not substantially recognize or interact with other molecules, even when present in the same sample, such as a biological sample. In some embodiments, the antibodies of this invention have a specificity such that the specific interaction with or binding to the antigen is at least about 2, or in some embodiments, at least about 5, or in some embodiments, at least about 10-fold greater than interaction or binding observed under the same reaction conditions with a molecule that does not include the antigenic determinant.

In particular, this invention provides an antibody capable of specifically binding to a unique edge portion of the polypeptide set forth in SEQ ID NO:18, the unique portion consisting of the amino acids sequence set forth in any one of SEQ ID NO: 462 and SEQ ID NO:463, but not with a known VEGFR-1 having the amino acid sequence set forth in any one of SEQ ID NO:359 and SEQ ID NO:360, respectively, for detecting preeclampsia in an in vitro biological sample.

The antibodies may be useful, in some embodiments, in detecting qualitative and/or quantitative changes in expression of the polypeptides of this invention. In some embodiments, changes in expression are associated with a particular disease or disorder, such that detection of such changes comprises a diagnostic method of this invention.

In one embodiment, this invention provides a diagnostic kit for detecting an inflammatory or artheroscleritic disease, comprising reagents which detect qualitative and/or quantitative changes in expression of a polypeptide of this invention.

The kit comprises an antibody according to the invention, optionally and preferably, the kit further comprises at least one reagent for performing an ELISA or a Western blot.

In some embodiments, this invention provides a diagnostic method for detection of a polypeptide disclosed in this invention, whereby expression, or relative changes in expression of the polypeptide herald the onset, severity, or prognosis of an individual with regard to the particular disease, disorder or condition.

The polypeptides disclosed in this invention may serve as markers or indicators of disease initation, severity and/or response to treatment, for the indicated disease, disorder or condition, and their use as such is to be considered part of this invention, and part of the methods of this invention.

In some embodiments, the polypeptides and/or methods of this invention may be useful in the diagnosis, treatment or assessment of the prognosis of a subject with acute and chronic inflammation, and/or CVS diseases including for a spectrum of diseases where an inflammatory process plays a substantial role. In some embodiments, the polypeptides and/or methods of this invention may be useful in the diagnosis, treatment or assessment of the prognosis of a subject with hypercholesterolemia, diabetes, atherosclerosis, inflammation that involves blood vessels - whether acute or chronic including but not limited to the coronary arteries and blood vessels of the brain, myocardial infarction, cerebral stroke, peripheral vascular disease, vasculitis, polyarteritis nodosa, ANCA associated small vessel vasculitis, Churg-Strauss syndrome, Henoch-Schonlein purpura, scleroderma, thromboangiitis obliterans, temporal arteritis, Takayasu's arteritis, hypersensitivity vasculitis, Kawasaki disease, Behçet syndrome, and their complications including but not limited to coronary disease, angina pectoris, deep vein thrombosis, renal disease, diabetic nephropathy, lupus nephritis, renal artery thrombosis, renal artery stenosis, atheroembolic disease of the renal arteries, renal vein thrombosis, hemolytic uremic syndrome, thrombotic thrombocytopenic purpura, arteriolar nephrosclerosis, preeclampsia, eclampsia, albuminuria, microalbuminuria, glomerulonephritis, renal failure, hypertension, uremia, cerebrovascular disease, peripheral vascular disease, intermittent claudication, abdominal angina; rheumatic / autoimmune diseases that involve systemic immune reaction including but not limited to rheumatoid arthritis, scleroderma, mixed connective tissue disease, Sjogren syndrome, ankylosing spondylitis, spondyloarthropathy, psoriasis, psoriatic arthritis, myositis and systemic lupus erythematosus; acute and/or chronic infective processes that involve systemic immune reaction including but not limited to pneumonia, bacteremia, sepsis, pyelonephritis, cellulitis, osteomyelitis, meningitis and viral hepatitis; malignant and idiopathic processes that involve systemic immune reaction and/or proliferation of immune cells including but not limited to granulomatous disorders, Wegener's granulomatosis, lymphomatoid granulomatosis / polymorphic reticulosis, idiopathic midline granuloma, multiple myeloma, Waldenstrom's macroglobulinemia, Castleman's disease, amyloidosis, lymphoma, histiocytosis, renal cell carcinoma and paraneoplastic syndromes; conditions where CRP was shown to have a positive correlation with the presence of the condition including but not limited to weight loss, anorexia-cachexia syndrome, extent of disease, recurrence in advanced cancer, diabetes (types 1 & 2), obesity, hypertension, preterm delivery; conditions which have similar symptoms, signs and complications as the conditions above and where the differential diagnosis between them and the conditions above is of clinical importance including but not limited to: other (non vascular) causes of heart disease, renal disease and cerebral disease; other (non rheumatic) causes of arthropathy and musculoskeletal pain; other causes of non-specific symptoms and signs such as fever of unknown origin, loss of appetite, weight loss, nonspecific pains, breathing difficulties, anxiety, or any combination thereof, or any disease disorder or condition associated with inflammation.

In some embodiments, this invention provides a method of detecting, treating and/or assessing prognosis of the disease, disorder or condition, comprising detecting the polypeptides of this invention. In some embodiments, such methods are also referred to herein as methods of screening for variant-detectable disease, whereby the detection of variant expression serves as an indicator for the disease. In some embodiments, such detection may make use of a biomarker, antibody or any method or assay as described herein.

This invention provides a method for screening for a inflammatory or artherosclerotic disease, comprising detecting expression of:
a. unique edge portion of a polypeptide having an amino acid sequence as set forth in SEQ ID NOs: 18;
b. wherein the unique edge portion consist of the amino acid sequence as set forth in SEQ ID NOs: 463 or 462 ;
c. an antibody capable of specifically binding to at least one epitope of a polypeptide comprising an amino acid sequence as set forth in SEQ ID NOs: 18, 463 or 463;
whereby qualitative or quantitative differences in expression as compared to an index sample is indicator for the treatment, diagnosis or assessment of prognosis of the disease, disorder or condition.

In some embodiments, the methods of this invention may be conducted on a cell or tissue or body fluid sample isolated from a subject having, predisposed to, or suspected of having the disease disorder or condition. In some embodiments, the methods are directed to the monitoring of disease progression and/or treatment efficacy and/or relapse of the indicated disease, disorder or condition. In another embodiment, the invention may be used for the selection of a particular therapy, or optimization of a given therapy for a disease, disorder or condition, the method comprising quantitatively and/or qualitatively determining or assessing expression of the polypeptides whereby differences in expression from an index sample, or a sample taken from a subject prior to the initiation of the therapy, or during the course of therapy, is indicative of the efficacy, or optimal activity of the therapy.

All technical and scientific terms used herein should be understood to have the meaning commonly understood by a person skilled in the art to which this invention belongs, as well as any other specified description. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). All of these are hereby incorporated by reference as if fully set forth herein.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

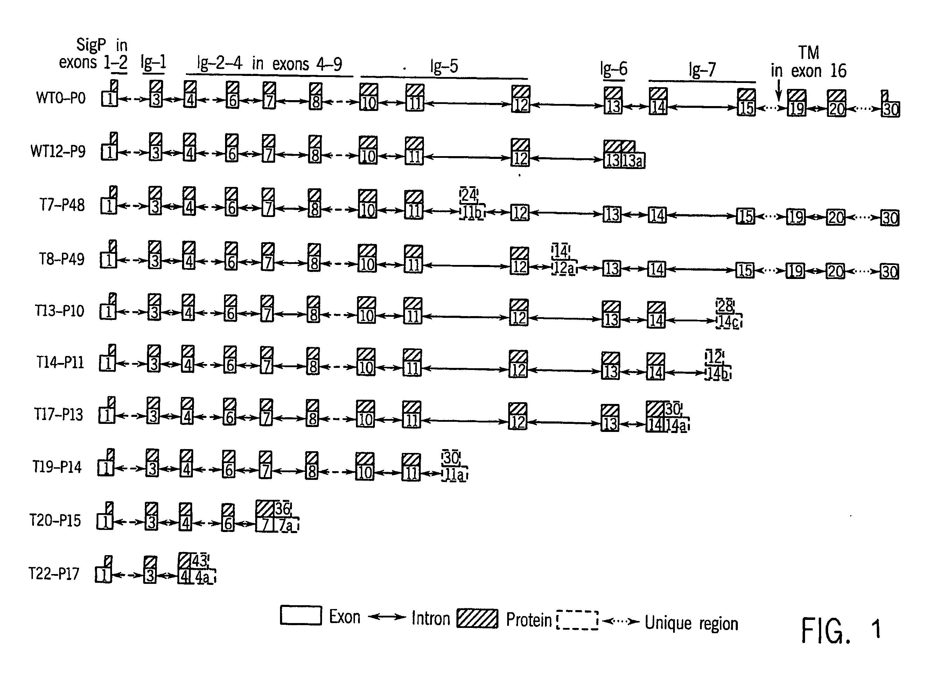

Figure 1 shows the structure of the HSFLT variants mRNA and protein. Exons are represented by white boxes, while introns are represented by two headed arrows. Proteins are shown in boxes with upper right to lower left fill. The unique regions are represented by white boxes with dashed frame. Figure 5 shows expression of Homo sapiens fms-related tyrosine kinase 1 (vascular endothelial growth factor/vascular permeability factor receptor) (FLT1) transcripts detectable by or according to seg20 - HSFLT_seg20 (SEQ ID NO:363) amplicon and primers HSFLT_seg20F (SEQ ID NO:361) and HSFLT_seg20R (SEQ ID NO:362) on a normal panel;

### DESCRIPTION OF EMBODIMENTS

The present invention provides the use of polypeptides, as further described herein as diagnostic markers.

In some embodiments, certain diseases are associated with differential expression, qualitatively or quantitatively, of the polypeptides of this invention. Assessment of such expression, in turn, may serve as a marker for a particular disease state, susceptibility, pathogenesis, etc., including any desired disease-specific event, whose analysis is useful, as will be appreciated by one skilled in the art. In one embodiment, such use as a marker is also referred to herein as the polypeptides being "variant disease markers".

The polypeptides of the present invention, alone or in combination, in some embodiments, can be used for, and in some embodiments are a part of the methods of prognosis, prediction, screening, early diagnosis, staging, therapy selection and treatment monitoring of a inflammatory or artherosclerotic disease. For example, in some embodiments, these markers may be used for the staging of the disease in a patient and/or monitoring the progression of the disease. Also, one or more of the markers may optionally be used in combination with one or more other disease markers (other than those described herein).

Biomolecular sequences (amino acid) uncovered using the methodology of the present invention and described herein can be efficiently utilized, in some embodiments, as tissue or pathological markers and/or as drugs or drug targets for treating or preventing a disease.

In some embodiments, these markers are released to the bloodstream under conditions of a particular disease, and/or are otherwise expressed at a much higher level and/or specifically expressed in tissue or cells afflicted with or demonstrating the disease. In some embodiments, the measurement of these markers, alone or in combination, in patient samples provides information that the diagnostician can correlate with a probable diagnosis of a particular disease and/or a condition that is indicative of a higher risk for a particular disease.

The present invention provides diagnostic assays for an inflammatory or atherosclerotic disease and/or an indicative condition, and methods of use of such markers for detection of the disease and/or an indicative condition, for example in a sample taken from a subject (patient), which in some embodiments, is a blood sample.

Some embodiments of this invention have been exemplified herein wherein cellular localization was determined via the use of four different software programs: (i) tmhmm (from Center for Biological Sequence Analysis, Technical University of Denmark DTU, www,cbs,dtu,dk/services/TMHMMKMHMM2.0b.guide.php) or (ii) tmpred (from EMBnet, maintained by the ISREC Bionformatics group and the LICR Information Technology Office, Ludwig Institute for Cancer Research, Swiss Institute of Bioinformatics, www.ch.embnet.org/software/TMPRED_form.html) for transmembrane region prediction; (iii) signalp_hmm or (iv) signalp_nn (both from Center for Biological Sequence Analysis, Technical University of Denmark DTU, www.cbs.dtu.dk/services/SignalP/background/prediction.php) for signal peptide prediction. The terms "signalp_hmm" and "signalp_nn" refer to two modes of operation for the program SignalP: hmm refers to Hidden Markov Model, while nn refers to neural networks. Localization was also determined through manual inspection of known protein localization and/or gene structure, and the use of heuristics by the individual inventor. In some cases for the manual inspection of cellular localization prediction inventors used the ProLoc computational platform [Einat Hazkani-Covo, Erez Levanon, Galit Rotman, Dan Graur and Amit Novik; (2004) "Evolution of multicellularity in metazoa: comparative analysis of the subcellular localization of proteins in Saccharomyces, Drosophila and Caenorhabditis." Cell Biology International 2004;28(3):171-8.], which predicts protein localization based on various parameters including, protein domains (e.g., prediction of trans-membranous regions and localization thereof within the protein), pI, protein length, amino acid composition, homology to pre-annotated proteins, recognition of sequence patterns which direct the protein to a certain organelle (such as, nuclear localization signal, NLS, mitochondria localization signal), signal peptide and anchor modeling and using unique domains from Pfam that are specific to a single compartment.

In some embodiments, the phrase "disease" refers to its commonly understood meaning, and includes, *inter alia,* any type of pathology and/or damage, including both chronic and acute damage, as well as a progress from acute to chronic damage.

In some embodiments, the phrase "marker" in the context of the present invention refers to a a peptide or a polypeptide, which is differentially present in a sample taken from patients (subjects) having one of the herein-described diseases or conditions, as compared to a comparable sample taken from subjects who do not have one the above-described diseases or conditions.

In some embodiments, the phrase "differentially present" refers to differences in the quantity or quality of a marker present in a sample taken from patients having one of the herein-described diseases or conditions as compared to a comparable sample taken from patients who do not have one of the herein-described diseases or conditions. For example, a nucleic acid fragment may optionally be differentially present between the two samples if the amount of the nucleic acid fragment in one sample is significantly different from the amount of the nucleic acid fragment in the other sample, for example as measured by hybridization and/or NAT-based assays. A polypeptide is differentially present between the two samples if the amount of the polypeptide in one sample is significantly different from the amount of the polypeptide in the other sample. It should be noted that if the marker is detectable in one sample and not detectable in the other, then such a marker can be considered to be differentially present. Optionally, a relatively low amount of up-regulation may serve as the marker, as described herein. One of ordinary skill in the art could easily determine such relative levels of the markers; further guidance is provided in the description of each individual marker below.

In some embodiments, the phrase "diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

In some embodiments, the phrase "qualitative" when in reference to differences in expression levels of a polynucleotide, polypeptide or cluster as described herein, refers to the presence versus absence of expression, or in some embodiments, the temporal regulation of expression, or in some embodiments, the timing of expression, or in some embodiments, the variant expressed, or in some embodiments, any post-translational modifications to the expressed molecule, and others, as will be appreciated by one skilled in the art. In some embodiments, the phrase "qualntitative" when in reference to differences in expression levels of a polynucleotide, polypeptide or cluster as described herein, refers to absolute differences in quantity of expression, as determined by any means, known in the art, or in other embodiments, relative differences, which may be statistically significant, or in some embodiments, when viewed as a whole or over a prolonged period of time, etc., indicate a trend in terms of differences in experession.

In some embodiments, the term "diagnosing" refers to classifying a disease or a symptom, determining a severity of the disease, monitoring disease progression, forecasting an outcome of a disease and/or prospects of recovery. The term "detecting" may also optionally encompass any of the above.

Diagnosis of a disease according to the present invention can, in some embodiments, be effected by determining a level of a polypeptide disclosed in the present invention in a biological sample obtained from the subject, wherein the level determined can be correlated with predisposition to, or presence or absence of the disease. It should be noted that a "biological sample obtained from the subject" may also optionally comprise a sample that has not been physically removed from the subject, as described in greater detail below.

In some embodiments, the term "level" refers to expression levels of protein or to DNA copy number of a marker of the present invention.

Typically the level of the marker in a biological sample obtained from the subject is different (i.e., increased or decreased) from the level of the same variant in a similar sample obtained from a healthy individual (examples of biological samples are described herein).

Numerous well known tissue or fluid collection methods can be utilized to collect the biological sample from the subject in order to determine the level of a polypeptide of the variant of interest in the subject.

Examples include, but are not limited to, fine needle biopsy, needle biopsy, core needle biopsy and surgical biopsy (e.g., brain biopsy), and lavage. Regardless of the procedure employed, once a biopsy/sample is obtained the level of the variant can be determined and a diagnosis can thus be made.

Determining the level of the same variant in normal tissues of the same origin is preferably effected along-side to detect an elevated expression and/or amplification and/or a decreased expression, of the variant as opposed to the normal tissues.

In some embodiments, the term "test amount" of a marker refers to an amount of a marker in a subject's sample that is consistent with a diagnosis of a particular disease or condition. A test amount can be either in absolute amount (e.g., microgram/ml) or a relative amount (e.g., relative intensity of signals).

In some embodiments, the term "control amount" of a marker can be any amount or a range of amounts to be compared against a test amount of a marker. For example, a control amount of a marker can be the amount of a marker in a patient with a particular disease or condition or a person without such a disease or condition. A control amount can be either in absolute amount (e.g., microgram/ml) or a relative amount (e.g., relative intensity of signals).

In some embodiments, the term "detect" refers to identifying the presence, absence or amount of the object to be detected.

In some embodiments, the term "label" includes any moiety or item detectable by spectroscopic, photo chemical, biochemical, immunochemical, or chemical means. For example, useful labels include ³²P, ³⁵S, fluorescent dyes, electron-dense reagents, enzymes (e.g., as commonly used in an ELISA), biotin-streptavadin, dioxigenin, haptens and proteins for which antisera or monoclonal antibodies are available. The label often generates a measurable signal, such as a radioactive, chromogenic, or fluorescent signal, that can be used to quantify the amount of bound label in a sample. The label can be incorporated in or attached to a primer or probe either covalently, or through ionic, van der Waals or hydrogen bonds, e.g., incorporation of radioactive nucleotides, or biotinylated nucleotides that are recognized by streptavadin. The label may be directly or indirectly detectable. Indirect detection can involve the binding of a second label to the first label, directly or indirectly. For example, the label can be the ligand of a binding partner, such as biotin, which is a binding partner for streptavidin. The binding partner may itself be directly detectable, for example, an antibody may be itself labeled with a fluorescent molecule. The binding partner also may be indirectly detectable.. Quantitation of the signal is achieved by, e.g., scintillation counting, densitometry, or flow cytometry.

Exemplary detectable labels, optionally and preferably for use with immunoassays, include but are not limited to magnetic beads, fluorescent dyes, radiolabels, enzymes (e.g., horse radish peroxide, alkaline phosphatase and others commonly used in an ELISA), and calorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

"Immunoassay" is an assay that uses an antibody to specifically bind an antigen. The immunoassay is characterized by the use of specific binding properties of a particular antibody to isolate, target, and/or quantify the antigen.

The phrase "specifically (or selectively) binds" to an antibody or "specifically (or selectively) immunoreactive with," or "specifically interacts or binds" when referring to a protein or peptide (or other epitope), refers, in some embodiments, to a binding reaction that is determinative of the presence of the protein in a heterogeneous population of proteins and other biologics. Thus, under designated immunoassay conditions, the specified antibodies bind to a particular protein at least two times greater than the background (non-specific signal) and do not substantially bind in a significant amount to other proteins present in the sample. Specific binding to an antibody under such conditions may require an antibody that is selected for its specificity for a particular protein. For example, polyclonal antibodies raised to seminal basic protein from specific species such as rat, mouse, or human can be selected to obtain only those polyclonal antibodies that are specifically immunoreactive with seminal basic protein and not with other proteins, except for polymorphic variants and alleles of seminal basic protein. This selection may be achieved by subtracting out antibodies that cross-react with seminal basic protein molecules from other species. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.

The term "an edge portion" refers to a connection between two portions of a splice variant according to the present invention that were not joined in the wild type or known protein. An edge may optionally arise due to a join between the above "known protein" portion of a variant and the tail, for example, and/or may occur if an internal portion of the wild type sequence is no longer present, such that two portions of the sequence are now contiguous in the splice variant that were not contiguous in the known protein. A "bridge" may optionally be an edge portion as described above, but may also include a join between a head and a "known protein" portion of a variant, or a join between a tail and a "known protein" portion of a variant, or a join between an insertion and a "known protein" portion of a variant.

In another embodiment, this invention provides a method for detecting the polypeptides of this invention in a biological sample, comprising: contacting a biological sample with an antibody specifically recognizing the polypeptides according to the present invention under conditions whereby the antibody specifically interacts with the polypeptides in the biological sample but do not recognize known corresponding proteins (wherein the known protein is discussed with regard to its splice variant(s) in the Examples below), and detecting said interaction; wherein the presence of an interaction correlates with the presence of a splice variant in the biological sample.

Each polypeptide marker of the present invention can be used alone or in combination, for various uses, including but not limited to, prognosis, prediction, screening, early diagnosis, determination of progression, therapy selection and treatment monitoring of the disease and/or an indicative condition, including a transition from an indicative condition to marker-detectable disease.

According to some embodiments of the present invention, any marker according to the present invention may optionally be used alone or combination. Such a combination may optionally comprise a plurality of markers described herein, optionally including any subcombination of markers, and/or a combination featuring at least one other marker, for example a known marker. Furthermore, such a combination may optionally and preferably be used as described above with regard to determining a ratio between a quantitative or semi-quantitative measurement of any marker described herein to any other marker described herein, and/or any other known marker, and/or any other marker. With regard to such a ratio between any marker described herein (or a combination thereof) and a known marker, more preferably the known marker comprises the "known protein" as described in greater detail below with regard to each cluster or gene.

In some embodiments of the present invention, there are provided of methods, uses, devices and assays for the diagnosis of an inflammatory or artheroscerotic disease or condition. Optionally a plurality of biomarkers (or markers) may be used with the present invention. The plurality of markers may optionally include any of the markers described herein, and/or one or more known markers. The plurality of markers is preferably then correlated with the disease or condition. For example, such correlating may optionally comprise determining the concentration of each of the plurality of markers, and individually comparing each marker concentration to a threshold level. Optionally, if the marker concentration is above or below the threshold level (depending upon the marker and/or the diagnostic test being performed), the marker concentration correlates with the disease or condition. Optionally and preferably, a plurality of marker concentrations correlate with the disease or condition.

Alternatively, such correlating may optionally comprise determining the concentration of each of the plurality of markers, calculating a single index value based on the concentration of each of the plurality of markers, and comparing the index value to a threshold level.

Also alternatively, such correlating may optionally comprise determining a temporal change in at least one of the markers, and wherein the temporal change is used in the correlating step.

Also alternatively, such correlating may optionally comprise determining whether at least "X" number of the plurality of markers has a concentration outside of a predetermined range and/or above or below a threshold (as described above). The value of "X" may optionally be one marker, a plurality of markers or all of the markers; alternatively or additionally, rather than including any marker in the count for "X", one or more specific markers of the plurality of markers may optionally be required to correlate with the disease or condition (according to a range and/or threshold).

Also alternatively, such correlating may optionally comprise determining whether a ratio of marker concentrations for two markers is outside a range and/or above or below a threshold. Optionally, if the ratio is above or below the threshold level and/or outside a range, the ratio correlates with the disease or condition.

Optionally, a combination of two or more these correlations may be used with a single panel and/or for correlating between a plurality of panels.

Optionally, the method distinguishes the diseases or conditions with a sensitivity of at least 70% at a specificity of at least 85% when compared to normal subjects. As used herein, sensitivity relates to the number of positive (diseased) samples detected out of the total number of positive samples present; specificity relates to the number of true negative (non-diseased) samples detected out of the total number of negative samples present. Preferably, the method distinguishes a disease or condition with a sensitivity of at least 80% at a specificity of at least 90% when compared to normal subjects. More preferably, the method distinguishes a disease or condition with a sensitivity of at least 90% at a specificity of at least 90% when compared to normal subjects. Also more preferably, the method distinguishes a disease or condition with a sensitivity of at least 70% at a specificity of at least 85% when compared to subjects exhibiting symptoms that mimic disease or condition symptoms.

A marker panel may be analyzed in a number of fashions well known to those of skill in the art. For example, each member of a panel may be compared to a "normal" value, or a value indicating a particular outcome. A particular diagnosis/prognosis may depend upon the comparison of each marker to this value; alternatively, if only a subset of markers are outside of a normal range, this subset may be indicative of a particular diagnosis/prognosis. The skilled artisan will also understand that diagnostic markers, differential diagnostic markers, prognostic markers, time of onset markers, disease or condition differentiating markers, etc., may be combined in a single assay or device. Markers may also be commonly used for multiple purposes by, for example, applying a different threshold or a different weighting factor to the marker for the different purpose(s).

In one embodiment, the panels comprise markers for the following purposes: diagnosis of a disease; diagnosis of disease and indication if the disease is in an acute phase and/or if an acute attack of the disease has occurred; diagnosis of disease and indication if the disease is in a non-acute phase and/or if a non-acute attack of the disease has occurred; indication whether a combination of acute and non-acute phases or attacks has occurred; diagnosis of a disease and prognosis of a subsequent adverse outcome; diagnosis of a disease and prognosis of a subsequent acute or non-acute phase or attack; disease progression (for example for cancer, such progression may include for example occurrence or recurrence of metastasis).

The above diagnoses may also optionally include differential diagnosis of the disease to distinguish it from other diseases, including those diseases that may feature one or more similar or identical symptoms.

In certain embodiments, one or more diagnostic or prognostic indicators are correlated to a condition or disease by merely the presence or absence of the indicator(s). In other embodiments, threshold level(s) of a diagnostic or prognostic indicator(s) can be established, and the level of the indicator(s) in a patient sample can simply be compared to the threshold level(s). The sensitivity and specificity of a diagnostic and/or prognostic test depends on more than just the analytical "quality" of the test--they also depend on the definition of what constitutes an abnormal result. In practice, Receiver Operating Characteristic curves, or "ROC" curves, are typically calculated by plotting the value of a variable versus its relative frequency in "normal" and "disease" populations, and/or by comparison of results from a subject before, during and/or after treatment. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition.

The horizontal axis of the ROC curve represents (1-specificity), which increases with the rate of false positives. The vertical axis of the curve represents sensitivity, which increases with the rate of true positives. Thus, for a particular cutoff selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

ROC curves can be used even when test results don't necessarily give an accurate number. As long as one can rank results, one can create an ROC curve. For example, results of a test on "disease" samples might be ranked according to degree (say 1=low, 2=normal, and 3=high). This ranking can be correlated to results in the "normal" population, and a ROC curve created. These methods are well known in the art (see for example Hanley et al., Radiology 143: 29-36 (1982), incorporated by reference as if fully set forth herein).

According to some embodiments of the present invention, individual markers and/or combinations (panels) of markers may optionally be used for diagnosis of time of onset of a disease or condition. The phrase "determining the prognosis" as used herein refers to methods by which the skilled artisan can predict the course or outcome of a condition in a patient. The term "prognosis" does not refer to the ability to predict the course or outcome of a condition with 100% accuracy, or even that a given course or outcome is more likely to occur than not. Instead, the skilled artisan will understand that the term "prognosis" refers to an increased probability that a certain course or outcome will occur; that is, that a course or outcome is more likely to occur in a patient exhibiting a given condition, when compared to those individuals not exhibiting the condition. For example, in individuals not exhibiting the condition, the chance of a given outcome may be about 3%. In some embodiments, a prognosis is about a 5% chance of a given outcome, about a 7% chance, about a 10% chance, about a 12% chance, about a 15% chance, about a 20% chance, about a 25% chance, about a 30% chance, about a 40% chance, about a 50% chance, about a 60% chance, about a 75% chance, about a 90% chance, and about a 95% chance. The term "about" in this context refers to +/-1%.

The skilled artisan will understand that associating a prognostic indicator with a predisposition to an adverse outcome is a statistical analysis. For example, a marker level of greater than 80 pg/mL may signal that a patient is more likely to suffer from an adverse outcome than patients with a level less than or equal to 80 pg/mL, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, e.g., Dowdy and Wearden, Statistics for Research, John Wiley & Sons, New York, 1983. In one embodiment the confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001. Exemplary statistical tests for associating a prognostic indicator with a predisposition to an adverse outcome are described hereinafter.

In other embodiments, a threshold degree of change in the level of a prognostic or diagnostic indicator can be established, and the degree of change in the level of the indicator in a patient sample can simply be compared to the threshold degree of change in the level. A preferred threshold change in the level for markers of the invention is about 5%, about 10%, about 15%, about 20%, about 25%, about 30%, about 50%, about 75%, about 100%, and about 150%. The term "about" in this context refers to +/-10%. In yet other embodiments, a "nomogram" can be established, by which a level of a prognostic or diagnostic indicator can be directly related to an associated disposition towards a given outcome. The skilled artisan is acquainted with the use of such nomograms to relate two numeric values with the understanding that the uncertainty in this measurement is the same as the uncertainty in the marker concentration because individual sample measurements are referenced, not population averages.

The present invention also relates to kits based upon such diagnostic methods or assays.

### Amino acid sequences and peptides

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

Polypeptide products can be biochemically synthesized such as by employing standard solid phase techniques. Such methods include but are not limited to exclusive solid phase synthesis, partial solid phase synthesis methods, fragment condensation, classical solution synthesis. These methods are preferably used when the peptide is relatively short (i.e., 10 kDa) and/or when it cannot be produced by recombinant techniques (i.e., not encoded by a nucleic acid sequence) and therefore involves different chemistry.

Solid phase polypeptide synthesis procedures are well known in the art and further described by John Morrow Stewart and Janis Dillaha Young, Solid Phase Peptide Syntheses (2nd Ed., Pierce Chemical Company, 1984).

Synthetic polypeptides can optionally be purified by preparative high performance liquid chromatography [Creighton T. (1983) Proteins, structures and molecular principles. WH Freeman and Co. N.Y.], after which their composition can be confirmed via amino acid sequencing.

In cases where large amounts of a polypeptide are desired, it can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

As used herein in the specification and in the claims section below the term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids. Non-conventional or modified amino acids can be incorporated in the polypeptides of this invention as well, as will be known to one skilled in the art.

Since the peptides of the present invention are utilized in diagnostics which require the peptides to be in soluble form, the peptides of the present invention may include one or more non-natural or natural polar amino acids, including but not limited to serine and threonine which are capable of increasing peptide solubility due to their hydroxyl-containing side chain.

The peptides of the present invention may be utilized in a linear form, although it will be appreciated that in cases where cyclicization does not severely interfere with peptide characteristics, cyclic forms of the peptide can also be utilized.

The peptides of present invention can be biochemically synthesized such as by using standard solid phase techniques. These methods include exclusive solid phase synthesis well known in the art, partial solid phase synthesis methods, fragment condensation, classical solution synthesis. These methods are preferably used when the peptide is relatively short (i.e., 10 kDa) and/or when it cannot be produced by recombinant techniques (i.e., not encoded by a nucleic acid sequence) and therefore involves different chemistry.

Synthetic peptides can be purified by preparative high performance liquid chromatography and the composition of which can be confirmed via amino acid sequencing.

In cases where large amounts of the peptides of the present invention are desired, the peptides of the present invention can be generated using recombinant techniques such as described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463 and also as described above.

### Antibodies:

"Antibody" refers to a polypeptide ligand that is preferably substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad-immunoglobulin variable region genes. Antibodies exist, e.g., as intact immunoglobulins or as a number of well characterized fragments produced by digestion with various peptidases. This includes, e.g., Fab' and F(ab)'₂ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region.

The functional fragments of antibodies, such as Fab, F(ab')2, and Fv that are capable of binding to macrophages, are described as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Monoclonal antibody development may optionally be performed according to any method that is known in the art. The method described below is provided for the purposes of description only and is not meant to be limiting in any way.

### Antibody Engineering in Phage Display Libraries:

Antibodies of this invention may be prepared through the use of phage display libraries, as is known in the art, for example, as described in PCT Application No. WO 94/18219, US Patent No. 6096551, both of which are hereby fully incorporated by reference, The method involves inducing mutagenesis in a complementarity determining region (CDR) of an immunoglobulin light chain gene for the purpose of producing light chain gene libraries for use in combination with heavy chain genes and gene libraries to produce antibody libraries of diverse and novel immuno-specificities. The method comprises amplifying a CDR portion of an immunoglobulin light chain gene by polymerase chain reaction (PCR) using a PCR primer oligonucleotide. The resultant gene portions are inserted into phagemids for production of a phage display library, wherein the engineered light chains are displayed by the phages, for example for testing their binding specificity.

Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using Papain produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)]. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (1972)]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. A scFv antibody fragment is an engineered antibody derivative that includes heavy- and light chain variable regions joined by a peptide linker. The minimal size of antibody molecules are those that still comprise the complete antigen binding site. ScFv antibody fragments are potentially more effective than unmodified IgG antibodies. The reduced size of 27-30 kDa permits them to penetrate tissues and solid tumors more readily. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)]. Optionally, there may be 1, 2 or 3 CDRs of different chains, but preferably there are 3 CDRs of 1 chain. The chain could be the heavy or the light chain.

Humanized forms of non-human (e.g., murine) antibodies, are chimeric molecules of immunoglobulins, immunoglobulin chains or fragments thereof (such as Fv, Fab, Fab', F(ab') or other antigen-binding subsequences of antibodies) which contain minimal sequence derived from non-human immunoglobulin, or fragments thereof may comprise the antibodies of this invention. Humanized antibodies are well known in the art. Methods for humanizing non-human antibodies are well known in the art, for example, as described in Winter and co-workers [Jones et al., Nature, 321:522-525 (1986); Riechmann et al., Nature 332:323-327 (1988); Verhoeyen et al., Science, 239:1534-1536 (1988)], U.S. Pat. No. 4,816,567, Hoogenboom and Winter, J. Mol. Biol., 227:381 (1991); Marks et al., J. Mol. Biol., 222:581 (1991), Cole et al., Monoclonal Antibodies and Cancer Therapy, Alan R. Liss, p. 77 (1985), Boerner et al., J. Immunol., 147(1):86-95 (1991), U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995), all of which are incorporated herein by reference.

Preferably, the antibody of this aspect of the present invention specifically binds at least one epitope of the polypeptide variants of the present invention. As used herein, the term "epitope" refers to any antigenic determinant on an antigen to which the paratope of an antibody binds.

Epitopic determinants usually consist of chemically active surface groupings of molecules such as amino acids or carbohydrate side chains and usually have specific three dimensional structural characteristics, as well as specific charge characteristics.

Optionally, a unique epitope may be created in a variant due to a change in one or more post-translational modifications, including but not limited to glycosylation and/or phosphorylation, as described below. Such a change may also cause a new epitope to be created, for example through removal of glycosylation at a particular site.

An epitope according to the present invention may also optionally comprise part or all of a unique sequence portion of a variant according to the present invention in combination with at least one other portion of the variant which is not contiguous to the unique sequence portion in the linear polypeptide itself, yet which are able to form an epitope in combination. One or more unique sequence portions may optionally combine with one or more other non-contiguous portions of the variant (including a portion which may have high homology to a portion of the known protein) to form an epitope.

### Immunoassays

In another embodiment of the present invention, an immunoassay can be used to qualitatively or quantitatively detect and analyze markers in a sample. This method comprises: providing an antibody that specifically binds to a marker; contacting a sample with the antibody; and detecting the presence of a complex of the antibody bound to the marker in the sample.

To prepare an antibody that specifically binds to a marker, purified protein markers can be used. Antibodies that specifically bind to a protein marker can be prepared using any suitable methods known in the art.

After the antibody is provided, a marker can be detected and/or quantified using any of a number of well recognized immunological binding assays. Useful assays include, for example, an enzyme immune assay (EIA) such as enzyme-linked immunosorbent assay (ELISA), a radioimmune assay (RIA), a Western blot assay, or a slot blot assay see, e.g., U.S. Pat. Nos. 4,366,241; 4,376,110; 4,517,288; and 4,837,168). Generally, a sample obtained from a subject can be contacted with the antibody that specifically binds the marker.

Optionally, the antibody can be fixed to a solid support to facilitate washing and subsequent isolation of the complex, prior to contacting the antibody with a sample. Examples of solid supports include but are not limited to glass or plastic in the form of, e.g., a microtiter plate, a stick, a bead, or a microbead. Antibodies can also be attached to a solid support.

After incubating the sample with antibodies, the mixture is washed and the antibody-marker complex formed can be detected. This can be accomplished by incubating the washed mixture with a detection reagent. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker are incubated simultaneously with the mixture.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10 °C to 40 °C.

The immunoassay can be used to determine a test amount of a marker in a sample from a subject. First, a test amount of a marker in a sample can be detected using the immunoassay methods described above. If a marker is present in the sample, it will form an antibody-marker complex with an antibody that specifically binds the marker under suitable incubation conditions described above. The amount of an antibody-marker complex can optionally be determined by comparing to a standard. As noted above, the test amount of marker need not be measured in absolute units, as long as the unit of measurement can be compared to a control amount and/or signal.

In some embodiments, antibodies which specifically interact with the polypeptides of the present invention and not with wild type proteins or other isoforms thereof, are used. Such antibodies are directed, for example, to the unique sequence portions of the polypeptide variants of the present invention, including but not limited to bridges, heads, tails and insertions described in greater detail below. Some embodiments of antibodies according to the present invention are described in greater detail with regard to the section entitled "Antibodies".

Radio-immunoassay (RIA): In one version, this method involves precipitation of the desired substrate and in the methods detailed hereinbelow, with a specific antibody and radiolabelled antibody binding protein (e.g., protein A labeled with I¹²⁵) immobilized on a precipitable carrier such as agarose beads. The number of counts in the precipitated pellet is proportional to the amount of substrate.

In an alternate version of the RIA, a labeled substrate and an unlabelled antibody binding protein are employed. A sample containing an unknown amount of substrate is added in varying amounts. The decrease in precipitated counts from the labeled substrate is proportional to the amount of substrate in the added sample.

Enzyme linked immunosorbent assay (ELISA): This method involves fixation of a sample (e.g., fixed cells or a proteinaceous solution) containing a protein substrate to a surface such as a well of a microtiter plate. A substrate specific antibody coupled to an enzyme is applied and allowed to bind to the substrate. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

Western blot: This method involves separation of a substrate from other protein by means of an acrylamide gel followed by transfer of the substrate to a membrane (e.g., nylon or PVDF). Presence of the substrate is then detected by antibodies specific to the substrate, which are in turn detected by antibody binding reagents. Antibody binding reagents may be, for example, protein A, or other antibodies. Antibody binding reagents may be radiolabelled or enzyme linked as described hereinabove. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis.

Immunohistochemical analysis: This method involves detection of a substrate in situ in fixed cells by substrate specific antibodies. The substrate specific antibodies may be enzyme linked or linked to fluorophores. Detection is by microscopy and subjective evaluation. If enzyme linked antibodies are employed, a colorimetric reaction may be required.

Fluorescence activated cell sorting (FACS): This method involves detection of a substrate in situ in cells by substrate specific antibodies. The substrate specific antibodies are linked to fluorophores. Detection is by means of a cell sorting machine which reads the wavelength of light emitted from each cell as it passes through a light beam. This method may employ two or more antibodies simultaneously.

### Display Libraries

Display library comprising a plurality of display vehicles (such as phages, viruses or bacteria) each displaying at least 6, at least 7, at least 8, at least 9, at least 10, 10-15, 12-17, 15-20, 15-30 or 20-50 consecutive amino acids derived from the polypeptide sequences of the present invention may be prepared.

Methods of constructing such display libraries are well known in the art. Such methods are described in, for example, Young AC, et al., "The three-dimensional structures of a polysaccharide binding antibody to Cryptococcus neoformans and its complex with a peptide from a phage display library: implications for the identification of peptide mimotopes" J Mol Biol 1997 Dec 12;274(4):622-34; Giebel LB et al. "Screening of cyclic peptide phage libraries identifies ligands that bind streptavidin with high affinities" Biochemistry 1995 Nov 28;34(47):15430-5; Davies EL et al., "Selection of specific phage-display antibodies using libraries derived from chicken immunoglobulin genes" J Immunol Methods 1995 Oct 12;186(1):125-35; Jones C RT al. "Current trends in molecular recognition and bioseparation" J Chromatogr A 1995 Jul 14;707(1):3-22; Deng SJ et al. "Basis for selection of improved carbohydrate-binding single-chain antibodies from synthetic gene libraries" Proc Natl Acad Sci U S A 1995 May 23;92(11):4992-6; and Deng SJ et al. "Selection of antibody single-chain variable fragments with improved carbohydrate binding by phage display" J Biol Chem 1994 Apr 1;269(13):9533-8, which are incorporated herein by reference.

### Theranostics:

The term theranostics describes the use of diagnostic testing to diagnose the disease, choose the correct treatment regime according to the results of diagnostic testing and/or monitor the patient response to therapy according to the results of diagnostic testing. Theranostic tests can be used to select patients for treatments that are particularly likely to benefit them and unlikely to produce side-effects. They can also provide an early and objective indication of treatment efficacy in individual patients, so that (if necessary) the treatment can be altered with a minimum of delay. For example: DAKO and Genentech together created HercepTest and Herceptin (trastuzumab) for the treatment of breast cancer, the first theranostic test approved simultaneously with a new therapeutic drug. In addition to HercepTest (which is an immunohistochemical test), other theranostic tests are in development which use traditional clinical chemistry, immunoassay, cell-based technologies and nucleic acid tests. PPGx's recently launched TPMT (thiopurine S-methyltransferase) test, which is enabling doctors to identify patients at risk for potentially fatal adverse reactions to 6-mercaptopurine, an agent used in the treatment of leukemia. Also, Nova Molecular pioneered SNP genotyping of the apolipoprotein E gene to predict Alzheimer's disease patients' responses to cholinomimetic therapies and it is now widely used in clinical trials of new drugs for this indication. Thus, the field of theranostics represents the intersection of diagnostic testing information that predicts the response of a patient to a treatment with the selection of the appropriate treatment for that particular patient.

### Surrogate markers:

A surrogate marker is a marker, that is detectable in a laboratory and/or according to a physical sign or symptom on the patient, and that is used in therapeutic trials as a substitute for a clinically meaningful endpoint. The surrogate marker is a direct measure of how a patient feels, functions, or survives which is expected to predict the effect of the therapy. The need for surrogate markers mainly arises when such markers can be measured earlier, more conveniently, or more frequently than the endpoints of interest in terms of the effect of a treatment on a patient, which are referred to as the clinical endpoints. Ideally, a surrogate marker should be biologically plausible, predictive of disease progression and measurable by standardized assays (including but not limited to traditional clinical chemistry, immunoassay, cell-based technologies, nucleic acid tests and imaging modalities).

Surrogate endpoints were used first mainly in the cardiovascular area. For example, antihypertensive drugs have been approved based on their effectiveness in lowering blood pressure. Similarly, in the past, cholesterol-lowering agents have been approved based on their ability to decrease serum cholesterol, not on the direct evidence that they decrease mortality from atherosclerotic heart disease. The measurement of cholesterol levels is now an accepted surrogate marker of atherosclerosis. In addition, currently two commonly used surrogate markers in HIV studies are CD4+ T cell counts and quantitative plasma HIV RNA (viral load). In some embodiments of this invention, the polypeptide/polynucleotide expression pattern may serve as a surrogate marker for a particular disease, as will be appreciated by one skilled in the art.

### EXAMPLES

### Acute and chronic inflammation and risk factors for CVS diseases

HSFLT variants, HSIIRa variants, HSPLGF variants, HUMSP18A variants, F05068 variants and/or HUMIL10 variants are potential markers for inflammation, including a spectrum of diseases where an inflammatory process plays a substantial role. In addition CRP levels and in particular baseline levels serve as a risk factor for various diseases, particularly cardiovascular diseases where inflammation is thought to participate in the pathogenesis. Conditions that may be diagnosed by these markers or variants of them include but are not limited to the presence, risk and/or extent of the following:
1. Conditions that entail an inflammatory process that involves blood vessels including but not limited to hypercholesterolemia, diabetes, atherosclerosis, inflammation that involves blood vessels - whether acute or chronic including but not limited to the coronary arteries and blood vessels of the brain, myocardial infarction, cerebral stroke, peripheral vascular disease, vasculitis, polyarteritis nodosa, ANCA associated small vessel vasculitis, Churg-Strauss syndrome, Henoch-Schonlein purpura, scleroderma, thromboangiitis obliterans, temporal arteritis, Takayasu's arteritis, hypersensitivity vasculitis, Kawasaki disease, Behçet syndrome, and their complications including but not limited to coronary disease, angina pectoris, deep vein thrombosis, renal disease, diabetic nephropathy, lupus nephritis, renal artery thrombosis, renal artery stenosis, atheroembolic disease of the renal arteries, renal vein thrombosis, hemolytic uremic syndrome, thrombotic thrombocytopenic purpura, arteriolar nephrosclerosis, preeclampsia, eclampsia, albuminuria, microalbuminuria, glomerulonephritis, renal failure, hypertension, uremia, cerebrovascular disease, peripheral vascular disease, intermittent claudication, abdominal angina.
2. Rheumatic / autoimmune diseases that involve systemic immune reaction including but not limited to rheumatoid arthritis, scleroderma, mixed connective tissue disease, Sjogren syndrome, ankylosing spondylitis, spondyloarthropathy, psoriasis, psoriatic arthritis, myositis and systemic lupus erythematosus.
3. Acute and/or chronic infective processes that involve systemic immune reaction including but not limited to pneumonia, bacteremia, sepsis, pyelonephritis, cellulitis, osteomyelitis, meningitis and viral hepatitis.
4. Malignant and idiopathic processes that involve systemic immune reaction and/or proliferation of immune cells including but not limited to granulomatous disorders, Wegener's granulomatosis, lymphomatoid granulomatosis / polymorphic reticulosis, idiopathic midline granuloma, multiple myeloma, Waldenstrom's macroglobulinemia, Castleman's disease, amyloidosis, lymphoma, histiocytosis, renal cell carcinoma and paraneoplastic syndromes.
5. Conditions where CRP was shown to have a positive correlation with the presence of the condition including but not limited to weight loss, anorexia-cachexia syndrome, extent of disease, recurrence in advanced cancer, diabetes (types 1 & 2), obesity, hypertension, preterm delivery.
6. Conditions which have similar symptoms, signs and complications as the conditions above and where the differential diagnosis between them and the conditions above is of clinical importance including but not limited to:
   a. Other (non vascular) causes of heart disease, renal disease and cerebral disease.
   b. Other (non rheumatic) causes of arthropathy and musculoskeletal pain.
   c. Other causes of non-specific symptoms and signs such as fever of unknown origin, loss of appetite, weight loss, nonspecific pains, breathing difficulties and anxiety.

### CLUSTER HSFLT

Cluster HSFLT features certain protein variants that are described in table 1.

**Table 1 - Proteins and their Corresponding Transcript Descriptions:**

| Protein Name | Corresponding Transcript(s) |
|---|---|
| HSFLT_P6 (SEQ ID NO:16) | HSFLT_T9 (SEQ ID NO:3) |
| HSFLT_P7 (SEQ ID NO:17) | HSFLT_T10 (SEQ ID NO:4) |
| HSFLT_P10 (SEQ ID NO:18) | HSFLT_T13 (SEQ ID NO:5) |
| HSFLT_P11 (SEQ ID NO:19) | HSFLT_T14 (SEQ ID NO:6) |
| HSFLT_P13 (SEQ ID NO:20) | HSFLT_T17 (SEQ ID NO:7) |
| HSFLT_P14 (SEQ ID NO:21) | HSFLT_T19 (SEQ ID NO:8) |
| HSFLT_P15 (SEQ ID NO:22) | HSFLT_T20 (SEQ ID NO:9) |
| HSFLT_P16 (SEQ ID NO:23) | HSFLT_T21 (SEQIDNO:10) |
| HSFLT_P17 (SEQ ID NO:24) | HSFLT_T22 (SEQ ID NO:11) |
| HSFLT_P18 (SEQ ID NO:25) | HSFLT_T23 (SEQ ID NO:12) |
| HSFLT_P19 (SEQ ID NO:26) | HSFLT_T24 (SEQ ID NO:13) |
| HSFLT_P20 (SEQ ID NO:27) | HSFLT_T25 (SEQ ID NO:14) |
| HSFLT_P21 (SEQ ID NO:28) | HSFLT_T26 (SEQ ID NO:15) |
| HSFLT_P41 (SEQ ID NO:29) | HSFLT_T21 (SEQIDNO:10) |
| HSFLT_P48 (SEQ ID NO:30) | HSFLT_T7 (SEQ ID NO:1) |
| HSFLT_P49 (SEQ ID NO:31) | HSFLT_T8 (SEQ ID NO:2) |

The sequences listed in Table 1 comprise variants of the known protein Vascular endothelial growth factor receptor 1 precursor (SwissProt accession identifier VGR1_HUMAN (SEQ ID NO: 359); known also according to the synonyms EC 2.7.1.112; VEGFR-1; Vascular permeability factor receptor; Tyrosine-protein kinase receptor FLT; Flt-1; Tyrosine-protein kinase FRT; Fms-like tyrosine kinase 1)), and may be referred to herein as "the corresponding native protein".

Protein Vascular endothelial growth factor receptor 1 precursor is associated with the following function(s): it is a receptor for VEGF, VEGFB and PGF, has tyrosine-protein kinase activity. The VEGF-kinase ligand/receptor signaling system plays a key role in vascular development and regulation of vascular permeability. Isoform SFlt1 may have an inhibitory role in angiogenesis. The sequence for protein Vascular endothelial growth factor receptor 1 precursor is given at the end of the application, as "Vascular endothelial growth factor receptor 1 precursor amino acid sequence". Known polymorphisms for this sequence include an SNP at amino acid position 779, having an L to F substitution.

The following GO Annotation(s) apply to the previously known protein. The following annotation(s) were found: positive regulation of cell proliferation; pregnancy; transmembrane receptor protein tyrosine kinase signaling pathway, which are annotation(s) related to Biological Process; receptor activity; vascular endothelial growth factor receptor activity, which are annotation(s) related to Molecular Function; and extracellular space; integral to plasma membrane, which are annotation(s) related to Cellular Component.

The GO assignment relies on information from one or more of the SwissProt/TremB1 Protein knowledgebase, available from <http://www.expasy.ch/sprot/>; or Locuslink, available from <http://www.ncbi.nlm.nih.gov/projects/LocusLink/>.

According to some embodiments of the present invention, variants of this cluster may optionally have one or more of the following utilities, as described below:
inflammation, pathological angiogenesis, monocyte recruitment that underlie chronic inflammatory disease.

Another non-limiting example of the utility of the variants of HSFLT cluster according to the present invention (amino acid and/or nucleic acid sequences of HSFLT) is using this marker as a surrogate marker for determining the efficacy of treatment for modulators, preferably inhibitors, of the VEGF-kinase ligand/receptor signaling system, which plays a key role in vascular development and regulation of vascular permeability.

In some embodiments, HSFLT_P10 of the present invention has an amino acid sequence homologous to or as set forth in SEQ ID NO:18, and may be encoded by transcript(s) HSFLT_T13 (SEQ ID NO:5). An alignment of HSFLT_P10 to known proteinvascular endothelial growth factor receptor 1 precursor is provided in the alignment table on the attached CD-ROM. A brief description of the relationship of the variant protein according to the present invention to each such aligned protein is as follows:
1. Comparison report between HSFLT_P10 (SEQ ID NO:18) and VGR1_HUMAN (SEQ ID NO: 359):
   A. An isolated chimeric polypeptide as set forth in HSFLT_P10 (SEQ ID NO:18), comprising a first amino acid sequence being at least 90% homologous to MVSYWDTGVLLCALLSCLLLTGSSSGSKLKDPELSLKGTQHIMQAGQTLHLQCRGEAAHK WSLPEMVSKESERLSITKSACGRNGKQFCSTLTLNTAQANHTGFYSCKYLAVPTSKKKETE SAIYIFISDTGRPFVEMYSEIPEIIHMTEGRELVIPCRVTSPNITVTLKKFPLDTLIPDGKRIIWD SRKGFIISNATYKEIGLLTCEATVNGHLYKTNYLTHRQTNTIIDVQISTPRPVKLLRGHTLVL NCTATTPLNTRVQMTWSYPDEKNKRASVRRRIDQSNSHANIFYSVLTIDKMQNKDKGLYT CRVRSGPSFKSVNTSVHIYDKAFITVKHRKQQVLETVAGKRSYRLSMKVKAFPSPEVVWLK DGLPATEKSARYLTRGYSLIIKDVTEEDAGNYTILLSIKQSNVFKNLTATLIVNVKPQIYEKA VSSFPDPALYPLGSRQILTCTAYGIPQPTIKWFWHPCNHNHSEARCDFCSNNEESFILDADSN MGNRIESITQRMAIIEGKNKMASTLVVADSRISGIYICIASNKVGTVGRNISFYITDVPNGFHV NLEKMPTEGEDLKLSCTVNKFLYRDVTWILLRTVNNRTMHYSISKQKMAITKEHSITLNLTI MNVSLQDSGTYACRARNVYTGEEILQKKEITIRDQEAPYLLRNLSDHTVAISSSTTLDCHAN GVPEPQITWFKNNHKIQQEP corresponding to amino acids 1 - 705 of VGR1_HUMAN (SEQ ID NO: 359), which also corresponds to amino acids 1 - 705 of HSFLT_P10 (SEQ ID NO:18), and a second amino acid sequence correspodning to a polypeptide having the sequence ELYTSTSPSSSSSSPLSSSSSSSSSSSS (SEQ ID NO: 462) corresponding to amino acids 706 - 733 of HSFLT_P10 (SEQ ID NO:18), wherein said first amino acid sequence and second amino acid sequence are contiguous and in a sequential order.
   B. An isolated polypeptide encoding for an edge portion of HSFLT_P10 (SEQ ID NO:18), consisting of the amino acid having the sequence ELYTSTSPSSSSSSPLSSSSSSSSSSSS (SEQ ID NO: 462) of HSFLT_P10 (SEQ ID NO:18).
2. Comparison report between HSFLT_P10 (SEQ ID NO:18) and P17948-2 (SEQ ID NO:360) :
   A. An isolated chimeric polypeptide as set forth in HSFLT_P10 (SEQ ID NO:18), comprising a first amino acid sequence MVSYWDTGVLLCALLSCLLLTGSSSGSKLKDPELSLKGTQHIMQAGQTLHLQCRGEAAHK WSLPEMVSKESERLSITKSACGRNGKQFCSTLTLNTAQANHTGFYSCKYLAVPTSKKKETE SAIYIFISDTGRPFVEMYSEIPEIIHMTEGRELVIPCRVTSPNITVTLKKFPLDTLIPDGKRIIWD SRKGFIISNATYKEIGLLTCEATVNGHLYKTNYLTHRQTNTIIDVQISTPRPVKLLRGHTLVL NCTATTPLNTRVQMTWSYPDEKNKRASVRRRIDQSNSHANIFYSVLTIDKMQNKDKGLYT CRVRSGPSFKSVNTSVHIYDKAFITVKHRKQQVLETVAGKRSYRLSMKVKAFPSPEVVWLK DGLPATEKSARYLTRGYSLIIKDVTEEDAGNYTILLSIKQSNVFKNLTATLIVNVKPQIYEKA VSSFPDPALYPLGSRQILTCTAYGIPQPTIKWFWHPCNHNHSEARCDFCSNNEESFILDADSN MGNRIESITQRMAIIEGKNKMASTLVVADSRISGIYICIASNKVGTVGRNISFYTTDVPNGFHV NLEKMPTEGEDLKLSCTVNKFLYRDVTWILLRTVNNRTMHYSISKQKMAITKEHSITLNLTI MNVSLQDSGTYACRARNVYTGEEILQKKEITIR corresponding to amino acids 1 - 656 of P17948-2 (SEQ ID NO:360), which also corresponds to amino acids 1 - 656 of HSFLT_P10 (SEQ ID NO:18), and a second amino acid having the sequence DQEAPYLLRNLSDHTVAISSSTTLDCHANGVPEPQITWFKNNHKIQQEPELYTSTSPSSSSSSP LSSSSSSSSSSSS (SEQ ID NO: 463) corresponding to amino acids 657 - 733 of HSFLT_P10 (SEQ ID NO:18), wherein said first amino acid sequence and second amino acid sequence are contiguous and in a sequential order.
   B. An isolated polypeptide encoding for an edge portion of HSFLT_P10 (SEQ ID NO:18), comprising an amino acid sequence having the sequence DQEAPYLLRNLSDHTVAISSSTTLDCHANGVPEPQITWFKNNHKIQQEPELYTSTSPSSSSSSP LSSSSSSSSSSSS (SEQ ID NO: 463) of HSFLT_P10 (SEQ ID NO:18).

The glycosylation sites of variant protein HSFLT_P10 (SEQ ID NO:18), as compared to the known protein Vascular endothelial growth factor receptor 1 precursor , are described in Table 2 (given according to their position(s) on the amino acid sequence in the first column; the second column indicates whether the glycosylation site is present in the variant protein; and the last column indicates whether the position is different on the variant protein).

**Table 2 - Glycosylation site(s)**

| Position(s) on known amino acid sequence | Present in variant protein? | Position(s) on variant protein |
|---|---|---|
| 100 | Yes | 100 |
| 164 | Yes | 164 |
| 196 | Yes | 196 |
| 251 | Yes | 251 |
| 323 | Yes | 323 |
| 402 | Yes | 402 |
| 417 | Yes | 417 |
| 474 | Yes | 474 |
| 547 | Yes | 547 |
| 597 | Yes | 597 |
| 620 | Yes | 620 |
| 625 | Yes | 625 |
| 666 | Yes | 666 |

The phosphorylation sites of variant protein HSFLT_P10 (SEQ ID NO:18), as compared to the known protein, are described in Table 3 (given according to their position(s) on the amino acid sequence in the first column; the second column indicates whether the phosphorylation site is present in the variant protein; and the last column indicates whether the position is different on the variant protein).

**Table 3 - Phosphorylation site(s)**

| Position(s) on known amino acid sequence | Present in variant protein? | Positions(s) on variant protein |
|---|---|---|
| 1053 | No | |
| 1169 | No | |
| 1213 | No | |
| 1242 | No | |
| 1327 | No | |
| 1333 | No | |

The variant protein has the following domains, as determined by using InterPro. The domains are described in Table 4:

**Table 4 - InterPro domain(s)**

| Domain description | Analysis type | Positions(s) on protein |
|---|---|---|
| Vascular endothelial growth factor receptor, VEGFR | FPrintScan | 89-107, 125-136, 184-194, 242-254, 390-407, 448-462 |
| Vascular endothelial growth factor receptor 1, VEGFR1 | FPrintScan | 26-41, 79-93, 130-155, 224-247, 273-290, 350-370, 375-389 |
| Immunoglobulin-like | HMMPfam | 245-313, 570-638, 675-731 |
| Immunoglobulin V-type | HMMSmart | 247-313, 572-638 |
| Immunoglobulin C2 type | HMMSmart | 149-214, 243-318, 348-412, 568-643, 673-732 |
| Immunoglobulin subtype | HMMSmart | 38-129, 143-224, 237-329, 344-425, 439-553, 562-658 |
| Immunoglobulin-like | ProfileScan | 32-107, 230-327, 349-404, 428-553, 556-654, 661-733 |

Variant protein HSFLT_P10 (SEQ ID NO:18) is encoded by the following transcript(s): HSFLT_T13 (SEQ ID NO:5), for which the coding portion starts at position 315 and ends at position 2513. The transcript also has the following SNPs as listed in Table 24 (given according to their position on the nucleotide sequence, with the alternative nucleic acid listed; the presence of known SNPs in variant protein HSFLT_P10 (SEQ ID NO:18) sequence provides support for the deduced sequence of this variant protein according to the present invention).

**Table5 - Nucleic acid SNPs**

| Polymorphism | SNP position(s) on nucleotide sequence |
|---|---|
| -> C | 823 |
| T -> C | 1063, 1342 |
| A -> G | 1165, 1325, 1495 |
| C -> T | 1533 |
| G -> A | 2018 |
| G -> T | 3301 |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A method for detecting the presence or severity of an inflammatory or atherosclerotic disease, disorder or condition, comprising detecting in an *in vitro* biological sample the expression of a unique edge portion of a polypeptide having the amino acid sequence set forth in SEQ ID NO:18, wherein said unique edge portion consists of the amino acid sequence set forth in any one of SEQ ID NOs: 463 and 462.

2. The method of claim 1, wherein the inflammatory disease is a chronic inflammatory disease.

3. The method of claim 2, wherein the chronic inflammatory disease is preeclampsia.

4. The method of any one of claims 1-3, wherein said detecting is conducted by immunoassay.

5. The method of claim 4,
wherein said detecting comprises detecting binding of an antibody specifically interacting with the unique edge portion of the polypeptide set forth in SEQ ID NO:18, said unique portion consisting of the amino acids sequence set forth in SEQ ID NO: 462, but not with a known VEGFR-1 having the amino acid sequence set forth in SEQ ID NO:359; or
wherein said detecting comprises detecting binding of an antibody specifically interacting with the unique edge portion of the polypeptide set forth in SEQ ID NO:18, said unique portion consisting of the amino acids sequence set forth in SEQ ID NO: 463, but not with a known VEGFR-1 having the amino acid sequence set forth in SEQ ID NO:360.

6. An antibody capable of specifically binding to a unique edge portion of the polypeptide set forth in SEQ ID NO:18, the unique portion consisting of the amino acids sequence set forth in SEQ ID NO:463, but not with a known VEGFR-1 having the amino acid sequence set forth in SEQ ID NO:360, for detecting preeclampsia in an *in vitro* biological sample.

7. A kit for detecting an inflammatory or atherosclerotic disease, comprising an antibody capable of specifically binding to a unique edge portion of the polypeptide set forth in SEQ ID NO:18, the unique portion consisting of the amino acids sequence set forth in any SEQ ID NO: 463, but not with a known VEGFR-1 having the amino acid sequence set forth in SEQ ID NO: 360.

8. The kit of claim 7, for detecting preeclampsia.

9. The kit of any one of claims 7-8, wherein said kit further comprises at least one reagent for performing an ELISA or a Western blot.

## Patentansprüche

1. Verfahren zum Nachweis der Anwesenheit oder Schwere einer entzündlichen oder atherosklerotischen Erkrankung, Störung oder Zustandes, welches umfasst. Erfassen der Expression eines einzigartigen Randbereichs eines Polypeptids mit der Aminosäuresequenz gemäß SEQ.ID.Nr. 18 *in vitro* in einer biologischen Probe, wobei der einzigartige Randbereich aus den Aminosäuresequenzen gemäß SEQ.ID.Nr. 463 und 462 besteht.

2. Verfahren nach Anspruch 1, wobei die entzündliche Erkrankung eine chronische entzündliche Erkrankung ist.

3. Verfahren nach Anspruch 2, wobei die chronische entzündliche Erkrankung Präeklampsie ist.

4. Verfahren nach einem der Ansprüche 1 - 3, wobei der Nachweis mittels eines Immunassays durchgeführt wird.

5. Verfahren nach Anspruch 4,
wobei die Erfassung umfasst. Erfassen der Bindung eines Antikörpers, der spezifisch mit dem einzigartigen Randbereich des Polypeptids gemäß SEQ.ID.Nr. 18 interagiert, worin der einzigartige Randbereich aus der Aminosäuresequenz gemäß SEQ.ID.Nr. 462 besteht, jedoch nicht mit einem bekannten VEGFR-1 mit der Aminosäuresequenz gemäß SEQ.ID.Nr. 359 interagiert; oder
wobei die Erfassung umfasst, Erfassen der Bindung eines Antikörpers, der spezifisch mit dem einzigartigen Randbereich des Polypeptids gemäß SEQ.ID.Nr. 18 interagiert, worin der einzigartige Randbereich aus der Aminosäuresequenz gemäß SEQ.ID.Nr. 463 besteht, jedoch nicht mit einem bekannten VEGFR-1 mit der Aminosäuresequenz gemäß SEQ.ID.Nr. 360 interagiert.

6. Antikörper, welcher spezifisch an einen einzigantigen Randbereich des Polypeptids gemäß SEQ.ID.Nr. 18 bindet, worin der einzigartige Randbereich aus der Aminosäuresequenz gemäß SEQ.ID.Nr. 463 besteht, jedoch nicht an einen bekannten VEGFR-1 mit der Aminosäuresequenz gemäß SEQ.ID.Nr. 360 bindet, um *in vitro* in einer biologischen Probe Präeklampsie nachzuweisen.

7. Kit zur Erfassung einer entzündlichen oder atherosklerotischen Erkrankung, welcher einen Antikörper umfasst, der spezifisch an einen einzigartigen Randbereich des Polypeptids gemäß SEQ.ID.Nr. 18 binden kann, worin der einzigartige Randbereich aus der Aminosäuresequenz gemäß SEQ.ID.Nr. 463 besteht, jedoch nicht an ein bekanntes VEGFR-1 mit der Aminosäuresequenz gemäß SEQ.ID.Nr. 360 bindet.

8. Kit nach Anspruch 8 zur Erfassung von Präeklampsie.

9. Kit nach einem der Ansprüche 7 - 8, worin der Kit weiter mindestens ein Reagenz zur Durchführung eines ELISA oder Western-Blots umfasst.

## Revendications

1. Procédé pour détecter la présence ou la gravité d'une maladie, un trouble ou une affection, inflammatoire ou athéroscléreux, comprenant la détection dans un échantillon biologique in vitro de l'expression d'une partie de bord unique d'un polypeptide possédant la séquence d'acides aminés présentée dans SEQ ID No. : 18, dans lequel ladite partie de bord unique est constituée de la séquence d'acides aminés présentée dans l'une quelconque des SEQ ID No. : 463 et 462.

2. Procédé selon la revendication 1, dans lequel la maladie inflammatoire est une maladie inflammatoire chronique.

3. Procédé selon la revendication 2, dans lequel la maladie inflammatoire chronique est la prééclampsie.

4. Procédé selon l'une quelconque des revendications 1 à 3, dans lequel ladite détection est effectuée par un dosage immunologique.

5. Procédé selon la revendication 4,
dans lequel ladite détection comprend la détection de la liaison d'un anticorps interagissant spécifiquement avec la partie de bord unique du polypeptide présenté dans SEQ ID No. : 18, ladite partie unique étant constituée de la séquence d'acides aminés présentée dans SEQ ID No. : 462, mais pas avec un VEGFR-1 connu possédant la séquence d'acides aminés présentée dans SEQ ID No. :359 ; ou
dans lequel ladite détection comprend la détection de la liaison d'un anticorps interagissant spécifiquement avec la partie de bord unique du polypeptide présenté dans SEQ ID No. : 18, ladite partie unique étant constituée de la séquence d'acides aminés présentée dans SEQ ID No. : 463, mais pas avec un VEGFR-1 connu possédant la séquence d'acides aminés présentée dans SEQ ID No. : 360.

6. Anticorps susceptible de se lier spécifiquement à une partie de bord unique du polypeptide présenté dans SEQ ID No. : 18, la partie unique étant constituée de la séquence d'acides aminés présentée dans SEQ ID No. : 463, mais pas avec un VEGFR-1 connu possédant la séquence d'acides aminés présentée dans SEQ ID No. : 360, pour détecter une prééclampsie dans un échantillon biologique in vitro.

7. Trousse pour détecter une maladie inflammatoire ou athéroscléreuse, comprenant un anticorps susceptible de se lier spécifiquement à une partie de bord unique du polypeptide présenté dans SEQ ID No. : 18, la partie unique étant constituée de la séquence d'acides aminés présentée dans n'importe quel SEQ ID No. : 463, mais pas avec un VEGFR-1 connu possédant la séquence d'acides aminés présentée dans SEQ ID No. :360.

8. Trousse selon la revendication 7, pour détecter la prééclampsie.

9. Trousse selon l'une quelconque des revendications 7 à 8, où ladite trousse comprend en outre au moins un réactif pour exécuter un dosage ELISA ou un buvardage de Western.


### SEQUENCE LISTING

<110> Compugen Ltd
<120> NOVEL NUCLEOTIDE AND AMINO ACID SEQUENCES, AND ASSAYS AND METHODS OF USE THEREOF FOR DIAGNOSIS
<130> COMP/025 PCT
<160> 592
<210> 1
   <211> 7235
   <212> DNA
   <213> Homo sapiens
<400> 1
   taaatcgccc ccgccctcgg ctgctcttca tcgaggtccg cgggaggctc ggagcgcgcc    60
   aggcggacac tcctctcggc tcctccccgg cagcggcggc ggctcggagc gggctccggg   120
   gctcgggtgc agcggccagc gggcgcctgg cggcgaggat tacccgggga agtggttgtc   180
   tcctggctgg agccgcgaga cgggcgctca gggcgcgggg ccggcggcgg cgaacgagag   240
   gacggactct ggcggccggg tcgttggccg cggggagcgc gggcaccggg cgagcaggcc   300
   gcgtcgcgct caccatggtc agctactggg acaccggggt cctgctgtgc gcgctgctca   360
   gctgtctgct tctcacagga tctagttcag gttcaaaatt aaaagatcct gaactgagtt   420
   taaaaggcac ccagcacatc atgcaagcag gccagacact gcatctccaa tgcagggggg   480
   aagcagccca taaatggtct ttgcctgaaa tggtgagtaa ggaaagcgaa aggctgagca   540
   taactaaatc tgcctgtgga agaaatggca aacaattctg cagtacttta accttgaaca   600
   cagctcaagc aaaccacact ggcttctaca gctgcaaata tctagctgta cctacttcaa   660
   agaagaagga aacagaatct gcaatctata tatttattag tgatacaggt agacctttcg   720
   tagagatgta cagtgaaatc cccgaaatta tacacatgac tgaaggaagg gagctcgtca   780
   ttccctgccg ggttacgtca cctaacatca ctgttacttt aaaaaagttt ccacttgaca   840
   ctttgatccc tgatggaaaa cgcataatct gggacagtag aaagggcttc atcatatcaa   900
   atgcaacgta caaagaaata gggcttctga cctgtgaagc aacagtcaat gggcatttgt   960
   ataagacaaa ctatctcaca catcgacaaa ccaatacaat catagatgtc caaataagca  1020
   caccacgccc agtcaaatta cttagaggcc atactcttgt cctcaattgt actgctacca  1080
   ctcccttgaa cacgagagtt caaatgacct ggagttaccc tgatgaaaaa aataagagag  1140
   cttccgtaag gcgacgaatt gaccaaagca attcccatgc caacatattc tacagtgttc  1200
   ttactattga caaaatgcag aacaaagaca aaggacttta tacttgtcgt gtaaggagtg  1260
   gaccatcatt caaatctgtt aacacctcag tgcatatata tgataaagca ttcatcactg  1320
   tgaaacatcg aaaacagcag gtgcttgaaa ccgtagctgg caagcggtct taccggctct  1380
   ctatgaaagt gaaggcattt ccctcgccgg aagttgtatg gttaaaagat gggttacctg 1440
   cgactgagaa atctgctcgc tatttgactc gtggctactc gttaattatc aaggacgtaa 1500
   ctgaagagga tgcagggaat tatacaatct tgctgagcat aaaacagtca aatgtgttta 1560
   aaaacctcac tgccactcta attgtcaatg tgaaacccca gatttacgaa aaggccgtgt 1620
   catcgtttcc agacccggct ctctacccac tgggcagcag acaaatcctg acttgtaccg 1680
   catatggtat ccctcaacct acaatcaagt ggttctggca cccctgtaac cataatcatt 1740
   ccgaagcaag gtgtgacttt tgttccaata atgaagagtc ctttatcctg gatgctgaca 1800
   gcaacatggg aaacagaatt gagagcatca ctcagcgcat ggcaataata gaaggaaaga 1860
   ataagcttcc accagctaac agttctttca tgttgccacc tacaagcttc tcttccaact 1920
   acttccattt ccttccgtga ctctaaacgg atggctagca ccttggttgt ggctgactct 1980
   agaatttctg gaatctacat ttgcatagct tccaataaag ttgggactgt gggaagaaac 2040
   ataagctttt atatcacaga tgtgccaaat gggtttcatg ttaacttgga aaaaatgccg 2100
   acggaaggag aggacctgaa actgtcttgc acagttaaca agttcttata cagagacgtt 2160
   acttggattt tactgcggac agttaataac agaacaatgc actacagtat tagcaagcaa 2220
   aaaatggcca tcactaagga gcactccatc actcttaatc ttaccatcat gaatgtttcc 2280
   ctgcaagatt caggcaccta tgcctgcaga gccaggaatg tatacacagg ggaagaaatc 2340
   ctccagaaga aagaaattac aatcagagat caggaagcac catacctcct gcgaaacctc 2400
   agtgatcaca cagtggccat cagcagttcc accactttag actgtcatgc taatggtgtc 2460
   cccgagcctc agatcacttg gtttaaaaac aaccacaaaa tacaacaaga gcctggaatt 2520
   attttaggac caggaagcag cacgctgttt attgaaagag tcacagaaga ggatgaaggt 2580
   gtctatcact gcaaagccac caaccagaag ggctctgtgg aaagttcagc atacctcact 2640
   gttcaaggaa cctcggacaa gtctaatctg gagctgatca ctctaacatg cacctgtgtg 2700
   gctgcgactc tcttctggct cctattaacc ctctttatcc gaaaaatgaa aaggtcttct 2760
   tctgaaataa agactgacta cctatcaatt ataatggacc cagatgaagt tcctttggat 2820
   gagcagtgtg agcggctccc ttatgatgcc agcaagtggg agtttgcccg ggagagactt 2880
   aaactgggca aatcacttgg aagaggggct tttggaaaag tggttcaagc atcagcattt 2940
   ggcattaaga aatcacctac gtgccggact gtggctgtga aaatgctgaa agagggggcc 3000
   acggccagcg agtacaaagc tctgatgact gagctaaaaa tcttgaccca cattggccac 3060
   catctgaacg tggttaacct gctgggagcc tgcaccaagc aaggagggcc tctgatggtg 3120
   attgttgaat actgcaaata tggaaatctc tccaactacc tcaagagcaa acgtgactta 3180
   ttttttctca acaaggatgc agcactacac atggagccta agaaagaaaa aatggagcca 3240
   ggcctggaac aaggcaagaa accaagacta gatagcgtca ccagcagcga aagctttgcg 3300
   agctccggct ttcaggaaga taaaagtctg agtgatgttg aggaagagga ggattctgac 3360
   ggtttctaca aggagcccat cactatggaa gatctgattt cttacagttt tcaagtggcc 3420
   agaggcatgg agttcctgtc ttccagaaag tgcattcatc gggacctggc agcgagaaac 3480
   attcttttat ctgagaacaa cgtggtgaag atttgtgatt ttggccttgc ccgggatatt 3540
   tataagaacc ccgattatgt gagaaaagga gatactcgac ttcctctgaa atggatggct 3600
   cctgaatcta tctttgacaa aatctacagc accaagagcg acgtgtggtc ttacggagta 3660
   ttgctgtggg aaatcttctc cttaggtggg tctccatacc caggagtaca aatggatgag 3720
   gacttttgca gtcgcctgag ggaaggcatg aggatgagag ctcctgagta ctctactcct 3780
   gaaatctatc agatcatgct ggactgctgg cacagagacc caaaagaaag gccaagattt 3840
   gcagaacttg tggaaaaact aggtgatttg cttcaagcaa atgtacaaca ggatggtaaa 3900
   gactacatcc caatcaatgc catactgaca ggaaatagtg ggtttacata ctcaactcct 3960
   gccttctctg aggacttctt caaggaaagt atttcagctc cgaagtttaa ttcaggaagc 4020
   tctgatgatg tcagatatgt aaatgctttc aagttcatga gcctggaaag aatcaaaacc 4080
   tttgaagaac ttttaccgaa tgccacctcc atgtttgatg actaccaggg cgacagcagc 4140
   actctgttgg cctctcccat gctgaagcgc ttcacctgga ctgacagcaa acccaaggcc 4200
   tcgctcaaga ttgacttgag agtaaccagt aaaagtaagg agtcggggct gtctgatgtc 4260
   agcaggccca gtttctgcca ttccagctgt gggcacgtca gcgaaggcaa gcgcaggttc 4320
   acctacgacc acgctgagct ggaaaggaaa atcgcgtgct gctccccgcc cccagactac 4380
   aactcggtgg tcctgtactc caccccaccc atctagagtt tgacacgaag ccttatttct 4440
   agaagcacat gtgtatttat acccccagga aactagcttt tgccagtatt atgcatatat 4500
   aagtttacac ctttatcttt ccatgggagc cagctgcttt ttgtgatttt tttaatagtg 4560
   cttttttttt tttgactaac aagaatgtaa ctccagatag agaaatagtg acaagtgaag 4620
   aacactactg ctaaatcctc atgttactca gtgttagaga aatccttcct aaacccaatg 4680
   acttccctgc tccaaccccc gccacctcag ggcacgcagg accagtttga ttgaggagct 4740
   gcactgatca cccaatgcat cacgtacccc actgggccag ccctgcagcc caaaacccag 4800
   ggcaacaagc ccgttagccc cagggatcac tggctggcct gagcaacatc tcgggagtcc 4860
   tctagcaggc ctaagacatg tgaggaggaa aaggaaaaaa agcaaaaagc aagggagaaa 4920
   agagaaaccg ggagaaggca tgagaaagaa tttgagacgc accatgtggg cacggagggg 4980
   gacggggctc agcaatgcca tttcagtggc ttcccagctc tgacccttct acatttgagg 5040
   gcccagccag gagcagatgg acagcgatga ggggacattt tctggattct gggaggcaag 5100
   aaaaggacaa atatcttttt tggaactaaa gcaaatttta gaactttacc tatggaagtg 5160
   gttctatgtc cattctcatt cgtggcatgt tttgatttgt agcactgagg gtggcactca 5220
   actctgagcc catacttttg gctcctctag taagatgcac tgaaaactta gccagagtta 5280
   ggttgtctcc aggccatgat ggccttacac tgaaaatgtc acattctatt ttgggtatta 5340
   atatatagtc cagacactta actcaatttc ttggtattat tctgttttgc acagttagtt 5400
   gtgaaagaaa gctgagaaga atgaaaatgc agtcctgagg agaggagttt tctccatatc 5460
   aaaacgaggg ctgatggagg aaaaaggtca ataaggtcaa gggaaaaccc cgtctctata 5520
   ccaaccaaac caattcacca acacagttgg gacccaaaac acaggaagtc agtcacgttt 5580
   ccttttcatt taatggggat tccactatct cacactaatc tgaaaggatg tggaagagca 5640
   ttagctggcg catattaagc actttaagct ccttgagtaa aaaggtggta tgtaatttat 5700
   gcaaggtatt tctccagttg ggactcagga tattagttaa tgagccatca ctagaagaaa 5760
   agcccatttt caactgcttt gaaacttgcc tggggtctga gcatgatggg aatagggaga 5820
   cagggtagga aagggcgcct actcttcagg gtctaaagat caagtgggcc ttggatcgct 5880
   aagctggctc tgtttgatgc tatttatgca agttagggtc tatgtattta tgatgtctgc 5940
   accttctgca gccagtcaga agctggagag gcaacagtgg attgctgctt cttggggaga 6000
   agagtatgct tccttttatc catgtaattt aactgtagaa cctgagctct aagtaaccga 6060
   agaatgtatg cctctgttct tatgtgccac atccttgttt aaaggctctc tgtatgaaga 6120
   gatgggaccg tcatcagcac attccctagt gagcctactg gctcctggca gcggcttttg 6180
   tggaagactc actagccaga agagaggagt gggacagtcc tctccaccaa gatctaaatc 6240
   caaacaaaag caggctagag ccagaagaga ggacaaatct ttgttcttcc tcttctttac 6300
   atacgcaaac cacctgtgac agctggcaat tttataaatc aggtaactgg aaggaggtta 6360
   aacacagaaa aaagaagacc tcagtcaatt ctctactttt tttttttttt ccaaatcaga 6420
   taatagccca gcaaatagtg ataacaaata aaaccttagc tattcatgtc ttgatttcaa 6480
   taattaattc ttaatcatta agagaccata ataaatactc cttttcaaga gaaaagcaaa 6540
   accattagaa ttgttactca gctccttcaa actcaggttt gtagcataca tgagtccatc 6600
   catcagtcaa agaatggttc catctggagt cttaatgtag aaagaaaaat ggagacttgt 6660
   aataatgagc tagttacaaa gtgcttgttc attaaaatag cactgaaaat tgaaacatga 6720
   attaactgat aatattccaa tcatttgcca tttatgacaa aaatggttgg cactaacaaa 6780
   gaacgagcac ttcctttcag agtttctgag ataatgtacg tggaacagtc tgggtggaat 6840
   ggggctgaaa ccatgtgcaa gtctgtgtct tgtcagtcca agaagtgaca ccgagatgtt 6900
   aattttaggg acccgtgcct tgtttcctag cccacaagaa tgcaaacatc aaacagatac 6960
   tcgctagcct catttaaatt gattaaagga ggagtgcatc tttggccgac agtggtgtaa 7020
   ctgtatgtgt gtgtgtgtgt gtgtgtgtgt gtgtgggtgt atgtgtgttt tgtgcataac 7080
   tatttaagga aactggaatt ttaaagttac ttttatacaa accaagaata tatgctacag 7140
   atataagaca gacatggttt ggtcctatat ttctagtcat gatgaatgta ttttgtatac 7200
   catcttcata taataaactt ccaaaaacac atata 7235
<210> 2
   <211> 7215
   <212> DNA
   <213> Homo sapiens
<400> 2
   taaatcgccc ccgccctcgg ctgctcttca tcgaggtccg cgggaggctc ggagcgcgcc 60
   aggcggacac tcctctcggc tcctccccgg cagcggcggc ggctcggagc gggctccggg 120
   gctcgggtgc agcggccagc gggcgcctgg cggcgaggat tacccgggga agtggttgtc 180
   tcctggctgg agccgcgaga cgggcgctca gggcgcgggg ccggcggcgg cgaacgagag 240
   gacggactct ggcggccggg tcgttggccg cggggagcgc gggcaccggg cgagcaggcc 300
   gcgtcgcgct caccatggtc agctactggg acaccggggt cctgctgtgc gcgctgctca 360
   gctgtctgct tctcacagga tctagttcag gttcaaaatt aaaagatcct gaactgagtt 420
   taaaaggcac ccagcacatc atgcaagcag gccagacact gcatctccaa tgcagggggg 480
   aagcagccca taaatggtct ttgcctgaaa tggtgagtaa ggaaagcgaa aggctgagca 540
   taactaaatc tgcctgtgga agaaatggca aacaattctg cagtacttta accttgaaca 600
   cagctcaagc aaaccacact ggcttctaca gctgcaaata tctagctgta cctacttcaa 660
   agaagaagga aacagaatct gcaatctata tatttattag tgatacaggt agacctttcg 720
   tagagatgta cagtgaaatc cccgaaatta tacacatgac tgaaggaagg gagctcgtca 780
   ttccctgccg ggttacgtca cctaacatca ctgttacttt aaaaaagttt ccacttgaca 840
   ctttgatccc tgatggaaaa cgcataatct gggacagtag aaagggcttc atcatatcaa 900
   atgcaacgta caaagaaata gggcttctga cctgtgaagc aacagtcaat gggcatttgt 960
   ataagacaaa ctatctcaca catcgacaaa ccaatacaat catagatgtc caaataagca 1020
   caccacgccc agtcaaatta cttagaggcc atactcttgt cctcaattgt actgctacca 1080
   ctcccttgaa cacgagagtt caaatgacct ggagttaccc tgatgaaaaa aataagagag 1140
   cttccgtaag gcgacgaatt gaccaaagca attcccatgc caacatattc tacagtgttc 1200
   ttactattga caaaatgcag aacaaagaca aaggacttta tacttgtcgt gtaaggagtg 1260
   gaccatcatt caaatctgtt aacacctcag tgcatatata tgataaagca ttcatcactg 1320
   tgaaacatcg aaaacagcag gtgcttgaaa ccgtagctgg caagcggtct taccggctct 1380
   ctatgaaagt gaaggcattt ccctcgccgg aagttgtatg gttaaaagat gggttacctg 1440
   cgactgagaa atctgctcgc tatttgactc gtggctactc gttaattatc aaggacgtaa 1500
   ctgaagagga tgcagggaat tatacaatct tgctgagcat aaaacagtca aatgtgttta 1560
   aaaacctcac tgccactcta attgtcaatg tgaaacccca gatttacgaa aaggccgtgt 1620
   catcgtttcc agacccggct ctctacccac tgggcagcag acaaatcctg acttgtaccg 1680
   catatggtat ccctcaacct acaatcaagt ggttctggca cccctgtaac cataatcatt   1740
   ccgaagcaag gtgtgacttt tgttccaata atgaagagtc ctttatcctg gatgctgaca   1800
   gcaacatggg aaacagaatt gagagcatca ctcagcgcat ggcaataata gaaggaaaga   1860
   ataagatggc tagcaccttg gttgtggctg actctagaat ttctggaatc tacatttgca   1920
   tagcttccaa taaagttggg actgtgggaa gaaacataag cttttatatc acagaattgt   1980
   caaactttga gtgccttcat ccttgctctc aggaatagaa ttctacctca tcggatctca   2040
   tgtgccaaat gggtttcatg ttaacttgga aaaaatgccg acggaaggag aggacctgaa   2100
   actgtcttgc acagttaaca agttcttata cagagacgtt acttggattt tactgcggac   2160
   agttaataac agaacaatgc actacagtat tagcaagcaa aaaatggcca tcactaagga   2220
   gcactccatc actcttaatc ttaccatcat gaatgtttcc ctgcaagatt caggcaccta   2280
   tgcctgcaga gccaggaatg tatacacagg ggaagaaatc ctccagaaga aagaaattac   2340
   aatcagagat caggaagcac catacctcct gcgaaacctc agtgatcaca cagtggccat   2400
   cagcagttcc accactttag actgtcatgc taatggtgtc cccgagcctc agatcacttg   2460
   gtttaaaaac aaccacaaaa tacaacaaga gcctggaatt attttaggac caggaagcag   2520
   cacgctgttt attgaaagag tcacagaaga ggatgaaggt gtctatcact gcaaagccac   2580
   caaccagaag ggctctgtgg aaagttcagc atacctcact gttcaaggaa cctcggacaa   2640
   gtctaatctg gagctgatca ctctaacatg cacctgtgtg gctgcgactc tcttctggct   2700
   cctattaacc ctctttatcc gaaaaatgaa aaggtcttct tctgaaataa agactgacta   2760
   cctatcaatt ataatggacc cagatgaagt tcctttggat gagcagtgtg agcggctccc   2820
   ttatgatgcc agcaagtggg agtttgcccg ggagagactt aaactgggca aatcacttgg   2880
   aagaggggct tttggaaaag tggttcaagc atcagcattt ggcattaaga aatcacctac   2940
   gtgccggact gtggctgtga aaatgctgaa agagggggcc acggccagcg agtacaaagc   3000
   tctgatgact gagctaaaaa tcttgaccca cattggccac catctgaacg tggttaacct   3060
   gctgggagcc tgcaccaagc aaggagggcc tctgatggtg attgttgaat actgcaaata   3120
   tggaaatctc tccaactacc tcaagagcaa acgtgactta ttttttctca acaaggatgc   3180
   agcactacac atggagccta agaaagaaaa aatggagcca ggcctggaac aaggcaagaa   3240
   accaagacta gatagcgtca ccagcagcga aagctttgcg agctccggct ttcaggaaga   3300
   taaaagtctg agtgatgttg aggaagagga ggattctgac ggtttctaca aggagcccat   3360
   cactatggaa gatctgattt cttacagttt tcaagtggcc agaggcatgg agttcctgtc   3420
   ttccagaaag tgcattcatc gggacctggc agcgagaaac attcttttat ctgagaacaa   3480
   cgtggtgaag atttgtgatt ttggccttgc ccgggatatt tataagaacc ccgattatgt   3540
   gagaaaagga gatactcgac ttcctctgaa atggatggct cctgaatcta tctttgacaa   3600
   aatctacagc accaagagcg acgtgtggtc ttacggagta ttgctgtggg aaatcttctc   3660
   cttaggtggg tctccatacc caggagtaca aatggatgag gacttttgca gtcgcctgag 3720
   ggaaggcatg aggatgagag ctcctgagta ctctactcct gaaatctatc agatcatgct 3780
   ggactgctgg cacagagacc caaaagaaag gccaagattt gcagaacttg tggaaaaact 3840
   aggtgatttg cttcaagcaa atgtacaaca ggatggtaaa gactacatcc caatcaatgc 3900
   catactgaca ggaaatagtg ggtttacata ctcaactcct gccttctctg aggacttctt 3960
   caaggaaagt atttcagctc cgaagtttaa ttcaggaagc tctgatgatg tcagatatgt 4020
   aaatgctttc aagttcatga gcctggaaag aatcaaaacc tttgaagaac ttttaccgaa 4080
   tgccacctcc atgtttgatg actaccaggg cgacagcagc actctgttgg cctctcccat 4140
   gctgaagcgc ttcacctgga ctgacagcaa acccaaggcc tcgctcaaga ttgacttgag 4200
   agtaaccagt aaaagtaagg agtcggggct gtctgatgtc agcaggccca gtttctgcca 4260
   ttccagctgt gggcacgtca gcgaaggcaa gcgcaggttc acctacgacc acgctgagct 4320
   ggaaaggaaa atcgcgtgct gctccccgcc cccagactac aactcggtgg tcctgtactc 4380
   caccccaccc atctagagtt tgacacgaag ccttatttct agaagcacat gtgtatttat 4440
   acccccagga aactagcttt tgccagtatt atgcatatat aagtttacac ctttatcttt 4500
   ccatgggagc cagctgcttt ttgtgatttt tttaatagtg cttttttttt tttgactaac 4560
   aagaatgtaa ctccagatag agaaatagtg acaagtgaag aacactactg ctaaatcctc 4620
   atgttactca gtgttagaga aatccttcct aaacccaatg acttccctgc tccaaccccc 4680
   gccacctcag ggcacgcagg accagtttga ttgaggagct gcactgatca cccaatgcat 4740
   cacgtacccc actgggccag ccctgcagcc caaaacccag ggcaacaagc ccgttagccc 4800
   cagggatcac tggctggcct gagcaacatc tcgggagtcc tctagcaggc ctaagacatg 4860
   tgaggaggaa aaggaaaaaa agcaaaaagc aagggagaaa agagaaaccg ggagaaggca 4920
   tgagaaagaa tttgagacgc accatgtggg cacggagggg gacggggctc agcaatgcca 4980
   tttcagtggc ttcccagctc tgacccttct acatttgagg gcccagccag gagcagatgg 5040
   acagcgatga ggggacattt tctggattct gggaggcaag aaaaggacaa atatcttttt 5100
   tggaactaaa gcaaatttta gaactttacc tatggaagtg gttctatgtc cattctcatt 5160
   cgtggcatgt tttgatttgt agcactgagg gtggcactca actctgagcc catacttttg 5220
   gctcctctag taagatgcac tgaaaactta gccagagtta ggttgtctcc aggccatgat 5280
   ggccttacac tgaaaatgtc acattctatt ttgggtatta atatatagtc cagacactta 5340
   actcaatttc ttggtattat tctgttttgc acagttagtt gtgaaagaaa gctgagaaga 5400
   atgaaaatgc agtcctgagg agaggagttt tctccatatc aaaacgaggg ctgatggagg 5460
   aaaaaggtca ataaggtcaa gggaaaaccc cgtctctata ccaaccaaac caattcacca 5520
   acacagttgg gacccaaaac acaggaagtc agtcacgttt ccttttcatt taatggggat 5580
   tccactatct cacactaatc tgaaaggatg tggaagagca ttagctggcg catattaagc 5640
   actttaagct ccttgagtaa aaaggtggta tgtaatttat gcaaggtatt tctccagttg 5700
   ggactcagga tattagttaa tgagccatca ctagaagaaa agcccatttt caactgcttt 5760
   gaaacttgcc tggggtctga gcatgatggg aatagggaga cagggtagga aagggcgcct 5820
   actcttcagg gtctaaagat caagtgggcc ttggatcgct aagctggctc tgtttgatgc 5880
   tatttatgca agttagggtc tatgtattta tgatgtctgc accttctgca gccagtcaga 5940
   agctggagag gcaacagtgg attgctgctt cttggggaga agagtatgct tccttttatc 6000
   catgtaattt aactgtagaa cctgagctct aagtaaccga agaatgtatg cctctgttct 6060
   tatgtgccac atccttgttt aaaggctctc tgtatgaaga gatgggaccg tcatcagcac 6120
   attccctagt gagcctactg gctcctggca gcggcttttg tggaagactc actagccaga 6180
   agagaggagt gggacagtcc tctccaccaa gatctaaatc caaacaaaag caggctagag 6240
   ccagaagaga ggacaaatct ttgttcttcc tcttctttac atacgcaaac cacctgtgac 6300
   agctggcaat tttataaatc aggtaactgg aaggaggtta aacacagaaa aaagaagacc 6360
   tcagtcaatt ctctactttt tttttttttt ccaaatcaga taatagccca gcaaatagtg 6420
   ataacaaata aaaccttagc tattcatgtc ttgatttcaa taattaattc ttaatcatta 6480
   agagaccata ataaatactc cttttcaaga gaaaagcaaa accattagaa ttgttactca 6540
   gctccttcaa actcaggttt gtagcataca tgagtccatc catcagtcaa agaatggttc 6600
   catctggagt cttaatgtag aaagaaaaat ggagacttgt aataatgagc tagttacaaa 6660
   gtgcttgttc attaaaatag cactgaaaat tgaaacatga attaactgat aatattccaa 6720
   tcatttgcca tttatgacaa aaatggttgg cactaacaaa gaacgagcac ttcctttcag 6780
   agtttctgag ataatgtacg tggaacagtc tgggtggaat ggggctgaaa ccatgtgcaa 6840
   gtctgtgtct tgtcagtcca agaagtgaca ccgagatgtt aattttaggg acccgtgcct 6900
   tgtttcctag cccacaagaa tgcaaacatc aaacagatac tcgctagcct catttaaatt 6960
   gattaaagga ggagtgcatc tttggccgac agtggtgtaa ctgtatgtgt gtgtgtgtgt 7020
   gtgtgtgtgt gtgtgggtgt atgtgtgttt tgtgcataac tatttaagga aactggaatt 7080
   ttaaagttac ttttatacaa accaagaata tatgctacag atataagaca gacatggttt 7140
   ggtcctatat ttctagtcat gatgaatgta ttttgtatac catcttcata taataaactt 7200
   ccaaaaacac atata 7215
<210> 3
   <211> 7447
   <212> DNA
   <213> Homo sapiens
<400> 3
   ccttcattct tacacatgcg gtggtggcca cagctgatgg gctacagtct ttgactgctt 60
   tagatcttgt cagctaaatg taaagttact ttgtcaatgg aatgctacta acagccgtaa 120
   tggatagatg agagtaagag aaacacttcg gaggaaattt tagtgtattt ttaagttgta 180
   ctgaggacgt ctgtcagcaa tcctttcttt tagattcagc acatgcttgt tttcacttat 240
   ttgtaaagta ccatagattt cagtccctga cttaaatttt tctgggtttt tcctttgctt 300
   gcactaaatt agaagagaag gttctttttt tcccaccgac tgcattcctg agtttacagg 360
   tagcaccttg atttactgaa acatgaaagc gaatccttta attgctttca gttcttcata 420
   aacaggatgt aattaatttc aaacccaagg aacaccggta atacattccg atgtaggcat 480
   agtccctgga agagaggcac tagtggcttc tggactagaa atctctgcca taggatcaca 540
   aatccagatc agcgccttca gcaagcaata gaaataaatg ataactggat agagcgaggg 600
   atgccaggtg tactttttaa aaggcgatag aaaaggtttc gagcttcttt gtggctgttt 660
   ggtatttcct ttataatctg ttgggggaag gtaatttgat ttttttcccc ctttttcaac 720
   tgggaataag gaatagtggt agttgggagt ctaccaccac tgaaattggg ctggcaacct 780
   ggcatcaggg gaatttggct gggccacttt atggagcaat gaccctttat aaataaagtt 840
   taatcccaag gcagagtgtt tatcttgtag tctttaaatg ttacattctt gatgtcagaa 900
   gaaggcataa ggagaaagtc agatcatgga ttattttctt ctgtttggac tcaccgtgct 960
   tgggaatact tctgagcatt agagagcact tcattcattg cagagtctct ggcctccgag 1020
   gctgccttca ccatcagcag cttcagcttc tgggagtttc ctttccagag gcagagctga 1080
   tgccttcctt gtgacacagc aggatcgtca gaatgacagc cccatttcca cttgacactt 1140
   tgatccctga tggaaaacgc ataatctggg acagtagaaa gggcttcatc atatcaaatg 1200
   caacgtacaa agaaataggg cttctgacct gtgaagcaac agtcaatggg catttgtata 1260
   agacaaacta tctcacacat cgacaaacca atacaatcat agatgtccaa ataagcacac 1320
   cacgcccagt caaattactt agaggccata ctcttgtcct caattgtact gctaccactc 1380
   ccttgaacac gagagttcaa atgacctgga gttaccctga tgaaaaaaat aagagagctt 1440
   ccgtaaggcg acgaattgac caaagcaatt cccatgccaa catattctac agtgttctta 1500
   ctattgacaa aatgcagaac aaagacaaag gactttatac ttgtcgtgta aggagtggac 1560
   catcattcaa atctgttaac acctcagtgc atatatatga taaagcattc atcactgtga 1620
   aacatcgaaa acagcaggtg cttgaaaccg tagctggcaa gcggtcttac cggctctcta 1680
   tgaaagtgaa ggcatttccc tcgccggaag ttgtatggtt aaaagatggg ttacctgcga 1740
   ctgagaaatc tgctcgctat ttgactcgtg gctactcgtt aattatcaag gacgtaactg 1800
   aagaggatgc agggaattat acaatcttgc tgagcataaa acagtcaaat gtgtttaaaa 1860
   acctcactgc cactctaatt gtcaatgtga aaccccagat ttacgaaaag gccgtgtcat 1920
   cgtttccaga cccggctctc tacccactgg gcagcagaca aatcctgact tgtaccgcat 1980
   atggtatccc tcaacctaca atcaagtggt tctggcaccc ctgtaaccat aatcattccg 2040
   aagcaaggtg tgacttttgt tccaataatg aagagtcctt tatcctggat gctgacagca 2100
   acatgggaaa cagaattgag agcatcactc agcgcatggc aataatagaa ggaaagaata 2160
   agatggctag caccttggtt gtggctgact ctagaatttc tggaatctac atttgcatag 2220
   cttccaataa agttgggact gtgggaagaa acataagctt ttatatcaca gatgtgccaa 2280
   atgggtttca tgttaacttg gaaaaaatgc cgacggaagg agaggacctg aaactgtctt 2340
   gcacagttaa caagttctta tacagagacg ttacttggat tttactgcgg acagttaata 2400
   acagaacaat gcactacagt attagcaagc aaaaaatggc catcactaag gagcactcca 2460
   tcactcttaa tcttaccatc atgaatgttt ccctgcaaga ttcaggcacc tatgcctgca 2520
   gagccaggaa tgtatacaca ggggaagaaa tcctccagaa gaaagaaatt acaatcagag 2580
   atcaggaagc accatacctc ctgcgaaacc tcagtgatca cacagtggcc atcagcagtt 2640
   ccaccacttt agactgtcat gctaatggtg tccccgagcc tcagatcact tggtttaaaa 2700
   acaaccacaa aatacaacaa gagcctggaa ttattttagg accaggaagc agcacgctgt 2760
   ttattgaaag agtcacagaa gaggatgaag gtgtctatca ctgcaaagcc accaaccaga 2820
   agggctctgt ggaaagttca gcatacctca ctgttcaagg aacctcggac aagtctaatc 2880
   tggagctgat cactctaaca tgcacctgtg tggctgcgac tctcttctgg ctcctattaa 2940
   ccctctttat ccgaaaaatg aaaaggtctt cttctgaaat aaagactgac tacctatcaa 3000
   ttataatgga cccagatgaa gttcctttgg atgagcagtg tgagcggctc ccttatgatg 3060
   ccagcaagtg ggagtttgcc cgggagagac ttaaactggg caaatcactt ggaagagggg 3120
   cttttggaaa agtggttcaa gcatcagcat ttggcattaa gaaatcacct acgtgccgga 3180
   ctgtggctgt gaaaatgctg aaagaggggg ccacggccag cgagtacaaa gctctgatga 3240
   ctgagctaaa aatcttgacc cacattggcc accatctgaa cgtggttaac ctgctgggag 3300
   cctgcaccaa gcaaggaggg cctctgatgg tgattgttga atactgcaaa tatggaaatc 3360
   tctccaacta cctcaagagc aaacgtgact tattttttct caacaaggat gcagcactac 3420
   acatggagcc taagaaagaa aaaatggagc caggcctgga acaaggcaag aaaccaagac 3480
   tagatagcgt caccagcagc gaaagctttg cgagctccgg ctttcaggaa gataaaagtc 3540
   tgagtgatgt tgaggaagag gaggattctg acggtttcta caaggagccc atcactatgg 3600
   aagatctgat ttcttacagt tttcaagtgg ccagaggcat ggagttcctg tcttccagaa 3660
   agtgcattca tcgggacctg gcagcgagaa acattctttt atctgagaac aacgtggtga 3720
   agatttgtga ttttggcctt gcccgggata tttataagaa ccccgattat gtgagaaaag 3780
   gagatactcg acttcctctg aaatggatgg ctcctgaatc tatctttgac aaaatctaca 3840
   gcaccaagag cgacgtgtgg tcttacggag tattgctgtg ggaaatcttc tccttaggtg 3900
   ggtctccata cccaggagta caaatggatg aggacttttg cagtcgcctg agggaaggca 3960
   tgaggatgag agctcctgag tactctactc ctgaaatcta tcagatcatg ctggactgct 4020
   ggcacagaga cccaaaagaa aggccaagat ttgcagaact tgtggaaaaa ctaggtgatt 4080
   tgcttcaagc aaatgtacaa caggatggta aagactacat cccaatcaat gccatactga 4140
   caggaaatag tgggtttaca tactcaactc ctgccttctc tgaggacttc ttcaaggaaa 4200
   gtatttcagc tccgaagttt aattcaggaa gctctgatga tgtcagatat gtaaatgctt 4260
   tcaagttcat gagcctggaa agaatcaaaa cctttgaaga acttttaccg aatgccacct 4320
   ccatgtttga tgactaccag ggcgacagca gcactctgtt ggcctctccc atgctgaagc 4380
   gcttcacctg gactgacagc aaacccaagg cctcgctcaa gattgacttg agagtaacca 4440
   gtaaaagtaa ggagtcgggg ctgtctgatg tcagcaggcc cagtttctgc cattccagct 4500
   gtgggcacgt cagcgaaggc aagcgcaggt tcacctacga ccacgctgag ctggaaagga 4560
   aaatcgcgtg ctgctccccg cccccagact acaactcggt ggtcctgtac tccaccccac 4620
   ccatctagag tttgacacga agccttattt ctagaagcac atgtgtattt atacccccag 4680
   gaaactagct tttgccagta ttatgcatat ataagtttac acctttatct ttccatggga 4740
   gccagctgct tttgtgattt tttttaatag tgcttttttt tttttgacta acaagaatgt 4800
   aactccagat agagaaatag tgacaagtga agaacactac tgctaaatcc tcatgttact 4860
   cagtgttaga gaaatccttc ctaaacccaa tgacttccct gctccaaccc ccgccacctc 4920
   agggcacgca ggaccagttt gattgaggag ctgcactgat cacccaatgc atcacgtacc 4980
   ccactgggcc agccctgcag cccaaaaccc agggcaacaa gcccgttagc cccagggatc 5040
   actggctggc ctgagcaaca tctcgggagt cctctagcag gcctaagaca tgtgaggagg 5100
   aaaaggaaaa aaagcaaaaa gcaagggaga aaagagaaac cgggagaagg catgagaaag 5160
   aatttgagac gcaccatgtg ggcacggagg gggacggggc tcagcaatgc catttcagtg 5220
   gcttcccagc tctgaccctt ctacatttga gggcccagcc aggagcagat ggacagcgat 5280
   gaggggacat tttctggatt ctgggaggca agaaaaggac aaatatcttt tttggaacta 5340
   aagcaaattt tagaacttta cctatggaag tggttctatg tccattctca ttcgtggcat 5400
   gttttgattt gtagcactga gggtggcact caactctgag cccatacttt tggctcctct 5460
   agtaagatgc actgaaaact tagccagagt taggttgtct ccaggccatg atggccttac 5520
   actgaaaatg tcacattcta ttttgggtat taatatatag tccagacact taactcaatt 5580
   tcttggtatt attctgtttt gcacagttag ttgtgaaaga aagctgagaa gaatgaaaat 5640
   gcagtcctga ggagaggagt tttctccata tcaaaacgag ggctgatgga ggaaaaaggt 5700
   caataaggtc aagggaaaac cccgtctcta taccaaccaa accaattcac caacacagtt 5760
   gggacccaaa acacaggaag tcagtcacgt ttccttttca tttaatgggg attccactat 5820
   ctcacactaa tctgaaagga tgtggaagag cattagctgg cgcatattaa gcactttaag 5880
   ctccttgagt aaaaaggtgg tatgtaattt atgcaaggta tttctccagt tgggactcag 5940
   gatattagtt aatgagccat cactagaaga aaagcccatt ttcaactgct ttgaaacttg 6000
   cctggggtct gagcatgatg ggaataggga gacagggtag gaaagggcgc ctactcttca 6060
   gggtctaaag atcaagtggg ccttggatcg ctaagctggc tctgtttgat gctatttatg 6120
   caagttaggg tctatgtatt tatgatgtct gcaccttctg cagccagtca gaagctggag 6180
   aggcaacagt ggattgctgc ttcttgggga gaagagtatg cttcctttta tccatgtaat 6240
   ttaactgtag aacctgagct ctaagtaacc gaagaatgta tgcctctgtt cttatgtgcc 6300
   acatccttgt ttaaaggctc tctgtatgaa gagatgggac cgtcatcagc acattcccta 6360
   gtgagcctac tggctcctgg cagcggcttt tgtggaagac tcactagcca gaagagagga 6420
   gtgggacagt cctctccacc aagatctaaa tccaaacaaa agcaggctag agccagaaga 6480
   gaggacaaat ctttgttctt cctcttcttt acatacgcaa accacctgtg acagctggca 6540
   attttataaa tcaggtaact ggaaggaggt taaacacaga aaaaagaaga cctcagtcaa 6600
   ttctctactt tttttttttt ttccaaatca gataatagcc cagcaaatag tgataacaaa 6660
   taaaacctta gctattcatg tcttgatttc aataattaat tcttaatcat taagagacca 6720
   taataaatac tccttttcaa gagaaaagca aaaccattag aattgttact cagctccttc 6780
   aaactcaggt ttgtagcata catgagtcca tccatcagtc aaagaatggt tccatctgga 6840
   gtcttaatgt agaaagaaaa atggagactt gtaataatga gctagttaca aagtgcttgt   6900
   tcattaaaat agcactgaaa attgaaacat gaattaactg ataatattcc aatcatttgc   6960
   catttatgac aaaaatggtt ggcactaaca aagaacgagc acttcctttc agagtttctg 7020
   agataatgta cgtggaacag tctgggtgga atggggctga aaccatgtgc aagtctgtgt 7080
   cttgtcagtc caagaagtga caccgagatg ttaattttag ggacccgtgc cttgtttcct 7140
   agcccacaag aatgcaaaca tcaaacagat actcgctagc ctcatttaaa ttgattaaag 7200
   gaggagtgca tctttggccg acagtggtgt aactgtatgt gtgtgtgtgt gtgtgtgtgt 7260
   gtgtgtgggt gtatgtgtgt tttgtgcata actatttaag gaaactggaa ttttaaagtt 7320
   acttttatac aaaccaagaa tatatgctac agatataaga cagacatggt ttggtcctat 7380
   atttctagtc atgatgaatg tattttgtat accatcttca tataataaac ttccaaaaac 7440
   acatata 7447
<210> 4
   <211> 6788
   <212> DNA
   <213> Homo sapiens
<400> 4
   gtctctaact gctaacctca ggtgatccgc ccacctcggc ctcccaaagt actggaatta 60
   caggcgtgag ccaccacgcc cggcctctta attggtttgt ctattgagtg tctgaccagc 120
   ttattatgaa gattcccgga ggccaaagag ttatgggctc caactgaaag tctgacatga   180
   caagtaggtc tatcctgggt agatgccact tgttgccaag gctgatagta aattatcata   240
   attactataa tttatcataa tgcaaatatc ttttgctata tgcaaaaaaa ctcaagaacc   300
   aattataaca aactagagtt tttaacctag tttgttataa gcaaactaca aagttttact   360
   attttcattt tagatgaaaa atcatgtttt cacaaactgc tggtgaaaca gtattgccaa   420
   ccagtgctaa taggaccttg ttaatgcatg cctcttcctt ttcagtttcc acttgacact   480
   ttgatccctg atggaaaacg cataatctgg gacagtagaa agggcttcat catatcaaat   540
   gcaacgtaca aagaaatagg gcttctgacc tgtgaagcaa cagtcaatgg gcatttgtat   600
   aagacaaact atctcacaca tcgacaaacc aatacaatca tagatgtcca aataagcaca   660
   ccacgcccag tcaaattact tagaggccat actcttgtcc tcaattgtac tgctaccact   720
   cccttgaaca cgagagttca aatgacctgg agttaccctg atgaaaaaaa taagagagct   780
   tccgtaaggc gacgaattga ccaaagcaat tcccatgcca acatattcta cagtgttctt   840
   actattgaca aaatgcagaa caaagacaaa ggactttata cttgtcgtgt aaggagtgga   900
   ccatcattca aatctgttaa cacctcagtg catatatatg ataaagcatt catcactgtg   960
   aaacatcgaa aacagcaggt gcttgaaacc gtagctggca agcggtctta ccggctctct  1020
   atgaaagtga aggcatttcc ctcgccggaa gttgtatggt taaaagatgg gttacctgcg  1080
   actgagaaat ctgctcgcta tttgactcgt ggctactcgt taattatcaa ggacgtaact  1140
   gaagaggatg cagggaatta tacaatcttg ctgagcataa aacagtcaaa tgtgtttaaa  1200
   aacctcactg ccactctaat tgtcaatgtg aaaccccaga tttacgaaaa ggccgtgtca  1260
   tcgtttccag acccggctct ctacccactg ggcagcagac aaatcctgac ttgtaccgca  1320
   tatggtatcc ctcaacctac aatcaagtgg ttctggcacc cctgtaacca taatcattcc  1380
   gaagcaaggt gtgacttttg ttccaataat gaagagtcct ttatcctgga tgctgacagc  1440
   aacatgggaa acagaattga gagcatcact cagcgcatgg caataataga aggaaagaat  1500
   aagatggcta gcaccttggt tgtggctgac tctagaattt ctggaatcta catttgcata  1560
   gcttccaata aagttgggac tgtgggaaga aacataagct tttatatcac agatgtgcca  1620
   aatgggtttc atgttaactt ggaaaaaatg ccgacggaag gagaggacct gaaactgtct  1680
   tgcacagtta acaagttctt atacagagac gttacttgga ttttactgcg gacagttaat  1740
   aacagaacaa tgcactacag tattagcaag caaaaaatgg ccatcactaa ggagcactcc  1800
   atcactctta atcttaccat catgaatgtt tccctgcaag attcaggcac ctatgcctgc  1860
   agagccagga atgtatacac aggggaagaa atcctccaga agaaagaaat tacaatcaga  1920
   gatcaggaag caccatacct cctgcgaaac ctcagtgatc acacagtggc catcagcagt  1980
   tccaccactt tagactgtca tgctaatggt gtccccgagc ctcagatcac ttggtttaaa  2040
   aacaaccaca aaatacaaca agagcctgga attattttag gaccaggaag cagcacgctg 2100
   tttattgaaa gagtcacaga agaggatgaa ggtgtctatc actgcaaagc caccaaccag 2160
   aagggctctg tggaaagttc agcatacctc actgttcaag gaacctcgga caagtctaat 2220
   ctggagctga tcactctaac atgcacctgt gtggctgcga ctctcttctg gctcctatta 2280
   accctcttta tccgaaaaat gaaaaggtct tcttctgaaa taaagactga ctacctatca 2340
   attataatgg acccagatga agttcctttg gatgagcagt gtgagcggct cccttatgat 2400
   gccagcaagt gggagtttgc ccgggagaga cttaaactgg gcaaatcact tggaagaggg 2460
   gcttttggaa aagtggttca agcatcagca tttggcatta agaaatcacc tacgtgccgg 2520
   actgtggctg tgaaaatgct gaaagagggg gccacggcca gcgagtacaa agctctgatg 2580
   actgagctaa aaatcttgac ccacattggc caccatctga acgtggttaa cctgctggga 2640
   gcctgcacca agcaaggagg gcctctgatg gtgattgttg aatactgcaa atatggaaat 2700
   ctctccaact acctcaagag caaacgtgac ttattttttc tcaacaagga tgcagcacta 2760
   cacatggagc ctaagaaaga aaaaatggag ccaggcctgg aacaaggcaa gaaaccaaga 2820
   ctagatagcg tcaccagcag cgaaagcttt gcgagctccg gctttcagga agataaaagt 2880
   ctgagtgatg ttgaggaaga ggaggattct gacggtttct acaaggagcc catcactatg 2940
   gaagatctga tttcttacag ttttcaagtg gccagaggca tggagttcct gtcttccaga 3000
   aagtgcattc atcgggacct ggcagcgaga aacattcttt tatctgagaa caacgtggtg 3060
   aagatttgtg attttggcct tgcccgggat atttataaga accccgatta tgtgagaaaa 3120
   ggagatactc gacttcctct gaaatggatg gctcctgaat ctatctttga caaaatctac 3180
   agcaccaaga gcgacgtgtg gtcttacgga gtattgctgt gggaaatctt ctccttaggt 3240
   gggtctccat acccaggagt acaaatggat gaggactttt gcagtcgcct gagggaaggc 3300
   atgaggatga gagctcctga gtactctact cctgaaatct atcagatcat gctggactgc 3360
   tggcacagag acccaaaaga aaggccaaga tttgcagaac ttgtggaaaa actaggtgat 3420
   ttgcttcaag caaatgtaca acaggatggt aaagactaca tcccaatcaa tgccatactg 3480
   acaggaaata gtgggtttac atactcaact cctgccttct ctgaggactt cttcaaggaa 3540
   agtatttcag ctccgaagtt taattcagga agctctgatg atgtcagata tgtaaatgct 3600
   ttcaagttca tgagcctgga aagaatcaaa acctttgaag aacttttacc gaatgccacc 3660
   tccatgtttg atgactacca gggcgacagc agcactctgt tggcctctcc catgctgaag 3720
   cgcttcacct ggactgacag caaacccaag gcctcgctca agattgactt gagagtaacc 3780
   agtaaaagta aggagtcggg gctgtctgat gtcagcaggc ccagtttctg ccattccagc 3840
   tgtgggcacg tcagcgaagg caagcgcagg ttcacctacg accacgctga gctggaaagg 3900
   aaaatcgcgt gctgctcccc gcccccagac tacaactcgg tggtcctgta ctccacccca 3960
   cccatctaga gtttgacacg aagccttatt tctagaagca catgtgtatt tataccccca 4020
   ggaaactagc ttttgccagt attatgcata tataagttta cacctttatc tttccatggg 4080
   agccagctgc tttttgtgat ttttttaata gtgctttttt ttttttgact aacaagaatg 4140
   taactccaga tagagaaata gtgacaagtg aagaacacta ctgctaaatc ctcatgttac 4200
   tcagtgttag agaaatcctt cctaaaccca atgacttccc tgctccaacc cccgccacct 4260
   cagggcacgc aggaccagtt tgattgagga gctgcactga tcacccaatg catcacgtac 4320
   cccactgggc cagccctgca gcccaaaacc cagggcaaca agcccgttag ccccagggat 4380
   cactggctgg cctgagcaac atctcgggag tcctctagca ggcctaagac atgtgaggag 4440
   gaaaaggaaa aaaagcaaaa agcaagggag aaaagagaaa ccgggagaag gcatgagaaa 4500
   gaatttgaga cgcaccatgt gggcacggag ggggacgggg ctcagcaatg ccatttcagt 4560
   ggcttcccag ctctgaccct tctacatttg agggcccagc caggagcaga tggacagcga 4620
   tgaggggaca ttttctggat tctgggaggc aagaaaagga caaatatctt ttttggaact 4680
   aaagcaaatt ttagaacttt acctatggaa gtggttctat gtccattctc attcgtggca 4740
   tgttttgatt tgtagcactg agggtggcac tcaactctga gcccatactt ttggctcctc 4800
   tagtaagatg cactgaaaac ttagccagag ttaggttgtc tccaggccat gatggcctta 4860
   cactgaaaat gtcacattct attttgggta ttaatatata gtccagacac ttaactcaat 4920
   ttcttggtat tattctgttt tgcacagtta gttgtgaaag aaagctgaga agaatgaaaa 4980
   tgcagtcctg aggagaggag ttttctccat atcaaaacga gggctgatgg aggaaaaagg 5040
   tcaataaggt caagggaaaa ccccgtctct ataccaacca aaccaattca ccaacacagt 5100
   tgggacccaa aacacaggaa gtcagtcacg tttccttttc atttaatggg gattccacta 5160
   tctcacacta atctgaaagg atgtggaaga gcattagctg gcgcatatta agcactttaa 5220
   gctccttgag taaaaaggtg gtatgtaatt tatgcaaggt atttctccag ttgggactca 5280
   ggatattagt taatgagcca tcactagaag aaaagcccat tttcaactgc tttgaaactt 5340
   gcctggggtc tgagcatgat gggaataggg agacagggta ggaaagggcg cctactcttc 5400
   agggtctaaa gatcaagtgg gccttggatc gctaagctgg ctctgtttga tgctatttat 5460
   gcaagttagg gtctatgtat ttatgatgtc tgcaccttct gcagccagtc agaagctgga 5520
   gaggcaacag tggattgctg cttcttgggg agaagagtat gcttcctttt atccatgtaa 5580
   tttaactgta gaacctgagc tctaagtaac cgaagaatgt atgcctctgt tcttatgtgc 5640
   cacatccttg tttaaaggct ctctgtatga agagatggga ccgtcatcag cacattccct 5700
   agtgagccta ctggctcctg gcagcggctt ttgtggaaga ctcactagcc agaagagagg 5760
   agtgggacag tcctctccac caagatctaa atccaaacaa aagcaggcta gagccagaag 5820
   agaggacaaa tctttgttct tcctcttctt tacatacgca aaccacctgt gacagctggc 5880
   aattttataa atcaggtaac tggaaggagg ttaaacacag aaaaaagaag acctcagtca 5940
   attctctact tttttttttt tttccaaatc agataatagc ccagcaaata gtgataacaa 6000
   ataaaacctt agctattcat gtcttgattt caataattaa ttcttaatca ttaagagacc 6060
   ataataaata ctccttttca agagaaaagc aaaaccatta gaattgttac tcagctcctt 6120
   caaactcagg tttgtagcat acatgagtcc atccatcagt caaagaatgg ttccatctgg 6180
   agtcttaatg tagaaagaaa aatggagact tgtaataatg agctagttac aaagtgcttg 6240
   ttcattaaaa tagcactgaa aattgaaaca tgaattaact gataatattc caatcatttg 6300
   ccatttatga caaaaatggt tggcactaac aaagaacgag cacttccttt cagagtttct 6360
   gagataatgt acgtggaaca gtctgggtgg aatggggctg aaaccatgtg caagtctgtg 6420
   tcttgtcagt ccaagaagtg acaccgagat gttaatttta gggacccgtg ccttgtttcc 6480
   tagcccacaa gaatgcaaac atcaaacaga tactcgctag cctcatttaa attgattaaa 6540
   ggaggagtgc atctttggcc gacagtggtg taactgtatg tgtgtgtgtg tgtgtgtgtg 6600
   tgtgtgtggg tgtatgtgtg ttttgtgcat aactatttaa ggaaactgga attttaaagt 6660
   tacttttata caaaccaaga atatatgcta cagatataag acagacatgg tttggtccta 6720
   tatttctagt catgatgaat gtattttgta taccatcttc atataataaa cttccaaaaa 6780
   cacatata 6788

<210> 5
   <211> 3903
   <212> DNA
   <213> Homo sapiens
<400> 5
   taaatcgccc ccgccctcgg ctgctcttca tcgaggtccg cgggaggctc ggagcgcgcc     60
   aggcggacac tcctctcggc tcctccccgg cagcggcggc ggctcggagc gggctccggg    120
   gctcgggtgc agcggccagc gggcgcctgg cggcgaggat tacccgggga agtggttgtc    180
   tcctggctgg agccgcgaga cgggcgctca gggcgcgggg ccggcggcgg cgaacgagag    240
   gacggactct ggcggccggg tcgttggccg cggggagcgc gggcaccggg cgagcaggcc    300
   gcgtcgcgct caccatggtc agctactggg acaccggggt cctgctgtgc gcgctgctca    360
   gctgtctgct tctcacagga tctagttcag gttcaaaatt aaaagatcct gaactgagtt    420
   taaaaggcac ccagcacatc atgcaagcag gccagacact gcatctccaa tgcagggggg    480
   aagcagccca taaatggtct ttgcctgaaa tggtgagtaa ggaaagcgaa aggctgagca    540
   taactaaatc tgcctgtgga agaaatggca aacaattctg cagtacttta accttgaaca    600
   cagctcaagc aaaccacact ggcttctaca gctgcaaata tctagctgta cctacttcaa    660
   agaagaagga aacagaatct gcaatctata tatttattag tgatacaggt agacctttcg    720
   tagagatgta cagtgaaatc cccgaaatta tacacatgac tgaaggaagg gagctcgtca    780
   ttccctgccg ggttacgtca cctaacatca ctgttacttt aaaaaagttt ccacttgaca    840
   ctttgatccc tgatggaaaa cgcataatct gggacagtag aaagggcttc atcatatcaa 900
   atgcaacgta caaagaaata gggcttctga cctgtgaagc aacagtcaat gggcatttgt 960
   ataagacaaa ctatctcaca catcgacaaa ccaatacaat catagatgtc caaataagca 1020
   caccacgccc agtcaaatta cttagaggcc atactcttgt cctcaattgt actgctacca 1080
   ctcccttgaa cacgagagtt caaatgacct ggagttaccc tgatgaaaaa aataagagag 1140
   cttccgtaag gcgacgaatt gaccaaagca attcccatgc caacatattc tacagtgttc 1200
   ttactattga caaaatgcag aacaaagaca aaggacttta tacttgtcgt gtaaggagtg 1260
   gaccatcatt caaatctgtt aacacctcag tgcatatata tgataaagca ttcatcactg 1320
   tgaaacatcg aaaacagcag gtgcttgaaa ccgtagctgg caagcggtct taccggctct 1380
   ctatgaaagt gaaggcattt ccctcgccgg aagttgtatg gttaaaagat gggttacctg 1440
   cgactgagaa atctgctcgc tatttgactc gtggctactc gttaattatc aaggacgtaa 1500
   ctgaagagga tgcagggaat tatacaatct tgctgagcat aaaacagtca aatgtgttta 1560
   aaaacctcac tgccactcta attgtcaatg tgaaacccca gatttacgaa aaggccgtgt 1620
   catcgtttcc agacccggct ctctacccac tgggcagcag acaaatcctg acttgtaccg 1680
   catatggtat ccctcaacct acaatcaagt ggttctggca cccctgtaac cataatcatt 1740
   ccgaagcaag gtgtgacttt tgttccaata atgaagagtc ctttatcctg gatgctgaca 1800
   gcaacatggg aaacagaatt gagagcatca ctcagcgcat ggcaataata gaaggaaaga 1860
   ataagatggc tagcaccttg gttgtggctg actctagaat ttctggaatc tacatttgca 1920
   tagcttccaa taaagttggg actgtgggaa gaaacataag cttttatatc acagatgtgc 1980
   caaatgggtt tcatgttaac ttggaaaaaa tgccgacgga aggagaggac ctgaaactgt 2040
   cttgcacagt taacaagttc ttatacagag acgttacttg gattttactg cggacagtta 2100
   ataacagaac aatgcactac agtattagca agcaaaaaat ggccatcact aaggagcact 2160
   ccatcactct taatcttacc atcatgaatg tttccctgca agattcaggc acctatgcct 2220
   gcagagccag gaatgtatac acaggggaag aaatcctcca gaagaaagaa attacaatca 2280
   gagatcagga agcaccatac ctcctgcgaa acctcagtga tcacacagtg gccatcagca 2340
   gttccaccac tttagactgt catgctaatg gtgtccccga gcctcagatc acttggttta 2400
   aaaacaacca caaaatacaa caagagcctg aactgtatac atcaacgtca ccatcgtcat 2460
   cgtcatcatc accattgtca tcatcatcat catcgtcatc atcatcatca tcatagctat 2520
   catcattatc atcatcatca tcatcatcat catagctacc atttattgaa aactattatg 2580
   tgtcaacttc aaagaactta tcctttagtt ggagagccaa gacaatcata acaataacaa 2640
   atggccgggc atggtggctc acgcctgtaa tcccagcact ttgggaggcc aaggcaggtg 2700
   gatcatttga ggtcaggagt tcaagaccag cctgaccaag atggtgaaat gctgtctcta 2760
   ttaaaaatac aaaattagcc gggcatggtg gctcatgcct gtaatgccag ctactcggga 2820
   ggctgagaca ggagaatcac ttgaacccag gaggcagagg ttgcagggag ccgagatcgt 2880
   gtactgcact ccagcctggg caacaagagc gaaactccgt ctcaaaaaac aaataaataa 2940
   ataaataaat aaacagacaa aattcacttt ttattctatt aaacttaaca tacatgcatt 3000
   aattgcctac tctgagcctg atcctttatt atattctaga gaaaatgaaa ataagcaaca 3060
   cataatcctc acctttgaaa aacaaacata ataggtacaa agtcagttca taaatgtgca 3120
   atgtgagtgg tataaaataa catcagataa aaattagcac ttctgatgac taatcagggg 3180
   agatttcatg gaggagatgt catctgaatt ggactttgat gaataaaaaa gagagttttg 3240
   atgacgaata ttagtttggc caaagtggat gaatataatg ttctctaagt caacaataca 3300
   gatagtcaac tgagcaacaa aacagaagtt gctggtcaga ctagtaggcc catttaggag 3360
   agcaacaagc cacttttgtg tgtggggggg aacaggaggc aaacacatgg cctgcattta 3420
   tgtaatcaaa taggtgaatt caaaagctct acagatttat ttactcaatg gtgaattgga 3480
   ttaaaattat attccatgct cttccctggg caatatggtg ctcacacata aaatgccaag 3540
   attgtccttc accttaaggt ataatgggaa gaaataaagc tatctacaga gtcccttgaa 3600
   aattacacct ttaaaattga cagttggaca tcttgaaaat ttagaccaca tcccatgaat 3660
   aaattaggcc atcatggtca aaaacttatc cacatcctcc attagcaacc aacataataa 3720
   gtaattcaaa tcctcacact gagtttcatc gtcagatact ttagaggaca ccacacgttt 3780
   ctacaaaaac agcccagtgt aaagttttac ttgtcctttt ggggctaatg ctttagcaaa 3840
   cgcataaaac caccagagtt tgcattctaa gctccaggaa ctgaggcttt ttgaacctta 3900
   aga 3903
<210> 6
   <211> 2623
   <212> DNA
   <213> Homo sapiens
<400> 6
   taaatcgccc ccgccctcgg ctgctcttca tcgaggtccg cgggaggctc ggagcgcgcc    60
   aggcggacac tcctctcggc tcctccccgg cagcggcggc ggctcggagc gggctccggg   120
   gctcgggtgc agcggccagc gggcgcctgg cggcgaggat tacccgggga agtggttgtc   180
   tcctggctgg agccgcgaga cgggcgctca gggcgcgggg ccggcggcgg cgaacgagag   240
   gacggactct ggcggccggg tcgttggccg cggggagcgc gggcaccggg cgagcaggcc   300
   gcgtcgcgct caccatggtc agctactggg acaccggggt cctgctgtgc gcgctgctca   360
   gctgtctgct tctcacagga tctagttcag gttcaaaatt aaaagatcct gaactgagtt   420
   taaaaggcac ccagcacatc atgcaagcag gccagacact gcatctccaa tgcagggggg   480
   aagcagccca taaatggtct ttgcctgaaa tggtgagtaa ggaaagcgaa aggctgagca   540
   taactaaatc tgcctgtgga agaaatggca aacaattctg cagtacttta accttgaaca    600
   cagctcaagc aaaccacact ggcttctaca gctgcaaata tctagctgta cctacttcaa    660
   agaagaagga aacagaatct gcaatctata tatttattag tgatacaggt agacctttcg    720
   tagagatgta cagtgaaatc cccgaaatta tacacatgac tgaaggaagg gagctcgtca    780
   ttccctgccg ggttacgtca cctaacatca ctgttacttt aaaaaagttt ccacttgaca    840
   ctttgatccc tgatggaaaa cgcataatct gggacagtag aaagggcttc atcatatcaa    900
   atgcaacgta caaagaaata gggcttctga cctgtgaagc aacagtcaat gggcatttgt    960
   ataagacaaa ctatctcaca catcgacaaa ccaatacaat catagatgtc caaataagca   1020
   caccacgccc agtcaaatta cttagaggcc atactcttgt cctcaattgt actgctacca   1080
   ctcccttgaa cacgagagtt caaatgacct ggagttaccc tgatgaaaaa aataagagag   1140
   cttccgtaag gcgacgaatt gaccaaagca attcccatgc caacatattc tacagtgttc   1200
   ttactattga caaaatgcag aacaaagaca aaggacttta tacttgtcgt gtaaggagtg   1260
   gaccatcatt caaatctgtt aacacctcag tgcatatata tgataaagca ttcatcactg   1320
   tgaaacatcg aaaacagcag gtgcttgaaa ccgtagctgg caagcggtct taccggctct   1380
   ctatgaaagt gaaggcattt ccctcgccgg aagttgtatg gttaaaagat gggttacctg   1440
   cgactgagaa atctgctcgc tatttgactc gtggctactc gttaattatc aaggacgtaa   1500
   ctgaagagga tgcagggaat tatacaatct tgctgagcat aaaacagtca aatgtgttta   1560
   aaaacctcac tgccactcta attgtcaatg tgaaacccca gatttacgaa aaggccgtgt   1620
   catcgtttcc agacccggct ctctacccac tgggcagcag acaaatcctg acttgtaccg   1680
   catatggtat ccctcaacct acaatcaagt ggttctggca cccctgtaac cataatcatt   1740
   ccgaagcaag gtgtgacttt tgttccaata atgaagagtc ctttatcctg gatgctgaca   1800
   gcaacatggg aaacagaatt gagagcatca ctcagcgcat ggcaataata gaaggaaaga   1860
   ataagatggc tagcaccttg gttgtggctg actctagaat ttctggaatc tacatttgca   1920
   tagcttccaa taaagttggg actgtgggaa gaaacataag cttttatatc acagatgtgc   1980
   caaatgggtt tcatgttaac ttggaaaaaa tgccgacgga aggagaggac ctgaaactgt   2040
   cttgcacagt taacaagttc ttatacagag acgttacttg gattttactg cggacagtta   2100
   ataacagaac aatgcactac agtattagca agcaaaaaat ggccatcact aaggagcact   2160
   ccatcactct taatcttacc atcatgaatg tttccctgca agattcaggc acctatgcct   2220
   gcagagccag gaatgtatac acaggggaag aaatcctcca gaagaaagaa attacaatca   2280
   gagatcagga agcaccatac ctcctgcgaa acctcagtga tcacacagtg gccatcagca   2340
   gttccaccac tttagactgt catgctaatg gtgtccccga gcctcagatc acttggttta   2400
   aaaacaacca caaaatacaa caagagcctg gctctgcaaa tacagcagtg aacaagaaaa   2460
   ctgaaatctg aagaaccaaa aatctagagt aattaagtga tgcatgtatt tacctgaaat    2520
   acgcttcttc aagcaacaac cctgtgtgtc ctgccttatt cgctattatc gtgtcaattg    2580
   gtctaaaaat ggaataaggc aaattaataa atgggcaatc aga                      2623
<210> 7
   <211> 2709
   <212> DNA
   <213> Homo sapiens
<400> 7
   taaatcgccc ccgccctcgg ctgctcttca tcgaggtccg cgggaggctc ggagcgcgcc 60
   aggcggacac tcctctcggc tcctccccgg cagcggcggc ggctcggagc gggctccggg 120
   gctcgggtgc agcggccagc gggcgcctgg cggcgaggat tacccgggga agtggttgtc 180
   tcctggctgg agccgcgaga cgggcgctca gggcgcgggg ccggcggcgg cgaacgagag 240
   gacggactct ggcggccggg tcgttggccg cggggagcgc gggcaccggg cgagcaggcc 300
   gcgtcgcgct caccatggtc agctactggg acaccggggt cctgctgtgc gcgctgctca 360
   gctgtctgct tctcacagga tctagttcag gttcaaaatt aaaagatcct gaactgagtt 420
   taaaaggcac ccagcacatc atgcaagcag gccagacact gcatctccaa tgcagggggg 480
   aagcagccca taaatggtct ttgcctgaaa tggtgagtaa ggaaagcgaa aggctgagca 540
   taactaaatc tgcctgtgga agaaatggca aacaattctg cagtacttta accttgaaca 600
   cagctcaagc aaaccacact ggcttctaca gctgcaaata tctagctgta cctacttcaa 660
   agaagaagga aacagaatct gcaatctata tatttattag tgatacaggt agacctttcg 720
   tagagatgta cagtgaaatc cccgaaatta tacacatgac tgaaggaagg gagctcgtca 780
   ttccctgccg ggttacgtca cctaacatca ctgttacttt aaaaaagttt ccacttgaca 840
   ctttgatccc tgatggaaaa cgcataatct gggacagtag aaagggcttc atcatatcaa 900
   atgcaacgta caaagaaata gggcttctga cctgtgaagc aacagtcaat gggcatttgt 960
   ataagacaaa ctatctcaca catcgacaaa ccaatacaat catagatgtc caaataagca 1020
   caccacgccc agtcaaatta cttagaggcc atactcttgt cctcaattgt actgctacca 1080
   ctcccttgaa cacgagagtt caaatgacct ggagttaccc tgatgaaaaa aataagagag 1140
   cttccgtaag gcgacgaatt gaccaaagca attcccatgc caacatattc tacagtgttc 1200
   ttactattga caaaatgcag aacaaagaca aaggacttta tacttgtcgt gtaaggagtg 1260
   gaccatcatt caaatctgtt aacacctcag tgcatatata tgataaagca ttcatcactg 1320
   tgaaacatcg aaaacagcag gtgcttgaaa ccgtagctgg caagcggtct taccggctct 1380
   ctatgaaagt gaaggcattt ccctcgccgg aagttgtatg gttaaaagat gggttacctg 1440
   cgactgagaa atctgctcgc tatttgactc gtggctactc gttaattatc aaggacgtaa 1500
   ctgaagagga tgcagggaat tatacaatct tgctgagcat aaaacagtca aatgtgttta 1560
   aaaacctcac tgccactcta attgtcaatg tgaaacccca gatttacgaa aaggccgtgt   1620
   catcgtttcc agacccggct ctctacccac tgggcagcag acaaatcctg acttgtaccg   1680
   catatggtat ccctcaacct acaatcaagt ggttctggca cccctgtaac cataatcatt   1740
   ccgaagcaag gtgtgacttt tgttccaata atgaagagtc ctttatcctg gatgctgaca   1800
   gcaacatggg aaacagaatt gagagcatca ctcagcgcat ggcaataata gaaggaaaga   1860
   ataagatggc tagcaccttg gttgtggctg actctagaat ttctggaatc tacatttgca   1920
   tagcttccaa taaagttggg actgtgggaa gaaacataag cttttatatc acagatgtgc   1980
   caaatgggtt tcatgttaac ttggaaaaaa tgccgacgga aggagaggac ctgaaactgt   2040
   cttgcacagt taacaagttc ttatacagag acgttacttg gattttactg cggacagtta   2100
   ataacagaac aatgcactac agtattagca agcaaaaaat ggccatcact aaggagcact   2160
   ccatcactct taatcttacc atcatgaatg tttccctgca agattcaggc acctatgcct   2220
   gcagagccag gaatgtatac acaggggaag aaatcctcca gaagaaagaa attacaatca   2280
   gagatcagga agcaccatac ctcctgcgaa acctcagtga tcacacagtg gccatcagca   2340
   gttccaccac tttagactgt catgctaatg gtgtccccga gcctcagatc acttggttta   2400
   aaaacaacca caaaatacaa caagagcctg gtaagagact tttttttctt ccatttatta   2460
   tttctcatct ttcatcagcc cccctttccc ttaacagtcc cgtcacctgc ttccagtatg   2520
   tatagaccta cttgctgata gagccccaaa cactgggatc tggttcatgc tgatagcaat   2580
   gtccagtgag gaatatttgg gccagttcct atgcagacca tattcctaac ccatagaaag   2640
   atcctgcaga atctattcag ctggaagtct ttaaatagtg cattcagaaa gaaatgagaa   2700
   gagccatgt                                                           2709
<210> 8
   <211> 2819
   <212> DNA
   <213> Homo sapiens
<400> 8
   taaatcgccc ccgccctcgg ctgctcttca tcgaggtccg cgggaggctc ggagcgcgcc     60
   aggcggacac tcctctcggc tcctccccgg cagcggcggc ggctcggagc gggctccggg    120
   gctcgggtgc agcggccagc gggcgcctgg cggcgaggat tacccgggga agtggttgtc    180
   tcctggctgg agccgcgaga cgggcgctca gggcgcgggg ccggcggcgg cgaacgagag    240
   gacggactct ggcggccggg tcgttggccg cggggagcgc gggcaccggg cgagcaggcc    300
   gcgtcgcgct caccatggtc agctactggg acaccggggt cctgctgtgc gcgctgctca    360
   gctgtctgct tctcacagga tctagttcag gttcaaaatt aaaagatcct gaactgagtt    420
   taaaaggcac ccagcacatc atgcaagcag gccagacact gcatctccaa tgcagggggg    480
   aagcagccca taaatggtct ttgcctgaaa tggtgagtaa ggaaagcgaa aggctgagca 540
   taactaaatc tgcctgtgga agaaatggca aacaattctg cagtacttta accttgaaca 600
   cagctcaagc aaaccacact ggcttctaca gctgcaaata tctagctgta cctacttcaa 660
   agaagaagga aacagaatct gcaatctata tatttattag tgatacaggt agacctttcg 720
   tagagatgta cagtgaaatc cccgaaatta tacacatgac tgaaggaagg gagctcgtca 780
   ttccctgccg ggttacgtca cctaacatca ctgttacttt aaaaaagttt ccacttgaca 840
   ctttgatccc tgatggaaaa cgcataatct gggacagtag aaagggcttc atcatatcaa 900
   atgcaacgta caaagaaata gggcttctga cctgtgaagc aacagtcaat gggcatttgt 960
   ataagacaaa ctatctcaca catcgacaaa ccaatacaat catagatgtc caaataagca 1020
   caccacgccc agtcaaatta cttagaggcc atactcttgt cctcaattgt actgctacca 1080
   ctcccttgaa cacgagagtt caaatgacct ggagttaccc tgatgaaaaa aataagagag 1140
   cttccgtaag gcgacgaatt gaccaaagca attcccatgc caacatattc tacagtgttc 1200
   ttactattga caaaatgcag aacaaagaca aaggacttta tacttgtcgt gtaaggagtg 1260
   gaccatcatt caaatctgtt aacacctcag tgcatatata tgataaagca ttcatcactg 1320
   tgaaacatcg aaaacagcag gtgcttgaaa ccgtagctgg caagcggtct taccggctct 1380
   ctatgaaagt gaaggcattt ccctcgccgg aagttgtatg gttaaaagat gggttacctg 1440
   cgactgagaa atctgctcgc tatttgactc gtggctactc gttaattatc aaggacgtaa 1500
   ctgaagagga tgcagggaat tatacaatct tgctgagcat aaaacagtca aatgtgttta 1560
   aaaacctcac tgccactcta attgtcaatg tgaaacccca gatttacgaa aaggccgtgt 1620
   catcgtttcc agacccggct ctctacccac tgggcagcag acaaatcctg acttgtaccg 1680
   catatggtat ccctcaacct acaatcaagt ggttctggca cccctgtaac cataatcatt 1740
   ccgaagcaag gtgtgacttt tgttccaata atgaagagtc ctttatcctg gatgctgaca 1800
   gcaacatggg aaacagaatt gagagcatca ctcagcgcat ggcaataata gaaggaaaga 1860
   ataagtattt ggatataagg actgaggaac agattttttc ctttattcag aagactcaga 1920
   cattgaaact cactgtctca tgcaaagctg ccttctagtc cagccagact acactgatct 1980
   acaatttgca cctgagctcc tttctctggc cctataaagt ttaaatttta attgtcaaat 2040
   aattttattt ttaatgagaa aatgtttgaa tttataattt aaaaatatat cccctgaaag 2100
   agaagtactc tagaaatgag ataaatttta ataaaactga tcataggata tgtttcacct 2160
   tatttttaac ttttcttctt tagtgataac cattgtattt atcaaatata ttttcaaacc 2220
   acataacaac agttgaagag cagcttgcag cttatgtaaa atgtcatcag agaagccaac 2280
   tagtatttct gtaaatagta gtagtattta atttatgtta cccatctctg cggattaaag 2340
   gaagtattct caaggaaaaa ttcaattaaa ggtgtagaca gaattttgcc aatagatctg 2400
   tgttattcaa cttaaagagt ctcaaggcga gaagcaatct ggtgtagaga ttaggagcat 2460
   aggcacggag gcagacacac ctgagcataa attctgccca ggcctgtctg tggtcctttg 2520
   catctttggg tgaatcatct aatctctcgg aacctcagtt gtcctacctg tagaataggg 2580
   taatataccc atctcacagg gttttgggga ggattaaatg agacaattcc tgtaaagtgc 2640
   cttgcatgct gcctggtgca ttgtaaggca tacataaagg ttcgcagaaa caaagaaata 2700
   atgtaaactc ccatcaacgt tgttttctgc ttctatttta atagaagctt tggagacatg 2760
   atttctgggg caggcttgac tacgagtaat gtgtcttgtt tggaaggtgt cattcacac 2819
<210> 9
   <211> 1731
   <212> DNA
   <213> Homo sapiens
<400> 9
   taaatcgccc ccgccctcgg ctgctcttca tcgaggtccg cgggaggctc ggagcgcgcc      60
   aggcggacac tcctctcggc tcctccccgg cagcggcggc ggctcggagc gggctccggg     120
   gctcgggtgc agcggccagc gggcgcctgg cggcgaggat tacccgggga agtggttgtc     180
   tcctggctgg agccgcgaga cgggcgctca gggcgcgggg ccggcggcgg cgaacgagag     240
   gacggactct ggcggccggg tcgttggccg cggggagcgc gggcaccggg cgagcaggcc     300
   gcgtcgcgct caccatggtc agctactggg acaccggggt cctgctgtgc gcgctgctca     360
   gctgtctgct tctcacagga tctagttcag gttcaaaatt aaaagatcct gaactgagtt     420
   taaaaggcac ccagcacatc atgcaagcag gccagacact gcatctccaa tgcagggggg     480
   aagcagccca taaatggtct ttgcctgaaa tggtgagtaa ggaaagcgaa aggctgagca     540
   taactaaatc tgcctgtgga agaaatggca aacaattctg cagtacttta accttgaaca     600
   cagctcaagc aaaccacact ggcttctaca gctgcaaata tctagctgta cctacttcaa     660
   agaagaagga aacagaatct gcaatctata tatttattag tgatacaggt agacctttcg     720
   tagagatgta cagtgaaatc cccgaaatta tacacatgac tgaaggaagg gagctcgtca     780
   ttccctgccg ggttacgtca cctaacatca ctgttacttt aaaaaagttt ccacttgaca     840
   ctttgatccc tgatggaaaa cgcataatct gggacagtag aaagggcttc atcatatcaa     900
   atgcaacgta caaagaaata gggcttctga cctgtgaagc aacagtcaat gggcatttgt     960
   ataagacaaa ctatctcaca catcgacaaa ccaatacaat catagatgtc caaataagca    1020
   caccacgccc agtcaaatta cttagaggcc atactcttgt cctcaattgt actgctacca    1080
   ctcccttgaa cacgagagtt caaatgacct ggagttaccc tgatgaaaaa aataagagag    1140
   cttccgtaag gcgacgaatt gaccaaagca attcccatgc caacatattc tacagtgttc    1200
   ttactattga caaaatgcag aacaaagaca aaggacttta tacttgtcgt gtaaggagtg    1260
   gaccatcatt caaatctgtt aacacctcag tgcatatata tggtaagcat agttcagcgt    1320
   tgccaactca tgctatgctt tctaatcatt gtagatgtct gtgttctcta aataagtcag    1380
   ttttctgttg gccaagagtt acattatcat gaatgaatac agcttggcca tggttccctc    1440
   ctccccctgt caaaaagcaa accacattaa gtactcttta catttaatac ttttgaattg    1500
   aacagagagt cctttcttta gatgcttatt atggctaata attcggttag aacctaatat    1560
   atttatgaaa ttcttagtgt tctgtataaa caaaacagaa aaagatgatg agttttatct    1620
   ttattatcta agaaaaattt ctcaggtact accatgaatt agagaaataa aaatatggta    1680
   tgctaaatca tttagaatta atgctatttt attcctgctt ttactctcaa g             1731
<210> 10
   <211> 4262
   <212> DNA
   <213> Homo sapiens
<400> 10
   ttgtgggctg catgcccagc ccaggcatgc agtggaaacg cagggcagga gagaggaggc      60
   cttcagagtg cagctggact tccttcccaa ccctcttgct tccttcagac cggggtcggg     120
   ttggcaaacc aagggcctct gatggtgatt gttgaatact gcaaatatgg aaatctctcc     180
   aactacctca agagcaaacg tgacttattt tttctcaaca aggatgcagc actacacatg     240
   gagcctaaga aagaaaaaat ggagccaggc ctggaacaag gcaagaaacc aagactagat     300
   agcgtcacca gcagcgaaag ctttgcgagc tccggctttc aggaagataa aagtctgagt     360
   gatgttgagg aagaggagga ttctgacggt ttctacaagg agcccatcac tatggaagat     420
   ctgatttctt acagttttca agtggccaga ggcatggagt tcctgtcttc cagaaagtgc     480
   attcatcggg acctggcagc gagaaacatt cttttatctg agaacaacgt ggtgaagatt     540
   tgtgattttg gccttgcccg ggatatttat aagaaccccg attatgtgag aaaaggagat     600
   actcgacttc ctctgaaatg gatggctcct gaatctatct ttgacaaaat ctacagcacc     660
   aagagcgacg tgtggtctta cggagtattg ctgtgggaaa tcttctcctt aggtgggtct     720
   ccatacccag gagtacaaat ggatgaggac ttttgcagtc gcctgaggga aggcatgagg     780
   atgagagctc ctgagtactc tactcctgaa atctatcaga tcatgctgga ctgctggcac     840
   agagacccaa aagaaaggcc aagatttgca gaacttgtgg aaaaactagg tgatttgctt     900
   caagcaaatg tacaacagga tggtaaagac tacatcccaa tcaatgccat actgacagga     960
   aatagtgggt ttacatactc aactcctgcc ttctctgagg acttcttcaa ggaaagtatt    1020
   tcagctccga agtttaattc aggaagctct gatgatgtca gatatgtaaa tgctttcaag    1080
   ttcatgagcc tggaaagaat caaaaccttt gaagaacttt taccgaatgc cacctccatg    1140
   tttgatgact accagggcga cagcagcact ctgttggcct ctcccatgct gaagcgcttc    1200
   acctggactg acagcaaacc caaggcctcg ctcaagattg acttgagagt aaccagtaaa    1260
   agtaaggagt cggggctgtc tgatgtcagc aggcccagtt tctgccattc cagctgtggg    1320
   cacgtcagcg aaggcaagcg caggttcacc tacgaccacg ctgagctgga aaggaaaatc 1380
   gcgtgctgct ccccgccccc agactacaac tcggtggtcc tgtactccac cccacccatc 1440
   tagagtttga cacgaagcct tatttctaga agcacatgtg tatttatacc cccaggaaac 1500
   tagcttttgc cagtattatg catatataag tttacacctt tatctttcca tgggagccag 1560
   ctgctttttg tgattttttt aatagtgctt tttttttttt gactaacaag aatgtaactc 1620
   cagatagaga aatagtgaca agtgaagaac actactgcta aatcctcatg ttactcagtg 1680
   ttagagaaat ccttcctaaa cccaatgact tccctgctcc aacccccgcc acctcagggc 1740
   acgcaggacc agtttgattg aggagctgca ctgatcaccc aatgcatcac gtaccccact 1800
   gggccagccc tgcagcccaa aacccagggc aacaagcccg ttagccccag ggatcactgg 1860
   ctggcctgag caacatctcg ggagtcctct agcaggccta agacatgtga ggaggaaaag 1920
   gaaaaaaagc aaaaagcaag ggagaaaaga gaaaccggga gaaggcatga gaaagaattt 1980
   gagacgcacc atgtgggcac ggagggggac ggggctcagc aatgccattt cagtggcttc 2040
   ccagctctga cccttctaca tttgagggcc cagccaggag cagatggaca gcgatgaggg 2100
   gacattttct ggattctggg aggcaagaaa aggacaaata tcttttttgg aactaaagca 2160
   aattttagaa ctttacctat ggaagtggtt ctatgtccat tctcattcgt ggcatgtttt 2220
   gatttgtagc actgagggtg gcactcaact ctgagcccat acttttggct cctctagtaa 2280
   gatgcactga aaacttagcc agagttaggt tgtctccagg ccatgatggc cttacactga 2340
   aaatgtcaca ttctattttg ggtattaata tatagtccag acacttaact caatttcttg 2400
   gtattattct gttttgcaca gttagttgtg aaagaaagct gagaagaatg aaaatgcagt 2460
   cctgaggaga ggagttttct ccatatcaaa acgagggctg atggaggaaa aaggtcaata 2520
   aggtcaaggg aaaaccccgt ctctatacca accaaaccaa ttcaccaaca cagttgggac 2580
   ccaaaacaca ggaagtcagt cacgtttcct tttcatttaa tggggattcc actatctcac 2640
   actaatctga aaggatgtgg aagagcatta gctggcgcat attaagcact ttaagctcct 2700
   tgagtaaaaa ggtggtatgt aatttatgca aggtatttct ccagttggga ctcaggatat 2760
   tagttaatga gccatcacta gaagaaaagc ccattttcaa ctgctttgaa acttgcctgg 2820
   ggtctgagca tgatgggaat agggagacag ggtaggaaag ggcgcctact cttcagggtc 2880
   taaagatcaa gtgggccttg gatcgctaag ctggctctgt ttgatgctat ttatgcaagt 2940
   tagggtctat gtatttatga tgtctgcacc ttctgcagcc agtcagaagc tggagaggca 3000
   acagtggatt gctgcttctt ggggagaaga gtatgcttcc ttttatccat gtaatttaac 3060
   tgtagaacct gagctctaag taaccgaaga atgtatgcct ctgttcttat gtgccacatc 3120
   cttgtttaaa ggctctctgt atgaagagat gggaccgtca tcagcacatt ccctagtgag 3180
   cctactggct cctggcagcg gcttttgtgg aagactcact agccagaaga gaggagtggg 3240
   acagtcctct ccaccaagat ctaaatccaa acaaaagcag gctagagcca gaagagagga 3300
   caaatctttg ttcttcctct tctttacata cgcaaaccac ctgtgacagc tggcaatttt 3360
   ataaatcagg taactggaag gaggttaaac acagaaaaaa gaagacctca gtcaattctc 3420
   tacttttttt tttttttcca aatcagataa tagcccagca aatagtgata acaaataaaa 3480
   ccttagctat tcatgtcttg atttcaataa ttaattctta atcattaaga gaccataata 3540
   aatactcctt ttcaagagaa aagcaaaacc attagaattg ttactcagct ccttcaaact 3600
   caggtttgta gcatacatga gtccatccat cagtcaaaga atggttccat ctggagtctt 3660
   aatgtagaaa gaaaaatgga gacttgtaat aatgagctag ttacaaagtg cttgttcatt 3720
   aaaatagcac tgaaaattga aacatgaatt aactgataat attccaatca tttgccattt 3780
   atgacaaaaa tggttggcac taacaaagaa cgagcacttc ctttcagagt ttctgagata 3840
   atgtacgtgg aacagtctgg gtggaatggg gctgaaacca tgtgcaagtc tgtgtcttgt 3900
   cagtccaaga agtgacaccg agatgttaat tttagggacc cgtgccttgt ttcctagccc 3960
   acaagaatgc aaacatcaaa cagatactcg ctagcctcat ttaaattgat taaaggagga 4020
   gtgcatcttt ggccgacagt ggtgtaactg tatgtgtgtg tgtgtgtgtg tgtgtgtgtg 4080
   tgggtgtatg tgtgttttgt gcataactat ttaaggaaac tggaatttta aagttacttt 4140
   tatacaaacc aagaatatat gctacagata taagacagac atggtttggt cctatatttc 4200
   tagtcatgat gaatgtattt tgtataccat cttcatataa taaacttcca aaaacacata 4260
   ta 4262
<210> 11
   <211> 956
   <212> DNA
   <213> Homo sapiens
<400> 11
   taaatcgccc ccgccctcgg ctgctcttca tcgaggtccg cgggaggctc ggagcgcgcc 60
   aggcggacac tcctctcggc tcctccccgg cagcggcggc ggctcggagc gggctccggg 120
   gctcgggtgc agcggccagc gggcgcctgg cggcgaggat tacccgggga agtggttgtc 180
   tcctggctgg agccgcgaga cgggcgctca gggcgcgggg ccggcggcgg cgaacgagag 240
   gacggactct ggcggccggg tcgttggccg cggggagcgc gggcaccggg cgagcaggcc 300
   gcgtcgcgct caccatggtc agctactggg acaccggggt cctgctgtgc gcgctgctca 360
   gctgtctgct tctcacagga tctagttcag gttcaaaatt aaaagatcct gaactgagtt 420
   taaaaggcac ccagcacatc atgcaagcag gccagacact gcatctccaa tgcagggggg 480
   aagcagccca taaatggtct ttgcctgaaa tggtgagtaa ggaaagcgaa aggctgagca 540
   taactaaatc tgcctgtgga agaaatggca aacaattctg cagtacttta accttgaaca 600
   cagctcaagc aaaccacact ggcttctaca gctgcaaata tctagctgta cctacttcaa 660
   agaagaagga aacagaatct gcaatctata tatttattag tgatacaggt agacctttcg 720
   tagagatgta cagtgaaatc cccgaaatta tacacatgac tgaaggaagg gagctcgtca 780
   ttccctgccg ggttacgtca cctaacatca ctgttacttt aaaaaaggta aatttgaaca 840
   ccgctattct cagcatactt tcactgcaaa taagcataat gaagttttac tcgttctatc 900
   tttctggaat aatttcctta caaacacctg ggcttctcag tggactttcc tgtaac 956
<210> 12
   <211> 3916
   <212> DNA
   <213> Homo sapiens
<400> 12
   attacaaaca gttacggttc taatgctttc cagattctga cggtttctac aaggagccca     60
   tcactatgga agatctgatt tcttacagtt ttcaagtggc cagaggcatg gagttcctgt    120
   cttccagaaa gtgcattcat cgggacctgg cagcgagaaa cattctttta tctgagaaca    180
   acgtggtgaa gatttgtgat tttggccttg cccgggatat ttataagaac cccgattatg    240
   tgagaaaagg agatactcga cttcctctga aatggatggc tcctgaatct atctttgaca    300
   aaatctacag caccaagagc gacgtgtggt cttacggagt attgctgtgg gaaatcttct    360
   ccttaggtgg gtctccatac ccaggagtac aaatggatga ggacttttgc agtcgcctga    420
   gggaaggcat gaggatgaga gctcctgagt actctactcc tgaaatctat cagatcatgc    480
   tggactgctg gcacagagac ccaaaagaaa ggccaagatt tgcagaactt gtggaaaaac    540
   taggtgattt gcttcaagca aatgtacaac aggatggtaa agactacatc ccaatcaatg    600
   ccatactgac aggaaatagt gggtttacat actcaactcc tgccttctct gaggacttct    660
   tcaaggaaag tatttcagct ccgaagttta attcaggaag ctctgatgat gtcagatatg    720
   taaatgcttt caagttcatg agcctggaaa gaatcaaaac ctttgaagaa cttttaccga    780
   atgccacctc catgtttgat gactaccagg gcgacagcag cactctgttg gcctctccca    840
   tgctgaagcg cttcacctgg actgacagca aacccaaggc ctcgctcaag attgacttga    900
   gagtaaccag taaaagtaag gagtcggggc tgtctgatgt cagcaggccc agtttctgcc    960
   attccagctg tgggcacgtc agcgaaggca agcgcaggtt cacctacgac cacgctgagc   1020
   tggaaaggaa aatcgcgtgc tgctccccgc ccccagacta caactcggtg gtcctgtact   1080
   ccaccccacc catctagagt ttgacacgaa gccttatttc tagaagcaca tgtgtattta   1140
   tacccccagg aaactagctt ttgccagtat tatgcatata taagtttaca cctttatctt   1200
   tccatgggag ccagctgctt tttgtgattt ttttaatagt gctttttttt ttttgactaa   1260
   caagaatgta actccagata gagaaatagt gacaagtgaa gaacactact gctaaatcct   1320
   catgttactc agtgttagag aaatccttcc taaacccaat gacttccctg ctccaacccc   1380
   cgccacctca gggcacgcag gaccagtttg attgaggagc tgcactgatc acccaatgca 1440
   tcacgtaccc cactgggcca gccctgcagc ccaaaaccca gggcaacaag cccgttagcc 1500
   ccagggatca ctggctggcc tgagcaacat ctcgggagtc ctctagcagg cctaagacat 1560
   gtgaggagga aaaggaaaaa aagcaaaaag caagggagaa aagagaaacc gggagaaggc 1620
   atgagaaaga atttgagacg caccatgtgg gcacggaggg ggacggggct cagcaatgcc 1680
   atttcagtgg cttcccagct ctgacccttc tacatttgag ggcccagcca ggagcagatg 1740
   gacagcgatg aggggacatt ttctggattc tgggaggcaa gaaaaggaca aatatctttt 1800
   ttggaactaa agcaaatttt agaactttac ctatggaagt ggttctatgt ccattctcat 1860
   tcgtggcatg ttttgatttg tagcactgag ggtggcactc aactctgagc ccatactttt 1920
   ggctcctcta gtaagatgca ctgaaaactt agccagagtt aggttgtctc caggccatga 1980
   tggccttaca ctgaaaatgt cacattctat tttgggtatt aatatatagt ccagacactt 2040
   aactcaattt cttggtatta ttctgttttg cacagttagt tgtgaaagaa agctgagaag 2100
   aatgaaaatg cagtcctgag gagaggagtt ttctccatat caaaacgagg gctgatggag 2160
   gaaaaaggtc aataaggtca agggaaaacc ccgtctctat accaaccaaa ccaattcacc 2220
   aacacagttg ggacccaaaa cacaggaagt cagtcacgtt tccttttcat ttaatgggga 2280
   ttccactatc tcacactaat ctgaaaggat gtggaagagc attagctggc gcatattaag 2340
   cactttaagc tccttgagta aaaaggtggt atgtaattta tgcaaggtat ttctccagtt 2400
   gggactcagg atattagtta atgagccatc actagaagaa aagcccattt tcaactgctt 2460
   tgaaacttgc ctggggtctg agcatgatgg gaatagggag acagggtagg aaagggcgcc 2520
   tactcttcag ggtctaaaga tcaagtgggc cttggatcgc taagctggct ctgtttgatg 2580
   ctatttatgc aagttagggt ctatgtattt atgatgtctg caccttctgc agccagtcag 2640
   aagctggaga ggcaacagtg gattgctgct tcttggggag aagagtatgc ttccttttat 2700
   ccatgtaatt taactgtaga acctgagctc taagtaaccg aagaatgtat gcctctgttc 2760
   ttatgtgcca catccttgtt taaaggctct ctgtatgaag agatgggacc gtcatcagca 2820
   cattccctag tgagcctact ggctcctggc agcggctttt gtggaagact cactagccag 2880
   aagagaggag tgggacagtc ctctccacca agatctaaat ccaaacaaaa gcaggctaga 2940
   gccagaagag aggacaaatc tttgttcttc ctcttcttta catacgcaaa ccacctgtga 3000
   cagctggcaa ttttataaat caggtaactg gaaggaggtt aaacacagaa aaaagaagac 3060
   ctcagtcaat tctctacttt tttttttttt tccaaatcag ataatagccc agcaaatagt 3120
   gataacaaat aaaaccttag ctattcatgt cttgatttca ataattaatt cttaatcatt 3180
   aagagaccat aataaatact ccttttcaag agaaaagcaa aaccattaga attgttactc 3240
   agctccttca aactcaggtt tgtagcatac atgagtccat ccatcagtca aagaatggtt 3300
   ccatctggag tcttaatgta gaaagaaaaa tggagacttg taataatgag ctagttacaa 3360
   agtgcttgtt cattaaaata gcactgaaaa ttgaaacatg aattaactga taatattcca    3420
   atcatttgcc atttatgaca aaaatggttg gcactaacaa agaacgagca cttcctttca    3480
   gagtttctga gataatgtac gtggaacagt ctgggtggaa tggggctgaa accatgtgca    3540
   agtctgtgtc ttgtcagtcc aagaagtgac accgagatgt taattttagg gacccgtgcc    3600
   ttgtttccta gcccacaaga atgcaaacat caaacagata ctcgctagcc tcatttaaat    3660
   tgattaaagg aggagtgcat ctttggccga cagtggtgta actgtatgtg tgtgtgtgtg    3720
   tgtgtgtgtg tgtgtgggtg tatgtgtgtt ttgtgcataa ctatttaagg aaactggaat    3780
   tttaaagtta cttttataca aaccaagaat atatgctaca gatataagac agacatggtt    3840
   tggtcctata tttctagtca tgatgaatgt attttgtata ccatcttcat ataataaact    3900
   tccaaaaaca catata                                                    3916
<210> 13
   <211> 1157
   <212> DNA
   <213> Homo sapiens
<400> 13
   taaatcgccc ccgccctcgg ctgctcttca tcgaggtccg cgggaggctc ggagcgcgcc 60
   aggcggacac tcctctcggc tcctccccgg cagcggcggc ggctcggagc gggctccggg 120
   gctcgggtgc agcggccagc gggcgcctgg cggcgaggat tacccgggga agtggttgtc 180
   tcctggctgg agccgcgaga cgggcgctca gggcgcgggg ccggcggcgg cgaacgagag 240
   gacggactct ggcggccggg tcgttggccg cggggagcgc gggcaccggg cgagcaggcc 300
   gcgtcgcgct caccatggtc agctactggg acaccggggt cctgctgtgc gcgctgctca 360
   gctgtctgct tctcacagga tctagttcag gttcaaaatt aaaagatcct gaactgagtt 420
   taaaaggcac ccagcacatc atgcaagcag gccagacact gcatctccaa tgcagggggg 480
   aagcagccca taaatggtct ttgcctgaaa tggtgagtaa ggaaagcgaa aggctgagca 540
   taactaaatc tgcctgtgga agaaatggca aacaattctg cagtacttta accttgaaca 600
   cagctcaagc aaaccacact ggcttctaca gctgcaaata tctagctgta cctacttcaa 660
   agaagaagga aacagaatct gcaatctata tatttattag tggtaagact tccattttct 720
   acattctgtt tgcttttgct ttgcaaatga gtcataaatc taccctgatc cattggaaag 780
   gttgctttcc tagtgagtat gaaagaaacg ggttaggaaa gagattccat cctagttgta 840
   gacactttcg tggatgtcaa ttttaaccag aattgtcaag gcccagttag tcactatgtg 900
   ctccacaaag agagagactg gtctgtatag gtcacctctg tacccacagc tcctagcaca 960
   gtatgtggcc catagttgga tgaataagtg aataaaagga ttttagttct atttcttagt 1020
   tattgagaag cccaaaatag aatacatgat ttttgtcatt taacacagct gcattctctt 1080
   atatgttgga actgtaaaat actgttgttt ctactaaatt aataaacgca atgttgctgt 1140
   tatctcgagt gttatat 1157
<210> 14
   <211> 4132
   <212> DNA
   <213> Homo sapiens
<400> 14
   tgatcagcta cacacaggaa ccttgctcac agccccggaa actccacact gtgggcttca      60
   ccttctggaa gtgacaaagt catgtacgca ggaccttccc caccagtctc cactgctgag     120
   cccataaagg gaggtgtgag gacttggaaa tgccacatgc tttttaagaa gccagctcaa     180
   accaaccttt gttacaactc gagaatgaaa taagtactac aaactattca gccattgata     240
   tttatggctg tatgactttt ttttttttta actttaagga catcgtccta cttctgtaag     300
   tgtggggagg agtactccac ttcacattga actgcagtga gcggtggggt ttcctttccg     360
   aattgctgtg tttcattcac agaactcaga ccttccatct gcagaggcca caggcacaac     420
   aaagaacctg ggtatccatg agctctggtg ggttggttag tctgccttgg tagacgtgtt     480
   ttccactgac cacaggacct ggcccagaca gccttttaag tgctggtgct ataaacccaa     540
   acctaaaaat gaagcagggt cacatagtac agaaagcttg ggctttatgc ggatgatgac     600
   agccctccct ttgtagtacg taaggcaatg cataggatga tcactgctct ccaactattt     660
   ctgttgctgt tttccccacc agctatcaga tcatgctgga ctgctggcac agagacccaa     720
   aagaaaggcc aagatttgca gaacttgtgg aaaaactagg tgatttgctt caagcaaatg     780
   tacaacagga tggtaaagac tacatcccaa tcaatgccat actgacagga aatagtgggt     840
   ttacatactc aactcctgcc ttctctgagg acttcttcaa ggaaagtatt tcagctccga     900
   agtttaattc aggaagctct gatgatgtca gatatgtaaa tgctttcaag ttcatgagcc     960
   tggaaagaat caaaaccttt gaagaacttt taccgaatgc cacctccatg tttgatgact    1020
   accagggcga cagcagcact ctgttggcct ctcccatgct gaagcgcttc acctggactg    1080
   acagcaaacc caaggcctcg ctcaagattg acttgagagt aaccagtaaa agtaaggagt    1140
   cggggctgtc tgatgtcagc aggcccagtt tctgccattc cagctgtggg cacgtcagcg    1200
   aaggcaagcg caggttcacc tacgaccacg ctgagctgga aaggaaaatc gcgtgctgct    1260
   ccccgccccc agactacaac tcggtggtcc tgtactccac cccacccatc tagagtttga    1320
   cacgaagcct tatttctaga agcacatgtg tatttatacc cccaggaaac tagcttttgc    1380
   cagtattatg catatataag tttacacctt tatctttcca tgggagccag ctgctttttg    1440
   tgattttttt aatagtgctt tttttttttt gactaacaag aatgtaactc cagatagaga    1500
   aatagtgaca agtgaagaac actactgcta aatcctcatg ttactcagtg ttagagaaat    1560
   ccttcctaaa cccaatgact tccctgctcc aacccccgcc acctcagggc acgcaggacc    1620
   agtttgattg aggagctgca ctgatcaccc aatgcatcac gtaccccact gggccagccc 1680
   tgcagcccaa aacccagggc aacaagcccg ttagccccag ggatcactgg ctggcctgag 1740
   caacatctcg ggagtcctct agcaggccta agacatgtga ggaggaaaag gaaaaaaagc 1800
   aaaaagcaag ggagaaaaga gaaaccggga gaaggcatga gaaagaattt gagacgcacc 1860
   atgtgggcac ggagggggac ggggctcagc aatgccattt cagtggcttc ccagctctga 1920
   cccttctaca tttgagggcc cagccaggag cagatggaca gcgatgaggg gacattttct 1980
   ggattctggg aggcaagaaa aggacaaata tcttttttgg aactaaagca aattttagaa 2040
   ctttacctat ggaagtggtt ctatgtccat tctcattcgt ggcatgtttt gatttgtagc 2100
   actgagggtg gcactcaact ctgagcccat acttttggct cctctagtaa gatgcactga 2160
   aaacttagcc agagttaggt tgtctccagg ccatgatggc cttacactga aaatgtcaca 2220
   ttctattttg ggtattaata tatagtccag acacttaact caatttcttg gtattattct 2280
   gttttgcaca gttagttgtg aaagaaagct gagaagaatg aaaatgcagt cctgaggaga 2340
   ggagttttct ccatatcaaa acgagggctg atggaggaaa aaggtcaata aggtcaaggg 2400
   aaaaccccgt ctctatacca accaaaccaa ttcaccaaca cagttgggac ccaaaacaca 2460
   ggaagtcagt cacgtttcct tttcatttaa tggggattcc actatctcac actaatctga 2520
   aaggatgtgg aagagcatta gctggcgcat attaagcact ttaagctcct tgagtaaaaa 2580
   ggtggtatgt aatttatgca aggtatttct ccagttggga ctcaggatat tagttaatga 2640
   gccatcacta gaagaaaagc ccattttcaa ctgctttgaa acttgcctgg ggtctgagca 2700
   tgatgggaat agggagacag ggtaggaaag ggcgcctact cttcagggtc taaagatcaa 2760
   gtgggccttg gatcgctaag ctggctctgt ttgatgctat ttatgcaagt tagggtctat 2820
   gtatttatga tgtctgcacc ttctgcagcc agtcagaagc tggagaggca acagtggatt 2880
   gctgcttctt ggggagaaga gtatgcttcc ttttatccat gtaatttaac tgtagaacct 2940
   gagctctaag taaccgaaga atgtatgcct ctgttcttat gtgccacatc cttgtttaaa 3000
   ggctctctgt atgaagagat gggaccgtca tcagcacatt ccctagtgag cctactggct 3060
   cctggcagcg gcttttgtgg aagactcact agccagaaga gaggagtggg acagtcctct 3120
   ccaccaagat ctaaatccaa acaaaagcag gctagagcca gaagagagga caaatctttg 3180
   ttcttcctct tctttacata cgcaaaccac ctgtgacagc tggcaatttt ataaatcagg 3240
   taactggaag gaggttaaac acagaaaaaa gaagacctca gtcaattctc tacttttttt 3300
   tttttttcca aatcagataa tagcccagca aatagtgata acaaataaaa ccttagctat 3360
   tcatgtcttg atttcaataa ttaattctta atcattaaga gaccataata aatactcctt 3420
   ttcaagagaa aagcaaaacc attagaattg ttactcagct ccttcaaact caggtttgta 3480
   gcatacatga gtccatccat cagtcaaaga atggttccat ctggagtctt aatgtagaaa 3540
   gaaaaatgga gacttgtaat aatgagctag ttacaaagtg cttgttcatt aaaatagcac   3600
   tgaaaattga aacatgaatt aactgataat attccaatca tttgccattt atgacaaaaa   3660
   tggttggcac taacaaagaa cgagcacttc ctttcagagt ttctgagata atgtacgtgg   3720
   aacagtctgg gtggaatggg gctgaaacca tgtgcaagtc tgtgtcttgt cagtccaaga   3780
   agtgacaccg agatgttaat tttagggacc cgtgccttgt ttcctagccc acaagaatgc   3840
   aaacatcaaa cagatactcg ctagcctcat ttaaattgat taaaggagga gtgcatcttt   3900
   ggccgacagt ggtgtaactg tatgtgtgtg tgtgtgtgtg tgtgtgtgtg tgggtgtatg   3960
   tgtgttttgt gcataactat ttaaggaaac tggaatttta aagttacttt tatacaaacc   4020
   aagaatatat gctacagata taagacagac atggtttggt cctatatttc tagtcatgat   4080
   gaatgtattt tgtataccat cttcatataa taaacttcca aaaacacata ta           4132
<210> 15
   <211> 3443
   <212> DNA
   <213> Homo sapiens
<400> 15
   tgtaatcaca cacacacaca aaatacaagc ctggcgctag cactcgatta tgccattaaa 60
   taatatttag ccgtgtagcc atgccaggtc actttgccac ctcacatcct tttcagagca 120
   cctgataaag tcataccact tccctgcaca tcatttctct cctgtgccat tgggcactca 180
   gacgagatga tgcctccagt ctctcctatg tctggcattc tctgatttca caacggacca 240
   gaatatgtaa atgctttcaa gttcatgagc ctggaaagaa tcaaaacctt tgaagaactt 300
   ttaccgaatg ccacctccat gtttgatgac taccagggcg acagcagcac tctgttggcc 360
   tctcccatgc tgaagcgctt cacctggact gacagcaaac ccaaggcctc gctcaagatt 420
   gacttgagag taaccagtaa aagtaaggag tcggggctgt ctgatgtcag caggcccagt 480
   ttctgccatt ccagctgtgg gcacgtcagc gaaggcaagc gcaggttcac ctacgaccac 540
   gctgagctgg aaaggaaaat cgcgtgctgc tccccgcccc cagactacaa ctcggtggtc 600
   ctgtactcca ccccacccat ctagagtttg acacgaagcc ttatttctag aagcacatgt 660
   gtatttatac ccccaggaaa ctagcttttg ccagtattat gcatatataa gtttacacct 720
   ttatctttcc atgggagcca gctgcttttt gtgatttttt taatagtgct tttttttttt 780
   tgactaacaa gaatgtaact ccagatagag aaatagtgac aagtgaagaa cactactgct 840
   aaatcctcat gttactcagt gttagagaaa tccttcctaa acccaatgac ttccctgctc 900
   caacccccgc cacctcaggg cacgcaggac cagtttgatt gaggagctgc actgatcacc 960
   caatgcatca cgtaccccac tgggccagcc ctgcagccca aaacccaggg caacaagccc 1020
   gttagcccca gggatcactg gctggcctga gcaacatctc gggagtcctc tagcaggcct 1080
   aagacatgtg aggaggaaaa ggaaaaaaag caaaaagcaa gggagaaaag agaaaccggg 1140
   agaaggcatg agaaagaatt tgagacgcac catgtgggca cggaggggga cggggctcag 1200
   caatgccatt tcagtggctt cccagctctg acccttctac atttgagggc ccagccagga 1260
   gcagatggac agcgatgagg ggacattttc tggattctgg gaggcaagaa aaggacaaat 1320
   atcttttttg gaactaaagc aaattttaga actttaccta tggaagtggt tctatgtcca 1380
   ttctcattcg tggcatgttt tgatttgtag cactgagggt ggcactcaac tctgagccca 1440
   tacttttggc tcctctagta agatgcactg aaaacttagc cagagttagg ttgtctccag 1500
   gccatgatgg ccttacactg aaaatgtcac attctatttt gggtattaat atatagtcca 1560
   gacacttaac tcaatttctt ggtattattc tgttttgcac agttagttgt gaaagaaagc 1620
   tgagaagaat gaaaatgcag tcctgaggag aggagttttc tccatatcaa aacgagggct 1680
   gatggaggaa aaaggtcaat aaggtcaagg gaaaaccccg tctctatacc aaccaaacca 1740
   attcaccaac acagttggga cccaaaacac aggaagtcag tcacgtttcc ttttcattta 1800
   atggggattc cactatctca cactaatctg aaaggatgtg gaagagcatt agctggcgca 1860
   tattaagcac tttaagctcc ttgagtaaaa aggtggtatg taatttatgc aaggtatttc 1920
   tccagttggg actcaggata ttagttaatg agccatcact agaagaaaag cccattttca 1980
   actgctttga aacttgcctg gggtctgagc atgatgggaa tagggagaca gggtaggaaa 2040
   gggcgcctac tcttcagggt ctaaagatca agtgggcctt ggatcgctaa gctggctctg 2100
   tttgatgcta tttatgcaag ttagggtcta tgtatttatg atgtctgcac cttctgcagc 2160
   cagtcagaag ctggagaggc aacagtggat tgctgcttct tggggagaag agtatgcttc 2220
   cttttatcca tgtaatttaa ctgtagaacc tgagctctaa gtaaccgaag aatgtatgcc 2280
   tctgttctta tgtgccacat ccttgtttaa aggctctctg tatgaagaga tgggaccgtc 2340
   atcagcacat tccctagtga gcctactggc tcctggcagc ggcttttgtg gaagactcac 2400
   tagccagaag agaggagtgg gacagtcctc tccaccaaga tctaaatcca aacaaaagca 2460
   ggctagagcc agaagagagg acaaatcttt gttcttcctc ttctttacat acgcaaacca 2520
   cctgtgacag ctggcaattt tataaatcag gtaactggaa ggaggttaaa cacagaaaaa 2580
   agaagacctc agtcaattct ctactttttt ttttttttcc aaatcagata atagcccagc 2640
   aaatagtgat aacaaataaa accttagcta ttcatgtctt gatttcaata attaattctt 2700
   aatcattaag agaccataat aaatactcct tttcaagaga aaagcaaaac cattagaatt 2760
   gttactcagc tccttcaaac tcaggtttgt agcatacatg agtccatcca tcagtcaaag 2820
   aatggttcca tctggagtct taatgtagaa agaaaaatgg agacttgtaa taatgagcta 2880
   gttacaaagt gcttgttcat taaaatagca ctgaaaattg aaacatgaat taactgataa 2940
   tattccaatc atttgccatt tatgacaaaa atggttggca ctaacaaaga acgagcactt 3000
   cctttcagag tttctgagat aatgtacgtg gaacagtctg ggtggaatgg ggctgaaacc 3060
   atgtgcaagt ctgtgtcttg tcagtccaag aagtgacacc gagatgttaa ttttagggac   3120
   ccgtgccttg tttcctagcc cacaagaatg caaacatcaa acagatactc gctagcctca   3180
   tttaaattga ttaaaggagg agtgcatctt tggccgacag tggtgtaact gtatgtgtgt   3240
   gtgtgtgtgt gtgtgtgtgt gtgggtgtat gtgtgttttg tgcataacta tttaaggaaa   3300
   ctggaatttt aaagttactt ttatacaaac caagaatata tgctacagat ataagacaga   3360
   catggtttgg tcctatattt ctagtcatga tgaatgtatt ttgtatacca tcttcatata   3420
   ataaacttcc aaaaacacat ata                                           3443
<210> 16
   <211> 1171
   <212> PRT
   <213> Homo sapiens
<400> 16
   Met Thr Ala Pro Phe Pro Leu Asp Thr Leu Ile Pro Asp Gly Lys Arg
   1               5                   10                  15
   Ile Ile Trp Asp Ser Arg Lys Gly Phe Ile Ile Ser Asn Ala Thr Tyr
               20                  25                  30
   Lys Glu Ile Gly Leu Leu Thr Cys Glu Ala Thr Val Asn Gly His Leu
           35                  40                  45
   Tyr Lys Thr Asn Tyr Leu Thr His Arg Gln Thr Asn Thr Ile Ile Asp
       50                  55                  60
   Val Gln Ile Ser Thr Pro Arg Pro Val Lys Leu Leu Arg Gly His Thr
   65                  70                  75                  80
   Leu Val Leu Asn Cys Thr Ala Thr Thr Pro Leu Asn Thr Arg Val Gln
                   85                  90                  95
   Met Thr Trp Ser Tyr Pro Asp Glu Lys Asn Lys Arg Ala Ser Val Arg
               100                 105                 110
   Arg Arg Ile Asp Gln Ser Asn Ser His Ala Asn Ile Phe Tyr Ser Val
           115                 120                 125
   Leu Thr Ile Asp Lys Met Gln Asn Lys Asp Lys Gly Leu Tyr Thr Cys
       130                 135                 140
   Arg Val Arg Ser Gly Pro Ser Phe Lys Ser Val Asn Thr Ser Val His
   145                 150                 155                 160
   Ile Tyr Asp Lys Ala Phe Ile Thr Val Lys His Arg Lys Gln Gln Val
                   165                 170                 175
   Leu Glu Thr Val Ala Gly Lys Arg Ser Tyr Arg Leu Ser Met Lys Val
   180 185 190
   Lys Ala Phe Pro Ser Pro Glu Val Val Trp Leu Lys Asp Gly Leu Pro
   195 200 205
   Ala Thr Glu Lys Ser Ala Arg Tyr Leu Thr Arg Gly Tyr Ser Leu Ile
   210 215 220
   Ile Lys Asp Val Thr Glu Glu Asp Ala Gly Asn Tyr Thr Ile Leu Leu
   225 230 235 240
   Ser Ile Lys Gln Ser Asn Val Phe Lys Asn Leu Thr Ala Thr Leu Ile
   245 250 255
   Val Asn Val Lys Pro Gln Ile Tyr Glu Lys Ala Val Ser Ser Phe Pro
   260 265 270
   Asp Pro Ala Leu Tyr Pro Leu Gly Ser Arg Gln Ile Leu Thr Cys Thr
   275 280 285
   Ala Tyr Gly Ile Pro Gln Pro Thr Ile Lys Trp Phe Trp His Pro Cys
   290 295 300
   Asn His Asn His Ser Glu Ala Arg Cys Asp Phe Cys Ser Asn Asn Glu
   305 310 315 320
   Glu Ser Phe Ile Leu Asp Ala Asp Ser Asn Met Gly Asn Arg Ile Glu
   325 330 335
   Ser Ile Thr Gln Arg Met Ala Ile Ile Glu Gly Lys Asn Lys Met Ala
   340 345 350
   Ser Thr Leu Val Val Ala Asp Ser Arg Ile Ser Gly Ile Tyr Ile Cys
   355 360 365
   Ile Ala Ser Asn Lys Val Gly Thr Val Gly Arg Asn Ile Ser Phe Tyr
   370 375 380
   Ile Thr Asp Val Pro Asn Gly Phe His Val Asn Leu Glu Lys Met Pro
   385 390 395 400
   Thr Glu Gly Glu Asp Leu Lys Leu Ser Cys Thr Val Asn Lys Phe Leu
   405 410 415
   Tyr Arg Asp Val Thr Trp Ile Leu Leu Arg Thr Val Asn Asn Arg Thr
   420 425 430
   Met His Tyr Ser Ile Ser Lys Gln Lys Met Ala Ile Thr Lys Glu His
   435 440 445
   Ser Ile Thr Leu Asn Leu Thr Ile Met Asn Val Ser Leu Gln Asp Ser
   450 455 460
   Gly Thr Tyr Ala Cys Arg Ala Arg Asn Val Tyr Thr Gly Glu Glu Ile
   465 470 475 480
   Leu Gln Lys Lys Glu Ile Thr Ile Arg Asp Gln Glu Ala Pro Tyr Leu
   485 490 495
   Leu Arg Asn Leu Ser Asp His Thr Val Ala Ile Ser Ser Ser Thr Thr
   500 505 510
   Leu Asp Cys His Ala Asn Gly Val Pro Glu Pro Gln Ile Thr Trp Phe
   515 520 525
   Lys Asn Asn His Lys Ile Gln Gln Glu Pro Gly Ile Ile Leu Gly Pro
   530 535 540
   Gly Ser Ser Thr Leu Phe Ile Glu Arg Val Thr Glu Glu Asp Glu Gly
   545 550 555 560
   Val Tyr His Cys Lys Ala Thr Asn Gln Lys Gly Ser Val Glu Ser Ser
   565 570 575
   Ala Tyr Leu Thr Val Gln Gly Thr Ser Asp Lys Ser Asn Leu Glu Leu
   580 585 590
   Ile Thr Leu Thr Cys Thr Cys Val Ala Ala Thr Leu Phe Trp Leu Leu
   595 600 605
   Leu Thr Leu Phe Ile Arg Lys Met Lys Arg Ser Ser Ser Glu Ile Lys
   610 615 620
   Thr Asp Tyr Leu Ser Ile Ile Met Asp Pro Asp Glu Val Pro Leu Asp
   625 630 635 640
   Glu Gln Cys Glu Arg Leu Pro Tyr Asp Ala Ser Lys Trp Glu Phe Ala
   645 650 655
   Arg Glu Arg Leu Lys Leu Gly Lys Ser Leu Gly Arg Gly Ala Phe Gly
   660 665 670
   Lys Val Val Gln Ala Ser Ala Phe Gly Ile Lys Lys Ser Pro Thr Cys
   675 680 685
   Arg Thr Val Ala Val Lys Met Leu Lys Glu Gly Ala Thr Ala Ser Glu
   690 695 700
   Tyr Lys Ala Leu Met Thr Glu Leu Lys Ile Leu Thr His Ile Gly His
   705 710 715 720
   His Leu Asn Val Val Asn Leu Leu Gly Ala Cys Thr Lys Gln Gly Gly
   725 730 735
   Pro Leu Met Val Ile Val Glu Tyr Cys Lys Tyr Gly Asn Leu Ser Asn
   740 745 750
   Tyr Leu Lys Ser Lys Arg Asp Leu Phe Phe Leu Asn Lys Asp Ala Ala
   755 760 765
   Leu His Met Glu Pro Lys Lys Glu Lys Met Glu Pro Gly Leu Glu Gln
   770 775 780
   Gly Lys Lys Pro Arg Leu Asp Ser Val Thr Ser Ser Glu Ser Phe Ala
   785 790 795 800
   Ser Ser Gly Phe Gln Glu Asp Lys Ser Leu Ser Asp Val Glu Glu Glu
   805 810 815
   Glu Asp Ser Asp Gly Phe Tyr Lys Glu Pro Ile Thr Met Glu Asp Leu
   820 825 830
   Ile Ser Tyr Ser Phe Gln Val Ala Arg Gly Met Glu Phe Leu Ser Ser
   835 840 845
   Arg Lys Cys Ile His Arg Asp Leu Ala Ala Arg Asn Ile Leu Leu Ser
   850 855 860
   Glu Asn Asn Val Val Lys Ile Cys Asp Phe Gly Leu Ala Arg Asp Ile
   865 870 875 880
   Tyr Lys Asn Pro Asp Tyr Val Arg Lys Gly Asp Thr Arg Leu Pro Leu
   885 890 895
   Lys Trp Met Ala Pro Glu Ser Ile Phe Asp Lys Ile Tyr Ser Thr Lys
   900 905 910
   Ser Asp Val Trp Ser Tyr Gly Val Leu Leu Trp Glu Ile Phe Ser Leu
   915 920 925
   Gly Gly Ser Pro Tyr Pro Gly Val Gln Met Asp Glu Asp Phe Cys Ser
   930 935 940
   Arg Leu Arg Glu Gly Met Arg Met Arg Ala Pro Glu Tyr Ser Thr Pro
   945 950 955 960
   Glu Ile Tyr Gln Ile Met Leu Asp Cys Trp His Arg Asp Pro Lys Glu
   965 970 975
   Arg Pro Arg Phe Ala Glu Leu Val Glu Lys Leu Gly Asp Leu Leu Gln
   980 985 990
   Ala Asn Val Gln Gln Asp Gly Lys Asp Tyr Ile Pro Ile Asn Ala Ile
   995 1000 1005
   Leu Thr Gly Asn Ser Gly Phe Thr Tyr Ser Thr Pro Ala Phe Ser
   1010 1015 1020
   Glu Asp Phe Phe Lys Glu Ser Ile Ser Ala Pro Lys Phe Asn Ser
   1025 1030 1035
   Gly Ser Ser Asp Asp Val Arg Tyr Val Asn Ala Phe Lys Phe Met
   1040 1045 1050
   Ser Leu Glu Arg Ile Lys Thr Phe Glu Glu Leu Leu Pro Asn Ala
   1055 1060 1065
   Thr Ser Met Phe Asp Asp Tyr Gln Gly Asp Ser Ser Thr Leu Leu
   1070 1075 1080
   Ala Ser Pro Met Leu Lys Arg Phe Thr Trp Thr Asp Ser Lys Pro
   1085 1090 1095
   Lys Ala Ser Leu Lys Ile Asp Leu Arg Val Thr Ser Lys Ser Lys
   1100 1105 1110
   Glu Ser Gly Leu Ser Asp Val Ser Arg Pro Ser Phe Cys His Ser
   1115 1120 1125
   Ser Cys Gly His Val Ser Glu Gly Lys Arg Arg Phe Thr Tyr Asp
   1130 1135 1140
   His Ala Glu Leu Glu Arg Lys Ile Ala Cys Cys Ser Pro Pro Pro
   1145 1150 1155
   Asp Tyr Asn Ser Val Val Leu Tyr Ser Thr Pro Pro Ile
   1160 1165 1170
<210> 17
   <211> 1173
   <212> PRT
   <213> Homo sapiens
<400> 17
   Met Pro Leu Pro Phe Gln Phe Pro Leu Asp Thr Leu Ile Pro Asp Gly
   1               5                   10                  15
   Lys Arg Ile Ile Trp Asp Ser Arg Lys Gly Phe Ile Ile Ser Asn Ala
               20                  25                  30
   Thr Tyr Lys Glu Ile Gly Leu Leu Thr Cys Glu Ala Thr Val Asn Gly
           35                  40                  45
   His Leu Tyr Lys Thr Asn Tyr Leu Thr His Arg Gln Thr Asn Thr Ile
       50                  55                  60
   Ile Asp Val Gln Ile Ser Thr Pro Arg Pro Val Lys Leu Leu Arg Gly
   65                  70                  75                  80
   His Thr Leu Val Leu Asn Cys Thr Ala Thr Thr Pro Leu Asn Thr Arg
                   85                  90                  95
   Val Gln Met Thr Trp Ser Tyr Pro Asp Glu Lys Asn Lys Arg Ala Ser
               100                 105                 110
   Val Arg Arg Arg Ile Asp Gln Ser Asn Ser His Ala Asn Ile Phe Tyr
           115                 120                 125
   Ser Val Leu Thr Ile Asp Lys Met Gln Asn Lys Asp Lys Gly Leu Tyr
       130                 135                 140
   Thr Cys Arg Val Arg Ser Gly Pro Ser Phe Lys Ser Val Asn Thr Ser
   145                 150                 155                 160
   Val His Ile Tyr Asp Lys Ala Phe Ile Thr Val Lys His Arg Lys Gln
                   165                 170                 175
   Gln Val Leu Glu Thr Val Ala Gly Lys Arg Ser Tyr Arg Leu Ser Met
               180                 185                 190
   Lys Val Lys Ala Phe Pro Ser Pro Glu Val Val Trp Leu Lys Asp Gly
           195                 200                 205
   Leu Pro Ala Thr Glu Lys Ser Ala Arg Tyr Leu Thr Arg Gly Tyr Ser
       210                 215                 220
   Leu Ile Ile Lys Asp Val Thr Glu Glu Asp Ala Gly Asn Tyr Thr Ile
   225                 230                 235                 240
   Leu Leu Ser Ile Lys Gln Ser Asn Val Phe Lys Asn Leu Thr Ala Thr
                   245                 250                 255
   Leu Ile Val Asn Val Lys Pro Gln Ile Tyr Glu Lys Ala Val Ser Ser
   260 265 270
   Phe Pro Asp Pro Ala Leu Tyr Pro Leu Gly Ser Arg Gln Ile Leu Thr
   275 280 285
   Cys Thr Ala Tyr Gly Ile Pro Gln Pro Thr Ile Lys Trp Phe Trp His
   290 295 300
   Pro Cys Asn His Asn His Ser Glu Ala Arg Cys Asp Phe Cys Ser Asn
   305 310 315 320
   Asn Glu Glu Ser Phe Ile Leu Asp Ala Asp Ser Asn Met Gly Asn Arg
   325 330 335
   Ile Glu Ser Ile Thr Gln Arg Met Ala Ile Ile Glu Gly Lys Asn Lys
   340 345 350
   Met Ala Ser Thr Leu Val Val Ala Asp Ser Arg Ile Ser Gly Ile Tyr
   355 360 365
   Ile Cys Ile Ala Ser Asn Lys Val Gly Thr Val Gly Arg Asn Ile Ser
   370 375 380
   Phe Tyr Ile Thr Asp Val Pro Asn Gly Phe His Val Asn Leu Glu Lys
   385 390 395 400
   Met Pro Thr Glu Gly Glu Asp Leu Lys Leu Ser Cys Thr Val Asn Lys
   405 410 415
   Phe Leu Tyr Arg Asp Val Thr Trp Ile Leu Leu Arg Thr Val Asn Asn
   420 425 430
   Arg Thr Met His Tyr Ser Ile Ser Lys Gln Lys Met Ala Ile Thr Lys
   435 440 445
   Glu His Ser Ile Thr Leu Asn Leu Thr Ile Met Asn Val Ser Leu Gln
   450 455 460
   Asp Ser Gly Thr Tyr Ala Cys Arg Ala Arg Asn Val Tyr Thr Gly Glu
   465 470 475 480
   Glu Ile Leu Gln Lys Lys Glu Ile Thr Ile Arg Asp Gln Glu Ala Pro
   485 490 495
   Tyr Leu Leu Arg Asn Leu Ser Asp His Thr Val Ala Ile Ser Ser Ser
   500 505 510
   Thr Thr Leu Asp Cys His Ala Asn Gly Val Pro Glu Pro Gln Ile Thr
   515 520 525
   Trp Phe Lys Asn Asn His Lys Ile Gln Gln Glu Pro Gly Ile Ile Leu
   530 535 540
   Gly Pro Gly Ser Ser Thr Leu Phe Ile Glu Arg Val Thr Glu Glu Asp
   545 550 555 560
   Glu Gly Val Tyr His Cys Lys Ala Thr Asn Gln Lys Gly Ser Val Glu
   565 570 575
   Ser Ser Ala Tyr Leu Thr Val Gln Gly Thr Ser Asp Lys Ser Asn Leu
   580 585 590
   Glu Leu Ile Thr Leu Thr Cys Thr Cys Val Ala Ala Thr Leu Phe Trp
   595 600 605
   Leu Leu Leu Thr Leu Phe Ile Arg Lys Met Lys Arg Ser Ser Ser Glu
   610 615 620
   Ile Lys Thr Asp Tyr Leu Ser Ile Ile Met Asp Pro Asp Glu Val Pro
   625 630 635 640
   Leu Asp Glu Gln Cys Glu Arg Leu Pro Tyr Asp Ala Ser Lys Trp Glu
   645 650 655
   Phe Ala Arg Glu Arg Leu Lys Leu Gly Lys Ser Leu Gly Arg Gly Ala
   660 665 670
   Phe Gly Lys Val Val Gln Ala Ser Ala Phe Gly Ile Lys Lys Ser Pro
   675 680 685
   Thr Cys Arg Thr Val Ala Val Lys Met Leu Lys Glu Gly Ala Thr Ala
   690 695 700
   Ser Glu Tyr Lys Ala Leu Met Thr Glu Leu Lys Ile Leu Thr His Ile
   705 710 715 720
   Gly His His Leu Asn Val Val Asn Leu Leu Gly Ala Cys Thr Lys Gln
   725 730 735
   Gly Gly Pro Leu Met Val Ile Val Glu Tyr Cys Lys Tyr Gly Asn Leu
   740 745 750
   Ser Asn Tyr Leu Lys Ser Lys Arg Asp Leu Phe Phe Leu Asn Lys Asp
   755 760 765
   Ala Ala Leu His Met Glu Pro Lys Lys Glu Lys Met Glu Pro Gly Leu
   770 775 780
   Glu Gln Gly Lys Lys Pro Arg Leu Asp Ser Val Thr Ser Ser Glu Ser
   785 790 795 800
   Phe Ala Ser Ser Gly Phe Gln Glu Asp Lys Ser Leu Ser Asp Val Glu
   805 810 815
   Glu Glu Glu Asp Ser Asp Gly Phe Tyr Lys Glu Pro Ile Thr Met Glu
   820 825 830
   Asp Leu Ile Ser Tyr Ser Phe Gln Val Ala Arg Gly Met Glu Phe Leu
   835 840 845
   Ser Ser Arg Lys Cys Ile His Arg Asp Leu Ala Ala Arg Asn Ile Leu
   850 855 860
   Leu Ser Glu Asn Asn Val Val Lys Ile Cys Asp Phe Gly Leu Ala Arg
   865 870 875 880
   Asp Ile Tyr Lys Asn Pro Asp Tyr Val Arg Lys Gly Asp Thr Arg Leu
   885 890 895
   Pro Leu Lys Trp Met Ala Pro Glu Ser Ile Phe Asp Lys Ile Tyr Ser
   900 905 910
   Thr Lys Ser Asp Val Trp Ser Tyr Gly Val Leu Leu Trp Glu Ile Phe
   915 920 925
   Ser Leu Gly Gly Ser Pro Tyr Pro Gly Val Gln Met Asp Glu Asp Phe
   930 935 940
   Cys Ser Arg Leu Arg Glu Gly Met Arg Met Arg Ala Pro Glu Tyr Ser
   945 950 955 960
   Thr Pro Glu Ile Tyr Gln Ile Met Leu Asp Cys Trp His Arg Asp Pro
   965 970 975
   Lys Glu Arg Pro Arg Phe Ala Glu Leu Val Glu Lys Leu Gly Asp Leu
   980 985 990
   Leu Gln Ala Asn Val Gln Gln Asp Gly Lys Asp Tyr Ile Pro Ile Asn
   995 1000 1005
   Ala Ile Leu Thr Gly Asn Ser Gly Phe Thr Tyr Ser Thr Pro Ala
   1010 1015 1020
   Phe Ser Glu Asp Phe Phe Lys Glu Ser Ile Ser Ala Pro Lys Phe
   1025 1030 1035
   Asn Ser Gly Ser Ser Asp Asp Val Arg Tyr Val Asn Ala Phe Lys
   1040 1045 1050
   Phe Met Ser Leu Glu Arg Ile Lys Thr Phe Glu Glu Leu Leu Pro
   1055 1060 1065
   Asn Ala Thr Ser Met Phe Asp Asp Tyr Gln Gly Asp Ser Ser Thr
   1070 1075 1080
   Leu Leu Ala Ser Pro Met Leu Lys Arg Phe Thr Trp Thr Asp Ser
   1085 1090 1095
   Lys Pro Lys Ala Ser Leu Lys Ile Asp Leu Arg Val Thr Ser Lys
   1100 1105 1110
   Ser Lys Glu Ser Gly Leu Ser Asp Val Ser Arg Pro Ser Phe Cys
   1115 1120 1125
   His Ser Ser Cys Gly His Val Ser Glu Gly Lys Arg Arg Phe Thr
   1130 1135 1140
   Tyr Asp His Ala Glu Leu Glu Arg Lys Ile Ala Cys Cys Ser Pro
   1145 1150 1155
   Pro Pro Asp Tyr Asn Ser Val Val Leu Tyr Ser Thr Pro Pro Ile
   1160 1165 1170

<210> 18
   <211> 733
   <212> PRT
   <213> Homo sapiens
<400> 18
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
   210 215 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
               340                 345                 350
   Tyr Arg Leu Ser Met Lys Val Lys Ala Phe Pro Ser Pro Glu Val Val
           355                 360                 365
   Trp Leu Lys Asp Gly Leu Pro Ala Thr Glu Lys Ser Ala Arg Tyr Leu
       370                 375                 380
   Thr Arg Gly Tyr Ser Leu Ile Ile Lys Asp Val Thr Glu Glu Asp Ala
   385                 390                 395                 400
   Gly Asn Tyr Thr Ile Leu Leu Ser Ile Lys Gln Ser Asn Val Phe Lys
                   405                 410                 415
   Asn Leu Thr Ala Thr Leu Ile Val Asn Val Lys Pro Gln Ile Tyr Glu
               420                 425                 430
   Lys Ala Val Ser Ser Phe Pro Asp Pro Ala Leu Tyr Pro Leu Gly Ser
           435                 440                 445
   Arg Gln Ile Leu Thr Cys Thr Ala Tyr Gly Ile Pro Gln Pro Thr Ile
       450                 455                 460
   Lys Trp Phe Trp His Pro Cys Asn His Asn His Ser Glu Ala Arg Cys
   465                 470                 475                 480
   Asp Phe Cys Ser Asn Asn Glu Glu Ser Phe Ile Leu Asp Ala Asp Ser
                   485                 490                 495
   Asn Met Gly Asn Arg Ile Glu Ser Ile Thr Gln Arg Met Ala Ile Ile
               500                 505                 510
   Glu Gly Lys Asn Lys Met Ala Ser Thr Leu Val Val Ala Asp Ser Arg
           515                 520                 525
   Ile Ser Gly Ile Tyr Ile Cys Ile Ala Ser Asn Lys Val Gly Thr Val
       530                 535                 540
   Gly Arg Asn Ile Ser Phe Tyr Ile Thr Asp Val Pro Asn Gly Phe His
   545                 550                 555                 560
   Val Asn Leu Glu Lys Met Pro Thr Glu Gly Glu Asp Leu Lys Leu Ser
                   565                 570                 575
   Cys Thr Val Asn Lys Phe Leu Tyr Arg Asp Val Thr Trp Ile Leu Leu
               580                 585                 590
   Arg Thr Val Asn Asn Arg Thr Met His Tyr Ser Ile Ser Lys Gln Lys
           595                 600                 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Asp Gln Glu Ala Pro Tyr Leu Leu Arg Asn Leu Ser Asp His Thr Val
   660 665 670
   Ala Ile Ser Ser Ser Thr Thr Leu Asp Cys His Ala Asn Gly Val Pro
   675 680 685
   Glu Pro Gln Ile Thr Trp Phe Lys Asn Asn His Lys Ile Gln Gln Glu
   690 695 700
   Pro Glu Leu Tyr Thr Ser Thr Ser Pro Ser Ser Ser Ser Ser Ser Pro
   705 710 715 720
   Leu Ser Ser Ser Ser Ser Ser Ser Ser Ser Ser Ser Ser
   725 730
<210> 19
   <211> 718
   <212> PRT
   <213> Homo sapiens
<400> 19
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
   210 215 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545 550 555 560
   Val Asn Leu Glu Lys Met Pro Thr Glu Gly Glu Asp Leu Lys Leu Ser
   565 570 575
   Cys Thr Val Asn Lys Phe Leu Tyr Arg Asp Val Thr Trp Ile Leu Leu
   580 585 590
   Arg Thr Val Asn Asn Arg Thr Met His Tyr Ser Ile Ser Lys Gln Lys
   595 600 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Asp Gln Glu Ala Pro Tyr Leu Leu Arg Asn Leu Ser Asp His Thr Val
   660 665 670
   Ala Ile Ser Ser Ser Thr Thr Leu Asp Cys His Ala Asn Gly Val Pro
   675 680 685
   Glu Pro Gln Ile Thr Trp Phe Lys Asn Asn His Lys Ile Gln Gln Glu
   690 695 700
   Pro Gly Ser Ala Asn Thr Ala Val Asn Lys Lys Thr Glu Ile
   705 710 715
<210> 20
   <211> 736
   <212> PRT
   <213> Homo sapiens
<400> 20
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
   210 215 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545 550 555 560
   Val Asn Leu Glu Lys Met Pro Thr Glu Gly Glu Asp Leu Lys Leu Ser
   565 570 575
   Cys Thr Val Asn Lys Phe Leu Tyr Arg Asp Val Thr Trp Ile Leu Leu
   580 585 590
   Arg Thr Val Asn Asn Arg Thr Met His Tyr Ser Ile Ser Lys Gln Lys
   595 600 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Asp Gln Glu Ala Pro Tyr Leu Leu Arg Asn Leu Ser Asp His Thr Val
   660 665 670
   Ala Ile Ser Ser Ser Thr Thr Leu Asp Cys His Ala Asn Gly Val Pro
   675 680 685
   Glu Pro Gln Ile Thr Trp Phe Lys Asn Asn His Lys Ile Gln Gln Glu
   690 695 700
   Pro Gly Lys Arg Leu Phe Phe Leu Pro Phe Ile Ile Ser His Leu Ser
   705 710 715 720
   Ser Ala Pro Leu Ser Leu Asn Ser Pro Val Thr Cys Phe Gln Tyr Val
   725 730 735
<210> 21
   <211> 547
   <212> PRT
   <213> Homo sapiens
<400> 21
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
           275                 280                 285
   Ala Asn Ile Phe Tyr Ser Val Leu Thr Ile Asp Lys Met Gln Asn Lys
       290                 295                 300
   Asp Lys Gly Leu Tyr Thr Cys Arg Val Arg Ser Gly Pro Ser Phe Lys
   305                 310                 315                 320
   Ser Val Asn Thr Ser Val His Ile Tyr Asp Lys Ala Phe Ile Thr Val
                   325                 330                 335
   Lys His Arg Lys Gln Gln Val Leu Glu Thr Val Ala Gly Lys Arg Ser
               340                 345                 350
   Tyr Arg Leu Ser Met Lys Val Lys Ala Phe Pro Ser Pro Glu Val Val
           355                 360                 365
   Trp Leu Lys Asp Gly Leu Pro Ala Thr Glu Lys Ser Ala Arg Tyr Leu
       370                 375                 380
   Thr Arg Gly Tyr Ser Leu Ile Ile Lys Asp Val Thr Glu Glu Asp Ala
   385                 390                 395                 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Tyr Leu Asp Ile Arg Thr Glu Glu Gln Ile Phe
   515 520 525
   Ser Phe Ile Gln Lys Thr Gln Thr Leu Lys Leu Thr Val Ser Cys Lys
   530 535 540
   Ala Ala Phe
   545
<210> 22
   <211> 365
   <212> PRT
   <213> Homo sapiens
<400> 22
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
   210 215 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Gly Lys His Ser Ser Ala Leu
   325 330 335
   Pro Thr His Ala Met Leu Ser Asn His Cys Arg Cys Leu Cys Ser Leu
   340 345 350
   Asn Lys Ser Val Phe Cys Trp Pro Arg Val Thr Leu Ser
   355 360 365
<210> 23
   <211> 433
   <212> PRT
   <213> Homo sapiens
<400> 23
   Met Val Ile Val Glu Tyr Cys Lys Tyr Gly Asn Leu Ser Asn Tyr Leu
   1 5 10 15
   Lys Ser Lys Arg Asp Leu Phe Phe Leu Asn Lys Asp Ala Ala Leu His
   20 25 30
   Met Glu Pro Lys Lys Glu Lys Met Glu Pro Gly Leu Glu Gln Gly Lys
   35 40 45
   Lys Pro Arg Leu Asp Ser Val Thr Ser Ser Glu Ser Phe Ala Ser Ser
   50 55 60
   Gly Phe Gln Glu Asp Lys Ser Leu Ser Asp Val Glu Glu Glu Glu Asp
   65 70 75 80
   Ser Asp Gly Phe Tyr Lys Glu Pro Ile Thr Met Glu Asp Leu Ile Ser
   85 90 95
   Tyr Ser Phe Gln Val Ala Arg Gly Met Glu Phe Leu Ser Ser Arg Lys
   100 105 110
   Cys Ile His Arg Asp Leu Ala Ala Arg Asn Ile Leu Leu Ser Glu Asn
   115 120 125
   Asn Val Val Lys Ile Cys Asp Phe Gly Leu Ala Arg Asp Ile Tyr Lys
   130 135 140
   Asn Pro Asp Tyr Val Arg Lys Gly Asp Thr Arg Leu Pro Leu Lys Trp
   145 150 155 160
   Met Ala Pro Glu Ser Ile Phe Asp Lys Ile Tyr Ser Thr Lys Ser Asp
   165 170 175
   Val Trp Ser Tyr Gly Val Leu Leu Trp Glu Ile Phe Ser Leu Gly Gly
   180 185 190
   Ser Pro Tyr Pro Gly Val Gln Met Asp Glu Asp Phe Cys Ser Arg Leu
   195 200 205
   Arg Glu Gly Met Arg Met Arg Ala Pro Glu Tyr Ser Thr Pro Glu Ile
   210 215 220
   Tyr Gln Ile Met Leu Asp Cys Trp His Arg Asp Pro Lys Glu Arg Pro
   225 230 235 240
   Arg Phe Ala Glu Leu Val Glu Lys Leu Gly Asp Leu Leu Gln Ala Asn
   245 250 255
   Val Gln Gln Asp Gly Lys Asp Tyr Ile Pro Ile Asn Ala Ile Leu Thr
   260 265 270
   Gly Asn Ser Gly Phe Thr Tyr Ser Thr Pro Ala Phe Ser Glu Asp Phe
   275 280 285
   Phe Lys Glu Ser Ile Ser Ala Pro Lys Phe Asn Ser Gly Ser Ser Asp
   290 295 300
   Asp Val Arg Tyr Val Asn Ala Phe Lys Phe Met Ser Leu Glu Arg Ile
   305 310 315 320
   Lys Thr Phe Glu Glu Leu Leu Pro Asn Ala Thr Ser Met Phe Asp Asp
   325 330 335
   Tyr Gln Gly Asp Ser Ser Thr Leu Leu Ala Ser Pro Met Leu Lys Arg
   340 345 350
   Phe Thr Trp Thr Asp Ser Lys Pro Lys Ala Ser Leu Lys Ile Asp Leu
   355 360 365
   Arg Val Thr Ser Lys Ser Lys Glu Ser Gly Leu Ser Asp Val Ser Arg
   370 375 380
   Pro Ser Phe Cys His Ser Ser Cys Gly His Val Ser Glu Gly Lys Arg
   385 390 395 400
   Arg Phe Thr Tyr Asp His Ala Glu Leu Glu Arg Lys Ile Ala Cys Cys
   405 410 415
   Ser Pro Pro Pro Asp Tyr Asn Ser Val Val Leu Tyr Ser Thr Pro Pro
   420 425 430
   Ile
<210> 24
   <211> 214
   <212> PRT
   <213> Homo sapiens
<400> 24
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Val Asn Leu Asn Thr
                   165                 170                 175
   Ala Ile Leu Ser Ile Leu Ser Leu Gln Ile Ser Ile Met Lys Phe Tyr
               180                 185                 190
   Ser Phe Tyr Leu Ser Gly Ile Ile Ser Leu Gln Thr Pro Gly Leu Leu
           195                 200                 205
   Ser Gly Leu Ser Cys Asn
       210
<210> 25
   <211> 343
   <212> PRT
   <213> Homo sapiens
<400> 25
   Met Glu Asp Leu Ile Ser Tyr Ser Phe Gln Val Ala Arg Gly Met Glu
   1               5                   10                  15
   Phe Leu Ser Ser Arg Lys Cys Ile His Arg Asp Leu Ala Ala Arg Asn
               20                  25                  30
   Ile Leu Leu Ser Glu Asn Asn Val Val Lys Ile Cys Asp Phe Gly Leu
           35                  40                  45
   Ala Arg Asp Ile Tyr Lys Asn Pro Asp Tyr Val Arg Lys Gly Asp Thr
       50                  55                  60
   Arg Leu Pro Leu Lys Trp Met Ala Pro Glu Ser Ile Phe Asp Lys Ile
   65                  70                  75                  80
   Tyr Ser Thr Lys Ser Asp Val Trp Ser Tyr Gly Val Leu Leu Trp Glu
                   85                  90                  95
   Ile Phe Ser Leu Gly Gly Ser Pro Tyr Pro Gly Val Gln Met Asp Glu
               100                 105                 110
   Asp Phe Cys Ser Arg Leu Arg Glu Gly Met Arg Met Arg Ala Pro Glu
           115                 120                 125
   Tyr Ser Thr Pro Glu Ile Tyr Gln Ile Met Leu Asp Cys Trp His Arg
       130                 135                 140
   Asp Pro Lys Glu Arg Pro Arg Phe Ala Glu Leu Val Glu Lys Leu Gly
   145                 150                 155                 160
   Asp Leu Leu Gln Ala Asn Val Gln Gln Asp Gly Lys Asp Tyr Ile Pro
                   165                 170                 175
   Ile Asn Ala Ile Leu Thr Gly Asn Ser Gly Phe Thr Tyr Ser Thr Pro
               180                 185                 190
   Ala Phe Ser Glu Asp Phe Phe Lys Glu Ser Ile Ser Ala Pro Lys Phe
           195                 200                 205
   Asn Ser Gly Ser Ser Asp Asp Val Arg Tyr Val Asn Ala Phe Lys Phe
       210                 215                 220
   Met Ser Leu Glu Arg Ile Lys Thr Phe Glu Glu Leu Leu Pro Asn Ala
   225 230 235 240
   Thr Ser Met Phe Asp Asp Tyr Gln Gly Asp Ser Ser Thr Leu Leu Ala
   245 250 255
   Ser Pro Met Leu Lys Arg Phe Thr Trp Thr Asp Ser Lys Pro Lys Ala
   260 265 270
   Ser Leu Lys Ile Asp Leu Arg Val Thr Ser Lys Ser Lys Glu Ser Gly
   275 280 285
   Leu Ser Asp Val Ser Arg Pro Ser Phe Cys His Ser Ser Cys Gly His
   290 295 300
   Val Ser Glu Gly Lys Arg Arg Phe Thr Tyr Asp His Ala Glu Leu Glu
   305 310 315 320
   Arg Lys Ile Ala Cys Cys Ser Pro Pro Pro Asp Tyr Asn Ser Val Val
   325 330 335
   Leu Tyr Ser Thr Pro Pro Ile
   340
<210> 26
   <211> 183
   <212> PRT
   <213> Homo sapiens
<400> 26
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Gly Lys Thr Ser Ile Phe Tyr Ile Leu Phe Ala Phe Ala Leu Gln
       130                 135                 140
   Met Ser His Lys Ser Thr Leu Ile His Trp Lys Gly Cys Phe Pro Ser
   145                 150                 155                 160
   Glu Tyr Glu Arg Asn Gly Leu Gly Lys Arg Phe His Pro Ser Cys Arg
                   165                 170                 175
   His Phe Arg Gly Cys Gln Phe
               180
<210> 27
   <211> 206
   <212> PRT
   <213> Homo sapiens
<400> 27
   Met Leu Asp Cys Trp His Arg Asp Pro Lys Glu Arg Pro Arg Phe Ala
   1 5 10 15
   Glu Leu Val Glu Lys Leu Gly Asp Leu Leu Gln Ala Asn Val Gln Gln
   20 25 30
   Asp Gly Lys Asp Tyr Ile Pro Ile Asn Ala Ile Leu Thr Gly Asn Ser
   35 40 45
   Gly Phe Thr Tyr Ser Thr Pro Ala Phe Ser Glu Asp Phe Phe Lys Glu
   50 55 60
   Ser Ile Ser Ala Pro Lys Phe Asn Ser Gly Ser Ser Asp Asp Val Arg
   65 70 75 80
   Tyr Val Asn Ala Phe Lys Phe Met Ser Leu Glu Arg Ile Lys Thr Phe
   85 90 95
   Glu Glu Leu Leu Pro Asn Ala Thr Ser Met Phe Asp Asp Tyr Gln Gly
   100 105 110
   Asp Ser Ser Thr Leu Leu Ala Ser Pro Met Leu Lys Arg Phe Thr Trp
   115 120 125
   Thr Asp Ser Lys Pro Lys Ala Ser Leu Lys Ile Asp Leu Arg Val Thr
   130 135 140
   Ser Lys Ser Lys Glu Ser Gly Leu Ser Asp Val Ser Arg Pro Ser Phe
   145 150 155 160
   Cys His Ser Ser Cys Gly His Val Ser Glu Gly Lys Arg Arg Phe Thr
   165 170 175
   Tyr Asp His Ala Glu Leu Glu Arg Lys Ile Ala Cys Cys Ser Pro Pro
   180 185 190
   Pro Asp Tyr Asn Ser Val Val Leu Tyr Ser Thr Pro Pro Ile
   195 200 205
<210> 28
   <211> 119
   <212> PRT
   <213> Homo sapiens
<400> 28
<210> 29
   <211> 480
   <212> PRT
   <213> Homo sapiens
<400> 29
   Leu Trp Ala Ala Cys Pro Ala Gln Ala Cys Ser Gly Asn Ala Gly Gln
   1               5                   10                  15
   Glu Arg Gly Gly Leu Gln Ser Ala Ala Gly Leu Pro Ser Gln Pro Ser
               20                  25                  30
   Cys Phe Leu Gln Thr Gly Val Gly Leu Ala Asn Gln Gly Pro Leu Met
           35                  40                  45
   Val Ile Val Glu Tyr Cys Lys Tyr Gly Asn Leu Ser Asn Tyr Leu Lys
       50                  55                  60
   Ser Lys Arg Asp Leu Phe Phe Leu Asn Lys Asp Ala Ala Leu His Met
   65                  70                  75                  80
   Glu Pro Lys Lys Glu Lys Met Glu Pro Gly Leu Glu Gln Gly Lys Lys
                   85                  90                  95
   Pro Arg Leu Asp Ser Val Thr Ser Ser Glu Ser Phe Ala Ser Ser Gly
               100                 105                 110
   Phe Gln Glu Asp Lys Ser Leu Ser Asp Val Glu Glu Glu Glu Asp Ser
           115                 120                 125
   Asp Gly Phe Tyr Lys Glu Pro Ile Thr Met Glu Asp Leu Ile Ser Tyr
       130                 135                 140
   Ser Phe Gln Val Ala Arg Gly Met Glu Phe Leu Ser Ser Arg Lys Cys
   145                 150                 155                 160
   Ile His Arg Asp Leu Ala Ala Arg Asn Ile Leu Leu Ser Glu Asn Asn
                   165                 170                 175
   Val Val Lys Ile Cys Asp Phe Gly Leu Ala Arg Asp Ile Tyr Lys Asn
               180                 185                 190
   Pro Asp Tyr Val Arg Lys Gly Asp Thr Arg Leu Pro Leu Lys Trp Met
           195                 200                 205
   Ala Pro Glu Ser Ile Phe Asp Lys Ile Tyr Ser Thr Lys Ser Asp Val
       210                 215                 220
   Trp Ser Tyr Gly Val Leu Leu Trp Glu Ile Phe Ser Leu Gly Gly Ser
   225                 230                 235                 240
   Pro Tyr Pro Gly Val Gln Met Asp Glu Asp Phe Cys Ser Arg Leu Arg
                   245                 250                 255
   Glu Gly Met Arg Met Arg Ala Pro Glu Tyr Ser Thr Pro Glu Ile Tyr
   260 265 270
   Gln Ile Met Leu Asp Cys Trp His Arg Asp Pro Lys Glu Arg Pro Arg
   275 280 285
   Phe Ala Glu Leu Val Glu Lys Leu Gly Asp Leu Leu Gln Ala Asn Val
   290 295 300
   Gln Gln Asp Gly Lys Asp Tyr Ile Pro Ile Asn Ala Ile Leu Thr Gly
   305 310 315 320
   Asn Ser Gly Phe Thr Tyr Ser Thr Pro Ala Phe Ser Glu Asp Phe Phe
   325 330 335
   Lys Glu Ser Ile Ser Ala Pro Lys Phe Asn Ser Gly Ser Ser Asp Asp
   340 345 350
   Val Arg Tyr Val Asn Ala Phe Lys Phe Met Ser Leu Glu Arg Ile Lys
   355 360 365
   Thr Phe Glu Glu Leu Leu Pro Asn Ala Thr Ser Met Phe Asp Asp Tyr
   370 375 380
   Gln Gly Asp Ser Ser Thr Leu Leu Ala Ser Pro Met Leu Lys Arg Phe
   385 390 395 400
   Thr Trp Thr Asp Ser Lys Pro Lys Ala Ser Leu Lys Ile Asp Leu Arg
   405 410 415
   Val Thr Ser Lys Ser Lys Glu Ser Gly Leu Ser Asp Val Ser Arg Pro
   420 425 430
   Ser Phe Cys His Ser Ser Cys Gly His Val Ser Glu Gly Lys Arg Arg
   435 440 445
   Phe Thr Tyr Asp His Ala Glu Leu Glu Arg Lys Ile Ala Cys Cys Ser
   450 455 460
   Pro Pro Pro Asp Tyr Asn Ser Val Val Leu Tyr Ser Thr Pro Pro Ile
   465 470 475 480
<210> 30
   <211> 541
   <212> PRT
   <213> Homo sapiens
<400> 30
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Leu Pro Pro Ala Asn Ser Ser Phe Met Leu Pro
   515 520 525
   Pro Thr Ser Phe Ser Ser Asn Tyr Phe His Phe Leu Pro
   530 535 540

<210> 31
   <211> 567
   <212> PRT
   <213> Homo sapiens
<400> 31
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
           275                 280                 285
   Ala Asn Ile Phe Tyr Ser Val Leu Thr Ile Asp Lys Met Gln Asn Lys
       290                 295                 300
   Asp Lys Gly Leu Tyr Thr Cys Arg Val Arg Ser Gly Pro Ser Phe Lys
   305                 310                 315                 320
   Ser Val Asn Thr Ser Val His Ile Tyr Asp Lys Ala Phe Ile Thr Val
                   325                 330                 335
   Lys His Arg Lys Gln Gln Val Leu Glu Thr Val Ala Gly Lys Arg Ser
               340                 345                 350
   Tyr Arg Leu Ser Met Lys Val Lys Ala Phe Pro Ser Pro Glu Val Val
           355                 360                 365
   Trp Leu Lys Asp Gly Leu Pro Ala Thr Glu Lys Ser Ala Arg Tyr Leu
       370                 375                 380
   Thr Arg Gly Tyr Ser Leu Ile Ile Lys Asp Val Thr Glu Glu Asp Ala
   385                 390                 395                 400
   Gly Asn Tyr Thr Ile Leu Leu Ser Ile Lys Gln Ser Asn Val Phe Lys
                   405                 410                 415
   Asn Leu Thr Ala Thr Leu Ile Val Asn Val Lys Pro Gln Ile Tyr Glu
               420                 425                 430
   Lys Ala Val Ser Ser Phe Pro Asp Pro Ala Leu Tyr Pro Leu Gly Ser
           435                 440                 445
   Arg Gln Ile Leu Thr Cys Thr Ala Tyr Gly Ile Pro Gln Pro Thr Ile
       450                 455                 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Glu Leu Ser Asn Phe Glu Cys
   545 550 555 560
   Leu His Pro Cys Ser Gln Glu
   565
<210> 32
   <211> 378
   <212> DNA
   <213> Artificial Organism
<220>
   <223> Synthetic oligonucleotide
<400> 32
   taaatcgccc ccgccctcgg ctgctcttca tcgaggtccg cgggaggctc ggagcgcgcc 60
   aggcggacac tcctctcggc tcctccccgg cagcggcggc ggctcggagc gggctccggg 120
   gctcgggtgc agcggccagc gggcgcctgg cggcgaggat tacccgggga agtggttgtc 180
   tcctggctgg agccgcgaga cgggcgctca gggcgcgggg ccggcggcgg cgaacgagag 240
   gacggactct ggcggccggg tcgttggccg cggggagcgc gggcaccggg cgagcaggcc 300
   gcgtcgcgct caccatggtc agctactggg acaccggggt cctgctgtgc gcgctgctca 360
   gctgtctgct tctcacag 378
<210> 33
   <211> 227
   <212> DNA
   <213> Artificial Organism
<220>
   <223> Synthetic oligonucleotide
<400> 33
   gggggaagca gcccataaat ggtctttgcc tgaaatggtg agtaaggaaa gcgaaaggct 60
   gagcataact aaatctgcct gtggaagaaa tggcaaacaa ttctgcagta ctttaacctt 120
   gaacacagct caagcaaacc acactggctt ctacagctgc aaatatctag ctgtacctac 180
   ttcaaagaag aaggaaacag aatctgcaat ctatatattt attagtg 227
<210> 34
   <211> 455
   <212> DNA
   <213> Artificial Organism
<220>
   <223> Synthetic oligonucleotide
<400> 34
   gtaagacttc cattttctac attctgtttg cttttgcttt gcaaatgagt cataaatcta 60
   ccctgatcca ttggaaaggt tgctttccta gtgagtatga aagaaacggg ttaggaaaga 120
   gattccatcc tagttgtaga cactttcgtg gatgtcaatt ttaaccagaa ttgtcaaggc 180
   ccagttagtc actatgtgct ccacaaagag agagactggt ctgtataggt cacctctgta 240
   cccacagctc ctagcacagt atgtggccca tagttggatg aataagtgaa taaaaggatt 300
   ttagttctat ttcttagtta ttgagaagcc caaaatagaa tacatgattt ttgtcattta 360
   acacagctgc attctcttat atgttggaac tgtaaaatac tgttgtttct actaaattaa 420
   taaacgcaat gttgctgtta tctcgagtgt tatat 455
<210> 35
   <211> 125
   <212> DNA
   <213> Artificial Organism
<220>
   <223> Synthetic oligonucleotide
<400> 35
   atacaggtag acctttcgta gagatgtaca gtgaaatccc cgaaattata cacatgactg 60
   aaggaaggga gctcgtcatt ccctgccggg ttacgtcacc taacatcact gttactttaa 120
   aaaag 125
<210> 36
   <211> 129
   <212> DNA
   <213> Artificial Organism
<220>
   <223> Synthetic oligonucleotide
<400> 36
   gtaaatttga acaccgctat tctcagcata ctttcactgc aaataagcat aatgaagttt 60
   tactcgttct atctttctgg aataatttcc ttacaaacac ctgggcttct cagtggactt 120
   tcctgtaac 129
<210> 37
   <211> 1124
   <212> DNA
   <213> Artificial Organism
<220>
   <223> Synthetic oligonucleotide
<400> 37
   ccttcattct tacacatgcg gtggtggcca cagctgatgg gctacagtct ttgactgctt 60
   tagatcttgt cagctaaatg taaagttact ttgtcaatgg aatgctacta acagccgtaa 120
   tggatagatg agagtaagag aaacacttcg gaggaaattt tagtgtattt ttaagttgta 180
   ctgaggacgt ctgtcagcaa tcctttcttt tagattcagc acatgcttgt tttcacttat 240
   ttgtaaagta ccatagattt cagtccctga cttaaatttt tctgggtttt tcctttgctt 300
   gcactaaatt agaagagaag gttctttttt tcccaccgac tgcattcctg agtttacagg 360
   tagcaccttg atttactgaa acatgaaagc gaatccttta attgctttca gttcttcata 420
   aacaggatgt aattaatttc aaacccaagg aacaccggta atacattccg atgtaggcat 480
   agtccctgga agagaggcac tagtggcttc tggactagaa atctctgcca taggatcaca 540
   aatccagatc agcgccttca gcaagcaata gaaataaatg ataactggat agagcgaggg 600
   atgccaggtg tactttttaa aaggcgatag aaaaggtttc gagcttcttt gtggctgttt 660
   ggtatttcct ttataatctg ttgggggaag gtaatttgat ttttttcccc ctttttcaac 720
   tgggaataag gaatagtggt agttgggagt ctaccaccac tgaaattggg ctggcaacct 780
   ggcatcaggg gaatttggct gggccacttt atggagcaat gaccctttat aaataaagtt 840
   taatcccaag gcagagtgtt tatcttgtag tctttaaatg ttacattctt gatgtcagaa 900
   gaaggcataa ggagaaagtc agatcatgga ttattttctt ctgtttggac tcaccgtgct 960
   tgggaatact tctgagcatt agagagcact tcattcattg cagagtctct ggcctccgag 1020
   gctgccttca ccatcagcag cttcagcttc tgggagtttc ctttccagag gcagagctga 1080
   tgccttcctt gtgacacagc aggatcgtca gaatgacagc ccca 1124
<210> 38
   <211> 465
   <212> DNA
   <213> Artificial Organism
<220>
   <223> Synthetic oligonucleotide
<400> 38
   gtctctaact gctaacctca ggtgatccgc ccacctcggc ctcccaaagt actggaatta      60
   caggcgtgag ccaccacgcc cggcctctta attggtttgt ctattgagtg tctgaccagc     120
   ttattatgaa gattcccgga ggccaaagag ttatgggctc caactgaaag tctgacatga     180
   caagtaggtc tatcctgggt agatgccact tgttgccaag gctgatagta aattatcata     240
   attactataa tttatcataa tgcaaatatc ttttgctata tgcaaaaaaa ctcaagaacc     300
   aattataaca aactagagtt tttaacctag tttgttataa gcaaactaca aagttttact     360
   attttcattt tagatgaaaa atcatgtttt cacaaactgc tggtgaaaca gtattgccaa     420
   ccagtgctaa taggaccttg ttaatgcatg cctcttcctt ttcag                     465
<210> 39
   <211> 163
   <212> DNA
   <213> Artificial Organism
<220>
   <223> Synthetic oligonucleotide
<400> 39
   tttccacttg acactttgat ccctgatgga aaacgcataa tctgggacag tagaaagggc      60
   ttcatcatat caaatgcaac gtacaaagaa atagggcttc tgacctgtga agcaacagtc     120
   aatgggcatt tgtataagac aaactatctc acacatcgac aaa                      163
<210> 40
   <211> 137
   <212> DNA
   <213> Artificial Organism
<220>
   <223> Synthetic oligonucleotide
<400> 40
   ccaatacaat catagatgtc caaataagca caccacgccc agtcaaatta cttagaggcc      60
   atactcttgt cctcaattgt actgctacca ctcccttgaa cacgagagtt caaatgacct     120
   ggagttaccc tgatgaa                                                    137
<210> 41
   <211> 175
   <212> DNA
   <213> Artificial Organism
<220>
   <223> Synthetic oligonucleotide
<400> 41
   aaaaataaga gagcttccgt aaggcgacga attgaccaaa gcaattccca tgccaacata 60
   ttctacagtg ttcttactat tgacaaaatg cagaacaaag acaaaggact ttatacttgt 120
   cgtgtaagga gtggaccatc attcaaatct gttaacacct cagtgcatat atatg 175
<210> 42
   <211> 429
   <212> DNA
   <213> Artificial Organism
<220>
   <223> Synthetic oligonucleotide
<400> 42
   gtaagcatag ttcagcgttg ccaactcatg ctatgctttc taatcattgt agatgtctgt 60
   gttctctaaa taagtcagtt ttctgttggc caagagttac attatcatga atgaatacag 120
   cttggccatg gttccctcct ccccctgtca aaaagcaaac cacattaagt actctttaca 180
   tttaatactt ttgaattgaa cagagagtcc tttctttaga tgcttattat ggctaataat 240
   tcggttagaa cctaatatat ttatgaaatt cttagtgttc tgtataaaca aaacagaaaa 300
   agatgatgag ttttatcttt attatctaag aaaaatttct caggtactac catgaattag 360
   agaaataaaa atatggtatg ctaaatcatt tagaattaat gctattttat tcctgctttt 420
   actctcaag 429
<210> 43
   <211> 170
   <212> DNA
   <213> Artificial Organism
<220>
   <223> Synthetic oligonucleotide
<400> 43
   gttaaaagat gggttacctg cgactgagaa atctgctcgc tatttgactc gtggctactc 60
   gttaattatc aaggacgtaa ctgaagagga tgcagggaat tatacaatct tgctgagcat 120
   aaaacagtca aatgtgttta aaaacctcac tgccactcta attgtcaatg 170
<210> 44
   <211> 160
   <212> DNA
   <213> Artificial Organism
<220>
   <223> Synthetic oligonucleotide
<400> 44
   tgaaacccca gatttacgaa aaggccgtgt catcgtttcc agacccggct ctctacccac 60
   tgggcagcag acaaatcctg acttgtaccg catatggtat ccctcaacct acaatcaagt 120
   ggttctggca cccctgtaac cataatcatt ccgaagcaag 160
<210> 45
   <211> 954
   <212> DNA
   <213> Artificial Organism
<220>
   <223> Synthetic oligonucleotide
<400> 45
   tatttggata taaggactga ggaacagatt ttttccttta ttcagaagac tcagacattg 60
   aaactcactg tctcatgcaa agctgccttc tagtccagcc agactacact gatctacaat 120
   ttgcacctga gctcctttct ctggccctat aaagtttaaa ttttaattgt caaataattt 180
   tatttttaat gagaaaatgt ttgaatttat aatttaaaaa tatatcccct gaaagagaag 240
   tactctagaa atgagataaa ttttaataaa actgatcata ggatatgttt caccttattt 300
   ttaacttttc ttctttagtg ataaccattg tatttatcaa atatattttc aaaccacata 360
   acaacagttg aagagcagct tgcagcttat gtaaaatgtc atcagagaag ccaactagta 420
   tttctgtaaa tagtagtagt atttaattta tgttacccat ctctgcggat taaaggaagt 480
   attctcaagg aaaaattcaa ttaaaggtgt agacagaatt ttgccaatag atctgtgtta 540
   ttcaacttaa agagtctcaa ggcgagaagc aatctggtgt agagattagg agcataggca 600
   cggaggcaga cacacctgag cataaattct gcccaggcct gtctgtggtc ctttgcatct 660
   ttgggtgaat catctaatct ctcggaacct cagttgtcct acctgtagaa tagggtaata 720
   tacccatctc acagggtttt ggggaggatt aaatgagaca attcctgtaa agtgccttgc 780
   atgctgcctg gtgcattgta aggcatacat aaaggttcgc agaaacaaag aaataatgta 840
   aactcccatc aacgttgttt tctgcttcta ttttaataga agctttggag acatgatttc 900
   tggggcaggc ttgactacga gtaatgtgtc ttgtttggaa ggtgtcattc acac 954
<210> 46
   <211> 309
   <212> DNA
   <213> Artificial Organism
<220>
   <223> Synthetic oligonucleotide
<400> 46
   atgtgccaaa tgggtttcat gttaacttgg aaaaaatgcc gacggaagga gaggacctga    60
   aactgtcttg cacagttaac aagttcttat acagagacgt tacttggatt ttactgcgga   120
   cagttaataa cagaacaatg cactacagta ttagcaagca aaaaatggcc atcactaagg   180
   agcactccat cactcttaat cttaccatca tgaatgtttc cctgcaagat tcaggcacct   240
   atgcctgcag agccaggaat gtatacacag gggaagaaat cctccagaag aaagaaatta   300
   caatcagag                                                           309
<210> 47
   <211> 147
   <212> DNA
   <213> Artificial Organism
<220>
   <223> Synthetic oligonucleotide
<400> 47
   atcaggaagc accatacctc ctgcgaaacc tcagtgatca cacagtggcc atcagcagtt    60
   ccaccacttt agactgtcat gctaatggtg tccccgagcc tcagatcact tggtttaaaa   120
   acaaccacaa aatacaacaa gagcctg                                       147
<210> 48
   <211> 279
   <212> DNA
   <213> Artificial Organism
<220>
   <223> Synthetic oligonucleotide
<400> 48
   gtaagagact ttttttcttt ccatttatta tttctcatct ttcatcagcc cccctttccc    60
   ttaacagtcc cgtcacctgc ttccagtatg tatagaccta cttgctgata gagccccaaa   120
   cactgggatc tggttcatgc tgatagcaat gtccagtgag gaatatttgg gccagttcct   180
   atgcagacca tattcctaac ccatagaaag atcctgcaga atctattcag ctggaagtct   240
   ttaaatagtg cattcagaaa gaaatgagaa gagccatgt                          279
<210> 49
   <211> 615
   <212> DNA
   <213> Artificial Organism
<220>
   <223> Synthetic oligonucleotide
<400> 49
   gctctgcaaa tacagcagtg aacaagaaaa ctgaaatctg aagaaccaaa aatctagagt      60
   aattaagtga tgcatgtatt tacctgaaat acgcttcttc aagcaacaac cctgtgtgtc     120
   ctgccttatt cgctattatc gtgtcaattg gtctaaaaat ggaataaggc aaattaataa     180
   atgggcaatc agaaaaattc tatggctcaa tcatggtatc aaaatatctt taatacattg     240
   gatagctgaa aagatacacc ctttgtgctt taattttcct atgttcatgc tgtgcataca     300
   ataagttctc aattgttgtt agctgtatat acagaaagga ggaagagata aggggaaatt     360
   agtaggggct ccaaattaga actgagtaaa gaagcaactg ggaactctta gaagggactg     420
   atgtggtgca aagtgttgaa agatgaaggt aatttaagac ctatttactt tgaagaggca     480
   ggtgaaaaga gagtagacaa gctgaagtga atataataca gagagccaga gataagagag     540
   gaaaatgcag tgtgaaggga ctaataccaa gaagcaccac acaagagttc aaagtcagag     600
   atgaatcctg tttga                                                      615
<210> 50
   <211> 1473
   <212> DNA
   <213> Artificial Organism
<220>
   <223> Synthetic oligonucleotide
<400> 50
   aactgtatac atcaacgtca ccatcgtcat cgtcatcatc accattgtca tcatcatcat      60
   catcgtcatc atcatcatca tcatagctat catcattatc atcatcatca tcatcatcat     120
   catagctacc atttattgaa aactattatg tgtcaacttc aaagaactta tcctttagtt     180
   ggagagccaa gacaatcata acaataacaa atggccgggc atggtggctc acgcctgtaa     240
   tcccagcact ttgggaggcc aaggcaggtg gatcatttga ggtcaggagt tcaagaccag     300
   cctgaccaag atggtgaaat gctgtctcta ttaaaaatac aaaattagcc gggcatggtg     360
   gctcatgcct gtaatgccag ctactcggga ggctgagaca ggagaatcac ttgaacccag     420
   gaggcagagg ttgcagggag ccgagatcgt gtactgcact ccagcctggg caacaagagc     480
   gaaactccgt ctcaaaaaac aaataaataa ataaataaat aaacagacaa aattcacttt     540
   ttattctatt aaacttaaca tacatgcatt aattgcctac tctgagcctg atcctttatt     600
   atattctaga gaaaatgaaa ataagcaaca cataatcctc acctttgaaa aacaaacata     660
   ataggtacaa agtcagttca taaatgtgca atgtgagtgg tataaaataa catcagataa     720
   aaattagcac ttctgatgac taatcagggg agatttcatg gaggagatgt catctgaatt     780
   ggactttgat gaataaaaaa gagagttttg atgacgaata ttagtttggc caaagtggat     840
   gaatataatg ttctctaagt caacaataca gatagtcaac tgagcaacaa aacagaagtt     900
   gctggtcaga ctagtaggcc catttaggag agcaacaagc cacttttgtg tgtggggggg     960
   aacaggaggc aaacacatgg cctgcattta tgtaatcaaa taggtgaatt caaaagctct    1020
   acagatttat ttactcaatg gtgaattgga ttaaaattat attccatgct cttccctggg    1080
   caatatggtg ctcacacata aaatgccaag attgtccttc accttaaggt ataatgggaa    1140
   gaaataaagc tatctacaga gtcccttgaa aattacacct ttaaaattga cagttggaca    1200
   tcttgaaaat ttagaccaca tcccatgaat aaattaggcc atcatggtca aaaacttatc    1260
   cacatcctcc attagcaacc aacataataa gtaattcaaa tcctcacact gagtttcatc    1320
   gtcagatact ttagaggaca ccacacgttt ctacaaaaac agcccagtgt aaagttttac    1380
   ttgtcctttt ggggctaatg ctttagcaaa cgcataaaac caccagagtt tgcattctaa    1440
   gctccaggaa ctgaggcttt ttgaacctta aga                                 1473
<210> 51
   <211> 132
   <212> DNA
   <213> Artificial Organism
<220>
   <223> Synthetic oligonucleotide
<400> 51
   gaattatttt aggaccagga agcagcacgc tgtttattga aagagtcaca gaagaggatg 60
   aaggtgtcta tcactgcaaa gccaccaacc agaagggctc tgtggaaagt tcagcatacc 120
   tcactgttca ag 132
<210> 52
   <211> 133
   <212> DNA
   <213> Artificial Organism
<220>
   <223> Synthetic oligonucleotide
<400> 52
   tcttcttctg aaataaagac tgactaccta tcaattataa tggacccaga tgaagttcct 60
   ttggatgagc agtgtgagcg gctcccttat gatgccagca agtgggagtt tgcccgggag 120
   agacttaaac tgg 133
<210> 53
   <211> 133
   <212> DNA
   <213> Artificial Organism
<220>
   <223> Synthetic oligonucleotide
<400> 53
   ttgtgggctg catgcccagc ccaggcatgc agtggaaacg cagggcagga gagaggaggc 60
   cttcagagtg cagctggact tccttcccaa ccctcttgct tccttcagac cggggtcggg 120
   ttggcaaacc aag 133
<210> 54
   <211> 157
   <212> DNA
   <213> Artificial Organism
<220>
   <223> Synthetic oligonucleotide
<400> 54
   gatgcagcac tacacatgga gcctaagaaa gaaaaaatgg agccaggcct ggaacaaggc 60
   aagaaaccaa gactagatag cgtcaccagc agcgaaagct ttgcgagctc cggctttcag 120
   gaagataaaa gtctgagtga tgttgaggaa gaggagg 157
<210> 55
   <211> 123
   <212> DNA
   <213> Artificial Organism
<220>
   <223> Synthetic oligonucleotide
<400> 55
   tgcattcatc gggacctggc agcgagaaac attcttttat ctgagaacaa cgtggtgaag      60
   atttgtgatt ttggccttgc ccgggatatt tataagaacc ccgattatgt gagaaaagga     120
   gat                                                                   123
<210> 56
   <211> 682
   <212> DNA
   <213> Artificial Organism
<220>
   <223> Synthetic oligonucleotide
<400> 56
   tgatcagcta cacacaggaa ccttgctcac agccccggaa actccacact gtgggcttca      60
   ccttctggaa gtgacaaagt catgtacgca ggaccttccc caccagtctc cactgctgag     120
   cccataaagg gaggtgtgag gacttggaaa tgccacatgc tttttaagaa gccagctcaa     180
   accaaccttt gttacaactc gagaatgaaa taagtactac aaactattca gccattgata     240
   tttatggctg tatgactttt ttttttttta actttaagga catcgtccta cttctgtaag     300
   tgtggggagg agtactccac ttcacattga actgcagtga gcggtggggt ttcctttccg     360
   aattgctgtg tttcattcac agaactcaga ccttccatct gcagaggcca caggcacaac     420
   aaagaacctg ggtatccatg agctctggtg ggttggttag tctgccttgg tagacgtgtt     480
   ttccactgac cacaggacct ggcccagaca gccttttaag tgctggtgct ataaacccaa     540
   acctaaaaat gaagcagggt cacatagtac agaaagcttg ggctttatgc ggatgatgac     600
   agccctccct ttgtagtacg taaggcaatg cataggatga tcactgctct ccaactattt     660
   ctgttgctgt tttccccacc ag                                              682
<210> 57
   <211> 242
   <212> DNA
   <213> Artificial Organism
<220>
   <223> Synthetic oligonucleotide
<400> 57
   tgtaatcaca cacacacaca aaatacaagc ctggcgctag cactcgatta tgccattaaa      60
   taatatttag ccgtgtagcc atgccaggtc actttgccac ctcacatcct tttcagagca 120
   cctgataaag tcataccact tccctgcaca tcatttctct cctgtgccat tgggcactca 180
   gacgagatga tgcctccagt ctctcctatg tctggcattc tctgatttca caacggacca 240
   ga 242
<210> 58
   <211> 1005
   <212> DNA
   <213> Artificial Organism
<220>
   <223> Synthetic oligonucleotide
<400> 58
   gtcctgtact ccaccccacc catctagagt ttgacacgaa gccttatttc tagaagcaca 60
   tgtgtattta tacccccagg aaactagctt ttgccagtat tatgcatata taagtttaca 120
   cctttatctt tccatgggag ccagctgctt tttgtgattt ttttaatagt gctttttttt 180
   ttttgactaa caagaatgta actccagata gagaaatagt gacaagtgaa gaacactact 240
   gctaaatcct catgttactc agtgttagag aaatccttcc taaacccaat gacttccctg 300
   ctccaacccc cgccacctca gggcacgcag gaccagtttg attgaggagc tgcactgatc 360
   acccaatgca tcacgtaccc cactgggcca gccctgcagc ccaaaaccca gggcaacaag 420
   cccgttagcc ccagggatca ctggctggcc tgagcaacat ctcgggagtc ctctagcagg 480
   cctaagacat gtgaggagga aaaggaaaaa aagcaaaaag caagggagaa aagagaaacc 540
   gggagaaggc atgagaaaga atttgagacg caccatgtgg gcacggaggg ggacggggct 600
   cagcaatgcc atttcagtgg cttcccagct ctgacccttc tacatttgag ggcccagcca 660
   ggagcagatg gacagcgatg aggggacatt ttctggattc tgggaggcaa gaaaaggaca 720
   aatatctttt ttggaactaa agcaaatttt agaactttac ctatggaagt ggttctatgt 780
   ccattctcat tcgtggcatg ttttgatttg tagcactgag ggtggcactc aactctgagc 840
   ccatactttt ggctcctcta gtaagatgca ctgaaaactt agccagagtt aggttgtctc 900
   caggccatga tggccttaca ctgaaaatgt cacattctat tttgggtatt aatatatagt 960
   ccagacactt aactcaattt cttggtatta ttctgttttg cacag 1005
<210> 59
   <211> 168
   <212> DNA
   <213> Artificial Organism
<220>
   <223> Synthetic oligonucleotide
<400> 59
   gtcaataagg tcaagggaaa accccgtctc tataccaacc aaaccaattc accaacacag      60
   ttgggaccca aaacacagga agtcagtcac gtttcctttt catttaatgg ggattccact     120
   atctcacact aatctgaaag gatgtggaag agcattagct ggcgcata                  168
<210> 60
   <211> 286
   <212> DNA
   <213> Artificial Organism
<220>
   <223> Synthetic oligonucleotide
<400> 60
   tgtaatttat gcaaggtatt tctccagttg ggactcagga tattagttaa tgagccatca      60
   ctagaagaaa agcccatttt caactgcttt gaaacttgcc tggggtctga gcatgatggg     120
   aatagggaga cagggtagga aagggcgcct actcttcagg gtctaaagat caagtgggcc     180
   ttggatcgct aagctggctc tgtttgatgc tatttatgca agttagggtc tatgtattta     240
   tgatgtctgc accttctgca gccagtcaga agctggagag gcaaca                    286
<210> 61
   <211> 2015
   <212> DNA
   <213> Homo sapiens
<400> 61
   tgtactctct gaggtgctct ggaaggaggg gcagctccac cctgggaggg actgtggccc      60
   aggtactgcc cgggtgctac tttatgggca gcagctcagt tgagttagag tctggaagac     120
   ctcagaagac ctcctgtcct atgaggccct ccccatggct ttagctgact tgtatgaaga     180
   aggaggtgga ggaggaggag aaggtgaaga caatgctgac tcaaaggggg gattataaaa     240
   ctaatcatca aagccaagaa ggcaagagca agcatgtacc gctgaaaaca caagataact     300
   gcataagtaa tgactttcag tgcagattca tagctaaccc ataaactgct ggggcaaaaa     360
   tcatcttgga aggctctgaa cctcagaaag gattcacaag acgatctgcc gaccctctgg     420
   gagaaaatcc agcaagatgc aagccttcag aatctgggat gttaaccaga agaccttcta     480
   tctgaggaac aaccaactag ttgctggata cttgcaagga ccaaatgtca atttagaaga     540
   aaagatagat gtggtaccca ttgagcctca tgctctgttc ttgggaatcc atggagggaa     600
   gatgtgcctg tcctgtgtca agtctggtga tgagaccaga ctccagctgg aggcagttaa     660
   catcactgac ctgagcgaga acagaaagca ggacaagcgc ttcgccttca tccgctcaga     720
   cagtggcccc accaccagtt ttgagtctgc cgcctgcccc ggttggttcc tctgcacagc     780
   gatggaagct gaccagcccg tcagcctcac caatatgcct gacgaaggcg tcatggtcac     840
   caaattctac ttccaggagg acgagtagta ctgcccaggc ctgcctgttc ccattcttgc     900
   atggcaagga ctgcagggac tgccagtccc cctgccccag ggctcccggc tatgggggca 960
   ctgaggacca gccattgagg ggtggaccct cagaaggcgt cacaacaacc tggtcacagg 1020
   actctgcctc ctcttcaact gaccagcctc catgctgcct ccagaatggt ctttctaatg 1080
   tgtgaatcag agcacagcag cccctgcaca aagcccttcc atgtcgcctc tgcattcagg 1140
   atcaaacccc gaccacctgc ccaacctgct ctcctcttgc cactgcctct tcctccctca 1200
   ttccaccttc ccatgccctg gatccatcag gccacttgat gacccccaac caagtggctc 1260
   ccacaccctg ttttacaaaa aagaaaagac cagtccatga gggaggtttt taagggtttg 1320
   tggaaaatga aaattaggat ttcatgattt ttttttttca gtccccgtga aggagagccc 1380
   ttcatttgga gattatgttc tttcggggag aggctgagga cttaaaatat tcctgcattt 1440
   gtgaaatgat ggtgaaagta agtggtagct tttcccttct ttttcttctt tttttgtgat 1500
   gtcccaactt gtaaaaatta aaagttatgg tactatgtta gccccataat tttttttttc 1560
   cttttaaaac acttccataa tctggactcc tctgtccagg cactgctgcc cagcctccaa 1620
   gctccatctc cactccagat tttttacagc tgcctgcagt actttacctc ctatcagaag 1680
   tttctcagct cccaaggctc tgagcaaatg tggctcctgg gggttctttc ttcctctgct 1740
   gaaggaataa attgctcctt gacattgtag agcttctggc acttggagac ttgtatgaaa 1800
   gatggctgtg cctctgcctg tctcccccac cgggctggga gctctgcaga gcaggaaaca 1860
   tgactcgtat atgtctcagg tccctgcagg gccaagcacc tagcctcgct cttggcaggt 1920
   actcagcgaa tgaatgctgt atatgttggg tgcaaagttc cctacttcct gtgacttcag 1980
   ctctgtttta caataaaatc ttgaaaatgc ctata 2015
<210> 62
   <211> 3161
   <212> DNA
   <213> Homo sapiens
<400> 62
   tgtactctct gaggtgctct ggaaggaggg gcagctccac cctgggaggg actgtggccc     60
   aggtactgcc cgggtgctac tttatgggca gcagctcagt tgagttagag tctggaagac    120
   ctcagaagac ctcctgtcct atgaggccct ccccatggct ttagagacga tctgccgacc    180
   ctctgggaga aaatccagca agatgcaagc cttcagaatc tgggatgtta accagaagac    240
   cttctatctg aggaacaacc aactagttgc tggatacttg caaggaccaa atgtcaattt    300
   agaaggtgag tggttgccag gaaagccaat gtatgtgggc atcacgtcac tttgcccgtc    360
   tgtctgcagc agcatggcct gcctgcacaa accctaggtg caatgtccta atccttgttg    420
   ggtctttgta ttcaagtttg aagctgggag ggcctggcta ctgaagggca catatgaggg    480
   cagcctgaag agggtgtgga gaggtagagt ctaggtcaga ggtcagtgcc tataggcaca    540
   gtggtcccag ggccacagct gggaagggca aataccagaa ggcaaggttg accattccct    600
   tcctcaagtg cctattaagg ctccatgttc ctatgttgtt caaaccctaa ctcaatccca     660
   aattaatcca ccatgtataa ggttgagcta tgtctcttat tcctggacac catactcagc     720
   catattctgg tccacacatt aaacaagctg gatgaccttg aagaagcttc acccactctg     780
   ttcctcagct ttcccttcag tgggatgata tcaactggac aacaggatgt gcgattcttt     840
   tagttccagc cttccaggat gttttcactc ccctgtttgt tgttgtagga tggtattacc     900
   tccaccttcc caccttccct atgccctggt tctgtctcct gtgcctcgct ctgaaagtgg     960
   atgagaccta caattcctgt cctggtagtt ctcctaatga acacactgaa gcacgaggaa    1020
   gctgagattt ttgttgctac atgagagcat ggaggcctct tagggagaga ggaggttcag    1080
   agactcctag gctcctgtgg agccccactc atggccttgt tcattttccc tgcccctcag    1140
   caacactcct attgacctgg agcacaggta tcctggggaa agtgagggaa atatggacat    1200
   cacatggaac aacatccagg agactcaggc ctctaggagt aactgggtag tgtgcatcct    1260
   ggggaaagtg agggaaatat ggacatcaca tggaacaaca tccaggagac tcaggcctct    1320
   aggagtaact gggtagtgtg catcctgggg aaagtgaggg aaatatggac atcacatgga    1380
   acaacatcca ggagactcag gcctctagga gtaactgggt agtgtgcatc ctggggaaag    1440
   tgagggaaat atggacatca catggaacaa catccaggag actcaggcct ctaggagtaa    1500
   ctgggtagtg tgcttggttt aatcttctgt ttagctgcag accaggaaga tgagacctct    1560
   ctgcccttct gacctcggga ttttagtttt gtggggacca ggggagatag aaaaataccc    1620
   agggtctctt cattattgct gcctcctctt ctattaacct gaccctcccc tctgttcttc    1680
   cccagaaaag atagatgtgg tacccattga gcctcatgct ctgttcttgg gaatccatgg    1740
   agggaagatg tgcctgtcct gtgtcaagtc tggtgatgag accagactcc agctggaggc    1800
   agttaacatc actgacctga gcgagaacag aaagcaggac aagcgcttcg ccttcatccg    1860
   ctcagacagt ggccccacca ccagttttga gtctgccgcc tgccccggtt ggttcctctg    1920
   cacagcgatg gaagctgacc agcccgtcag cctcaccaat atgcctgacg aaggcgtcat    1980
   ggtcaccaaa ttctacttcc aggaggacga gtagtactgc ccaggcctgc ctgttcccat    2040
   tcttgcatgg caaggactgc agggactgcc agtccccctg ccccagggct cccggctatg    2100
   ggggcactga ggaccagcca ttgaggggtg gaccctcaga aggcgtcaca acaacctggt    2160
   cacaggactc tgcctcctct tcaactgacc agcctccatg ctgcctccag aatggtcttt    2220
   ctaatgtgtg aatcagagca cagcagcccc tgcacaaagc ccttccatgt cgcctctgca    2280
   ttcaggatca aaccccgacc acctgcccaa cctgctctcc tcttgccact gcctcttcct    2340
   ccctcattcc accttcccat gccctggatc catcaggcca cttgatgacc cccaaccaag    2400
   tggctcccac accctgtttt acaaaaaaga aaagaccagt ccatgaggga ggtttttaag    2460
   ggtttgtgga aaatgaaaat taggatttca tgattttttt ttttcagtcc ccgtgaagga    2520
   gagcccttca tttggagatt atgttctttc ggggagaggc tgaggactta aaatattcct 2580
   gcatttgtga aatgatggtg aaagtaagtg gtagcttttc ccttcttttt cttctttttt 2640
   tgtgatgtcc caacttgtaa aaattaaaag ttatggtact atgttagccc cataattttt 2700
   tttttccttt taaaacactt ccataatctg gactcctctg tccaggcact gctgcccagc 2760
   ctccaagctc catctccact ccagattttt tacagctgcc tgcagtactt tacctcctat 2820
   cagaagtttc tcagctccca aggctctgag caaatgtggc tcctgggggt tctttcttcc 2880
   tctgctgaag gaataaattg ctccttgaca ttgtagagct tctggcactt ggagacttgt 2940
   atgaaagatg gctgtgcctc tgcctgtctc ccccaccggg ctgggagctc tgcagagcag 3000
   gaaacatgac tcgtatatgt ctcaggtccc tgcagggcca agcacctagc ctcgctcttg 3060
   gcaggtactc agcgaatgaa tgctgtatat gttgggtgca aagttcccta cttcctgtga 3120
   cttcagctct gttttacaat aaaatcttga aaatgcctat a 3161
<210> 63
   <211> 3436
   <212> DNA
   <213> Homo sapiens
<400> 63
   gcctttctgc tcaaatctgt tttttaaaaa gtcaacaaac agactctggg tacctgtcag      60
   gaacagtagg gagtttggtt tccattgtgc tcttcttccc aggaactcaa tgaaggggaa     120
   atagaaatct taattttggg gaaattgcac aggggaaaaa ggggagggaa tcagttacaa     180
   cactccattg cgacacttag tggggttgaa agtgacaaca gcaagggttt ctctttttgg     240
   aaatgcgagg agggtatttc cgcttctcgc agtggggcag ggtggcagac gcctagcttg     300
   ggtgagtgac tatttcttta taaaccacaa ctctgggccc gcaatggcag tccactgcct     360
   tgctgcagtc acagaatgga aatctgcaga ggcctccgca gtcacctaat cactctcctc     420
   ctcttcctgt tccattcaga gacgatctgc cgaccctctg ggagaaaatc cagcaagatg     480
   caagccttca gaatctggga tgttaaccag aagaccttct atctgaggaa caaccaacta     540
   gttgctggat acttgcaagg accaaatgtc aatttagaag gtgagtggtt gccaggaaag     600
   ccaatgtatg tgggcatcac gtcactttgc ccgtctgtct gcagcagcat ggcctgcctg     660
   cacaaaccct aggtgcaatg tcctaatcct tgttgggtct ttgtattcaa gtttgaagct     720
   gggagggcct ggctactgaa gggcacatat gagggcagcc tgaagagggt gtggagaggt     780
   agagtctagg tcagaggtca gtgcctatag gcacagtggt cccagggcca cagctgggaa     840
   gggcaaatac cagaaggcaa ggttgaccat tcccttcctc aagtgcctat taaggctcca     900
   tgttcctatg ttgttcaaac cctaactcaa tcccaaatta atccaccatg tataaggttg     960
   agctatgtct cttattcctg gacaccatac tcagccatat tctggtccac acattaaaca    1020
   agctggatga ccttgaagaa gcttcaccca ctctgttcct cagctttccc ttcagtggga    1080
   tgatatcaac tggacaacag gatgtgcgat tcttttagtt ccagccttcc aggatgtttt 1140
   cactcccctg tttgttgttg taggatggta ttacctccac cttcccacct tccctatgcc 1200
   ctggttctgt ctcctgtgcc tcgctctgaa agtggatgag acctacaatt cctgtcctgg 1260
   tagttctcct aatgaacaca ctgaagcacg aggaagctga gatttttgtt gctacatgag 1320
   agcatggagg cctcttaggg agagaggagg ttcagagact cctaggctcc tgtggagccc 1380
   cactcatggc cttgttcatt ttccctgccc ctcagcaaca ctcctattga cctggagcac 1440
   aggtatcctg gggaaagtga gggaaatatg gacatcacat ggaacaacat ccaggagact 1500
   caggcctcta ggagtaactg ggtagtgtgc atcctgggga aagtgaggga aatatggaca 1560
   tcacatggaa caacatccag gagactcagg cctctaggag taactgggta gtgtgcatcc 1620
   tggggaaagt gagggaaata tggacatcac atggaacaac atccaggaga ctcaggcctc 1680
   taggagtaac tgggtagtgt gcatcctggg gaaagtgagg gaaatatgga catcacatgg 1740
   aacaacatcc aggagactca ggcctctagg agtaactggg tagtgtgctt ggtttaatct 1800
   tctgtttagc tgcagaccag gaagatgaga cctctctgcc cttctgacct cgggatttta 1860
   gttttgtggg gaccagggga gatagaaaaa tacccagggt ctcttcatta ttgctgcctc 1920
   ctcttctatt aacctgaccc tcccctctgt tcttccccag aaaagataga tgtggtaccc 1980
   attgagcctc atgctctgtt cttgggaatc catggaggga agatgtgcct gtcctgtgtc 2040
   aagtctggtg atgagaccag actccagctg gaggcagtta acatcactga cctgagcgag 2100
   aacagaaagc aggacaagcg cttcgccttc atccgctcag acagtggccc caccaccagt 2160
   tttgagtctg ccgcctgccc cggttggttc ctctgcacag cgatggaagc tgaccagccc 2220
   gtcagcctca ccaatatgcc tgacgaaggc gtcatggtca ccaaattcta cttccaggag 2280
   gacgagtagt actgcccagg cctgcctgtt cccattcttg catggcaagg actgcaggga 2340
   ctgccagtcc ccctgcccca gggctcccgg ctatgggggc actgaggacc agccattgag 2400
   gggtggaccc tcagaaggcg tcacaacaac ctggtcacag gactctgcct cctcttcaac 2460
   tgaccagcct ccatgctgcc tccagaatgg tctttctaat gtgtgaatca gagcacagca 2520
   gcccctgcac aaagcccttc catgtcgcct ctgcattcag gatcaaaccc cgaccacctg 2580
   cccaacctgc tctcctcttg ccactgcctc ttcctccctc attccacctt cccatgccct 2640
   ggatccatca ggccacttga tgacccccaa ccaagtggct cccacaccct gttttacaaa 2700
   aaagaaaaga ccagtccatg agggaggttt ttaagggttt gtggaaaatg aaaattagga 2760
   tttcatgatt tttttttttc agtccccgtg aaggagagcc cttcatttgg agattatgtt 2820
   ctttcgggga gaggctgagg acttaaaata ttcctgcatt tgtgaaatga tggtgaaagt 2880
   aagtggtagc ttttcccttc tttttcttct ttttttgtga tgtcccaact tgtaaaaatt 2940
   aaaagttatg gtactatgtt agccccataa tttttttttt ccttttaaaa cacttccata 3000
   atctggactc ctctgtccag gcactgctgc ccagcctcca agctccatct ccactccaga 3060
   ttttttacag ctgcctgcag tactttacct cctatcagaa gtttctcagc tcccaaggct 3120
   ctgagcaaat gtggctcctg ggggttcttt cttcctctgc tgaaggaata aattgctcct 3180
   tgacattgta gagcttctgg cacttggaga cttgtatgaa agatggctgt gcctctgcct 3240
   gtctccccca ccgggctggg agctctgcag agcaggaaac atgactcgta tatgtctcag 3300
   gtccctgcag ggccaagcac ctagcctcgc tcttggcagg tactcagcga atgaatgctg 3360
   tatatgttgg gtgcaaagtt ccctacttcc tgtgacttca gctctgtttt acaataaaat 3420
   cttgaaaatg cctata 3436
<210> 64
   <211> 2051
   <212> DNA
   <213> Homo sapiens
<400> 64
   gcctttctgc tcaaatctgt tttttaaaaa gtcaacaaac agactctggg tacctgtcag      60
   gaacagtagg gagtttggtt tccattgtgc tcttcttccc aggaactcaa tgaaggggaa     120
   atagaaatct taattttggg gaaattgcac aggggaaaaa ggggagggaa tcagttacaa     180
   cactccattg cgacacttag tggggttgaa agtgacaaca gcaagggttt ctctttttgg     240
   aaatgcgagg agggtatttc cgcttctcgc agtggggcag ggtggcagac gcctagcttg     300
   ggtgagtgac tatttcttta taaaccacaa ctctgggccc gcaatggcag tccactgcct     360
   tgctgcagtc acagaatgga aatctgcaga ggcctccgca gtcacctaat cactctcctc     420
   ctcttcctgt tccattcaga gacgatctgc cgaccctctg ggagaaaatc cagcaagatg     480
   caagccttca gaatctggga tgttaaccag aagaccttct atctgaggaa caaccaacta     540
   gttgctggat acttgcaagg accaaatgtc aatttagaag atagatgtgg tacccattga     600
   gcctcatgct ctgttcttgg gaatccatgg agggaagatg tgcctgtcct gtgtcaagtc     660
   tggtgatgag accagactcc agctggaggc agttaacatc actgacctga gcgagaacag     720
   aaagcaggac aagcgcttcg ccttcatccg ctcagacagt ggccccacca ccagttttga     780
   gtctgccgcc tgccccggtt ggttcctctg cacagcgatg gaagctgacc agcccgtcag     840
   cctcaccaat atgcctgacg aaggcgtcat ggtcaccaaa ttctacttcc aggaggacga     900
   gtagtactgc ccaggcctgc ctgttcccat tcttgcatgg caaggactgc agggactgcc     960
   agtccccctg ccccagggct cccggctatg ggggcactga ggaccagcca ttgaggggtg    1020
   gaccctcaga aggcgtcaca acaacctggt cacaggactc tgcctcctct tcaactgacc    1080
   agcctccatg ctgcctccag aatggtcttt ctaatgtgtg aatcagagca cagcagcccc    1140
   tgcacaaagc ccttccatgt cgcctctgca ttcaggatca aaccccgacc acctgcccaa    1200
   cctgctctcc tcttgccact gcctcttcct ccctcattcc accttcccat gccctggatc    1260
   catcaggcca cttgatgacc cccaaccaag tggctcccac accctgtttt acaaaaaaga 1320
   aaagaccagt ccatgaggga ggtttttaag ggtttgtgga aaatgaaaat taggatttca 1380
   tgattttttt ttttcagtcc ccgtgaagga gagcccttca tttggagatt atgttctttc 1440
   ggggagaggc tgaggactta aaatattcct gcatttgtga aatgatggtg aaagtaagtg 1500
   gtagcttttc ccttcttttt cttctttttt tgtgatgtcc caacttgtaa aaattaaaag 1560
   ttatggtact atgttagccc cataattttt tttttccttt taaaacactt ccataatctg 1620
   gactcctctg tccaggcact gctgcccagc ctccaagctc catctccact ccagattttt 1680
   tacagctgcc tgcagtactt tacctcctat cagaagtttc tcagctccca aggctctgag 1740
   caaatgtggc tcctgggggt tctttcttcc tctgctgaag gaataaattg ctccttgaca 1800
   ttgtagagct tctggcactt ggagacttgt atgaaagatg gctgtgcctc tgcctgtctc 1860
   ccccaccggg ctgggagctc tgcagagcag gaaacatgac tcgtatatgt ctcaggtccc 1920
   tgcagggcca agcacctagc ctcgctcttg gcaggtactc agcgaatgaa tgctgtatat 1980
   gttgggtgca aagttcccta cttcctgtga cttcagctct gttttacaat aaaatcttga 2040
   aaatgcctat a 2051
<210> 65
   <211> 64
   <212> PRT
   <213> Homo sapiens
<400> 65
   Met Gln Ala Phe Arg Ile Trp Asp Val Asn Gln Lys Thr Phe Tyr Leu
   1 5 10 15
   Arg Asn Asn Gln Leu Val Ala Gly Tyr Leu Gln Gly Pro Asn Val Asn
   20 25 30
   Leu Glu Gly Glu Trp Leu Pro Gly Lys Pro Met Tyr Val Gly Ile Thr
   35 40 45
   Ser Leu Cys Pro Ser Val Cys Ser Ser Met Ala Cys Leu His Lys Pro
   50 55 60
<210> 66
   <211> 98
   <212> PRT
   <213> Homo sapiens
<400> 66
   Met Glu Ile Cys Arg Gly Leu Arg Ser His Leu Ile Thr Leu Leu Leu
   1 5 10 15
   Phe Leu Phe His Ser Glu Thr Ile Cys Arg Pro Ser Gly Arg Lys Ser
   20 25 30
   Ser Lys Met Gln Ala Phe Arg Ile Trp Asp Val Asn Gln Lys Thr Phe
   35 40 45
   Tyr Leu Arg Asn Asn Gln Leu Val Ala Gly Tyr Leu Gln Gly Pro Asn
   50 55 60
   Val Asn Leu Glu Gly Glu Trp Leu Pro Gly Lys Pro Met Tyr Val Gly
   65 70 75 80
   Ile Thr Ser Leu Cys Pro Ser Val Cys Ser Ser Met Ala Cys Leu His
   85 90 95
   Lys Pro
<210> 67
   <211> 80
   <212> PRT
   <213> Homo sapiens
<400> 67
   Met Ala Leu Glu Thr Ile Cys Arg Pro Ser Gly Arg Lys Ser Ser Lys
   1 5 10 15
   Met Gln Ala Phe Arg Ile Trp Asp Val Asn Gln Lys Thr Phe Tyr Leu
   20 25 30
   Arg Asn Asn Gln Leu Val Ala Gly Tyr Leu Gln Gly Pro Asn Val Asn
   35 40 45
   Leu Glu Gly Glu Trp Leu Pro Gly Lys Pro Met Tyr Val Gly Ile Thr
   50 55 60
   Ser Leu Cys Pro Ser Val Cys Ser Ser Met Ala Cys Leu His Lys Pro
   65 70 75 80
<210> 68
   <211> 27
   <212> PRT
   <213> Homo sapiens
<400> 68
   Met Ala Leu Ala Asp Leu Tyr Glu Glu Gly Gly Gly Gly Gly Gly Glu
   1 5 10 15
   Gly Glu Asp Asn Ala Asp Ser Lys Gly Gly Leu
   20 25
<210> 69
   <211> 74
   <212> PRT
   <213> Homo sapiens
<400> 69
   Met Glu Ile Cys Arg Gly Leu Arg Ser His Leu Ile Thr Leu Leu Leu
   1 5 10 15
   Phe Leu Phe His Ser Glu Thr Ile Cys Arg Pro Ser Gly Arg Lys Ser
   20 25 30
   Ser Lys Met Gln Ala Phe Arg Ile Trp Asp Val Asn Gln Lys Thr Phe
   35 40 45
   Tyr Leu Arg Asn Asn Gln Leu Val Ala Gly Tyr Leu Gln Gly Pro Asn
   50 55 60
   Val Asn Leu Glu Asp Arg Cys Gly Thr His
   65 70
<210> 70
   <211> 40
   <212> PRT
   <213> Homo sapiens
<400> 70
   Met Gln Ala Phe Arg Ile Trp Asp Val Asn Gln Lys Thr Phe Tyr Leu
   1 5 10 15
   Arg Asn Asn Gln Leu Val Ala Gly Tyr Leu Gln Gly Pro Asn Val Asn
   20 25 30
   Leu Glu Asp Arg Cys Gly Thr His
   35 40
<210> 71
   <211> 164
   <212> DNA
   <213> Artificial Organism
<220>
   <223> Synthetic oligonucleotide
<400> 71
   tgtactctct gaggtgctct ggaaggaggg gcagctccac cctgggaggg actgtggccc 60
   aggtactgcc cgggtgctac tttatgggca gcagctcagt tgagttagag tctggaagac 120
   ctcagaagac ctcctgtcct atgaggccct ccccatggct ttag 164
<210> 72
   <211> 63
   <212> DNA
   <213> Artificial Organism
<220>
   <223> Synthetic oligonucleotide
<400> 72
   ctgacttgta tgaagaagga ggtggaggag gaggagaagg tgaagacaat gctgactcaa 60
   agg 63
<210> 73
   <211> 171
   <212> DNA
   <213> Artificial Organism
<220>
   <223> Synthetic oligonucleotide
<400> 73
   ggggattata aaactaatca tcaaagccaa gaaggcaaga gcaagcatgt accgctgaaa 60
   acacaagata actgcataag taatgacttt cagtgcagat tcatagctaa cccataaact 120
   gctggggcaa aaatcatctt ggaaggctct gaacctcaga aaggattcac a 171
<210> 74
   <211> 301
   <212> DNA
   <213> Artificial Organism
<220>
   <223> Synthetic oligonucleotide
<400> 74
   gcctttctgc tcaaatctgt tttttaaaaa gtcaacaaac agactctggg tacctgtcag 60
   gaacagtagg gagtttggtt tccattgtgc tcttcttccc aggaactcaa tgaaggggaa 120
   atagaaatct taattttggg gaaattgcac aggggaaaaa ggggagggaa tcagttacaa 180
   cactccattg cgacacttag tggggttgaa agtgacaaca gcaagggttt ctctttttgg 240
   aaatgcgagg agggtatttc cgcttctcgc agtggggcag ggtggcagac gcctagcttg 300
   g 301
<210> 75
   <211> 138
   <212> DNA
   <213> Artificial Organism
<220>
   <223> Synthetic oligonucleotide
<400> 75
   gtgagtgact atttctttat aaaccacaac tctgggcccg caatggcagt ccactgcctt 60
   gctgcagtca cagaatggaa atctgcagag gcctccgcag tcacctaatc actctcctcc 120
   tcttcctgtt ccattcag 138

<210> 76
   <211> 52
   <212> DNA
   <213> Artificial Organism
<220>
   <223> Synthetic oligonucleotide
<400> 76
   agacgatctg ccgaccctct gggagaaaat ccagcaagat gcaagccttc ag 52
<210> 77
   <211> 89
   <212> DNA
   <213> Artificial Organism
<220>
   <223> Synthetic oligonucleotide
<400> 77
   aatctgggat gttaaccaga agaccttcta tctgaggaac aaccaactag ttgctggata 60
   cttgcaagga ccaaatgtca atttagaag 89
<210> 78
   <211> 898
   <212> DNA
   <213> Artificial Organism
<220>
   <223> Synthetic oligonucleotide
<400> 78
   gtgagtggtt gccaggaaag ccaatgtatg tgggcatcac gtcactttgc ccgtctgtct      60
   gcagcagcat ggcctgcctg cacaaaccct aggtgcaatg tcctaatcct tgttgggtct     120
   ttgtattcaa gtttgaagct gggagggcct ggctactgaa gggcacatat gagggcagcc     180
   tgaagagggt gtggagaggt agagtctagg tcagaggtca gtgcctatag gcacagtggt     240
   cccagggcca cagctgggaa gggcaaatac cagaaggcaa ggttgaccat tcccttcctc     300
   aagtgcctat taaggctcca tgttcctatg ttgttcaaac cctaactcaa tcccaaatta     360
   atccaccatg tataaggttg agctatgtct cttattcctg gacaccatac tcagccatat     420
   tctggtccac acattaaaca agctggatga ccttgaagaa gcttcaccca ctctgttcct     480
   cagctttccc ttcagtggga tgatatcaac tggacaacag gatgtgcgat tcttttagtt     540
   ccagccttcc aggatgtttt cactcccctg tttgttgttg taggatggta ttacctccac     600
   cttcccacct tccctatgcc ctggttctgt ctcctgtgcc tcgctctgaa agtggatgag     660
   acctacaatt cctgtcctgg tagttctcct aatgaacaca ctgaagcacg aggaagctga     720
   gatttttgtt gctacatgag agcatggagg cctcttaggg agagaggagg ttcagagact     780
   cctaggctcc tgtggagccc cactcatggc cttgttcatt ttccctgccc ctcagcaaca     840
   ctcctattga cctggagcac aggtatcctg gggaaagtga gggaaatatg gacatcac       898
<210> 79
   <211> 86
   <212> DNA
   <213> Artificial Organism
<220>
   <223> Synthetic oligonucleotide
<400> 79
   atggaacaac atccaggaga ctcaggcctc taggagtaac tgggtagtgt gcatcctggg      60
   gaaagtgagg gaaatatgga catcac                                           86
<210> 80
   <211> 86
   <212> DNA
   <213> Artificial Organism
<220>
   <223> Synthetic oligonucleotide
<400> 80
   atggaacaac atccaggaga ctcaggcctc taggagtaac tgggtagtgt gcatcctggg 60
   gaaagtgagg gaaatatgga catcac 86
<210> 81
   <211> 86
   <212> DNA
   <213> Artificial Organism
<220>
   <223> Synthetic oligonucleotide
<400> 81
   atggaacaac atccaggaga ctcaggcctc taggagtaac tgggtagtgt gcatcctggg 60
   gaaagtgagg gaaatatgga catcac 86
<210> 82
   <211> 224
   <212> DNA
   <213> Artificial Organism
<220>
   <223> Synthetic oligonucleotide
<400> 82
   atggaacaac atccaggaga ctcaggcctc taggagtaac tgggtagtgt gcttggttta 60
   atcttctgtt tagctgcaga ccaggaagat gagacctctc tgcccttctg acctcgggat 120
   tttagttttg tggggaccag gggagataga aaaataccca gggtctcttc attattgctg 180
   cctcctcttc tattaacctg accctcccct ctgttcttcc ccag 224
<210> 83
   <211> 5
   <212> DNA
   <213> Artificial Organism
<220>
   <223> Synthetic oligonucleotide
<400> 83
   aaaag 5
<210> 84
   <211> 102
   <212> DNA
   <213> Artificial Organism
<220>
   <223> Synthetic oligonucleotide
<400> 84
   atagatgtgg tacccattga gcctcatgct ctgttcttgg gaatccatgg agggaagatg 60
   tgcctgtcct gtgtcaagtc tggtgatgag accagactcc ag 102
<210> 85
   <211> 6
   <212> DNA
   <213> Artificial Organism
<220>
   <223> Synthetic oligonucleotide
<400> 85
   ctggag 6
<210> 86
   <211> 4
   <212> DNA
   <213> Artificial Organism
<220>
   <223> Synthetic oligonucleotide
<400> 86
   gcag 4
<210> 87
   <211> 76
   <212> DNA
   <213> Artificial Organism
<220>
   <223> Synthetic oligonucleotide
<400> 87
   ttaacatcac tgacctgagc gagaacagaa agcaggacaa gcgcttcgcc ttcatccgct 60
   cagacagtgg ccccac 76
<210> 88
   <211> 78
   <212> DNA
   <213> Artificial Organism
<220>
   <223> Synthetic oligonucleotide
<400> 88
   caccagtttt gagtctgccg cctgccccgg ttggttcctc tgcacagcga tggaagctga 60
   ccagcccgtc agcctcac 78
<210> 89
   <211> 248
   <212> DNA
   <213> Artificial Organism
<220>
   <223> Synthetic oligonucleotide
<400> 89
   caatatgcct gacgaaggcg tcatggtcac caaattctac ttccaggagg acgagtagta      60
   ctgcccaggc ctgcctgttc ccattcttgc atggcaagga ctgcagggac tgccagtccc     120
   cctgccccag ggctcccggc tatgggggca ctgaggacca gccattgagg ggtggaccct     180
   cagaaggcgt cacaacaacc tggtcacagg actctgcctc ctcttcaact gaccagcctc     240
   catgctgc                                                              248
<210> 90
   <211> 399
   <212> DNA
   <213> Artificial Organism
<220>
   <223> Synthetic oligonucleotide
<400> 90
   ctccagaatg gtctttctaa tgtgtgaatc agagcacagc agcccctgca caaagccctt      60
   ccatgtcgcc tctgcattca ggatcaaacc ccgaccacct gcccaacctg ctctcctctt     120
   gccactgcct cttcctccct cattccacct tcccatgccc tggatccatc aggccacttg     180
   atgaccccca accaagtggc tcccacaccc tgttttacaa aaaagaaaag accagtccat     240
   gagggaggtt tttaagggtt tgtggaaaat gaaaattagg atttcatgat tttttttttt     300
   cagtccccgt gaaggagagc ccttcatttg gagattatgt tctttcgggg agaggctgag     360
   gacttaaaat attcctgcat ttgtgaaatg atggtgaaa                            399
<210> 91
   <211> 142
   <212> DNA
   <213> Artificial Organism
<220>
   <223> Synthetic oligonucleotide
<400> 91
   gtaagtggta gcttttccct tctttttctt ctttttttgt gatgtcccaa cttgtaaaaa 60
   ttaaaagtta tggtactatg ttagccccat aatttttttt ttccttttaa aacacttcca 120
   taatctggac tcctctgtcc ag 142
<210> 92
   <211> 139
   <212> DNA
   <213> Artificial Organism
<220>
   <223> Synthetic oligonucleotide
<400> 92
   gcactgctgc ccagcctcca agctccatct ccactccaga ttttttacag ctgcctgcag 60
   tactttacct cctatcagaa gtttctcagc tcccaaggct ctgagcaaat gtggctcctg 120
   ggggttcttt cttcctctg 139
<210> 93
   <211> 79
   <212> DNA
   <213> Artificial Organism
<220>
   <223> Synthetic oligonucleotide
<400> 93
   ctgaaggaat aaattgctcc ttgacattgt agagcttctg gcacttggag acttgtatga 60
   aagatggctg tgcctctgc 79
<210> 94
   <211> 29
   <212> DNA
   <213> Artificial Organism
<220>
   <223> Synthetic oligonucleotide
<400> 94
   ctgtctcccc caccgggctg ggagctctg 29
<210> 95
   <211> 169
   <212> DNA
   <213> Artificial Organism
<220>
   <223> Synthetic oligonucleotide
<400> 95
   cagagcagga aacatgactc gtatatgtct caggtccctg cagggccaag cacctagcct 60
   cgctcttggc aggtactcag cgaatgaatg ctgtatatgt tgggtgcaaa gttccctact 120
   tcctgtgact tcagctctgt tttacaataa aatcttgaaa atgcctata 169
<210> 96
   <211> 1943
   <212> DNA
   <213> Homo sapiens
<400> 96
   cctcgcacgc actgcgggct ccggcgctgc gggctggccg ggcgctgcgg gctgaccggg 60
   cgctccggga actcggctcg ggaacctcgt ctgcggtggg cggggccggc ccggagcccc 120
   gccccggctc agtccctgaa acccaggcgc ggaccggctg cagtctcaga agggagctgc 180
   tgtctgcgga ggaaactgca tcgacggacg gccgcccagc tacgggagga cctggagtgg 240
   cactgggcgc ccgacggacc atccccggga cccgcctgcc cctcggcgcc ccgccccgcc 300
   gggccgctcc ccgtcgggtt ccccagccac agccttacct acgggctcct gactccgcaa 360
   ggcttccaga agatgctcga accaccggcc ggggcctcgg ggcagcagtg agggaggcgt 420
   ccagcccccc actcagctct tctcctcctg tgccaggggc tccccggggg atgagcatgg 480
   tggttttccc tcggagcccc ctggctcggg acgtctgaga agatgccggt catgaggctg 540
   ttcccttgct tcctgcagct cctggccggg ctggcgctgc ctgctgtgcc cccccagcag 600
   tgggccttgt ctgctgggaa cggctcgtca gaggtggaag tggtaccctt ccaggaagtg 660
   tggggccgca gctactgccg ggcgctggag aggctggtgg acgtcgtgtc cgagtacccc 720
   agcgaggtgg agcacatgtt cagcccatcc tgtgtctccc tgctgcgctg caccggctgc 780
   tgcggcgatg agaatctgca ctgtgtgccg gtggagacgg ccaatgtcac catgcagctc 840
   ctaaagatcc gttctgggga ccggccctcc tacgtggagc tgacgttctc tcagcacgtt 900
   cgctgcgaat gccggcctct gcgggagaag atgaagccgg aaagccacct tgtcctgacg    960
   cttggcttat tgcaggagga gacccaaggg cagggggaag aggaggagag agaagcagag    1020
   acccacagac tgccacctgt gcggcgatgc tgttccccgg aggtaaccca ccccttggag    1080
   gagagagacc ccgcacccgg ctcgtgtatt tattaccgtc acactcttca gtgactcctg    1140
   ctggtacctg ccctctattt attagccaac tgtttccctg ctgaatgcct cgctcccttc    1200
   aagacgaggg gcagggaagg acaggaccct caggaattca gtgccttcaa caacgtgaga    1260
   gaaagagaga agccagccac agacccctgg gagcttccgc tttgaaagaa gcaagacacg    1320
   tggcctcgtg aggggcaagc taggccccag aggccctgga ggtctccagg ggcctgcaga    1380
   aggaaagaag ggggccctgc tacctgttct tgggcctcag gctctgcaca gtcaagcagc    1440
   ccttgctttc ggagctcctg tccaaagtag ggatgcggat cctgctgggg ccgccacggc    1500
   ctggctggtg ggaaggccgg cagcgggcgg aggggatcca gccacttccc cctcttcttc    1560
   tgaagatcag aacattcagc tctggagaac agtggttgcc tgggggcttt tgccactcct    1620
   tgtcccccgt gatctcccct cacactttgc catttgcttg tactgggaca ttgttctttc    1680
   cggccaaggt gccaccaccc tgccccccct aagagacaca tacagagtgg gccccgggct    1740
   ggagaaagag ctgcctggat gagaaacagc tcagccagtg gggatgaggt caccagggga    1800
   ggagcctgtg cgtcccagct gaaggcagtg gcaggggagc aggttcccca agggccctgg    1860
   cacccccaca agctgtccct gcagggccat ctgactgcca agccagattc tcttgaataa    1920
   agtattctag tgtggaaacg ctg                                            1943
<210> 97
   <211> 2215
   <212> DNA
   <213> Homo sapiens
<400> 97
   ctggagccct ggtggctttg ctaaaagcat ctgtccacta atcccaggcg ggtccctggc 60
   cttgtggtca gggtcgaggc cccatggcca accaggcgtc cccaggacct ctgacctggc 120
   ctggtggggg tggcggagga gccagctgaa gaagctgttg gtggggaggc tggccctgcc 180
   cccaccaggg ctcaggactg agccgtgggc tcctgggtgg gctggggacg agacaagggg 240
   tctcgctgtg ccttttctcc tgggtgggtg gccggaggtc ctgagtgcag ggcctgggga 300
   agtgagcagg gcccttttcc caggagccgg gtgagtctgg ctgggagcca gcttgggcag 360
   tgaaatcctg acaaagccaa tgaactgcct tcccactgcg ttgtcccact gccctgggag 420
   tccccagccc cagcccaaga gtaggagtgg gctgggcagt ccctgaggag tccgagcagc 480
   ccagccaccc gcctgcctgc tgcagggctg gccagaggtg taacgttacc ggctgcacca 540
   gctcatggcg ccacccgctc cagcccctca gctcccacca gctgatcacc tccccctggg 600
   ctctggcacc cagccaaggc gggctgtgca gggcccagca gggttggcct gggggctggg 660
   tagaaggcag acccgcagga ttggaagatc tttgggatgc agcaggccaa gggtctccaa 720
   aagcttctag cctcccatcc ttttccaaat gaggaaaact gaggccagag aggggtggga 780
   cctaacctac ccagggtcca gtgagttttt agtggtactc aggctgctct gagacagggc 840
   agagcatgtg cagcaagtgg gaaggaaccc agaggggccc ctaccttctt gccatcacac 900
   ttggcccaag agtgcagagc tccaaggtac ccccagctca cctctgcact tttctgtcca 960
   tagcagtggg ccttgtctgc tgggaacggc tcgtcagagg tggaagtggt acccttccag 1020
   gaagtgtggg gccgcagcta ctgccgggcg ctggagaggc tggtggacgt cgtgtccgag 1080
   taccccagcg aggtggagca catgttcagc ccatcctgtg tctccctgct gcgctgcacc 1140
   ggctgctgcg gcgatgagaa tctgcactgt gtgccggtgg agacggccaa tgtcaccatg 1200
   cagctcctaa agatccgttc tggggaccgg ccctcctacg tggagctgac gttctctcag 1260
   cacgttcgct gcgaatgccg gcctctgcgg gagaagatga agccggaaag gtgcggcgat 1320
   gctgttcccc ggaggtaacc caccccttgg aggagagaga ccccgcaccc ggctcgtgta 1380
   tttattaccg tcacactctt cagtgactcc tgctggtacc tgccctctat ttattagcca 1440
   actgtttccc tgctgaatgc ctcgctccct tcaagacgag gggcagggaa ggacaggacc 1500
   ctcaggaatt cagtgccttc aacaacgtga gagaaagaga gaagccagcc acagacccct 1560
   gggagcttcc gctttgaaag aagcaagaca cgtggcctcg tgaggggcaa gctaggcccc 1620
   agaggccctg gaggtctcca ggggcctgca gaaggaaaga agggggccct gctacctgtt 1680
   cttgggcctc aggctctgca cagtcaagca gcccttgctt tcggagctcc tgtccaaagt 1740
   agggatgcgg atcctgctgg ggccgccacg gcctggctgg tgggaaggcc ggcagcgggc 1800
   ggaggggatc cagccacttc cccctcttct tctgaagatc agaacattca gctctggaga 1860
   acagtggttg cctgggggct tttgccactc cttgtccccc gtgatctccc ctcacacttt 1920
   gccatttgct tgtactggga cattgttctt tccggccaag gtgccaccac cctgcccccc 1980
   ctaagagaca catacagagt gggccccggg ctggagaaag agctgcctgg atgagaaaca 2040
   gctcagccag tggggatgag gtcaccaggg gaggagcctg tgcgtcccag ctgaaggcag 2100
   tggcagggga gcaggttccc caagggccct ggcaccccca caagctgtcc ctgcagggcc 2160
   atctgactgc caagccagat tctcttgaat aaagtattct agtgtggaaa cgctg 2215
<210> 98
   <211> 2015
   <212> DNA
   <213> Homo sapiens
<400> 98
   cctcgcacgc actgcgggct ccggcgctgc gggctggccg ggcgctgcgg gctgaccggg 60
   cgctccggga actcggctcg ggaacctcgt ctgcggtggg cggggccggc ccggagcccc 120
   gccccggctc agtccctgaa acccaggcgc ggaccggctg cagtctcaga agggagctgc 180
   tgtctgcgga ggaaactgca tcgacggacg gccgcccagc tacgggagga cctggagtgg 240
   cactgggcgc ccgacggacc atccccggga cccgcctgcc cctcggcgcc ccgccccgcc 300
   gggccgctcc ccgtcgggtt ccccagccac agccttacct acgggctcct gactccgcaa 360
   ggcttccaga agatgctcga accaccggcc ggggcctcgg ggcagcagtg agggaggcgt 420
   ccagcccccc actcagctct tctcctcctg tgccaggggc tccccggggg atgagcatgg 480
   tggttttccc tcggagcccc ctggctcggg acgtctgaga agatgccggt catgaggctg 540
   ttcccttgct tcctgcagct cctggccggg ctggcgctgc ctgctgtgcc cccccagcag 600
   tgggccttgt ctgctgggaa cggctcgtca gaggtggaag tggtaccctt ccaggaagtg 660
   tggggccgca gctactgccg ggcgctggag aggctggtgg acgtcgtgtc cgagtacccc 720
   agcgaggtgg agcacatgtt cagcccatcc tgtgtctccc tgctgcgctg caccggctgc 780
   tgcggcgatg agaatctgca ctgtgtgccg gtggagacgg ccaatgtcac catgcagctc 840
   ctaaagatcc gttctgggga ccggccctcc tacgtggagc tgacgttctc tcagcacgtt 900
   cgctgcgaat gccggcctct gcgggagaag atgaagccgg aaaggtaagt ggtttggctg 960
   gggctcgggg ctattctcgg gcctgccagc ctctgtccta gcatggggtt ccccagccac 1020
   cttgtcctga cgcttggctt attgcaggag gagacccaag ggcaggggga agaggaggag 1080
   agagaagcag agacccacag actgccacct gtgcggcgat gctgttcccc ggaggtaacc 1140
   caccccttgg aggagagaga ccccgcaccc ggctcgtgta tttattaccg tcacactctt 1200
   cagtgactcc tgctggtacc tgccctctat ttattagcca actgtttccc tgctgaatgc 1260
   ctcgctccct tcaagacgag gggcagggaa ggacaggacc ctcaggaatt cagtgccttc 1320
   aacaacgtga gagaaagaga gaagccagcc acagacccct gggagcttcc gctttgaaag 1380
   aagcaagaca cgtggcctcg tgaggggcaa gctaggcccc agaggccctg gaggtctcca 1440
   ggggcctgca gaaggaaaga agggggccct gctacctgtt cttgggcctc aggctctgca 1500
   cagtcaagca gcccttgctt tcggagctcc tgtccaaagt agggatgcgg atcctgctgg 1560
   ggccgccacg gcctggctgg tgggaaggcc ggcagcgggc ggaggggatc cagccacttc 1620
   cccctcttct tctgaagatc agaacattca gctctggaga acagtggttg cctgggggct 1680
   tttgccactc cttgtccccc gtgatctccc ctcacacttt gccatttgct tgtactggga 1740
   cattgttctt tccggccaag gtgccaccac cctgcccccc ctaagagaca catacagagt 1800
   gggccccggg ctggagaaag agctgcctgg atgagaaaca gctcagccag tggggatgag 1860
   gtcaccaggg gaggagcctg tgcgtcccag ctgaaggcag tggcagggga gcaggttccc 1920
   caagggccct ggcaccccca caagctgtcc ctgcagggcc atctgactgc caagccagat 1980
   tctcttgaat aaagtattct agtgtggaaa cgctg 2015
<210> 99
   <211> 203
   <212> PRT
   <213> Homo sapiens
<400> 99
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Ser His Leu Val
       130                 135                 140
   Leu Thr Leu Gly Leu Leu Gln Glu Glu Thr Gln Gly Gln Gly Glu Glu
   145                 150                 155                 160
   Glu Glu Arg Glu Ala Glu Thr His Arg Leu Pro Pro Val Arg Arg Cys
                   165                 170                 175
   Cys Ser Pro Glu Val Thr His Pro Leu Glu Glu Arg Asp Pro Ala Pro
               180                 185                 190
   Gly Ser Cys Ile Tyr Tyr Arg His Thr Leu Gln
           195                 200
<210> 100
   <211> 110
   <212> PRT
   <213> Homo sapiens
<400> 100
   Trp Ser Pro Gly Gly Phe Ala Lys Ser Ile Cys Pro Leu Ile Pro Gly
   1               5                   10                  15
   Gly Ser Leu Ala Leu Trp Ser Gly Ser Arg Pro His Gly Gln Pro Gly
               20                  25                  30
   Val Pro Arg Thr Ser Asp Leu Ala Trp Trp Gly Trp Arg Arg Ser Gln
           35                  40                  45
   Leu Lys Lys Leu Leu Val Gly Arg Leu Ala Leu Pro Pro Pro Gly Leu
       50                  55                  60
   Arg Thr Glu Pro Trp Ala Pro Gly Trp Ala Gly Asp Glu Thr Arg Gly
   65                  70                  75                  80
   Leu Ala Val Pro Phe Leu Leu Gly Gly Trp Pro Glu Val Leu Ser Ala
                   85                  90                  95
   Gly Pro Gly Glu Val Ser Arg Ala Leu Phe Pro Gly Ala Gly
               100                 105                 110
<210> 101
   <211> 141
   <212> PRT
   <213> Homo sapiens
<400> 101
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg
       130                 135                 140
<210> 102
   <211> 103
   <212> PRT
   <213> Homo sapiens
<400> 102
   Met Cys Ser Lys Trp Glu Gly Thr Gln Arg Gly Pro Tyr Leu Leu Ala
   1               5                   10                  15
   Ile Thr Leu Gly Pro Arg Val Gln Ser Ser Lys Val Pro Pro Ala His
               20                  25                  30
   Leu Cys Thr Phe Leu Ser Ile Ala Val Gly Leu Val Cys Trp Glu Arg
           35                  40                  45
   Leu Val Arg Gly Gly Ser Gly Thr Leu Pro Gly Ser Val Gly Pro Gln
       50                  55                  60
   Leu Leu Pro Gly Ala Gly Glu Ala Gly Gly Arg Arg Val Arg Val Pro
   65                  70                  75                  80
   Gln Arg Gly Gly Ala His Val Gln Pro Ile Leu Cys Leu Pro Ala Ala
                   85                  90                  95
   Leu His Arg Leu Leu Arg Arg
               100
<210> 103
   <211> 597
   <212> DNA
   <213> Artificial Organism
<220>
   <223> Synthetic oligonucleotide
<400> 103
   cctcgcacgc actgcgggct ccggcgctgc gggctggccg ggcgctgcgg gctgaccggg 60
   cgctccggga actcggctcg ggaacctcgt ctgcggtggg cggggccggc ccggagcccc 120
   gccccggctc agtccctgaa acccaggcgc ggaccggctg cagtctcaga agggagctgc 180
   tgtctgcgga ggaaactgca tcgacggacg gccgcccagc tacgggagga cctggagtgg 240
   cactgggcgc ccgacggacc atccccggga cccgcctgcc cctcggcgcc ccgccccgcc 300
   gggccgctcc ccgtcgggtt ccccagccac agccttacct acgggctcct gactccgcaa 360
   ggcttccaga agatgctcga accaccggcc ggggcctcgg ggcagcagtg agggaggcgt 420
   ccagcccccc actcagctct tctcctcctg tgccaggggc tccccggggg atgagcatgg 480
   tggttttccc tcggagcccc ctggctcggg acgtctgaga agatgccggt catgaggctg 540
   ttcccttgct tcctgcagct cctggccggg ctggcgctgc ctgctgtgcc cccccag 597
<210> 104
   <211> 963
   <212> DNA
   <213> Artificial Organism
<220>
   <223> Synthetic oligonucleotide
<400> 104
   ctggagccct ggtggctttg ctaaaagcat ctgtccacta atcccaggcg ggtccctggc     60
   cttgtggtca gggtcgaggc cccatggcca accaggcgtc cccaggacct ctgacctggc    120
   ctggtggggg tggcggagga gccagctgaa gaagctgttg gtggggaggc tggccctgcc    180
   cccaccaggg ctcaggactg agccgtgggc tcctgggtgg gctggggacg agacaagggg    240
   tctcgctgtg ccttttctcc tgggtgggtg gccggaggtc ctgagtgcag ggcctgggga    300
   agtgagcagg gcccttttcc caggagccgg gtgagtctgg ctgggagcca gcttgggcag    360
   tgaaatcctg acaaagccaa tgaactgcct tcccactgcg ttgtcccact gccctgggag    420
   tccccagccc cagcccaaga gtaggagtgg gctgggcagt ccctgaggag tccgagcagc    480
   ccagccaccc gcctgcctgc tgcagggctg gccagaggtg taacgttacc ggctgcacca    540
   gctcatggcg ccacccgctc cagcccctca gctcccacca gctgatcacc tccccctggg    600
   ctctggcacc cagccaaggc gggctgtgca gggcccagca gggttggcct gggggctggg    660
   tagaaggcag acccgcagga ttggaagatc tttgggatgc agcaggccaa gggtctccaa    720
   aagcttctag cctcccatcc ttttccaaat gaggaaaact gaggccagag aggggtggga    780
   cctaacctac ccagggtcca gtgagttttt agtggtactc aggctgctct gagacagggc    840
   agagcatgtg cagcaagtgg gaaggaaccc agaggggccc ctaccttctt gccatcacac    900
   ttggcccaag agtgcagagc tccaaggtac ccccagctca cctctgcact tttctgtcca    960
   tag                                                                  963
<210> 105
   <211> 3
   <212> DNA
   <213> Artificial Organism
<220>
   <223> Synthetic oligonucleotide
<400> 105
   cag 3
<210> 106
   <211> 40
   <212> DNA
   <213> Artificial Organism
<220>
   <223> Synthetic oligonucleotide
<400> 106
   tgggccttgt ctgctgggaa cggctcgtca gaggtggaag 40
<210> 107
   <211> 197
   <212> DNA
   <213> Artificial Organism
<220>
   <223> Synthetic oligonucleotide
<400> 107
   tggtaccctt ccaggaagtg tggggccgca gctactgccg ggcgctggag aggctggtgg 60
   acgtcgtgtc cgagtacccc agcgaggtgg agcacatgtt cagcccatcc tgtgtctccc 120
   tgctgcgctg caccggctgc tgcggcgatg agaatctgca ctgtgtgccg gtggagacgg 180
   ccaatgtcac catgcag 197
<210> 108
   <211> 77
   <212> DNA
   <213> Artificial Organism
<220>
   <223> Synthetic oligonucleotide
<400> 108
   ctcctaaaga tccgttctgg ggaccggccc tcctacgtgg agctgacgtt ctctcagcac 60
   gttcgctgcg aatgccg 77
<210> 109
   <211> 30
   <212> DNA
   <213> Artificial Organism
<220>
   <223> Synthetic oligonucleotide
<400> 109
   gcctctgcgg gagaagatga agccggaaag 30
<210> 110
   <211> 72
   <212> DNA
   <213> Artificial Organism
<220>
   <223> Synthetic oligonucleotide
<400> 110
   gtaagtggtt tggctggggc tcggggctat tctcgggcct gccagcctct gtcctagcat 60
   ggggttcccc ag 72
<210> 111
   <211> 31
   <212> DNA
   <213> Artificial Organism
<220>
   <223> Synthetic oligonucleotide
<400> 111
   ccaccttgtc ctgacgcttg gcttattgca g 31
<210> 112
   <211> 63
   <212> DNA
   <213> Artificial Organism
<220>
   <223> Synthetic oligonucleotide
<400> 112
   gaggagaccc aagggcaggg ggaagaggag gagagagaag cagagaccca cagactgcca 60
   cct 63
<210> 113
   <211> 905
   <212> DNA
   <213> Artificial Organism
<220>
   <223> Synthetic oligonucleotide
<400> 113
   gtgcggcgat gctgttcccc ggaggtaacc caccccttgg aggagagaga ccccgcaccc 60
   ggctcgtgta tttattaccg tcacactctt cagtgactcc tgctggtacc tgccctctat 120
   ttattagcca actgtttccc tgctgaatgc ctcgctccct tcaagacgag gggcagggaa 180
   ggacaggacc ctcaggaatt cagtgccttc aacaacgtga gagaaagaga gaagccagcc 240
   acagacccct gggagcttcc gctttgaaag aagcaagaca cgtggcctcg tgaggggcaa 300
   gctaggcccc agaggccctg gaggtctcca ggggcctgca gaaggaaaga agggggccct 360
   gctacctgtt cttgggcctc aggctctgca cagtcaagca gcccttgctt tcggagctcc     420
   tgtccaaagt agggatgcgg atcctgctgg ggccgccacg gcctggctgg tgggaaggcc     480
   ggcagcgggc ggaggggatc cagccacttc cccctcttct tctgaagatc agaacattca     540
   gctctggaga acagtggttg cctgggggct tttgccactc cttgtccccc gtgatctccc     600
   ctcacacttt gccatttgct tgtactggga cattgttctt tccggccaag gtgccaccac     660
   cctgcccccc ctaagagaca catacagagt gggccccggg ctggagaaag agctgcctgg     720
   atgagaaaca gctcagccag tggggatgag gtcaccaggg gaggagcctg tgcgtcccag     780
   ctgaaggcag tggcagggga gcaggttccc caagggccct ggcaccccca caagctgtcc    840
   ctgcagggcc atctgactgc caagccagat tctcttgaat aaagtattct agtgtggaaa     900
   cgctg                                                                 905
<210> 114
   <211> 3475
   <212> DNA
   <213> Homo sapiens
<400> 114
   tgtaaatgct cttctgacta atgcaaacca tgtgtccata gaaccagaag atttttccag 60
   gggaaaagag cccccacgcc ccgcccagct ataaggggcc atgcaccaag cagggtaccc 120
   aggctgcaga ggtgccatgg ctgagtcaca cctgctgcag tggctgctgc tgctgctgcc 180
   cacgctctgt ggcccaggca ctgctgcctg gaccacctca tccttggcct gtgcccaggg 240
   ccctgagttc tggtgccaaa gcctggagca agcattgcag tgcagagccc tagggcattg 300
   cctacaggaa gtctggggac atgtgggagc cgatgaccta tgccaagagt gtgaggacat 360
   cgtccacatc cttaacaaga tggccaagga ggccattttc caggacacga tgaggaagtt 420
   cctggagcag gagtgcaacg tcctcccctt gaagctgctc atgccccagt gcaaccaagt 480
   gcttgacgac tacttccccc tggtcatcga ctacttccag aaccagactg actcaaacgg 540
   catctgtatg cacctgggcc tgtgcaaatc ccggcagcca gagccagagc aggagccagg 600
   gatgtcagac cccctgccca aacctctgcg ggaccctctg ccagaccctc tgctggacaa 660
   gctcgtcctc cctgtgctgc ccggggccct ccaggcgagg cctgggcctc acacacagga 720
   tctctccgag cagcaattcc ccattcctct cccctattgc tggctctgca gggctctgat 780
   caagcggatc caagccatga ttcccaaggg tgcgctagct gtggcagtgg cccaggtgtg 840
   ccgcgtggta cctctggtgg cgggcggcat ctgccagtgc ctggctgagc gctactccgt 900
   catcctgctc gacacgctgc tgggccgcat gctgccccag ctggtctgcc gcctcgtcct 960
   ccggtgctcc atggatgaca gcgctggccc aagtgagccc actgccccct ccttagccca 1020
   atgcctgctc tcctcctccc cctaccctgc cactgcatga ccctctccct ctgtggtccc 1080
   actgcaatgc accaaggagg acagaaacca aacacctctg tagggtggcc ttgcctgctt 1140
   tccccctaat gctcacatct ccagggtcgc cgacaggaga atggctgccg cgagactctg 1200
   agtgccacct ctgcatgtcc gtgaccaccc aggccgggaa cagcagcgag caggccatac 1260
   cacaggcaat gctccaggcc tgtgttggct cctggctgga cagggaaaag tgcaagcaat 1320
   ttgtggagca gcacacgccc cagctgctga ccctggtgcc caggggctgg gatgcccaca 1380
   ccacctgcca ggccctcggg gtgtgtggga ccatgtccag ccctctccag tgtatccaca 1440
   gccccgacct ttgatgagaa ctcagctgtc cagctgcaaa ggaaaagcca agtgagacgg 1500
   gctctgggac catggtgacc aggctcttcc cctgctccct ggccctcgcc agctgccagg 1560
   ctgaaaagaa gcctcagctc ccacaccgcc ctcctcaccg cccttcctcg gcagtcactt 1620
   ccactggtgg accacgggcc cccagccctg tgtcggcctt gtctgtctca gctcaaccac 1680
   agtctgacac cagagcccac ttccatcctc tctggtgtga ggcacagcga gggcagcatc 1740
   tggaggagct ctgcagcctc cacacctacc acgacctccc agggctgggc tcaggaaaaa 1800
   ccagccactg ctttacagga cagggggttg aagctgagcc ccgcctcaca cccaccccca 1860
   tgcactcaaa gattggattt tacagctact tgcaattcaa aattcagaag aataaaaaat 1920
   gggaacatac agaactctaa aagatagaca tcagaaattg ttaagttaag ctttttcaaa 1980
   aaatcagcaa ttccccagcg tagtcaaggg tggacactgc acgctctggc atgatgggat 2040
   ggcgaccggg caagctttct tcctcgagat gctctgctgc ttgagagcta ttgctttgtt 2100
   aagatataaa aaggggtttc tttttgtctt tctgtaaggt ggacttccag cttttgattg 2160
   aaagtcctag ggtgattcta tttctgctgt gatttatctg ctgaaagctc agctggggtt 2220
   gtgcaagcta gggacccatt cctgtgtaat acaatgtctg caccaatgct aataaagtcc 2280
   tattctcttt tatgagaaag aaaaagacac cgtcctttaa agtgctgcag tatggccaga 2340
   cgtggtggct cacacctgca atcccagcac cttaggaggc cgaggcagga ggatccttga 2400
   ggtcaggagt tcgagaccag cctcgccaac atggtgaaac cccatttcta ctaaaaatac 2460
   aaaaaattag ccaagtgtgg tggcatatgc ctgtaatccc aactactcag aaggccgagg 2520
   caggagaatt acttgaacgc aggagaatca ctgcagccca ggaggcagag gttgcagtga 2580
   gccgagattg caccactgca ctccagcctg ggtgacagag caagactcca tctcagtaaa 2640
   taaataaata aataaaaagc gctgcagtag ctgtggcctc accctgaagt cagcgggccc 2700
   aggcctacct cactctctcc cttggcagag aagcagacgt ccatagctcc tctccctcac 2760
   aagcgctccc agcctgccct ccagctgctg ctctcccctc ccagtctcta ctcactggga 2820
   tgaggttagg tcatgaggac accaaaaacc taaaaataaa caaaaagcca aacaagcctt 2880
   agcttttctt aaagactgaa atgcctggaa gtgtcccttt atttataaaa taacttttgt 2940
   catatttctt atacatgttt cttgtaagaa attcagaaac tacagacaaa gagagtggaa 3000
   attacccact gtcaggcctc tgagcccaag ctaagccatc atatcccctg tgccctgcac 3060
   gtatacaccc agatggcctg aagcaactga agatccacaa aagaagtgaa aatagccagt   3120
   tcctgcctta actgatgaca ttccaccatt gtgatttgtt cctgccccac cctaactgat   3180
   caattgacct tgtgacaata caccttcccc acccttgaga aggtgctttg taatattctc   3240
   cccacccacc ccacgcccgc acccccgcac ccttaagaag gtattttgta atattctctc   3300
   cgccattgag aatgtgcttt gtaagatcca ccccctgccc acaaaaaatt gctcctaact   3360
   ccaccgccta tcccaaacct acaagaacta atgataatcc caccaccctt tgctgactct   3420
   ttttggactc agcccacctg cacccaggtg attaaaaagc tttattgttc acaca        3475
<210> 115
   <211> 3473
   <212> DNA
   <213> Homo sapiens
<400> 115
   tgtaaatgct cttctgacta atgcaaacca tgtgtccata gaaccagaag atttttccag 60
   gggaaaagag cccccacgcc ccgcccagct ataaggggcc atgcaccaag cagggtaccc 120
   aggctgcaga ggtgccatgg ctgagtcaca cctgctgcag tggctgctgc tgctgctgcc 180
   cacgctctgt ggcccaggca ctgctgcctg gaccacctca tccttggcct gtgcccaggg 240
   ccctgagttc tggtgccaaa gcctggagca agcattgcag tgcagagccc tagggcattg 300
   cctacaggaa gtctggggac atgtgggagc cgatgaccta tgccaagagt gtgaggacat 360
   cgtccacatc cttaacaaga tggccaagga ggccattttc caggacacga tgaggaagtt 420
   cctggagcag gagtgcaacg tcctcccctt gaagctgctc atgccccagt gcaaccaagt 480
   gcttgacgac tacttccccc tggtcatcga ctacttccag aaccagactg actcaaacgg 540
   catctgtatg cacctgggcc tgtgcaaatc ccggcagcca gagccagagc aggagccagg 600
   gatgtcagac cccctgccca aacctctgcg ggaccctctg ccagaccctc tgctggacaa 660
   gctcgtcctc cctgtgctgc ccggggccct ccaggcgagg cctgggcctc acacacagga 720
   tctctccgag cagcaattcc ccattcctct cccctattgc tggctctgca gggctctgat 780
   caagcggatc caagccatga ttcccaaggg tgcgctagct gtggcagtgg cccaggtgtg 840
   ccgcgtggta cctctggtgg cgggcggcat ctgccagtgc ctggctgagc gctactccgt 900
   catcctgctc gacacgctgc tgggccgcat gctgccccag ctggtctgcc gcctcgtcct 960
   ccggtgctcc atggatgaca gcgctggccc aaggtcgccg acaggagaat ggctgccgcg 1020
   agactctgag tgccacctct gcatgtccgt gaccacccag gccgggaaca gcagcgagca 1080
   ggccatacca caggcaatgc tccaggcctg tgttggctcc tggctggaca gggaaaagct 1140
   ggctccagtt tgttaagggt ttcaactgca gccagaggtg ttccgtgagg gctgatggag 1200
   gagtcgggag ggagccctag agtgatccag agatgtggag aggccaggac cacacgacag 1260
   gagagtcctg caaagggacc ctccacagct gtgtgtctcc ttcctcagtg caagcaattt 1320
   gtggagcagc acacgcccca gctgctgacc ctggtgccca ggggctggga tgcccacacc 1380
   acctgccagg ccctcggggt gtgtgggacc atgtccagcc ctctccagtg tatccacagc 1440
   cccgaccttt gatgagaact cagctgtcca gctgcaaagg aaaagccaag tgagacgggc 1500
   tctgggacca tggtgaccag gctcttcccc tgctccctgg ccctcgccag ctgccaggct 1560
   gaaaagaagc ctcagctccc acaccgccct cctcaccgcc cttcctcggc agtcacttcc 1620
   actggtggac cacgggcccc cagccctgtg tcggccttgt ctgtctcagc tcaaccacag 1680
   tctgacacca gagcccactt ccatcctctc tggtgtgagg cacagcgagg gcagcatctg 1740
   gaggagctct gcagcctcca cacctaccac gacctcccag ggctgggctc aggaaaaacc 1800
   agccactgct ttacaggaca gggggttgaa gctgagcccc gcctcacacc cacccccatg 1860
   cactcaaaga ttggatttta cagctacttg caattcaaaa ttcagaagaa taaaaaatgg 1920
   gaacatacag aactctaaaa gatagacatc agaaattgtt aagttaagct ttttcaaaaa 1980
   atcagcaatt ccccagcgta gtcaagggtg gacactgcac gctctggcat gatgggatgg 2040
   cgaccgggca agctttcttc ctcgagatgc tctgctgctt gagagctatt gctttgttaa 2100
   gatataaaaa ggggtttctt tttgtctttc tgtaaggtgg acttccagct tttgattgaa 2160
   agtcctaggg tgattctatt tctgctgtga tttatctgct gaaagctcag ctggggttgt 2220
   gcaagctagg gacccattcc tgtgtaatac aatgtctgca ccaatgctaa taaagtccta 2280
   ttctctttta tgagaaagaa aaagacaccg tcctttaaag tgctgcagta tggccagacg 2340
   tggtggctca cacctgcaat cccagcacct taggaggccg aggcaggagg atccttgagg 2400
   tcaggagttc gagaccagcc tcgccaacat ggtgaaaccc catttctact aaaaatacaa 2460
   aaaattagcc aagtgtggtg gcatatgcct gtaatcccaa ctactcagaa ggccgaggca 2520
   ggagaattac ttgaacgcag gagaatcact gcagcccagg aggcagaggt tgcagtgagc 2580
   cgagattgca ccactgcact ccagcctggg tgacagagca agactccatc tcagtaaata 2640
   aataaataaa taaaaagcgc tgcagtagct gtggcctcac cctgaagtca gcgggcccag 2700
   gcctacctca ctctctccct tggcagagaa gcagacgtcc atagctcctc tccctcacaa 2760
   gcgctcccag cctgccctcc agctgctgct ctcccctccc agtctctact cactgggatg 2820
   aggttaggtc atgaggacac caaaaaccta aaaataaaca aaaagccaaa caagccttag 2880
   cttttcttaa agactgaaat gcctggaagt gtccctttat ttataaaata acttttgtca 2940
   tatttcttat acatgtttct tgtaagaaat tcagaaacta cagacaaaga gagtggaaat 3000
   tacccactgt caggcctctg agcccaagct aagccatcat atcccctgtg ccctgcacgt 3060
   atacacccag atggcctgaa gcaactgaag atccacaaaa gaagtgaaaa tagccagttc 3120
   ctgccttaac tgatgacatt ccaccattgt gatttgttcc tgccccaccc taactgatca 3180
   attgaccttg tgacaataca ccttccccac ccttgagaag gtgctttgta atattctccc 3240
   cacccacccc acgcccgcac ccccgcaccc ttaagaaggt attttgtaat attctctccg 3300
   ccattgagaa tgtgctttgt aagatccacc ccctgcccac aaaaaattgc tcctaactcc 3360
   accgcctatc ccaaacctac aagaactaat gataatccca ccaccctttg ctgactcttt 3420
   ttggactcag cccacctgca cccaggtgat taaaaagctt tattgttcac aca 3473
<210> 116
   <211> 4744
   <212> DNA
   <213> Homo sapiens
<400> 116
   tgtaaatgct cttctgacta atgcaaacca tgtgtccata gaaccagaag atttttccag 60
   gggaaaagag cccccacgcc ccgcccagct ataaggggcc atgcaccaag cagggtaccc 120
   aggctgcaga ggtgccatgg ctgagtcaca cctgctgcag tggctgctgc tgctgctgcc 180
   cacgctctgt ggcccaggca ctgctgcctg gaccacctca tccttggcct gtgcccaggg 240
   ccctgagttc tggtgccaaa gcctggagca agcattgcag tgcagagccc tagggcattg 300
   cctacaggaa gtctggggac atgtgggagc cgatgaccta tgccaagagt gtgaggacat 360
   cgtccacatc cttaacaaga tggccaagga ggccattttc caggacacga tgaggaagtt 420
   cctggagcag gagtgcaacg tcctcccctt gaagctgctc atgccccagt gcaaccaagt 480
   gcttgacgac tacttccccc tggtcatcga ctacttccag aaccagactg actcaaacgg 540
   catctgtatg cacctgggcc tgtgcaaatc ccggcagcca gagccagagc aggagccagg 600
   gatgtcagac cccctgccca aacctctgcg ggaccctctg ccagaccctc tgctggacaa 660
   gctcgtcctc cctgtgctgc ccggggccct ccaggcgagg cctgggcctc acacacagga 720
   tctctccgag cagcaattcc ccattcctct cccctattgc tggctctgca gggctctgat 780
   caagcggatc caagccatga ttcccaaggt gaggcatcca gggcctcaca gagcccagga 840
   gcacacgcat acctgtagct ccctgcagct cccacctctc tcccaactca cacccccgtc 900
   aggacccagc tggctgccag aagttaggag gggagagagc cgcttgtgca ttgcccccac 960
   ccaggggacc ctggggctca ggctcaggcc tggtaggtgc caggcctaca gttcatgcaa 1020
   caaacattaa gcccccactg tatggaggtg ccacgccagg agccaaagta caaaaacgga 1080
   caagacgcag ctttgtcctc cagcagctca ccatctgatg gagaaagatc cccagaggtc 1140
   tctgtagaaa ggttgctttg atctttcaag aggggaattt ccacagatag attccccatc 1200
   cttgcctgag tccaacttgg agtcttccag acctgcagtg gctattgtcc aatggccccg 1260
   ccagcccagg gctaccttgc ccaaattggg gcccaaatga ggaaaggccc tgccccctca 1320
   gcctttccca gatagggttg cgtgggccac caggggcaca aggcagcagg tgaggttcct 1380
   gctgaggcag gtggttcact tgagcccagg agttcaagac cagcttgggc aacatggcga 1440
   aaccccgtct ctactaagaa tacaaaaatt agccagatgt gacaggtgcc tgtagtccca 1500
   gctactcggg aggctgaggc aggagaatca cttgaaccca ggaggcggag gttgcagtga 1560
   gccgacatca cgccactgta ctctagcctg ggtgacagag caagactctg tctcaaaaaa 1620
   aaagaaagaa ggaaagatca ctgcagagat tgcagtgaga ggtgatggga cagggacgga 1680
   gctgagggct ggcctgggga tgcatttggg aggtgggccc actgctattg ggcatggatg 1740
   ggcctggagc gtgaggacca gggaggactc caaagtgact tttacacact ggccagagca 1800
   accagccctc tgtaatgcca gcagctgaga tggggagact aaagaagaaa acaggtttga 1860
   gcaaaaaaac agagagctcc ctcctggcca tgttgagttc aagatgcctg tgtgaagtgc 1920
   aggagaggag agtcaggcaa gcagctgaat cccaagcatt gggggaaggt caggtccacc 1980
   atgtcagtct gagagtcact agctgtgggc cagagccttt ggggccagac gtaggtctga 2040
   agctggctcc tacactcagt gaccctgtgt gagtcccctg catcccctgg actctctgat 2100
   ccccagtgtc cttatttgtg aatagccttg ccctcccttc tagaagagaa tgagggaatg 2160
   cgtaggaagt gcccagctgg gtgctgggca gagagtggag gcttgccaag tgaaggtccc 2220
   atgctggcct ctctccgccc ccgccccagg gtgcgctagc tgtggcagtg gcccaggtgt 2280
   gccgcgtggt acctctggtg gcgggcggca tctgccagtg cctggctgag cgctactccg 2340
   tcatcctgct cgacacgctg ctgggccgca tgctgcccca gctggtctgc cgcctcgtcc 2400
   tccggtgctc catggatgac agcgctggcc caaggtcgcc gacaggagaa tggctgccgc 2460
   gagactctga gtgccacctc tgcatgtccg tgaccaccca ggccgggaac agcagcgagc 2520
   aggccatacc acaggcaatg ctccaggcct gtgttggctc ctggctggac agggaaaagt 2580
   gcaagcaatt tgtggagcag cacacgcccc agctgctgac cctggtgccc aggggctggg 2640
   atgcccacac cacctgccag gccctcgggg tgtgtgggac catgtccagc cctctccagt 2700
   gtatccacag ccccgacctt tgatgagaac tcagctgtcc agctgcaaag gaaaagccaa 2760
   gtgagacggg ctctgggacc atggtgacca ggctcttccc ctgctccctg gccctcgcca 2820
   gctgccaggc tgaaaagaag cctcagctcc cacaccgccc tcctcaccgc ccttcctcgg 2880
   cagtcacttc cactggtgga ccacgggccc ccagccctgt gtcggccttg tctgtctcag 2940
   ctcaaccaca gtctgacacc agagcccact tccatcctct ctggtgtgag gcacagcgag 3000
   ggcagcatct ggaggagctc tgcagcctcc acacctacca cgacctccca gggctgggct 3060
   caggaaaaac cagccactgc tttacaggac agggggttga agctgagccc cgcctcacac 3120
   ccacccccat gcactcaaag attggatttt acagctactt gcaattcaaa attcagaaga 3180
   ataaaaaatg ggaacataca gaactctaaa agatagacat cagaaattgt taagttaagc 3240
   tttttcaaaa aatcagcaat tccccagcgt agtcaagggt ggacactgca cgctctggca 3300
   tgatgggatg gcgaccgggc aagctttctt cctcgagatg ctctgctgct tgagagctat 3360
   tgctttgtta agatataaaa aggggtttct ttttgtcttt ctgtaaggtg gacttccagc 3420
   ttttgattga aagtcctagg gtgattctat ttctgctgtg atttatctgc tgaaagctca 3480
   gctggggttg tgcaagctag ggacccattc ctgtgtaata caatgtctgc accaatgcta 3540
   ataaagtcct attctctttt atgagaaaga aaaagacacc gtcctttaaa gtgctgcagt 3600
   atggccagac gtggtggctc acacctgcaa tcccagcacc ttaggaggcc gaggcaggag 3660
   gatccttgag gtcaggagtt cgagaccagc ctcgccaaca tggtgaaacc ccatttctac 3720
   taaaaataca aaaaattagc caagtgtggt ggcatatgcc tgtaatccca actactcaga 3780
   aggccgaggc aggagaatta cttgaacgca ggagaatcac tgcagcccag gaggcagagg 3840
   ttgcagtgag ccgagattgc accactgcac tccagcctgg gtgacagagc aagactccat 3900
   ctcagtaaat aaataaataa ataaaaagcg ctgcagtagc tgtggcctca ccctgaagtc 3960
   agcgggccca ggcctacctc actctctccc ttggcagaga agcagacgtc catagctcct 4020
   ctccctcaca agcgctccca gcctgccctc cagctgctgc tctcccctcc cagtctctac 4080
   tcactgggat gaggttaggt catgaggaca ccaaaaacct aaaaataaac aaaaagccaa 4140
   acaagcctta gcttttctta aagactgaaa tgcctggaag tgtcccttta tttataaaat 4200
   aacttttgtc atatttctta tacatgtttc ttgtaagaaa ttcagaaact acagacaaag 4260
   agagtggaaa ttacccactg tcaggcctct gagcccaagc taagccatca tatcccctgt 4320
   gccctgcacg tatacaccca gatggcctga agcaactgaa gatccacaaa agaagtgaaa 4380
   atagccagtt cctgccttaa ctgatgacat tccaccattg tgatttgttc ctgccccacc 4440
   ctaactgatc aattgacctt gtgacaatac accttcccca cccttgagaa ggtgctttgt 4500
   aatattctcc ccacccaccc cacgcccgca cccccgcacc cttaagaagg tattttgtaa 4560
   tattctctcc gccattgaga atgtgctttg taagatccac cccctgccca caaaaaattg 4620
   ctcctaactc caccgcctat cccaaaccta caagaactaa tgataatccc accacccttt 4680
   gctgactctt tttggactca gcccacctgc acccaggtga ttaaaaagct ttattgttca 4740
   caca 4744
<210> 117
   <211> 4125
   <212> DNA
   <213> Homo sapiens
<400> 117
   tgtaaatgct cttctgacta atgcaaacca tgtgtccata gaaccagaag atttttccag      60
   gggaaaagag cccccacgcc ccgcccagct ataaggggcc atgcaccaag cagggtaccc     120
   aggctgcaga ggtgccatgg ctgagtcaca cctgctgcag tggctgctgc tgctgctgcc     180
   cacgctctgt ggcccaggca ctgctgcctg gaccacctca tccttggcct gtgcccaggg     240
   ccctgagttc tggtgccaaa gcctggagca agcattgcag tgcagagccc tagggcattg     300
   cctacaggaa gtctggggac atgtgggagc cgatgaccta tgccaagagt gtgaggacat     360
   cgtccacatc cttaacaaga tggccaagga ggccattttc caggacacga tgaggaagtt 420
   cctggagcag gagtgcaacg tcctcccctt gaagctgctc atgccccagt gcaaccaagt 480
   gcttgacgac tacttccccc tggtcatcga ctacttccag aaccagactg tgagggctgc 540
   aagctcacct cctgcctgcc tccccacgca ggcccctgtg cccacccatg gggagccaca 600
   cacacagcac cccagccagc cagacacaca cacacacaca cacacacaca cagcacccaa 660
   gccggccaga cacaaacaca cagcacccca gccagccgga cacacacaca cacacacaca 720
   caacacccca gctggccgga cacacacaca cacagtaccc cagctggccg gacacacaca 780
   cacacagcac cctatccaga cacatacaca cacacagtac cccagccagc tggaaacaca 840
   cacacacaca gcactccatc cagacacata cccacacagt accccagcca gccagacaca 900
   cacacacaca cacacacaca cacacacaca gcacacacac agcaccccag ctggccacac 960
   acacacacac acacaccctg tccacaaagg gcctaggaaa ctacgtgccc ttcagccatg 1020
   cacccgacca tgggccccca ggttcaggtg cacacggtgg gcctgtacgc tcacacaccc 1080
   ttacaccctc actctcacac acatgcttac acacttattc attctcacat atatgctcat 1140
   gctcattcac acacaatccc ggccacctgc cctaaagtcc ccacacagcc ctatctttgc 1200
   cttttgtccc cccacataga gttctaaacc acagcacccc cactaggcct gcttcctccc 1260
   attccagtgg tccctgagcc cttgggccgg cctgaatagg ggtgggcttc cctcccagac 1320
   cctaacactc ccaccctgtg ctgtgcccca ggactcaaac ggcatctgta tgcacctggg 1380
   cctgtgcaaa tcccggcagc cagagccaga gcaggagcca gggatgtcag accccctgcc 1440
   caaacctctg cgggaccctc tgccagaccc tctgctggac aagctcgtcc tccctgtgct 1500
   gcccggggcc ctccaggcga ggcctgggcc tcacacacag gatctctccg agcagcaatt 1560
   ccccattcct ctcccctatt gctggctctg cagggctctg atcaagcgga tccaagccat 1620
   gattcccaag ggtgcgctag ctgtggcagt ggcccaggtg tgccgcgtgg tacctctggt 1680
   ggcgggcggc atctgccagt gcctggctga gcgctactcc gtcatcctgc tcgacacgct 1740
   gctgggccgc atgctgcccc agctggtctg ccgcctcgtc ctccggtgct ccatggatga 1800
   cagcgctggc ccaaggtcgc cgacaggaga atggctgccg cgagactctg agtgccacct 1860
   ctgcatgtcc gtgaccaccc aggccgggaa cagcagcgag caggccatac cacaggcaat 1920
   gctccaggcc tgtgttggct cctggctgga cagggaaaag tgcaagcaat ttgtggagca 1980
   gcacacgccc cagctgctga ccctggtgcc caggggctgg gatgcccaca ccacctgcca 2040
   ggccctcggg gtgtgtggga ccatgtccag ccctctccag tgtatccaca gccccgacct 2100
   ttgatgagaa ctcagctgtc cagctgcaaa ggaaaagcca agtgagacgg gctctgggac 2160
   catggtgacc aggctcttcc cctgctccct ggccctcgcc agctgccagg ctgaaaagaa 2220
   gcctcagctc ccacaccgcc ctcctcaccg cccttcctcg gcagtcactt ccactggtgg 2280
   accacgggcc cccagccctg tgtcggcctt gtctgtctca gctcaaccac agtctgacac 2340
   cagagcccac ttccatcctc tctggtgtga ggcacagcga gggcagcatc tggaggagct 2400
   ctgcagcctc cacacctacc acgacctccc agggctgggc tcaggaaaaa ccagccactg 2460
   ctttacagga cagggggttg aagctgagcc ccgcctcaca cccaccccca tgcactcaaa 2520
   gattggattt tacagctact tgcaattcaa aattcagaag aataaaaaat gggaacatac 2580
   agaactctaa aagatagaca tcagaaattg ttaagttaag ctttttcaaa aaatcagcaa 2640
   ttccccagcg tagtcaaggg tggacactgc acgctctggc atgatgggat ggcgaccggg 2700
   caagctttct tcctcgagat gctctgctgc ttgagagcta ttgctttgtt aagatataaa 2760
   aaggggtttc tttttgtctt tctgtaaggt ggacttccag cttttgattg aaagtcctag 2820
   ggtgattcta tttctgctgt gatttatctg ctgaaagctc agctggggtt gtgcaagcta 2880
   gggacccatt cctgtgtaat acaatgtctg caccaatgct aataaagtcc tattctcttt 2940
   tatgagaaag aaaaagacac cgtcctttaa agtgctgcag tatggccaga cgtggtggct 3000
   cacacctgca atcccagcac cttaggaggc cgaggcagga ggatccttga ggtcaggagt 3060
   tcgagaccag cctcgccaac atggtgaaac cccatttcta ctaaaaatac aaaaaattag 3120
   ccaagtgtgg tggcatatgc ctgtaatccc aactactcag aaggccgagg caggagaatt 3180
   acttgaacgc aggagaatca ctgcagccca ggaggcagag gttgcagtga gccgagattg 3240
   caccactgca ctccagcctg ggtgacagag caagactcca tctcagtaaa taaataaata 3300
   aataaaaagc gctgcagtag ctgtggcctc accctgaagt cagcgggccc aggcctacct 3360
   cactctctcc cttggcagag aagcagacgt ccatagctcc tctccctcac aagcgctccc 3420
   agcctgccct ccagctgctg ctctcccctc ccagtctcta ctcactggga tgaggttagg 3480
   tcatgaggac accaaaaacc taaaaataaa caaaaagcca aacaagcctt agcttttctt 3540
   aaagactgaa atgcctggaa gtgtcccttt atttataaaa taacttttgt catatttctt 3600
   atacatgttt cttgtaagaa attcagaaac tacagacaaa gagagtggaa attacccact 3660
   gtcaggcctc tgagcccaag ctaagccatc atatcccctg tgccctgcac gtatacaccc 3720
   agatggcctg aagcaactga agatccacaa aagaagtgaa aatagccagt tcctgcctta 3780
   actgatgaca ttccaccatt gtgatttgtt cctgccccac cctaactgat caattgacct 3840
   tgtgacaata caccttcccc acccttgaga aggtgctttg taatattctc cccacccacc 3900
   ccacgcccgc acccccgcac ccttaagaag gtattttgta atattctctc cgccattgag 3960
   aatgtgcttt gtaagatcca ccccctgccc acaaaaaatt gctcctaact ccaccgccta 4020
   tcccaaacct acaagaacta atgataatcc caccaccctt tgctgactct ttttggactc 4080
   agcccacctg cacccaggtg attaaaaagc tttattgttc acaca 4125
<210> 118
   <211> 2646
   <212> DNA
   <213> Homo sapiens
<400> 118
   tgtaaatgct cttctgacta atgcaaacca tgtgtccata gaaccagaag atttttccag 60
   gggaaaagag cccccacgcc ccgcccagct ataaggggcc atgcaccaag cagggtaccc 120
   aggctgcaga ggtgccatgg ctgagtcaca cctgctgcag tggctgctgc tgctgctgcc 180
   cacgctctgt ggcccaggca ctgctgcctg gaccacctca tccttggcct gtgcccaggg 240
   ccctgagttc tggtgccaaa gcctggagca agcattgcag tgcagagccc tagggcattg 300
   cctacaggaa gtctggggac atgtgggagc cgatgaccta tgccaagagt gtgaggacat 360
   cgtccacatc cttaacaaga tggccaagga ggccattttc caggacacga tgaggaagtt 420
   cctggagcag gagtgcaacg tcctcccctt gaagctgctc atgccccagt gcaaccaagt 480
   gcttgacgac tacttccccc tggtcatcga ctacttccag aaccagactg actcaaacgg 540
   catctgtatg cacctgggcc tgtgcaaatc ccggcagcca gagccagagc aggagccagg 600
   gatgtcagac cccctgccca aacctctgcg ggaccctctg ccagaccctc tgctggacaa 660
   gctcgtcctc cctgtgctgc ccggggccct ccaggcgagg cctgggcctc acacacagga 720
   tctctccgag cagcaattcc ccattcctct cccctattgc tggctctgca gggctctgat 780
   caagcggatc caagccatga ttcccaaggg tgcgctagct gtggcagtgg cccaggtgtg 840
   ccgcgtggta cctctggtgg cgggcggcat ctgccagtgc ctggctgagc gctactccgt 900
   catcctgctc gacacgctgc tgggccgcat gctgccccag ctggtctgcc gcctcgtcct 960
   ccggtgctcc atggatgaca gcgctggccc aaggtcgccg acaggagaat ggctgccgcg 1020
   agactctgag tgccacctct gcatgtccgt gaccacccag gccgggaaca gcagcgagca 1080
   ggccatacca caggcaatgc tccaggcctg tgttggctcc tggctggaca gggaaaagct 1140
   ggctccagtt tgttaagggt ttcaactgca gccagaggtg ttccgtgagg gctgatggag 1200
   gagtcgggag ggagccctag agtgatccag agatgtggag aggccaggac cacacgacag 1260
   gagagtcctg caaagggacc ctccacagct gtgtgtctcc ttcctcagtg caagcaattt 1320
   gtggagcagc acacgcccca gctgctgacc ctggtgccca ggggctggga tgcccacacc 1380
   acctgccagg ccctcggggt gtgtgggacc atgtccagcc ctctccagtg tatccacagc 1440
   cccgaccttt gatgagaact cagctgtcca gaaaaagaca ccgtccttta aagtgctgca 1500
   gtatggccag acgtggtggc tcacacctgc aatcccagca ccttaggagg ccgaggcagg 1560
   aggatccttg aggtcaggag ttcgagacca gcctcgccaa catggtgaaa ccccatttct 1620
   actaaaaata caaaaaatta gccaagtgtg gtggcatatg cctgtaatcc caactactca 1680
   gaaggccgag gcaggagaat tacttgaacg caggagaatc actgcagccc aggaggcaga 1740
   ggttgcagtg agccgagatt gcaccactgc actccagcct gggtgacaga gcaagactcc 1800
   atctcagtaa ataaataaat aaataaaaag cgctgcagta gctgtggcct caccctgaag   1860
   tcagcgggcc caggcctacc tcactctctc ccttggcaga gaagcagacg tccatagctc   1920
   ctctccctca caagcgctcc cagcctgccc tccagctgct gctctcccct cccagtctct   1980
   actcactggg atgaggttag gtcatgagga caccaaaaac ctaaaaataa acaaaaagcc   2040
   aaacaagcct tagcttttct taaagactga aatgcctgga agtgtccctt tatttataaa   2100
   ataacttttg tcatatttct tatacatgtt tcttgtaaga aattcagaaa ctacagacaa   2160
   agagagtgga aattacccac tgtcaggcct ctgagcccaa gctaagccat catatcccct   2220
   gtgccctgca cgtatacacc cagatggcct gaagcaactg aagatccaca aaagaagtga   2280
   aaatagccag ttcctgcctt aactgatgac attccaccat tgtgatttgt tcctgcccca   2340
   ccctaactga tcaattgacc ttgtgacaat acaccttccc cacccttgag aaggtgcttt   2400
   gtaatattct ccccacccac cccacgcccg cacccccgca cccttaagaa ggtattttgt   2460
   aatattctct ccgccattga gaatgtgctt tgtaagatcc accccctgcc cacaaaaaat   2520
   tgctcctaac tccaccgcct atcccaaacc tacaagaact aatgataatc ccaccaccct   2580
   ttgctgactc tttttggact cagcccacct gcacccaggt gattaaaaag ctttattgtt   2640
   cacaca                                                              2646

<210> 119
   <211> 3375
   <212> DNA
   <213> Homo sapiens
<400> 119
   tgtaaatgct cttctgacta atgcaaacca tgtgtccata gaaccagaag atttttccag     60
   gggaaaagag cccccacgcc ccgcccagct ataaggggcc atgcaccaag cagggtaccc    120
   aggctgcaga ggtgccatgg ctgagtcaca cctgctgcag tggctgctgc tgctgctgcc    180
   cacgctctgt ggcccaggca ctgctgcctg gaccacctca tccttggcct gtgcccaggg    240
   ccctgagttc tggtgccaaa gcctggagca agcattgcag tgcagagccc tagggcattg    300
   cctacaggaa gtctggggac atgtgggagc cagaacctcc ccattggagc tgggagcctc    360
   ccccactcat gtgtcctcca ccttggggcc cctccctccc caggatgacc tatgccaaga    420
   gtgtgaggac atcgtccaca tccttaacaa gatggccaag gaggccattt tccaggacac    480
   gatgaggaag ttcctggagc aggagtgcaa cgtcctcccc ttgaagctgc tcatgcccca    540
   gtgcaaccaa gtgcttgacg actacttccc cctggtcatc gactacttcc agaaccagac    600
   tgactcaaac ggcatctgta tgcacctggg cctgtgcaaa tcccggcagc cagagccaga    660
   gcaggagcca gggatgtcag accccctgcc caaacctctg cgggaccctc tgccagaccc    720
   tctgctggac aagctcgtcc tccctgtgct gcccggggcc ctccaggcga ggcctgggcc    780
   tcacacacag gatctctccg agcagcaatt ccccattcct ctcccctatt gctggctctg    840
   cagggctctg atcaagcgga tccaagccat gattcccaag ggtgcgctag ctgtggcagt    900
   ggcccaggtg tgccgcgtgg tacctctggt ggcgggcggc atctgccagt gcctggctga    960
   gcgctactcc gtcatcctgc tcgacacgct gctgggccgc atgctgcccc agctggtctg   1020
   ccgcctcgtc ctccggtgct ccatggatga cagcgctggc ccaaggtcgc cgacaggaga   1080
   atggctgccg cgagactctg agtgccacct ctgcatgtcc gtgaccaccc aggccgggaa   1140
   cagcagcgag caggccatac cacaggcaat gctccaggcc tgtgttggct cctggctgga   1200
   cagggaaaag tgcaagcaat ttgtggagca gcacacgccc cagctgctga ccctggtgcc   1260
   caggggctgg gatgcccaca ccacctgcca ggccctcggg gtgtgtggga ccatgtccag   1320
   ccctctccag tgtatccaca gccccgacct ttgatgagaa ctcagctgtc cagctgcaaa   1380
   ggaaaagcca agtgagacgg gctctgggac catggtgacc aggctcttcc cctgctccct   1440
   ggccctcgcc agctgccagg ctgaaaagaa gcctcagctc ccacaccgcc ctcctcaccg   1500
   cccttcctcg gcagtcactt ccactggtgg accacgggcc cccagccctg tgtcggcctt   1560
   gtctgtctca gctcaaccac agtctgacac cagagcccac ttccatcctc tctggtgtga   1620
   ggcacagcga gggcagcatc tggaggagct ctgcagcctc cacacctacc acgacctccc   1680
   agggctgggc tcaggaaaaa ccagccactg ctttacagga cagggggttg aagctgagcc   1740
   ccgcctcaca cccaccccca tgcactcaaa gattggattt tacagctact tgcaattcaa   1800
   aattcagaag aataaaaaat gggaacatac agaactctaa aagatagaca tcagaaattg   1860
   ttaagttaag ctttttcaaa aaatcagcaa ttccccagcg tagtcaaggg tggacactgc   1920
   acgctctggc atgatgggat ggcgaccggg caagctttct tcctcgagat gctctgctgc   1980
   ttgagagcta ttgctttgtt aagatataaa aaggggtttc tttttgtctt tctgtaaggt   2040
   ggacttccag cttttgattg aaagtcctag ggtgattcta tttctgctgt gatttatctg   2100
   ctgaaagctc agctggggtt gtgcaagcta gggacccatt cctgtgtaat acaatgtctg   2160
   caccaatgct aataaagtcc tattctcttt tatgagaaag aaaaagacac cgtcctttaa   2220
   agtgctgcag tatggccaga cgtggtggct cacacctgca atcccagcac cttaggaggc   2280
   cgaggcagga ggatccttga ggtcaggagt tcgagaccag cctcgccaac atggtgaaac   2340
   cccatttcta ctaaaaatac aaaaaattag ccaagtgtgg tggcatatgc ctgtaatccc   2400
   aactactcag aaggccgagg caggagaatt acttgaacgc aggagaatca ctgcagccca   2460
   ggaggcagag gttgcagtga gccgagattg caccactgca ctccagcctg ggtgacagag   2520
   caagactcca tctcagtaaa taaataaata aataaaaagc gctgcagtag ctgtggcctc   2580
   accctgaagt cagcgggccc aggcctacct cactctctcc cttggcagag aagcagacgt   2640
   ccatagctcc tctccctcac aagcgctccc agcctgccct ccagctgctg ctctcccctc   2700
   ccagtctcta ctcactggga tgaggttagg tcatgaggac accaaaaacc taaaaataaa   2760
   caaaaagcca aacaagcctt agcttttctt aaagactgaa atgcctggaa gtgtcccttt 2820
   atttataaaa taacttttgt catatttctt atacatgttt cttgtaagaa attcagaaac 2880
   tacagacaaa gagagtggaa attacccact gtcaggcctc tgagcccaag ctaagccatc 2940
   atatcccctg tgccctgcac gtatacaccc agatggcctg aagcaactga agatccacaa 3000
   aagaagtgaa aatagccagt tcctgcctta actgatgaca ttccaccatt gtgatttgtt 3060
   cctgccccac cctaactgat caattgacct tgtgacaata caccttcccc acccttgaga 3120
   aggtgctttg taatattctc cccacccacc ccacgcccgc acccccgcac ccttaagaag 3180
   gtattttgta atattctctc cgccattgag aatgtgcttt gtaagatcca ccccctgccc 3240
   acaaaaaatt gctcctaact ccaccgccta tcccaaacct acaagaacta atgataatcc 3300
   caccaccctt tgctgactct ttttggactc agcccacctg cacccaggtg attaaaaagc 3360
   tttattgttc acaca 3375
<210> 120
   <211> 3645
   <212> DNA
   <213> Homo sapiens
<400> 120
   tgtaaatgct cttctgacta atgcaaacca tgtgtccata gaaccagaag atttttccag 60
   gggaaaagag cccccacgcc ccgcccagct ataaggggcc atgcaccaag cagggtaccc 120
   aggctgcaga ggtgccatgg ctgagtcaca cctgctgcag tggctgctgc tgctgctgcc 180
   cacgctctgt ggcccaggca ctgctgcctg gaccacctca tccttggcct gtgcccaggg 240
   ccctgagttc tggtgccaaa gcctggagca agcattgcag tgcagagccc tagggcattg 300
   cctacaggaa gtctggggac atgtgggagc cgatgaccta tgccaagagt gtgaggacat 360
   cgtccacatc cttaacaaga tggccaagga ggccattttc caggacacga tgaggaagtt 420
   cctggagcag gagtgcaacg tcctcccctt gaagctgctc atgccccagt gcaaccaagt 480
   gcttgacgac tacttccccc tggtcatcga ctacttccag aaccagactg actcaaacgg 540
   catctgtatg cacctgggcc tgtgcaaatc ccggcagcca gagccagagc aggagccagg 600
   gatgtcagac cccctgccca aacctctgcg ggaccctctg ccagaccctc tgctggacaa 660
   gctcgtcctc cctgtgctgc ccggggccct ccaggcgagg cctgggcctc acacacagga 720
   tctctccgag cagcaattcc ccattcctct cccctattgc tggctctgca gggctctgat 780
   caagcggatc caagccatga ttcccaaggg tgcgctagct gtggcagtgg cccaggtgtg 840
   ccgcgtggta cctctggtgg cgggcggcat ctgccagtgc ctggctgagc gctactccgt 900
   catcctgctc gacacgctgc tgggccgcat gctgccccag ctggtctgcc gcctcgtcct 960
   ccggtgctcc atggatgaca gcgctggccc aagtgagccc actgccccct ccttagccca 1020
   atgcctgctc tcctcctccc cctaccctgc cactgcatga ccctctccct ctgtggtccc 1080
   actgcaatgc accaaggagg acagaaacca aacacctctg tagggtggcc ttgcctgctt 1140
   tccccctaat gctcacatct ccagggtcgc cgacaggaga atggctgccg cgagactctg 1200
   agtgccacct ctgcatgtcc gtgaccaccc aggccgggaa cagcagcgag caggccatac 1260
   cacaggcaat gctccaggcc tgtgttggct cctggctgga cagggaaaag ctggctccag 1320
   tttgttaagg gtttcaactg cagccagagg tgttccgtga gggctgatgg aggagtcggg 1380
   agggagccct agagtgatcc agagatgtgg agaggccagg accacacgac aggagagtcc 1440
   tgcaaaggga ccctccacag ctgtgtgtct ccttcctcag tgcaagcaat ttgtggagca 1500
   gcacacgccc cagctgctga ccctggtgcc caggggctgg gatgcccaca ccacctgcca 1560
   ggccctcggg gtgtgtggga ccatgtccag ccctctccag tgtatccaca gccccgacct 1620
   ttgatgagaa ctcagctgtc cagctgcaaa ggaaaagcca agtgagacgg gctctgggac 1680
   catggtgacc aggctcttcc cctgctccct ggccctcgcc agctgccagg ctgaaaagaa 1740
   gcctcagctc ccacaccgcc ctcctcaccg cccttcctcg gcagtcactt ccactggtgg 1800
   accacgggcc cccagccctg tgtcggcctt gtctgtctca gctcaaccac agtctgacac 1860
   cagagcccac ttccatcctc tctggtgtga ggcacagcga gggcagcatc tggaggagct 1920
   ctgcagcctc cacacctacc acgacctccc agggctgggc tcaggaaaaa ccagccactg 1980
   ctttacagga cagggggttg aagctgagcc ccgcctcaca cccaccccca tgcactcaaa 2040
   gattggattt tacagctact tgcaattcaa aattcagaag aataaaaaat gggaacatac 2100
   agaactctaa aagatagaca tcagaaattg ttaagttaag ctttttcaaa aaatcagcaa 2160
   ttccccagcg tagtcaaggg tggacactgc acgctctggc atgatgggat ggcgaccggg 2220
   caagctttct tcctcgagat gctctgctgc ttgagagcta ttgctttgtt aagatataaa 2280
   aaggggtttc tttttgtctt tctgtaaggt ggacttccag cttttgattg aaagtcctag 2340
   ggtgattcta tttctgctgt gatttatctg ctgaaagctc agctggggtt gtgcaagcta 2400
   gggacccatt cctgtgtaat acaatgtctg caccaatgct aataaagtcc tattctcttt 2460
   tatgagaaag aaaaagacac cgtcctttaa agtgctgcag tatggccaga cgtggtggct 2520
   cacacctgca atcccagcac cttaggaggc cgaggcagga ggatccttga ggtcaggagt 2580
   tcgagaccag cctcgccaac atggtgaaac cccatttcta ctaaaaatac aaaaaattag 2640
   ccaagtgtgg tggcatatgc ctgtaatccc aactactcag aaggccgagg caggagaatt 2700
   acttgaacgc aggagaatca ctgcagccca ggaggcagag gttgcagtga gccgagattg 2760
   caccactgca ctccagcctg ggtgacagag caagactcca tctcagtaaa taaataaata 2820
   aataaaaagc gctgcagtag ctgtggcctc accctgaagt cagcgggccc aggcctacct 2880
   cactctctcc cttggcagag aagcagacgt ccatagctcc tctccctcac aagcgctccc 2940
   agcctgccct ccagctgctg ctctcccctc ccagtctcta ctcactggga tgaggttagg 3000
   tcatgaggac accaaaaacc taaaaataaa caaaaagcca aacaagcctt agcttttctt    3060
   aaagactgaa atgcctggaa gtgtcccttt atttataaaa taacttttgt catatttctt    3120
   atacatgttt cttgtaagaa attcagaaac tacagacaaa gagagtggaa attacccact    3180
   gtcaggcctc tgagcccaag ctaagccatc atatcccctg tgccctgcac gtatacaccc    3240
   agatggcctg aagcaactga agatccacaa aagaagtgaa aatagccagt tcctgcctta    3300
   actgatgaca ttccaccatt gtgatttgtt cctgccccac cctaactgat caattgacct    3360
   tgtgacaata caccttcccc acccttgaga aggtgctttg taatattctc cccacccacc    3420
   ccacgcccgc acccccgcac ccttaagaag gtattttgta atattctctc cgccattgag    3480
   aatgtgcttt gtaagatcca ccccctgccc acaaaaaatt gctcctaact ccaccgccta    3540
   tcccaaacct acaagaacta atgataatcc caccaccctt tgctgactct ttttggactc    3600
   agcccacctg cacccaggtg attaaaaagc tttattgttc acaca                    3645
<210> 121
   <211> 3119
   <212> DNA
   <213> Homo sapiens
<400> 121
   tgtaaatgct cttctgacta atgcaaacca tgtgtccata gaaccagaag atttttccag     60
   gggaaaagag cccccacgcc ccgcccagct ataaggggcc atgcaccaag cagggtaccc    120
   aggctgcaga ggtgccatgg ctgagtcaca cctgctgcag tggctgctgc tgctgctgcc    180
   cacgctctgt ggcccaggca ctgctgcctg gaccacctca tccttggcct gtgcccaggg    240
   ccctgagttc tggtgccaaa gcctggagca agcattgcag tgcagagccc tagggcattg    300
   cctacaggaa gtctggggac atgtgggagc cgatgaccta tgccaagagt gtgaggacat    360
   cgtccacatc cttaacaaga tggccaagga ggccattttc caggacacga tgaggaagtt    420
   cctggagcag gagtgcaacg tcctcccctt gaagctgctc atgccccagt gcaaccaagt    480
   gcttgacgac tacttccccc tggtcatcga ctacttccag aaccagactg actcaaacgg    540
   catctgtatg cacctgggcc tgtgcaaatc ccggcagcca gagccagagc aggagccagg    600
   gatgtcagac cccctgccca aacctctgcg ggaccctctg ccagaccctc tgctggacaa    660
   gctcgtcctc cctgtgctgc ccggggccct ccaggcgagg cctgggcctc acacacagga    720
   tctctccgag cagcaattcc ccattcctct cccctattgc tggctctgca gggctctgat    780
   caagcggatc caagccatga ttcccaaggg tcgccgacag gagaatggct gccgcgagac    840
   tctgagtgcc acctctgcat gtccgtgacc acccaggccg ggaacagcag cgagcaggcc    900
   ataccacagg caatgctcca ggcctgtgtt ggctcctggc tggacaggga aaagtgcaag    960
   caatttgtgg agcagcacac gccccagctg ctgaccctgg tgcccagggg ctgggatgcc   1020
   cacaccacct gccaggccct cggggtgtgt gggaccatgt ccagccctct ccagtgtatc   1080
   cacagccccg acctttgatg agaactcagc tgtccagctg caaaggaaaa gccaagtgag 1140
   acgggctctg ggaccatggt gaccaggctc ttcccctgct ccctggccct cgccagctgc 1200
   caggctgaaa agaagcctca gctcccacac cgccctcctc accgcccttc ctcggcagtc 1260
   acttccactg gtggaccacg ggcccccagc cctgtgtcgg ccttgtctgt ctcagctcaa 1320
   ccacagtctg acaccagagc ccacttccat cctctctggt gtgaggcaca gcgagggcag 1380
   catctggagg agctctgcag cctccacacc taccacgacc tcccagggct gggctcagga 1440
   aaaaccagcc actgctttac aggacagggg gttgaagctg agccccgcct cacacccacc 1500
   cccatgcact caaagattgg attttacagc tacttgcaat tcaaaattca gaagaataaa 1560
   aaatgggaac atacagaact ctaaaagata gacatcagaa attgttaagt taagcttttt 1620
   caaaaaatca gcaattcccc agcgtagtca agggtggaca ctgcacgctc tggcatgatg 1680
   ggatggcgac cgggcaagct ttcttcctcg agatgctctg ctgcttgaga gctattgctt 1740
   tgttaagata taaaaagggg tttctttttg tctttctgta aggtggactt ccagcttttg 1800
   attgaaagtc ctagggtgat tctatttctg ctgtgattta tctgctgaaa gctcagctgg 1860
   ggttgtgcaa gctagggacc cattcctgtg taatacaatg tctgcaccaa tgctaataaa 1920
   gtcctattct cttttatgag aaagaaaaag acaccgtcct ttaaagtgct gcagtatggc 1980
   cagacgtggt ggctcacacc tgcaatccca gcaccttagg aggccgaggc aggaggatcc 2040
   ttgaggtcag gagttcgaga ccagcctcgc caacatggtg aaaccccatt tctactaaaa 2100
   atacaaaaaa ttagccaagt gtggtggcat atgcctgtaa tcccaactac tcagaaggcc 2160
   gaggcaggag aattacttga acgcaggaga atcactgcag cccaggaggc agaggttgca 2220
   gtgagccgag attgcaccac tgcactccag cctgggtgac agagcaagac tccatctcag 2280
   taaataaata aataaataaa aagcgctgca gtagctgtgg cctcaccctg aagtcagcgg 2340
   gcccaggcct acctcactct ctcccttggc agagaagcag acgtccatag ctcctctccc 2400
   tcacaagcgc tcccagcctg ccctccagct gctgctctcc cctcccagtc tctactcact 2460
   gggatgaggt taggtcatga ggacaccaaa aacctaaaaa taaacaaaaa gccaaacaag 2520
   ccttagcttt tcttaaagac tgaaatgcct ggaagtgtcc ctttatttat aaaataactt 2580
   ttgtcatatt tcttatacat gtttcttgta agaaattcag aaactacaga caaagagagt 2640
   ggaaattacc cactgtcagg cctctgagcc caagctaagc catcatatcc cctgtgccct 2700
   gcacgtatac acccagatgg cctgaagcaa ctgaagatcc acaaaagaag tgaaaatagc 2760
   cagttcctgc cttaactgat gacattccac cattgtgatt tgttcctgcc ccaccctaac 2820
   tgatcaattg accttgtgac aatacacctt ccccaccctt gagaaggtgc tttgtaatat 2880
   tctccccacc caccccacgc ccgcaccccc gcacccttaa gaaggtattt tgtaatattc 2940
   tctccgccat tgagaatgtg ctttgtaaga tccaccccct gcccacaaaa aattgctcct 3000
   aactccaccg cctatcccaa acctacaaga actaatgata atcccaccac cctttgctga   3060
   ctctttttgg actcagccca cctgcaccca ggtgattaaa aagctttatt gttcacaca    3119
<210> 122
   <211> 2394
   <212> DNA
   <213> Homo sapiens
<400> 122
   tgtaaatgct cttctgacta atgcaaacca tgtgtccata gaaccagaag atttttccag      60
   gggaaaagag cccccacgcc ccgcccagct ataaggggcc atgcaccaag cagggtaccc     120
   aggctgcaga ggtgccatgg ctgagtcaca cctgctgcag tggctgctgc tgctgctgcc     180
   cacgctctgt ggcccaggca ctgctgcctg gaccacctca tccttggcct gtgcccaggg     240
   ccctgagttc tggtgccaaa gcctggagca agcattgcag tgcagagccc tagggcattg     300
   cctacaggaa gtctggggac atgtgggagc cgatgaccta tgccaagagt gtgaggacat     360
   cgtccacatc cttaacaaga tggccaagga ggccattttc caggacacga tgaggaagtt     420
   cctggagcag gagtgcaacg tcctcccctt gaagctgctc atgccccagt gcaaccaagt     480
   gcttgacgac tacttccccc tggtcatcga ctacttccag aaccagactg actcaaacgg     540
   catctgtatg cacctgggcc tgtgcaaatc ccggcagcca gagccagagc aggagccagg     600
   gatgtcagac cccctgccca aacctctgcg ggaccctctg ccagaccctc tgctggacaa     660
   gctcgtcctc cctgtgctgc ccggggccct ccaggcgagg cctgggcctc acacacagga     720
   tctctccgag cagcaattcc ccattcctct cccctattgc tggctctgca gggctctgat    780
   caagcggatc caagccatga ttcccaaggg tgcgctagct gtggcagtgg cccaggtgtg     840
   ccgcgtggta cctctggtgg cgggcggcat ctgccagtgc ctggctgagc gctactccgt     900
   catcctgctc gacacgctgc tgggccgcat gctgccccag ctggtctgcc gcctcgtcct     960
   ccggtgctcc atggatgaca gcgctggccc aaggtcgccg acaggagaat ggctgccgcg    1020
   agactctgag tgccacctct gcatgtccgt gaccacccag gccgggaaca gcagcgagca    1080
   ggccatacca caggcaatgc tccaggcctg tgttggctcc tggctggaca gggaaaagtg    1140
   caagcaattt gtggagcagc acacgcccca gctgctgacc ctggtgccca ggggctggga    1200
   tgcccacacc acctgccaga aaaagacacc gtcctttaaa gtgctgcagt atggccagac    1260
   gtggtggctc acacctgcaa tcccagcacc ttaggaggcc gaggcaggag gatccttgag    1320
   gtcaggagtt cgagaccagc ctcgccaaca tggtgaaacc ccatttctac taaaaataca    1380
   aaaaattagc caagtgtggt ggcatatgcc tgtaatccca actactcaga aggccgaggc    1440
   aggagaatta cttgaacgca ggagaatcac tgcagcccag gaggcagagg ttgcagtgag    1500
   ccgagattgc accactgcac tccagcctgg gtgacagagc aagactccat ctcagtaaat    1560
   aaataaataa ataaaaagcg ctgcagtagc tgtggcctca ccctgaagtc agcgggccca    1620
   ggcctacctc actctctccc ttggcagaga agcagacgtc catagctcct ctccctcaca 1680
   agcgctccca gcctgccctc cagctgctgc tctcccctcc cagtctctac tcactgggat 1740
   gaggttaggt catgaggaca ccaaaaacct aaaaataaac aaaaagccaa acaagcctta 1800
   gcttttctta aagactgaaa tgcctggaag tgtcccttta tttataaaat aacttttgtc 1860
   atatttctta tacatgtttc ttgtaagaaa ttcagaaact acagacaaag agagtggaaa 1920
   ttacccactg tcaggcctct gagcccaagc taagccatca tatcccctgt gccctgcacg 1980
   tatacaccca gatggcctga agcaactgaa gatccacaaa agaagtgaaa atagccagtt 2040
   cctgccttaa ctgatgacat tccaccattg tgatttgttc ctgccccacc ctaactgatc 2100
   aattgacctt gtgacaatac accttcccca cccttgagaa ggtgctttgt aatattctcc 2160
   ccacccaccc cacgcccgca cccccgcacc cttaagaagg tattttgtaa tattctctcc 2220
   gccattgaga atgtgctttg taagatccac cccctgccca caaaaaattg ctcctaactc 2280
   caccgcctat cccaaaccta caagaactaa tgataatccc accacccttt gctgactctt 2340
   tttggactca gcccacctgc acccaggtga ttaaaaagct ttattgttca caca 2394
<210> 123
   <211> 3213
   <212> DNA
   <213> Homo sapiens
<400> 123
   tgtaaatgct cttctgacta atgcaaacca tgtgtccata gaaccagaag atttttccag     60
   gggaaaagag cccccacgcc ccgcccagct ataaggggcc atgcaccaag cagggtaccc    120
   aggctgcaga ggtgccatgg ctgagtcaca cctgctgcag tggctgctgc tgctgctgcc    180
   cacgctctgt ggcccaggca ctgctgcctg gaccacctca tccttggcct gtgcccaggg    240
   ccctgagttc tggtgccaaa gcctggagca agcattgcag tgcagagccc tagggcattg    300
   cctacaggaa gtctggggac atgtgggagc cgatgaccta tgccaagagt gtgaggacat    360
   cgtccacatc cttaacaaga tggccaagga ggccattttc caggacacga tgaggaagtt    420
   cctggagcag gagtgcaacg tcctcccctt gaagctgctc atgccccagt gcaaccaagt    480
   gcttgacgac tacttccccc tggtcatcga ctacttccag aaccagactg actcaaacgg    540
   catctgtatg cacctgggcc tgtgcaaatc ccggcagcca gagccagagc aggagccagg    600
   gatgtcagac cccctgccca aacctctgcg ggaccctctg ccagaccctc tgctggacaa    660
   gctcgtcctc cctgtgctgc ccggggccct ccaggcgagg cctgggcctc acacacaggg    720
   tgcgctagct gtggcagtgg cccaggtgtg ccgcgtggta cctctggtgg cgggcggcat    780
   ctgccagtgc ctggctgagc gctactccgt catcctgctc gacacgctgc tgggccgcat    840
   gctgccccag ctggtctgcc gcctcgtcct ccggtgctcc atggatgaca gcgctggccc    900
   aaggtcgccg acaggagaat ggctgccgcg agactctgag tgccacctct gcatgtccgt 960
   gaccacccag gccgggaaca gcagcgagca ggccatacca caggcaatgc tccaggcctg 1020
   tgttggctcc tggctggaca gggaaaagtg caagcaattt gtggagcagc acacgcccca 1080
   gctgctgacc ctggtgccca ggggctggga tgcccacacc acctgccagg ccctcggggt 1140
   gtgtgggacc atgtccagcc ctctccagtg tatccacagc cccgaccttt gatgagaact 1200
   cagctgtcca gctgcaaagg aaaagccaag tgagacgggc tctgggacca tggtgaccag 1260
   gctcttcccc tgctccctgg ccctcgccag ctgccaggct gaaaagaagc ctcagctccc 1320
   acaccgccct cctcaccgcc cttcctcggc agtcacttcc actggtggac cacgggcccc 1380
   cagccctgtg tcggccttgt ctgtctcagc tcaaccacag tctgacacca gagcccactt 1440
   ccatcctctc tggtgtgagg cacagcgagg gcagcatctg gaggagctct gcagcctcca 1500
   cacctaccac gacctcccag ggctgggctc aggaaaaacc agccactgct ttacaggaca 1560
   gggggttgaa gctgagcccc gcctcacacc cacccccatg cactcaaaga ttggatttta 1620
   cagctacttg caattcaaaa ttcagaagaa taaaaaatgg gaacatacag aactctaaaa 1680
   gatagacatc agaaattgtt aagttaagct ttttcaaaaa atcagcaatt ccccagcgta 1740
   gtcaagggtg gacactgcac gctctggcat gatgggatgg cgaccgggca agctttcttc 1800
   ctcgagatgc tctgctgctt gagagctatt gctttgttaa gatataaaaa ggggtttctt 1860
   tttgtctttc tgtaaggtgg acttccagct tttgattgaa agtcctaggg tgattctatt 1920
   tctgctgtga tttatctgct gaaagctcag ctggggttgt gcaagctagg gacccattcc 1980
   tgtgtaatac aatgtctgca ccaatgctaa taaagtccta ttctctttta tgagaaagaa 2040
   aaagacaccg tcctttaaag tgctgcagta tggccagacg tggtggctca cacctgcaat 2100
   cccagcacct taggaggccg aggcaggagg atccttgagg tcaggagttc gagaccagcc 2160
   tcgccaacat ggtgaaaccc catttctact aaaaatacaa aaaattagcc aagtgtggtg 2220
   gcatatgcct gtaatcccaa ctactcagaa ggccgaggca ggagaattac ttgaacgcag 2280
   gagaatcact gcagcccagg aggcagaggt tgcagtgagc cgagattgca ccactgcact 2340
   ccagcctggg tgacagagca agactccatc tcagtaaata aataaataaa taaaaagcgc 2400
   tgcagtagct gtggcctcac cctgaagtca gcgggcccag gcctacctca ctctctccct 2460
   tggcagagaa gcagacgtcc atagctcctc tccctcacaa gcgctcccag cctgccctcc 2520
   agctgctgct ctcccctccc agtctctact cactgggatg aggttaggtc atgaggacac 2580
   caaaaaccta aaaataaaca aaaagccaaa caagccttag cttttcttaa agactgaaat 2640
   gcctggaagt gtccctttat ttataaaata acttttgtca tatttcttat acatgtttct 2700
   tgtaagaaat tcagaaacta cagacaaaga gagtggaaat tacccactgt caggcctctg 2760
   agcccaagct aagccatcat atcccctgtg ccctgcacgt atacacccag atggcctgaa 2820
   gcaactgaag atccacaaaa gaagtgaaaa tagccagttc ctgccttaac tgatgacatt 2880
   ccaccattgt gatttgttcc tgccccaccc taactgatca attgaccttg tgacaataca 2940
   ccttccccac ccttgagaag gtgctttgta atattctccc cacccacccc acgcccgcac 3000
   ccccgcaccc ttaagaaggt attttgtaat attctctccg ccattgagaa tgtgctttgt 3060
   aagatccacc ccctgcccac aaaaaattgc tcctaactcc accgcctatc ccaaacctac 3120
   aagaactaat gataatccca ccaccctttg ctgactcttt ttggactcag cccacctgca 3180
   cccaggtgat taaaaagctt tattgttcac aca 3213
<210> 124
   <211> 3177
   <212> DNA
   <213> Homo sapiens
<400> 124
   tgtaaatgct cttctgacta atgcaaacca tgtgtccata gaaccagaag atttttccag 60
   gggaaaagag cccccacgcc ccgcccagct ataaggggcc atgcaccaag cagggtaccc 120
   aggctgcaga ggtgccatgg ctgagtcaca cctgctgcag tggctgctgc tgctgctgcc 180
   cacgctctgt ggcccaggca ctgctgcctg gaccacctca tccttggcct gtgcccaggg 240
   ccctgagttc tggtgccaaa gcctggagca agcattgcag tgcagagccc tagggcattg 300
   cctacaggaa gtctggggac atgtgggagc cgatgaccta tgccaagagt gtgaggacat 360
   cgtccacatc cttaacaaga tggccaagga ggccattttc caggactcaa acggcatctg 420
   tatgcacctg ggcctgtgca aatcccggca gccagagcca gagcaggagc cagggatgtc 480
   agaccccctg cccaaacctc tgcgggaccc tctgccagac cctctgctgg acaagctcgt 540
   cctccctgtg ctgcccgggg ccctccaggc gaggcctggg cctcacacac aggatctctc 600
   cgagcagcaa ttccccattc ctctccccta ttgctggctc tgcagggctc tgatcaagcg 660
   gatccaagcc atgattccca agggtgcgct agctgtggca gtggcccagg tgtgccgcgt 720
   ggtacctctg gtggcgggcg gcatctgcca gtgcctggct gagcgctact ccgtcatcct 780
   gctcgacacg ctgctgggcc gcatgctgcc ccagctggtc tgccgcctcg tcctccggtg 840
   ctccatggat gacagcgctg gcccaaggtc gccgacagga gaatggctgc cgcgagactc 900
   tgagtgccac ctctgcatgt ccgtgaccac ccaggccggg aacagcagcg agcaggccat 960
   accacaggca atgctccagg cctgtgttgg ctcctggctg gacagggaaa agtgcaagca 1020
   atttgtggag cagcacacgc cccagctgct gaccctggtg cccaggggct gggatgccca 1080
   caccacctgc caggccctcg ggtgtgtggg gaccatgtcc agccctctcc agtgtatcca 1140
   cagccccgac ctttgatgag aactcagctg tccagctgca aaggaaaagc caagtgagac 1200
   gggctctggg accatggtga ccaggctctt cccctgctcc ctggccctcg ccagctgcca 1260
   ggctgaaaag aagcctcagc tcccacaccg ccctcctcac cgcccttcct cggcagtcac 1320
   ttccactggt ggaccacggg cccccagccc tgtgtcggcc ttgtctgtct cagctcaacc 1380
   acagtctgac accagagccc acttccatcc tctctggtgt gaggcacagc gagggcagca 1440
   tctggaggag ctctgcagcc tccacaccta ccacgacctc ccagggctgg gctcaggaaa 1500
   aaccagccac tgctttacag gacagggggt tgaagctgag ccccgcctca cacccacccc 1560
   catgcactca aagattggat tttacagcta cttgcaattc aaaattcaga agaataaaaa 1620
   atgggaacat acagaactct aaaagataga catcagaaat tgttaagtta agctttttca 1680
   aaaaatcagc aattccccag cgtagtcaag ggtggacact gcacgctctg gcatgatggg 1740
   atggcgaccg ggcaagcttt cttcctcgag atgctctgct gcttgagagc tattgctttg 1800
   ttaagatata aaaaggggtt tctttttgtc tttctgtaag gtggacttcc agcttttgat 1860
   tgaaagtcct agggtgattc tatttctgct gtgatttatc tgctgaaagc tcagctgggg 1920
   ttgtgcaagc tagggaccca ttcctgtgta atacaatgtc tgcaccaatg ctaataaagt 1980
   cctattctct tttatgagaa agaaaaagac accgtccttt aaagtgctgc agtatggcca 2040
   gacgtggtgg ctcacacctg caatcccagc accttaggag gccgaggcag gaggatcctt 2100
   gaggtcagga gttcgagacc agcctcgcca acatggtgaa accccatttc tactaaaaat 2160
   acaaaaaatt agccaagtgt ggtggcatat gcctgtaatc ccaactactc agaaggccga 2220
   ggcaggagaa ttacttgaac gcaggagaat cactgcagcc caggaggcag aggttgcagt 2280
   gagccgagat tgcaccactg cactccagcc tgggtgacag agcaagactc catctcagta 2340
   aataaataaa taaataaaaa gcgctgcagt agctgtggcc tcaccctgaa gtcagcgggc 2400
   ccaggcctac ctcactctct cccttggcag agaagcagac gtccatagct cctctccctc 2460
   acaagcgctc ccagcctgcc ctccagctgc tgctctcccc tcccagtctc tactcactgg 2520
   gatgaggtta ggtcatgagg acaccaaaaa cctaaaaata aacaaaaagc caaacaagcc 2580
   ttagcttttc ttaaagactg aaatgcctgg aagtgtccct ttatttataa aataactttt 2640
   gtcatatttc ttatacatgt ttcttgtaag aaattcagaa actacagaca aagagagtgg 2700
   aaattaccca ctgtcaggcc tctgagccca agctaagcca tcatatcccc tgtgccctgc 2760
   acgtatacac ccagatggcc tgaagcaact gaagatccac aaaagaagtg aaaatagcca 2820
   gttcctgcct taactgatga cattccacca ttgtgatttg ttcctgcccc accctaactg 2880
   atcaattgac cttgtgacaa tacaccttcc ccacccttga gaaggtgctt tgtaatattc 2940
   tccccaccca ccccacgccc gcacccccgc acccttaaga aggtattttg taatattctc 3000
   tccgccattg agaatgtgct ttgtaagatc caccccctgc ccacaaaaaa ttgctcctaa 3060
   ctccaccgcc tatcccaaac ctacaagaac taatgataat cccaccaccc tttgctgact 3120
   ctttttggac tcagcccacc tgcacccagg tgattaaaaa gctttattgt tcacaca 3177
<210> 125
   <211> 3226
   <212> DNA
   <213> Homo sapiens
<400> 125
   catagaataa agatggatgg gatgggatgg gatgggatgg gatgacacag aataaagatg      60
   gatggattgg gatggatgaa tagaagagat ggatgggata aattgatatg gatgagatgg     120
   gacaagttgg gctggtgggc agctgcatgt gccttggagt gctctgttgg cctcttccta     180
   agagaacctc cccattggag ctgggagcct cccccactca tgtgtcctcc accttggggc     240
   ccctccctcc ccaggatgac ctatgccaag agtgtgagga catcgtccac atccttaaca     300
   agatggccaa ggaggccatt ttccaggaca cgatgaggaa gttcctggag caggagtgca     360
   acgtcctccc cttgaagctg ctcatgcccc agtgcaacca agtgcttgac gactacttcc     420
   ccctggtcat cgactacttc cagaaccaga ctgactcaaa cggcatctgt atgcacctgg     480
   gcctgtgcaa atcccggcag ccagagccag agcaggagcc agggatgtca gaccccctgc    540
   ccaaacctct gcgggaccct ctgccagacc ctctgctgga caagctcgtc ctccctgtgc     600
   tgcccggggc cctccaggcg aggcctgggc ctcacacaca ggatctctcc gagcagcaat     660
   tccccattcc tctcccctat tgctggctct gcagggctct gatcaagcgg atccaagcca     720
   tgattcccaa gggtgcgcta gctgtggcag tggcccaggt gtgccgcgtg gtacctctgg     780
   tggcgggcgg catctgccag tgcctggctg agcgctactc cgtcatcctg ctcgacacgc     840
   tgctgggccg catgctgccc cagctggtct gccgcctcgt cctccggtgc tccatggatg     900
   acagcgctgg cccaaggtcg ccgacaggag aatggctgcc gcgagactct gagtgccacc     960
   tctgcatgtc cgtgaccacc caggccggga acagcagcga gcaggccata ccacaggcaa    1020
   tgctccaggc ctgtgttggc tcctggctgg acagggaaaa gtgcaagcaa tttgtggagc    1080
   agcacacgcc ccagctgctg accctggtgc ccaggggctg ggatgcccac accacctgcc    1140
   aggccctcgg ggtgtgtggg accatgtcca gccctctcca gtgtatccac agccccgacc    1200
   tttgatgaga actcagctgt ccagctgcaa aggaaaagcc aagtgagacg ggctctggga    1260
   ccatggtgac caggctcttc ccctgctccc tggccctcgc cagctgccag gctgaaaaga    1320
   agcctcagct cccacaccgc cctcctcacc gcccttcctc ggcagtcact tccactggtg    1380
   gaccacgggc ccccagccct gtgtcggcct tgtctgtctc agctcaacca cagtctgaca    1440
   ccagagccca cttccatcct ctctggtgtg aggcacagcg agggcagcat ctggaggagc    1500
   tctgcagcct ccacacctac cacgacctcc cagggctggg ctcaggaaaa accagccact    1560
   gctttacagg acagggggtt gaagctgagc cccgcctcac acccaccccc atgcactcaa    1620
   agattggatt ttacagctac ttgcaattca aaattcagaa gaataaaaaa tgggaacata    1680
   cagaactcta aaagatagac atcagaaatt gttaagttaa gctttttcaa aaaatcagca    1740
   attccccagc gtagtcaagg gtggacactg cacgctctgg catgatggga tggcgaccgg    1800
   gcaagctttc ttcctcgaga tgctctgctg cttgagagct attgctttgt taagatataa 1860
   aaaggggttt ctttttgtct ttctgtaagg tggacttcca gcttttgatt gaaagtccta 1920
   gggtgattct atttctgctg tgatttatct gctgaaagct cagctggggt tgtgcaagct 1980
   agggacccat tcctgtgtaa tacaatgtct gcaccaatgc taataaagtc ctattctctt 2040
   ttatgagaaa gaaaaagaca ccgtccttta aagtgctgca gtatggccag acgtggtggc 2100
   tcacacctgc aatcccagca ccttaggagg ccgaggcagg aggatccttg aggtcaggag 2160
   ttcgagacca gcctcgccaa catggtgaaa ccccatttct actaaaaata caaaaaatta 2220
   gccaagtgtg gtggcatatg cctgtaatcc caactactca gaaggccgag gcaggagaat 2280
   tacttgaacg caggagaatc actgcagccc aggaggcaga ggttgcagtg agccgagatt 2340
   gcaccactgc actccagcct gggtgacaga gcaagactcc atctcagtaa ataaataaat 2400
   aaataaaaag cgctgcagta gctgtggcct caccctgaag tcagcgggcc caggcctacc 2460
   tcactctctc ccttggcaga gaagcagacg tccatagctc ctctccctca caagcgctcc 2520
   cagcctgccc tccagctgct gctctcccct cccagtctct actcactggg atgaggttag 2580
   gtcatgagga caccaaaaac ctaaaaataa acaaaaagcc aaacaagcct tagcttttct 2640
   taaagactga aatgcctgga agtgtccctt tatttataaa ataacttttg tcatatttct 2700
   tatacatgtt tcttgtaaga aattcagaaa ctacagacaa agagagtgga aattacccac 2760
   tgtcaggcct ctgagcccaa gctaagccat catatcccct gtgccctgca cgtatacacc 2820
   cagatggcct gaagcaactg aagatccaca aaagaagtga aaatagccag ttcctgcctt 2880
   aactgatgac attccaccat tgtgatttgt tcctgcccca ccctaactga tcaattgacc 2940
   ttgtgacaat acaccttccc cacccttgag aaggtgcttt gtaatattct ccccacccac 3000
   cccacgcccg cacccccgca cccttaagaa ggtattttgt aatattctct ccgccattga 3060
   gaatgtgctt tgtaagatcc accccctgcc cacaaaaaat tgctcctaac tccaccgcct 3120
   atcccaaacc tacaagaact aatgataatc ccaccaccct ttgctgactc tttttggact 3180
   cagcccacct gcacccaggt gattaaaaag ctttattgtt cacaca 3226
<210> 126
   <211> 4048
   <212> DNA
   <213> Homo sapiens
<400> 126
   catagaataa agatggatgg gatgggatgg gatgggatgg gatgacacag aataaagatg 60
   gatggattgg gatggatgaa tagaagagat ggatgggata aattgatatg gatgagatgg 120
   gacaagttgg gctggtgggc agctgcatgt gccttggagt gctctgttgg cctcttccta 180
   agagaacctc cccattggag ctgggagcct cccccactca tgtgtcctcc accttggggc 240
   ccctccctcc ccaggatgac ctatgccaag agtgtgagga catcgtccac atccttaaca 300
   agatggccaa ggaggccatt ttccaggaca cgatgaggaa gttcctggag caggagtgca 360
   acgtcctccc cttgaagctg ctcatgcccc agtgcaacca agtgcttgac gactacttcc 420
   ccctggtcat cgactacttc cagaaccaga ctgtgagggc tgcaagctca cctcctgcct 480
   gcctccccac gcaggcccct gtgcccaccc atggggagcc acacacacag caccccagcc 540
   agccagacac acacacacac acacacacac acacagcacc caagccggcc agacacaaac 600
   acacagcacc ccagccagcc ggacacacac acacacacac acacaacacc ccagctggcc 660
   ggacacacac acacacagta ccccagctgg ccggacacac acacacacag caccctatcc 720
   agacacatac acacacacag taccccagcc agctggaaac acacacacac acagcactcc 780
   atccagacac atacccacac agtaccccag ccagccagac acacacacac acacacacac 840
   acacacacac acagcacaca cacagcaccc cagctggcca cacacacaca cacacacacc 900
   ctgtccacaa agggcctagg aaactacgtg cccttcagcc atgcacccga ccatgggccc 960
   ccaggttcag gtgcacacgg tgggcctgta cgctcacaca cccttacacc ctcactctca 1020
   cacacatgct tacacactta ttcattctca catatatgct catgctcatt cacacacaat 1080
   cccggccacc tgccctaaag tccccacaca gccctatctt tgccttttgt ccccccacat 1140
   agagttctaa accacagcac ccccactagg cctgcttcct cccattccag tggtccctga 1200
   gcccttgggc cggcctgaat aggggtgggc ttccctccca gaccctaaca ctcccaccct 1260
   gtgctgtgcc ccaggactca aacggcatct gtatgcacct gggcctgtgc aaatcccggc 1320
   agccagagcc agagcaggag ccagggatgt cagaccccct gcccaaacct ctgcgggacc 1380
   ctctgccaga ccctctgctg gacaagctcg tcctccctgt gctgcccggg gccctccagg 1440
   cgaggcctgg gcctcacaca caggatctct ccgagcagca attccccatt cctctcccct 1500
   attgctggct ctgcagggct ctgatcaagc ggatccaagc catgattccc aagggtgcgc 1560
   tagctgtggc agtggcccag gtgtgccgcg tggtacctct ggtggcgggc ggcatctgcc 1620
   agtgcctggc tgagcgctac tccgtcatcc tgctcgacac gctgctgggc cgcatgctgc 1680
   cccagctggt ctgccgcctc gtcctccggt gctccatgga tgacagcgct ggcccaaggt 1740
   cgccgacagg agaatggctg ccgcgagact ctgagtgcca cctctgcatg tccgtgacca 1800
   cccaggccgg gaacagcagc gagcaggcca taccacaggc aatgctccag gcctgtgttg 1860
   gctcctggct ggacagggaa aagtgcaagc aatttgtgga gcagcacacg ccccagctgc 1920
   tgaccctggt gcccaggggc tgggatgccc acaccacctg ccaggccctc ggggtgtgtg 1980
   ggaccatgtc cagccctctc cagtgtatcc acagccccga cctttgatga gaactcagct 2040
   gtccagctgc aaaggaaaag ccaagtgaga cgggctctgg gaccatggtg accaggctct 2100
   tcccctgctc cctggccctc gccagctgcc aggctgaaaa gaagcctcag ctcccacacc 2160
   gccctcctca ccgcccttcc tcggcagtca cttccactgg tggaccacgg gcccccagcc 2220
   ctgtgtcggc cttgtctgtc tcagctcaac cacagtctga caccagagcc cacttccatc 2280
   ctctctggtg tgaggcacag cgagggcagc atctggagga gctctgcagc ctccacacct 2340
   accacgacct cccagggctg ggctcaggaa aaaccagcca ctgctttaca ggacaggggg 2400
   ttgaagctga gccccgcctc acacccaccc ccatgcactc aaagattgga ttttacagct 2460
   acttgcaatt caaaattcag aagaataaaa aatgggaaca tacagaactc taaaagatag 2520
   acatcagaaa ttgttaagtt aagctttttc aaaaaatcag caattcccca gcgtagtcaa 2580
   gggtggacac tgcacgctct ggcatgatgg gatggcgacc gggcaagctt tcttcctcga 2640
   gatgctctgc tgcttgagag ctattgcttt gttaagatat aaaaaggggt ttctttttgt 2700
   ctttctgtaa ggtggacttc cagcttttga ttgaaagtcc tagggtgatt ctatttctgc 2760
   tgtgatttat ctgctgaaag ctcagctggg gttgtgcaag ctagggaccc attcctgtgt 2820
   aatacaatgt ctgcaccaat gctaataaag tcctattctc ttttatgaga aagaaaaaga 2880
   caccgtcctt taaagtgctg cagtatggcc agacgtggtg gctcacacct gcaatcccag 2940
   caccttagga ggccgaggca ggaggatcct tgaggtcagg agttcgagac cagcctcgcc 3000
   aacatggtga aaccccattt ctactaaaaa tacaaaaaat tagccaagtg tggtggcata 3060
   tgcctgtaat cccaactact cagaaggccg aggcaggaga attacttgaa cgcaggagaa 3120
   tcactgcagc ccaggaggca gaggttgcag tgagccgaga ttgcaccact gcactccagc 3180
   ctgggtgaca gagcaagact ccatctcagt aaataaataa ataaataaaa agcgctgcag 3240
   tagctgtggc ctcaccctga agtcagcggg cccaggccta cctcactctc tcccttggca 3300
   gagaagcaga cgtccatagc tcctctccct cacaagcgct cccagcctgc cctccagctg 3360
   ctgctctccc ctcccagtct ctactcactg ggatgaggtt aggtcatgag gacaccaaaa 3420
   acctaaaaat aaacaaaaag ccaaacaagc cttagctttt cttaaagact gaaatgcctg 3480
   gaagtgtccc tttatttata aaataacttt tgtcatattt cttatacatg tttcttgtaa 3540
   gaaattcaga aactacagac aaagagagtg gaaattaccc actgtcaggc ctctgagccc 3600
   aagctaagcc atcatatccc ctgtgccctg cacgtataca cccagatggc ctgaagcaac 3660
   tgaagatcca caaaagaagt gaaaatagcc agttcctgcc ttaactgatg acattccacc 3720
   attgtgattt gttcctgccc caccctaact gatcaattga ccttgtgaca atacaccttc 3780
   cccacccttg agaaggtgct ttgtaatatt ctccccaccc accccacgcc cgcacccccg 3840
   cacccttaag aaggtatttt gtaatattct ctccgccatt gagaatgtgc tttgtaagat 3900
   ccaccccctg cccacaaaaa attgctccta actccaccgc ctatcccaaa cctacaagaa 3960
   ctaatgataa tcccaccacc ctttgctgac tctttttgga ctcagcccac ctgcacccag 4020
   gtgattaaaa agctttattg ttcacaca 4048
<210> 127
   <211> 319
   <212> PRT
   <213> Homo sapiens
<400> 127
   Met His Gln Ala Gly Tyr Pro Gly Cys Arg Gly Ala Met Ala Glu Ser
   1               5                   10                  15
   His Leu Leu Gln Trp Leu Leu Leu Leu Leu Pro Thr Leu Cys Gly Pro
               20                  25                  30
   Gly Thr Ala Ala Trp Thr Thr Ser Ser Leu Ala Cys Ala Gln Gly Pro
           35                  40                  45
   Glu Phe Trp Cys Gln Ser Leu Glu Gln Ala Leu Gln Cys Arg Ala Leu
       50                  55                  60
   Gly His Cys Leu Gln Glu Val Trp Gly His Val Gly Ala Asp Asp Leu
   65                  70                  75                  80
   Cys Gln Glu Cys Glu Asp Ile Val His Ile Leu Asn Lys Met Ala Lys
                   85                  90                  95
   Glu Ala Ile Phe Gln Asp Thr Met Arg Lys Phe Leu Glu Gln Glu Cys
               100                 105                 110
   Asn Val Leu Pro Leu Lys Leu Leu Met Pro Gln Cys Asn Gln Val Leu
           115                 120                 125
   Asp Asp Tyr Phe Pro Leu Val Ile Asp Tyr Phe Gln Asn Gln Thr Asp
       130                 135                 140
   Ser Asn Gly Ile Cys Met His Leu Gly Leu Cys Lys Ser Arg Gln Pro
   145                 150                 155                 160
   Glu Pro Glu Gln Glu Pro Gly Met Ser Asp Pro Leu Pro Lys Pro Leu
                   165                 170                 175
   Arg Asp Pro Leu Pro Asp Pro Leu Leu Asp Lys Leu Val Leu Pro Val
               180                 185                 190
   Leu Pro Gly Ala Leu Gln Ala Arg Pro Gly Pro His Thr Gln Asp Leu
           195                 200                 205
   Ser Glu Gln Gln Phe Pro Ile Pro Leu Pro Tyr Cys Trp Leu Cys Arg
       210                 215                 220
   Ala Leu Ile Lys Arg Ile Gln Ala Met Ile Pro Lys Gly Ala Leu Ala
   225                 230                 235                 240
   Val Ala Val Ala Gln Val Cys Arg Val Val Pro Leu Val Ala Gly Gly
   245 250 255
   Ile Cys Gln Cys Leu Ala Glu Arg Tyr Ser Val Ile Leu Leu Asp Thr
   260 265 270
   Leu Leu Gly Arg Met Leu Pro Gln Leu Val Cys Arg Leu Val Leu Arg
   275 280 285
   Cys Ser Met Asp Asp Ser Ala Gly Pro Ser Glu Pro Thr Ala Pro Ser
   290 295 300
   Leu Ala Gln Cys Leu Leu Ser Ser Ser Pro Tyr Pro Ala Thr Ala
   305 310 315
<210> 128
   <211> 365
   <212> PRT
   <213> Homo sapiens
<400> 128
<210> 129
   <211> 344
   <212> PRT
   <213> Homo sapiens
<400> 129
   Met Asp Glu Met Gly Gln Val Gly Leu Val Gly Ser Cys Met Cys Leu
   1               5                   10                  15
   Gly Val Leu Cys Trp Pro Leu Pro Lys Arg Thr Ser Pro Leu Glu Leu
               20                  25                  30
   Gly Ala Ser Pro Thr His Val Ser Ser Thr Leu Gly Pro Leu Pro Pro
           35                  40                  45
   Gln Asp Asp Leu Cys Gln Glu Cys Glu Asp Ile Val His Ile Leu Asn
       50                  55                  60
   Lys Met Ala Lys Glu Ala Ile Phe Gln Asp Thr Met Arg Lys Phe Leu
   65                  70                  75                  80
   Glu Gln Glu Cys Asn Val Leu Pro Leu Lys Leu Leu Met Pro Gln Cys
                   85                  90                  95
   Asn Gln Val Leu Asp Asp Tyr Phe Pro Leu Val Ile Asp Tyr Phe Gln
               100                 105                 110
   Asn Gln Thr Val Arg Ala Ala Ser Ser Pro Pro Ala Cys Leu Pro Thr
           115                 120                 125
   Gln Ala Pro Val Pro Thr His Gly Glu Pro His Thr Gln His Pro Ser
       130                 135                 140
   Gln Pro Asp Thr His Thr His Thr His Thr His Thr Ala Pro Lys Pro
   145                 150                 155                 160
   Ala Arg His Lys His Thr Ala Pro Gln Pro Ala Gly His Thr His Thr
                   165                 170                 175
   His Thr His Asn Thr Pro Ala Gly Arg Thr His Thr His Thr Val Pro
               180                 185                 190
   Gln Leu Ala Gly His Thr His Thr Gln His Pro Ile Gln Thr His Thr
           195                 200                 205
   His Thr Gln Tyr Pro Ser Gln Leu Glu Thr His Thr His Thr Ala Leu
       210                 215                 220
   His Pro Asp Thr Tyr Pro His Ser Thr Pro Ala Ser Gln Thr His Thr
   225                 230                 235                 240
   His Thr His Thr His Thr His Thr Gln His Thr His Ser Thr Pro Ala
                   245                 250                 255
   Gly His Thr His Thr His Thr His Pro Val His Lys Gly Pro Arg Lys
               260                 265                 270
   Leu Arg Ala Leu Gln Pro Cys Thr Arg Pro Trp Ala Pro Arg Phe Arg
   275 280 285
   Cys Thr Arg Trp Ala Cys Thr Leu Thr His Pro Tyr Thr Leu Thr Leu
   290 295 300
   Thr His Met Leu Thr His Leu Phe Ile Leu Thr Tyr Met Leu Met Leu
   305 310 315 320
   Ile His Thr Gln Ser Arg Pro Pro Ala Leu Lys Ser Pro His Ser Pro
   325 330 335
   Ile Phe Ala Phe Cys Pro Pro Thr
   340
<210> 130
   <211> 307
   <212> PRT
   <213> Homo sapiens
<400> 130
   Met Ala Glu Ser His Leu Leu Gln Trp Leu Leu Leu Leu Leu Pro Thr
   1 5 10 15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Leu Gly Leu Cys Lys
   130 135 140
   Ser Arg Gln Pro Glu Pro Glu Gln Glu Pro Gly Met Ser Asp Pro Leu
   145 150 155 160
   Pro Lys Pro Leu Arg Asp Pro Leu Pro Asp Pro Leu Leu Asp Lys Leu
   165 170 175
   Val Leu Pro Val Leu Pro Gly Ala Leu Gln Ala Arg Pro Gly Pro His
   180 185 190
   Thr Gln Asp Leu Ser Glu Gln Gln Phe Pro Ile Pro Leu Pro Tyr Cys
   195 200 205
   Trp Leu Cys Arg Ala Leu Ile Lys Arg Ile Gln Ala Met Ile Pro Lys
   210 215 220
   Gly Ala Leu Ala Val Ala Val Ala Gln Val Cys Arg Val Val Pro Leu
   225 230 235 240
   Val Ala Gly Gly Ile Cys Gln Cys Leu Ala Glu Arg Tyr Ser Val Ile
   245 250 255
   Leu Leu Asp Thr Leu Leu Gly Arg Met Leu Pro Gln Leu Val Cys Arg
   260 265 270
   Leu Val Leu Arg Cys Ser Met Asp Asp Ser Ala Gly Pro Ser Glu Pro
   275 280 285
   Thr Ala Pro Ser Leu Ala Gln Cys Leu Leu Ser Ser Ser Pro Tyr Pro
   290 295 300
   Ala Thr Ala
   305
<210> 131
   <211> 339
   <212> PRT
   <213> Homo sapiens
<400> 131
   Met Ala Glu Ser His Leu Leu Gln Trp Leu Leu Leu Leu Leu Pro Thr
   1               5                   10                  15
   Leu Cys Gly Pro Gly Thr Ala Ala Trp Thr Thr Ser Ser Leu Ala Cys
               20                  25                  30
   Ala Gln Gly Pro Glu Phe Trp Cys Gln Ser Leu Glu Gln Ala Leu Gln
           35                  40                  45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Leu Gly Leu Cys Lys
   130 135 140
   Ser Arg Gln Pro Glu Pro Glu Gln Glu Pro Gly Met Ser Asp Pro Leu
   145 150 155 160
   Pro Lys Pro Leu Arg Asp Pro Leu Pro Asp Pro Leu Leu Asp Lys Leu
   165 170 175
   Val Leu Pro Val Leu Pro Gly Ala Leu Gln Ala Arg Pro Gly Pro His
   180 185 190
   Thr Gln Asp Leu Ser Glu Gln Gln Phe Pro Ile Pro Leu Pro Tyr Cys
   195 200 205
   Trp Leu Cys Arg Ala Leu Ile Lys Arg Ile Gln Ala Met Ile Pro Lys
   210 215 220
   Gly Ala Leu Ala Val Ala Val Ala Gln Val Cys Arg Val Val Pro Leu
   225 230 235 240
   Val Ala Gly Gly Ile Cys Gln Cys Leu Ala Glu Arg Tyr Ser Val Ile
   245 250 255
   Leu Leu Asp Thr Leu Leu Gly Arg Met Leu Pro Gln Leu Val Cys Arg
   260 265 270
   Leu Val Leu Arg Cys Ser Met Asp Asp Ser Ala Gly Pro Arg Ser Pro
   275 280 285
   Thr Gly Glu Trp Leu Pro Arg Asp Ser Glu Cys His Leu Cys Met Ser
   290 295 300
   Val Thr Thr Gln Ala Gly Asn Ser Ser Glu Gln Ala Ile Pro Gln Ala
   305 310 315 320
   Met Leu Gln Ala Cys Val Gly Ser Trp Leu Asp Arg Glu Lys Leu Ala
   325 330 335
   Pro Val Cys
<210> 132
   <211> 297
   <212> PRT
   <213> Homo sapiens
<400> 132
   Met Ala Glu Ser His Leu Leu Gln Trp Leu Leu Leu Leu Leu Pro Thr
   1 5 10 15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Leu Gly Leu Cys Lys
   130 135 140
   Ser Arg Gln Pro Glu Pro Glu Gln Glu Pro Gly Met Ser Asp Pro Leu
   145 150 155 160
   Pro Lys Pro Leu Arg Asp Pro Leu Pro Asp Pro Leu Leu Asp Lys Leu
   165 170 175
   Val Leu Pro Val Leu Pro Gly Ala Leu Gln Ala Arg Pro Gly Pro His
   180 185 190
   Thr Gln Asp Leu Ser Glu Gln Gln Phe Pro Ile Pro Leu Pro Tyr Cys
   195 200 205
   Trp Leu Cys Arg Ala Leu Ile Lys Arg Ile Gln Ala Met Ile Pro Lys
   210 215 220
   Val Arg His Pro Gly Pro His Arg Ala Gln Glu His Thr His Thr Cys
   225 230 235 240
   Ser Ser Leu Gln Leu Pro Pro Leu Ser Gln Leu Thr Pro Pro Ser Gly
   245 250 255
   Pro Ser Trp Leu Pro Glu Val Arg Arg Gly Glu Ser Arg Leu Cys Ile
   260 265 270
   Ala Pro Thr Gln Gly Thr Leu Gly Leu Arg Leu Arg Pro Gly Arg Cys
   275 280 285
   Gln Ala Tyr Ser Ser Cys Asn Lys His
   290 295
<210> 133
   <211> 360
   <212> PRT
   <213> Homo sapiens
<400> 133
   Met Ala Glu Ser His Leu Leu Gln Trp Leu Leu Leu Leu Leu Pro Thr
   1 5 10 15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Val Arg Ala Ala Ser Ser Pro Pro Ala Cys Leu Pro Thr
   130 135 140
   Gln Ala Pro Val Pro Thr His Gly Glu Pro His Thr Gln His Pro Ser
   145 150 155 160
   Gln Pro Asp Thr His Thr His Thr His Thr His Thr Ala Pro Lys Pro
   165 170 175
   Ala Arg His Lys His Thr Ala Pro Gln Pro Ala Gly His Thr His Thr
   180 185 190
   His Thr His Asn Thr Pro Ala Gly Arg Thr His Thr His Thr Val Pro
   195 200 205
   Gln Leu Ala Gly His Thr His Thr Gln His Pro Ile Gln Thr His Thr
   210 215 220
   His Thr Gln Tyr Pro Ser Gln Leu Glu Thr His Thr His Thr Ala Leu
   225 230 235 240
   His Pro Asp Thr Tyr Pro His Ser Thr Pro Ala Ser Gln Thr His Thr
   245 250 255
   His Thr His Thr His Thr His Thr Gln His Thr His Ser Thr Pro Ala
   260 265 270
   Gly His Thr His Thr His Thr His Pro Val His Lys Gly Pro Arg Lys
   275 280 285
   Leu Arg Ala Leu Gln Pro Cys Thr Arg Pro Trp Ala Pro Arg Phe Arg
   290 295 300
   Cys Thr Arg Trp Ala Cys Thr Leu Thr His Pro Tyr Thr Leu Thr Leu
   305 310 315 320
   Thr His Met Leu Thr His Leu Phe Ile Leu Thr Tyr Met Leu Met Leu
   325 330 335
   Ile His Thr Gln Ser Arg Pro Pro Ala Leu Lys Ser Pro His Ser Pro
   340 345 350
   Ile Phe Ala Phe Cys Pro Pro Thr
   355 360
<210> 134
   <211> 405
   <212> PRT
   <213> Homo sapiens

<400> 134
   Met Ala Glu Ser His Leu Leu Gln Trp Leu Leu Leu Leu Leu Pro Thr
   1               5                   10                  15
   Leu Cys Gly Pro Gly Thr Ala Ala Trp Thr Thr Ser Ser Leu Ala Cys
               20                  25                  30
   Ala Gln Gly Pro Glu Phe Trp Cys Gln Ser Leu Glu Gln Ala Leu Gln
           35                  40                  45
   Cys Arg Ala Leu Gly His Cys Leu Gln Glu Val Trp Gly His Val Gly
       50                  55                  60
   Ala Arg Thr Ser Pro Leu Glu Leu Gly Ala Ser Pro Thr His Val Ser
   65                  70                  75                  80
   Ser Thr Leu Gly Pro Leu Pro Pro Gln Asp Asp Leu Cys Gln Glu Cys
                   85                  90                  95
   Glu Asp Ile Val His Ile Leu Asn Lys Met Ala Lys Glu Ala Ile Phe
               100                 105                 110
   Gln Asp Thr Met Arg Lys Phe Leu Glu Gln Glu Cys Asn Val Leu Pro
           115                 120                 125
   Leu Lys Leu Leu Met Pro Gln Cys Asn Gln Val Leu Asp Asp Tyr Phe
       130                 135                 140
   Pro Leu Val Ile Asp Tyr Phe Gln Asn Gln Thr Asp Ser Asn Gly Ile
   145                 150                 155                 160
   Cys Met His Leu Gly Leu Cys Lys Ser Arg Gln Pro Glu Pro Glu Gln
                   165                 170                 175
   Glu Pro Gly Met Ser Asp Pro Leu Pro Lys Pro Leu Arg Asp Pro Leu
               180                 185                 190
   Pro Asp Pro Leu Leu Asp Lys Leu Val Leu Pro Val Leu Pro Gly Ala
           195                 200                 205
   Leu Gln Ala Arg Pro Gly Pro His Thr Gln Asp Leu Ser Glu Gln Gln
       210                 215                 220
   Phe Pro Ile Pro Leu Pro Tyr Cys Trp Leu Cys Arg Ala Leu Ile Lys
   225                 230                 235                 240
   Arg Ile Gln Ala Met Ile Pro Lys Gly Ala Leu Ala Val Ala Val Ala
   245 250 255
   Gln Val Cys Arg Val Val Pro Leu Val Ala Gly Gly Ile Cys Gln Cys
   260 265 270
   Leu Ala Glu Arg Tyr Ser Val Ile Leu Leu Asp Thr Leu Leu Gly Arg
   275 280 285
   Met Leu Pro Gln Leu Val Cys Arg Leu Val Leu Arg Cys Ser Met Asp
   290 295 300
   Asp Ser Ala Gly Pro Arg Ser Pro Thr Gly Glu Trp Leu Pro Arg Asp
   305 310 315 320
   Ser Glu Cys His Leu Cys Met Ser Val Thr Thr Gln Ala Gly Asn Ser
   325 330 335
   Ser Glu Gln Ala Ile Pro Gln Ala Met Leu Gln Ala Cys Val Gly Ser
   340 345 350
   Trp Leu Asp Arg Glu Lys Cys Lys Gln Phe Val Glu Gln His Thr Pro
   355 360 365
   Gln Leu Leu Thr Leu Val Pro Arg Gly Trp Asp Ala His Thr Thr Cys
   370 375 380
   Gln Ala Leu Gly Val Cys Gly Thr Met Ser Ser Pro Leu Gln Cys Ile
   385 390 395 400
   His Ser Pro Asp Leu
   405
<210> 135
   <211> 243
   <212> PRT
   <213> Homo sapiens
<400> 135
   Met Ala Glu Ser His Leu Leu Gln Trp Leu Leu Leu Leu Leu Pro Thr
   1 5 10 15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Leu Gly Leu Cys Lys
   130 135 140
   Ser Arg Gln Pro Glu Pro Glu Gln Glu Pro Gly Met Ser Asp Pro Leu
   145 150 155 160
   Pro Lys Pro Leu Arg Asp Pro Leu Pro Asp Pro Leu Leu Asp Lys Leu
   165 170 175
   Val Leu Pro Val Leu Pro Gly Ala Leu Gln Ala Arg Pro Gly Pro His
   180 185 190
   Thr Gln Asp Leu Ser Glu Gln Gln Phe Pro Ile Pro Leu Pro Tyr Cys
   195 200 205
   Trp Leu Cys Arg Ala Leu Ile Lys Arg Ile Gln Ala Met Ile Pro Lys
   210 215 220
   Gly Arg Arg Gln Glu Asn Gly Cys Arg Glu Thr Leu Ser Ala Thr Ser
   225 230 235 240
   Ala Cys Pro
<210> 136
   <211> 385
   <212> PRT
   <213> Homo sapiens
<400> 136
   Met Ala Glu Ser His Leu Leu Gln Trp Leu Leu Leu Leu Leu Pro Thr
   1 5 10 15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Thr Met Arg Lys Phe Leu
   85 90 95
   Glu Gln Glu Cys Asn Val Leu Pro Leu Lys Leu Leu Met Pro Gln Cys
   100 105 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Leu Gly Leu Cys Lys
   130 135 140
   Ser Arg Gln Pro Glu Pro Glu Gln Glu Pro Gly Met Ser Asp Pro Leu
   145 150 155 160
   Pro Lys Pro Leu Arg Asp Pro Leu Pro Asp Pro Leu Leu Asp Lys Leu
   165 170 175
   Val Leu Pro Val Leu Pro Gly Ala Leu Gln Ala Arg Pro Gly Pro His
   180 185 190
   Thr Gln Asp Leu Ser Glu Gln Gln Phe Pro Ile Pro Leu Pro Tyr Cys
   195 200 205
   Trp Leu Cys Arg Ala Leu Ile Lys Arg Ile Gln Ala Met Ile Pro Lys
   210 215 220
   Gly Ala Leu Ala Val Ala Val Ala Gln Val Cys Arg Val Val Pro Leu
   225 230 235 240
   Val Ala Gly Gly Ile Cys Gln Cys Leu Ala Glu Arg Tyr Ser Val Ile
   245 250 255
   Leu Leu Asp Thr Leu Leu Gly Arg Met Leu Pro Gln Leu Val Cys Arg
   260 265 270
   Leu Val Leu Arg Cys Ser Met Asp Asp Ser Ala Gly Pro Arg Ser Pro
   275 280 285
   Thr Gly Glu Trp Leu Pro Arg Asp Ser Glu Cys His Leu Cys Met Ser
   290 295 300
   Val Thr Thr Gln Ala Gly Asn Ser Ser Glu Gln Ala Ile Pro Gln Ala
   305 310 315 320
   Met Leu Gln Ala Cys Val Gly Ser Trp Leu Asp Arg Glu Lys Cys Lys
   325 330 335
   Gln Phe Val Glu Gln His Thr Pro Gln Leu Leu Thr Leu Val Pro Arg
   340 345 350
   Gly Trp Asp Ala His Thr Thr Cys Gln Lys Lys Thr Pro Ser Phe Lys
   355 360 365
   Val Leu Gln Tyr Gly Gln Thr Trp Trp Leu Thr Pro Ala Ile Pro Ala
   370 375 380
   Pro
   385
<210> 137
   <211> 351
   <212> PRT
   <213> Homo sapiens
<400> 137
   Met Ala Glu Ser His Leu Leu Gln Trp Leu Leu Leu Leu Leu Pro Thr
   1               5                   10                  15
   Leu Cys Gly Pro Gly Thr Ala Ala Trp Thr Thr Ser Ser Leu Ala Cys
               20                  25                  30
   Ala Gln Gly Pro Glu Phe Trp Cys Gln Ser Leu Glu Gln Ala Leu Gln
           35                  40                  45
   Cys Arg Ala Leu Gly His Cys Leu Gln Glu Val Trp Gly His Val Gly
       50                  55                  60
   Ala Asp Asp Leu Cys Gln Glu Cys Glu Asp Ile Val His Ile Leu Asn
   65                  70                  75                  80
   Lys Met Ala Lys Glu Ala Ile Phe Gln Asp Thr Met Arg Lys Phe Leu
                   85                  90                  95
   Glu Gln Glu Cys Asn Val Leu Pro Leu Lys Leu Leu Met Pro Gln Cys
               100                 105                 110
   Asn Gln Val Leu Asp Asp Tyr Phe Pro Leu Val Ile Asp Tyr Phe Gln
   115 120 125
   Asn Gln Thr Asp Ser Asn Gly Ile Cys Met His Leu Gly Leu Cys Lys
   130 135 140
   Ser Arg Gln Pro Glu Pro Glu Gln Glu Pro Gly Met Ser Asp Pro Leu
   145 150 155 160
   Pro Lys Pro Leu Arg Asp Pro Leu Pro Asp Pro Leu Leu Asp Lys Leu
   165 170 175
   Val Leu Pro Val Leu Pro Gly Ala Leu Gln Ala Arg Pro Gly Pro His
   180 185 190
   Thr Gln Gly Ala Leu Ala Val Ala Val Ala Gln Val Cys Arg Val Val
   195 200 205
   Pro Leu Val Ala Gly Gly Ile Cys Gln Cys Leu Ala Glu Arg Tyr Ser
   210 215 220
   Val Ile Leu Leu Asp Thr Leu Leu Gly Arg Met Leu Pro Gln Leu Val
   225 230 235 240
   Cys Arg Leu Val Leu Arg Cys Ser Met Asp Asp Ser Ala Gly Pro Arg
   245 250 255
   Ser Pro Thr Gly Glu Trp Leu Pro Arg Asp Ser Glu Cys His Leu Cys
   260 265 270
   Met Ser Val Thr Thr Gln Ala Gly Asn Ser Ser Glu Gln Ala Ile Pro
   275 280 285
   Gln Ala Met Leu Gln Ala Cys Val Gly Ser Trp Leu Asp Arg Glu Lys
   290 295 300
   Cys Lys Gln Phe Val Glu Gln His Thr Pro Gln Leu Leu Thr Leu Val
   305 310 315 320
   Pro Arg Gly Trp Asp Ala His Thr Thr Cys Gln Ala Leu Gly Val Cys
   325 330 335
   Gly Thr Met Ser Ser Pro Leu Gln Cys Ile His Ser Pro Asp Leu
   340 345 350
<210> 138
   <211> 339
   <212> PRT
   <213> Homo sapiens
<400> 138
   Met Ala Glu Ser His Leu Leu Gln Trp Leu Leu Leu Leu Leu Pro Thr
   1 5 10 15
   Leu Cys Gly Pro Gly Thr Ala Ala Trp Thr Thr Ser Ser Leu Ala Cys
   20 25 30
   Ala Gln Gly Pro Glu Phe Trp Cys Gln Ser Leu Glu Gln Ala Leu Gln
   35 40 45
   Cys Arg Ala Leu Gly His Cys Leu Gln Glu Val Trp Gly His Val Gly
   50 55 60
   Ala Asp Asp Leu Cys Gln Glu Cys Glu Asp Ile Val His Ile Leu Asn
   65 70 75 80
   Lys Met Ala Lys Glu Ala Ile Phe Gln Asp Ser Asn Gly Ile Cys Met
   85 90 95
   His Leu Gly Leu Cys Lys Ser Arg Gln Pro Glu Pro Glu Gln Glu Pro
   100 105 110
   Gly Met Ser Asp Pro Leu Pro Lys Pro Leu Arg Asp Pro Leu Pro Asp
   115 120 125
   Pro Leu Leu Asp Lys Leu Val Leu Pro Val Leu Pro Gly Ala Leu Gln
   130 135 140
   Ala Arg Pro Gly Pro His Thr Gln Asp Leu Ser Glu Gln Gln Phe Pro
   145 150 155 160
   Ile Pro Leu Pro Tyr Cys Trp Leu Cys Arg Ala Leu Ile Lys Arg Ile
   165 170 175
   Gln Ala Met Ile Pro Lys Gly Ala Leu Ala Val Ala Val Ala Gln Val
   180 185 190
   Cys Arg Val Val Pro Leu Val Ala Gly Gly Ile Cys Gln Cys Leu Ala
   195 200 205
   Glu Arg Tyr Ser Val Ile Leu Leu Asp Thr Leu Leu Gly Arg Met Leu
   210 215 220
   Pro Gln Leu Val Cys Arg Leu Val Leu Arg Cys Ser Met Asp Asp Ser
   225 230 235 240
   Ala Gly Pro Arg Ser Pro Thr Gly Glu Trp Leu Pro Arg Asp Ser Glu
   245 250 255
   Cys His Leu Cys Met Ser Val Thr Thr Gln Ala Gly Asn Ser Ser Glu
   260 265 270
   Gln Ala Ile Pro Gln Ala Met Leu Gln Ala Cys Val Gly Ser Trp Leu
   275 280 285
   Asp Arg Glu Lys Cys Lys Gln Phe Val Glu Gln His Thr Pro Gln Leu
   290 295 300
   Leu Thr Leu Val Pro Arg Gly Trp Asp Ala His Thr Thr Cys Gln Ala
   305 310 315 320
   Leu Gly Val Cys Gly Thr Met Ser Ser Pro Leu Gln Cys Ile His Ser
   325 330 335
   Pro Asp Leu
<210> 139
   <211> 68
   <212> DNA
   <213> Artificial Organism
<220>
   <223> Synthetic oligonucleotide
<400> 139
   tgtaaatgct cttctgacta atgcaaacca tgtgtccata gaaccagaag atttttccag 60
   gggaaaag 68
<210> 140
   <211> 135
   <212> DNA
   <213> Artificial Organism
<220>
   <223> Synthetic oligonucleotide
<400> 140
   agcccccacg ccccgcccag ctataagggg ccatgcacca agcagggtac ccaggctgca 60
   gaggtgccat ggctgagtca cacctgctgc agtggctgct gctgctgctg cccacgctct 120
   gtggcccagg cactg 135
<210> 141
   <211> 25
   <212> DNA
   <213> Artificial Organism
<220>
   <223> Synthetic oligonucleotide
<400> 141
   ctgcctggac cacctcatcc ttggc 25
<210> 142
   <211> 103
   <212> DNA
   <213> Artificial Organism
<220>
   <223> Synthetic oligonucleotide
<400> 142
   ctgtgcccag ggccctgagt tctggtgcca aagcctggag caagcattgc agtgcagagc 60
   cctagggcat tgcctacagg aagtctgggg acatgtggga gcc 103
<210> 143
   <211> 182
   <212> DNA
   <213> Artificial Organism
<220>
   <223> Synthetic oligonucleotide
<400> 143
   catagaataa agatggatgg gatgggatgg gatgggatgg gatgacacag aataaagatg 60
   gatggattgg gatggatgaa tagaagagat ggatgggata aattgatatg gatgagatgg 120
   gacaagttgg gctggtgggc agctgcatgt gccttggagt gctctgttgg cctcttccta 180
   ag 182
<210> 144
   <211> 72
   <212> DNA
   <213> Artificial Organism
<220>
   <223> Synthetic oligonucleotide
<400> 144
   agaacctccc cattggagct gggagcctcc cccactcatg tgtcctccac cttggggccc 60
   ctccctcccc ag 72
<210> 145
   <211> 72
   <212> DNA
   <213> Artificial Organism
<220>
   <223> Synthetic oligonucleotide
<400> 145
   gatgacctat gccaagagtg tgaggacatc gtccacatcc ttaacaagat ggccaaggag 60
   gccattttcc ag 72
<210> 146
   <211> 36
   <212> DNA
   <213> Artificial Organism
<220>
   <223> Synthetic oligonucleotide
<400> 146
   gacacgatga ggaagttcct ggagcaggag tgcaac 36
<210> 147
   <211> 90
   <212> DNA
   <213> Artificial Organism
<220>
   <223> Synthetic oligonucleotide
<400> 147
   gtcctcccct tgaagctgct catgccccag tgcaaccaag tgcttgacga ctacttcccc 60
   ctggtcatcg actacttcca gaaccagact 90
<210> 148
   <211> 186
   <212> DNA
   <213> Artificial Organism
<220>
   <223> Synthetic oligonucleotide
<400> 148
   gtgagggctg caagctcacc tcctgcctgc ctccccacgc aggcccctgt gcccacccat 60
   ggggagccac acacacagca ccccagccag ccagacacac acacacacac acacacacac 120
   acagcaccca agccggccag acacaaacac acagcacccc agccagccgg acacacacac 180
   acacac 186
<210> 149
   <211> 64
   <212> DNA
   <213> Artificial Organism
<220>
   <223> Synthetic oligonucleotide
<400> 149
   acacacaaca ccccagctgg ccggacacac acacacacag taccccagct ggccggacac 60
   acac 64
<210> 150
   <211> 572
   <212> DNA
   <213> Artificial Organism
<220>
   <223> Synthetic oligonucleotide
<400> 150
   acacacagca ccctatccag acacatacac acacacagta ccccagccag ctggaaacac 60
   acacacacac agcactccat ccagacacat acccacacag taccccagcc agccagacac 120
   acacacacac acacacacac acacacacac agcacacaca cagcacccca gctggccaca 180
   cacacacaca cacacaccct gtccacaaag ggcctaggaa actacgtgcc cttcagccat 240
   gcacccgacc atgggccccc aggttcaggt gcacacggtg ggcctgtacg ctcacacacc 300
   cttacaccct cactctcaca cacatgctta cacacttatt cattctcaca tatatgctca 360
   tgctcattca cacacaatcc cggccacctg ccctaaagtc cccacacagc cctatctttg 420
   ccttttgtcc ccccacatag agttctaaac cacagcaccc ccactaggcc tgcttcctcc 480
   cattccagtg gtccctgagc ccttgggccg gcctgaatag gggtgggctt ccctcccaga 540
   ccctaacact cccaccctgt gctgtgcccc ag 572
<210> 151
   <211> 24
   <212> DNA
   <213> Artificial Organism
<220>
   <223> Synthetic oligonucleotide
<400> 151
   gactcaaacg gcatctgtat gcac 24
<210> 152
   <211> 50
   <212> DNA
   <213> Artificial Organism
<220>
   <223> Synthetic oligonucleotide
<400> 152
   ctgggcctgt gcaaatcccg gcagccagag ccagagcagg agccagggat 50
<210> 153
   <211> 20
   <212> DNA
   <213> Artificial Organism
<220>
   <223> Synthetic oligonucleotide
<400> 153
   gtcagacccc ctgcccaaac 20
<210> 154
   <211> 29
   <212> DNA
   <213> Artificial Organism
<220>
   <223> Synthetic oligonucleotide
<400> 154
   ctctgcggga ccctctgcca gaccctctg 29
<210> 155
   <211> 28
   <212> DNA
   <213> Artificial Organism
<220>
   <223> Synthetic oligonucleotide
<400> 155
   ctggacaagc tcgtcctccc tgtgctgc 28
<210> 156
   <211> 38
   <212> DNA
   <213> Artificial Organism
<220>
   <223> Synthetic oligonucleotide
<400> 156
   ccggggccct ccaggcgagg cctgggcctc acacacag 38
<210> 157
   <211> 90
   <212> DNA
   <213> Artificial Organism
<220>
   <223> Synthetic oligonucleotide
<400> 157
   gatctctccg agcagcaatt ccccattcct ctcccctatt gctggctctg cagggctctg 60
   atcaagcgga tccaagccat gattcccaag 90
<210> 158
   <211> 554
   <212> DNA
   <213> Artificial Organism
<220>
   <223> Synthetic oligonucleotide
<400> 158
   gtgaggcatc cagggcctca cagagcccag gagcacacgc atacctgtag ctccctgcag 60
   ctcccacctc tctcccaact cacacccccg tcaggaccca gctggctgcc agaagttagg 120
   aggggagaga gccgcttgtg cattgccccc acccagggga ccctggggct caggctcagg 180
   cctggtaggt gccaggccta cagttcatgc aacaaacatt aagcccccac tgtatggagg 240
   tgccacgcca ggagccaaag tacaaaaacg gacaagacgc agctttgtcc tccagcagct 300
   caccatctga tggagaaaga tccccagagg tctctgtaga aaggttgctt tgatctttca 360
   agaggggaat ttccacagat agattcccca tccttgcctg agtccaactt ggagtcttcc 420
   agacctgcag tggctattgt ccaatggccc cgccagccca gggctacctt gcccaaattg 480
   gggcccaaat gaggaaaggc cctgccccct cagcctttcc cagatagggt tgcgtgggcc 540
   accaggggca caag 554
<210> 159
   <211> 61
   <212> DNA
   <213> Artificial Organism
<220>
   <223> Synthetic oligonucleotide
<400> 159
   gcagcaggtg aggttcctgc tgaggcaggt ggttcacttg agcccaggag ttcaagacca 60
   g 61
<210> 160
   <211> 368
   <212> DNA
   <213> Artificial Organism
<220>
   <223> Synthetic oligonucleotide
<400> 160
   cttgggcaac atggcgaaac cccgtctcta ctaagaatac aaaaattagc cagatgtgac      60
   aggtgcctgt agtcccagct actcgggagg ctgaggcagg agaatcactt gaacccagga     120
   ggcggaggtt gcagtgagcc gacatcacgc cactgtactc tagcctgggt gacagagcaa     180
   gactctgtct caaaaaaaaa gaaagaagga aagatcactg cagagattgc agtgagaggt     240
   gatgggacag ggacggagct gagggctggc ctggggatgc atttgggagg tgggcccact     300
   gctattgggc atggatgggc ctggagcgtg aggaccaggg aggactccaa agtgactttt     360
   acacactg                                                              368
<210> 161
   <211> 396
   <212> DNA
   <213> Artificial Organism
<220>
   <223> Synthetic oligonucleotide
<400> 161
   gccagagcaa ccagccctct gtaatgccag cagctgagat ggggagacta aagaagaaaa      60
   caggtttgag caaaaaaaca gagagctccc tcctggccat gttgagttca agatgcctgt     120
   gtgaagtgca ggagaggaga gtcaggcaag cagctgaatc ccaagcattg ggggaaggtc      180
   aggtccacca tgtcagtctg agagtcacta gctgtgggcc agagcctttg gggccagacg     240
   taggtctgaa gctggctcct acactcagtg accctgtgtg agtcccctgc atcccctgga     300
   ctctctgatc cccagtgtcc ttatttgtga atagccttgc cctcccttct agaagagaat     360
   gagggaatgc gtaggaagtg cccagctggg tgctgg                               396
<210> 162
   <211> 62
   <212> DNA
   <213> Artificial Organism
<220>
   <223> Synthetic oligonucleotide
<400> 162
   gcagagagtg gaggcttgcc aagtgaaggt cccatgctgg cctctctccg cccccgcccc      60
   ag                                                                     62
<210> 163
   <211> 184
   <212> DNA
   <213> Artificial Organism
<220>
   <223> Synthetic oligonucleotide
<400> 163
   ggtgcgctag ctgtggcagt ggcccaggtg tgccgcgtgg tacctctggt ggcgggcggc 60
   atctgccagt gcctggctga gcgctactcc gtcatcctgc tcgacacgct gctgggccgc 120
   atgctgcccc agctggtctg ccgcctcgtc ctccggtgct ccatggatga cagcgctggc 180
   ccaa 184
<210> 164
   <211> 172
   <212> DNA
   <213> Artificial Organism
<220>
   <223> Synthetic oligonucleotide
<400> 164
   gtgagcccac tgccccctcc ttagcccaat gcctgctctc ctcctccccc taccctgcca 60
   ctgcatgacc ctctccctct gtggtcccac tgcaatgcac caaggaggac agaaaccaaa 120
   cacctctgta gggtggcctt gcctgctttc cccctaatgc tcacatctcc ag 172
<210> 165
   <211> 12
   <212> DNA
   <213> Artificial Organism
<220>
   <223> Synthetic oligonucleotide
<400> 165
   ggtcgccgac ag 12
<210> 166
   <211> 32
   <212> DNA
   <213> Artificial Organism
<220>
   <223> Synthetic oligonucleotide
<400> 166
   gagaatggct gccgcgagac tctgagtgcc ac 32
<210> 167
   <211> 8
   <212> DNA
   <213> Artificial Organism
<220>
   <223> Synthetic oligonucleotide
<400> 167
   ctctgcat 8
<210> 168
   <211> 44
   <212> DNA
   <213> Artificial Organism
<220>
   <223> Synthetic oligonucleotide
<400> 168
   gtccgtgacc acccaggccg ggaacagcag cgagcaggcc atac 44
<210> 169
   <211> 29
   <212> DNA
   <213> Artificial Organism
<220>
   <223> Synthetic oligonucleotide
<400> 169
   cacaggcaat gctccaggcc tgtgttggc 29
<210> 170
   <211> 6
   <212> DNA
   <213> Artificial Organism
<220>
   <223> Synthetic oligonucleotide
<400> 170
   tcctgg 6
<210> 171
   <211> 15
   <212> DNA
   <213> Artificial Organism
<220>
   <223> Synthetic oligonucleotide
<400> 171
   ctggacaggg aaaag 15
<210> 172
   <211> 170
   <212> DNA
   <213> Artificial Organism
<220>
   <223> Synthetic oligonucleotide
<400> 172
   ctggctccag tttgttaagg gtttcaactg cagccagagg tgttccgtga gggctgatgg 60
   aggagtcggg agggagccct agagtgatcc agagatgtgg agaggccagg accacacgac 120
   aggagagtcc tgcaaaggga ccctccacag ctgtgtgtct ccttcctcag 170
<210> 173
   <211> 33
   <212> DNA
   <213> Artificial Organism
<220>
   <223> Synthetic oligonucleotide
<400> 173
   tgcaagcaat ttgtggagca gcacacgccc cag 33
<210> 174
   <211> 48
   <212> DNA
   <213> Artificial Organism
<220>
   <223> Synthetic oligonucleotide
<400> 174
   ctgctgaccc tggtgcccag gggctgggat gcccacacca cctgccag 48
<210> 175
   <211> 23
   <212> DNA
   <213> Artificial Organism
<220>
   <223> Synthetic oligonucleotide
<400> 175
   gccctcgggg tgtgtgggac cat 23
<210> 176
   <211> 16
   <212> DNA
   <213> Artificial Organism
<220>
   <223> Synthetic oligonucleotide
<400> 176
   gtccagccct ctccag 16
<210> 177
   <211> 43
   <212> DNA
   <213> Artificial Organism
<220>
   <223> Synthetic oligonucleotide
<400> 177
   tgtatccaca gccccgacct ttgatgagaa ctcagctgtc cag 43
<210> 178
   <211> 58
   <212> DNA
   <213> Artificial Organism
<220>
   <223> Synthetic oligonucleotide
<400> 178
   ctgcaaagga aaagccaagt gagacgggct ctgggaccat ggtgaccagg ctcttccc 58
<210> 179
   <211> 46
   <212> DNA
   <213> Artificial Organism
<220>
   <223> Synthetic oligonucleotide
<400> 179
   ctgctccctg gccctcgcca gctgccaggc tgaaaagaag cctcag 46
<210> 180
   <211> 68
   <212> DNA
   <213> Artificial Organism
<220>
   <223> Synthetic oligonucleotide
<400> 180
   ctcccacacc gccctcctca ccgcccttcc tcggcagtca cttccactgg tggaccacgg 60
   gcccccag 68
<210> 181
   <211> 37
   <212> DNA
   <213> Artificial Organism
<220>
   <223> Synthetic oligonucleotide
<400> 181
   ccctgtgtcg gccttgtctg tctcagctca accacag 37
<210> 182
   <211> 74
   <212> DNA
   <213> Artificial Organism
<220>
   <223> Synthetic oligonucleotide
<400> 182
   tctgacacca gagcccactt ccatcctctc tggtgtgagg cacagcgagg gcagcatctg 60
   gaggagctct gcag 74
<210> 183
   <211> 544
   <212> DNA
   <213> Artificial Organism
<220>
   <223> Synthetic oligonucleotide
<400> 183
   cctccacacc taccacgacc tcccagggct gggctcagga aaaaccagcc actgctttac 60
   aggacagggg gttgaagctg agccccgcct cacacccacc cccatgcact caaagattgg 120
   attttacagc tacttgcaat tcaaaattca gaagaataaa aaatgggaac atacagaact 180
   ctaaaagata gacatcagaa attgttaagt taagcttttt caaaaaatca gcaattcccc 240
   agcgtagtca agggtggaca ctgcacgctc tggcatgatg ggatggcgac cgggcaagct 300
   ttcttcctcg agatgctctg ctgcttgaga gctattgctt tgttaagata taaaaagggg 360
   tttctttttg tctttctgta aggtggactt ccagcttttg attgaaagtc ctagggtgat 420
   tctatttctg ctgtgattta tctgctgaaa gctcagctgg ggttgtgcaa gctagggacc 480
   cattcctgtg taatacaatg tctgcaccaa tgctaataaa gtcctattct cttttatgag 540
   aaag 544
<210> 184
   <211> 133
   <212> DNA
   <213> Artificial Organism
<220>
   <223> Synthetic oligonucleotide
<400> 184
   aaaaagacac cgtcctttaa agtgctgcag tatggccaga cgtggtggct cacacctgca 60
   atcccagcac cttaggaggc cgaggcagga ggatccttga ggtcaggagt tcgagaccag 120
   cctcgccaac atg 133
<210> 185
   <211> 173
   <212> DNA
   <213> Artificial Organism
<220>
   <223> Synthetic oligonucleotide
<400> 185
   gtgaaacccc atttctacta aaaatacaaa aaattagcca agtgtggtgg catatgcctg      60
   taatcccaac tactcagaag gccgaggcag gagaattact tgaacgcagg agaatcactg     120
   cagcccagga ggcagaggtt gcagtgagcc gagattgcac cactgcactc cag            173
<210> 186
   <211> 388
   <212> DNA
   <213> Artificial Organism
<220>
   <223> Synthetic oligonucleotide
<400> 186
   cctgggtgac agagcaagac tccatctcag taaataaata aataaataaa aagcgctgca      60
   gtagctgtgg cctcaccctg aagtcagcgg gcccaggcct acctcactct ctcccttggc     120
   agagaagcag acgtccatag ctcctctccc tcacaagcgc tcccagcctg ccctccagct     180
   gctgctctcc cctcccagtc tctactcact gggatgaggt taggtcatga ggacaccaaa     240
   aacctaaaaa taaacaaaaa gccaaacaag ccttagcttt tcttaaagac tgaaatgcct     300
   ggaagtgtcc ctttatttat aaaataactt ttgtcatatt tcttatacat gtttcttgta     360
   agaaattcag aaactacaga caaagaga                                        388
<210> 187
   <211> 112
   <212> DNA
   <213> Artificial Organism
<220>
   <223> Synthetic oligonucleotide
<400> 187
   gtggaaatta cccactgtca ggcctctgag cccaagctaa gccatcatat cccctgtgcc      60
   ctgcacgtat acacccagat ggcctgaagc aactgaagat ccacaaaaga ag             112
<210> 188
   <211> 733
   <212> DNA
   <213> Artificial Organism
<220>
   <223> Synthetic oligonucleotide
<400> 188
   tgaaaatagc cagttcctgc cttaactgat gacattccac cattgtgatt tgttcctgcc      60
   ccaccctaac tgatcaattg accttgtgac aatacacctt ccccaccctt gagaaggtgc     120
   tttgtaatat tctccccacc caccccacgc ccgcaccccc gcacccttaa gaaggtattt     180
   tgtaatattc tctccgccat tgagaatgtg ctttgtaaga tccaccccct gcccacaaaa    240
   aattgctcct aactccaccg cctatcccaa acctacaaga actaatgata atcccaccac     300
   cctttgctga ctctttttgg actcagccca cctgcaccca ggtgattaaa aagctttatt     360
   gttcacacaa agcctgtttg gtagtctctt cacagggaag catgtgacac ccacaatccc     420
   acctagccca ggagagagct acggcagggt gtgtgttttg acactgagct tggggctttt     480
   tccatcttct ccccacagcc tctggctcca cacctccacc gttcaagcgc cagaaagagc     540
   tgtctatgca gcctgctctt gggcctgggg atgagacaca caattcattg gctcctggat     600
   tttaagtaga catttgtaaa tctatagcta actactgtcc ttaaagccat tgtttccatt     660
   acaaaatcca actctctgag agaaaagggt gttttaaatt taaaaaaata aaaacaaaaa     720
   agtttgattg aga                                                        733
<210> 189
   <211> 1735
   <212> DNA
   <213> Homo sapiens
<400> 189
   aagaaaggga aggcaaccgg gcagcccagg ccccgccccg ccgctccccc acccgtgcgc 60
   ttataaagca caggaaccag agctggccac tcagtggttt cttggtgaca ctggatagaa 120
   cagctcaagc cttgccactt cgggcttctc actgcagctg ggcttggact tcggagtttt 180
   gccattgcca gtgggacgtc tgagactttc tccttcaagt acttggcaga tcactctctt 240
   agcagggtct gcgcttcgca gccgggatga agctggtttc cgtcgccctg atgtacctgg 300
   gttcgctcgc cttcctaggc gctgacaccg ctcggttgga tgtcgcgtcg gagtttcgaa 360
   agaagtgagt ccgggcagcg ccttccccct tgctggtacc tggcaggcaa ggggaactga 420
   ccgttggtcc cgaaggtcta gaagtgaatg ggagcaggga caggcctggg cgtcacctga 480
   acgcacgcga atcgggtctg cttgtgtttt ccaggtggaa taagtgggct ctgagtcgtg 540
   ggaagaggga actgcggatg tccagcagct accccaccgg gctcgctgac gtgaaggccg 600
   ggcctgccca gacccttatt cggccccagg acatgaaggg tgcctctcga agccccgaag 660
   acagcagtcc ggatgccgcc cgcatccgag tcaagcgcta ccgccagagc atgaacaact 720
   tccagggcct ccggagcttt ggctgccgct tcgggacgtg cacggtgcag aagctggcac 780
   accagatcta ccagttcaca gataaggaca aggacaacgt cgcccccagg agcaagatca 840
   gcccccaggg ctacggccgc cggcgccggc gctccctgcc cgaggccggc ccgggtcgga 900
   ctctggtgtc ttctaagcca caagcacacg gggctccagc ccccccgagt ggaagtgctc 960
   cccactttct ttaggattta ggcgcccatg gtacaaggaa tagtcgcgca agcatcccgc 1020
   tggtgcctcc cgggacgaag gacttcccga gcggtgtggg gaccgggctc tgacagccct 1080
   gcggagaccc tgagtccggg aggcaccgtc cggcggcgag ctctggcttt gcaagggccc 1140
   ctccttctgg gggcttcgct tccttagcct tgctcaggtg caagtgcccc agggggcggg 1200
   gtgcagaaga atccgagtgt ttgccaggct taaggagagg agaaactgag aaatgaatgc 1260
   tgagaccccc ggagcagggg tctgagccac agccgtgctc gcccacaaac tgatttctca 1320
   cggcgtgtca ccccaccagg gcgcaagcct cactattact tgaactttcc aaaacctaaa 1380
   gaggaaaagt gcaatgcgtg ttgtacatac agaggtaact atcaatattt aagtttgttg 1440
   ctgtcaagat tttttttgta acttcaaata tagagatatt tttgtacgtt atatattgta 1500
   ttaagggcat tttaaaagca attatattgt cctcccctat tttaagacgt gaatgtctca 1560
   gcgaggtgta aagttgttcg ccgcgtggaa tgtgagtgtg tttgtgtgca tgaaagagaa 1620
   agactgatta cctcctgtgt ggaagaagga aacaccgagt ctctgtataa tctatttaca 1680
   taaaatgggt gatatgcgaa cagcaaacca ataaactgtc tcaatgctga ttcat 1735
<210> 190
   <211> 1589
   <212> DNA
   <213> Homo sapiens
<400> 190
<210> 191
   <211> 1818
   <212> DNA
   <213> Homo sapiens
<400> 191
   aagaaaggga aggcaaccgg gcagcccagg ccccgccccg ccgctccccc acccgtgcgc      60
   ttataaagca caggaaccag agctggccac tcagtggttt cttggtgaca ctggatagaa     120
   cagctcaagc cttgccactt cgggcttctc actgcagctg ggcttggact tcggagtttt     180
   gccattgcca gtgggacgtc tgagactttc tccttcaagt acttggcaga tcactctctt     240
   agcagggtct gcgcttcgca gccgggatga agctggtttc cgtcgccctg atgtacctgg     300
   gttcgctcgc cttcctaggc gctgacaccg ctcggttgga tgtcgcgtcg gagtttcgaa     360
   agaagtggaa taagtgggct ctgagtcgtg ggaagaggga actgcggatg tccagcagct     420
   accccaccgg gctcgctgac gtgaaggccg ggcctgccca gacccttatt cggccccagg     480
   acatgaaggg tgcctctcga agccccgaag acaggtaact acgccctgtg ctgtccaggg     540
   acgggaggga aggaaggtgt gcgggaggag ttctctgtct ccactcccct ggcccggggg     600
   atcgtcgggg ctggaccgca gctcagatgg cgcgagcagt ttccagctcc ctctggctct     660
   agaatggctc ccgttcccgg tgttggggcc aaagctctgc ttgatggggt ctcaagttgc     720
   ctttcttccc cctccccccg cccgcagcag tccggatgcc gcccgcatcc gagtcaagcg     780
   ctaccgccag agcatgaaca acttccaggg cctccggagc tttggctgcc gcttcgggac     840
   gtgcacggtg cagaagctgg cacaccagat ctaccagttc acagataagg acaaggacaa     900
   cgtcgccccc aggagcaaga tcagccccca gggctacggc cgccggcgcc ggcgctccct     960
   gcccgaggcc ggcccgggtc ggactctggt gtcttctaag ccacaagcac acggggctcc    1020
   agcccccccg agtggaagtg ctccccactt tctttaggat ttaggcgccc atggtacaag    1080
   gaatagtcgc gcaagcatcc cgctggtgcc tcccgggacg aaggacttcc cgagcggtgt    1140
   ggggaccggg ctctgacagc cctgcggaga ccctgagtcc gggaggcacc gtccggcggc    1200
   gagctctggc tttgcaaggg cccctccttc tgggggcttc gcttccttag ccttgctcag    1260
   gtgcaagtgc cccagggggc ggggtgcaga agaatccgag tgtttgccag gcttaaggag     1320
   aggagaaact gagaaatgaa tgctgagacc cccggagcag gggtctgagc cacagccgtg    1380
   ctcgcccaca aactgatttc tcacggcgtg tcaccccacc agggcgcaag cctcactatt    1440
   acttgaactt tccaaaacct aaagaggaaa agtgcaatgc gtgttgtaca tacagaggta    1500
   actatcaata tttaagtttg ttgctgtcaa gatttttttt gtaacttcaa atatagagat 1560
   atttttgtac gttatatatt gtattaaggg cattttaaaa gcaattatat tgtcctcccc 1620
   tattttaaga cgtgaatgtc tcagcgaggt gtaaagttgt tcgccgcgtg gaatgtgagt 1680
   gtgtttgtgt gcatgaaaga gaaagactga ttacctcctg tgtggaagaa ggaaacaccg 1740
   agtctctgta taatctattt acataaaatg ggtgatatgc gaacagcaaa ccaataaact 1800
   gtctcaatgc tgattcat 1818
<210> 192
   <211> 1616
   <212> DNA
   <213> Homo sapiens
<400> 192
   aagaaaggga aggcaaccgg gcagcccagg ccccgccccg ccgctccccc acccgtgcgc      60
   ttataaagca caggaaccag agctggccac tcagtggttt cttggtgaca ctggatagaa     120
   cagctcaagc cttgccactt cgggcttctc actgcagctg ggcttggact tcggagtttt     180
   gccattgcca gtgggacgtc tgagactttc tccttcaagt acttggcaga tcactctctt     240
   agcagggtct gcgcttcgca gccgggatga agctggtttc cgtcgccctg atgtacctgg     300
   gttcgctcgc cttcctaggc gctgacaccg ctcggttgga tgtcgcgtcg gagtttcgaa     360
   agaagtggaa taagtgggct ctgagtcgtg ggaagaggga actgcggatg tccagcagct     420
   accccaccgg gctcgctgac gtgaaggccg ggcctgccca gacccttatt cggccccagg     480
   acatgaaggg tgcctctcga agccccgaag acagttgcct ttcttccccc tccccccgcc     540
   cgcagcagtc cggatgccgc ccgcatccga gtcaagcgct accgccagag catgaacaac     600
   ttccagggcc tccggagctt tggctgccgc ttcgggacgt gcacggtgca gaagctggca     660
   caccagatct accagttcac agataaggac aaggacaacg tcgcccccag gagcaagatc     720
   agcccccagg gctacggccg ccggcgccgg cgctccctgc ccgaggccgg cccgggtcgg     780
   actctggtgt cttctaagcc acaagcacac ggggctccag cccccccgag tggaagtgct     840
   ccccactttc tttaggattt aggcgcccat ggtacaagga atagtcgcgc aagcatcccg     900
   ctggtgcctc ccgggacgaa ggacttcccg agcggtgtgg ggaccgggct ctgacagccc     960
   tgcggagacc ctgagtccgg gaggcaccgt ccggcggcga gctctggctt tgcaagggcc    1020
   cctccttctg ggggcttcgc ttccttagcc ttgctcaggt gcaagtgccc cagggggcgg    1080
   ggtgcagaag aatccgagtg tttgccaggc ttaaggagag gagaaactga gaaatgaatg    1140
   ctgagacccc cggagcaggg gtctgagcca cagccgtgct cgcccacaaa ctgatttctc    1200
   acggcgtgtc accccaccag ggcgcaagcc tcactattac ttgaactttc caaaacctaa    1260
   agaggaaaag tgcaatgcgt gttgtacata cagaggtaac tatcaatatt taagtttgtt    1320
   gctgtcaaga ttttttttgt aacttcaaat atagagatat ttttgtacgt tatatattgt    1380
   attaagggca ttttaaaagc aattatattg tcctccccta ttttaagacg tgaatgtctc    1440
   agcgaggtgt aaagttgttc gccgcgtgga atgtgagtgt gtttgtgtgc atgaaagaga    1500
   aagactgatt acctcctgtg tggaagaagg aaacaccgag tctctgtata atctatttac    1560
   ataaaatggg tgatatgcga acagcaaacc aataaactgt ctcaatgctg attcat        1616
<210> 193
   <211> 139
   <212> PRT
   <213> Homo sapiens
<400> 193
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Cys Leu Ser Ser Pro Ser Pro Arg Pro Gln Gln Ser Gly
                   85                  90                  95
   Cys Arg Pro His Pro Ser Gln Ala Leu Pro Pro Glu His Glu Gln Leu
               100                 105                 110
   Pro Gly Pro Pro Glu Leu Trp Leu Pro Leu Arg Asp Val His Gly Ala
           115                 120                 125
   Glu Ala Gly Thr Pro Asp Leu Pro Val His Arg
       130                 135
<210> 194
   <211> 33
   <212> PRT
   <213> Homo sapiens
<400> 194
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys
<210> 195
   <211> 83
   <212> PRT
   <213> Homo sapiens

<400> 195
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Arg
<210> 196
   <211> 245
   <212> DNA
   <213> Artificial Organism
<220>
   <223> Synthetic oligonucleotide
<400> 196
   aagaaaggga aggcaaccgg gcagcccagg ccccgccccg ccgctccccc acccgtgcgc 60
   ttataaagca caggaaccag agctggccac tcagtggttt cttggtgaca ctggatagaa 120
   cagctcaagc cttgccactt cgggcttctc actgcagctg ggcttggact tcggagtttt 180
   gccattgcca gtgggacgtc tgagactttc tccttcaagt acttggcaga tcactctctt 240
   agcag 245
<210> 197
   <211> 119
   <212> DNA
   <213> Artificial Organism
<220>
   <223> Synthetic oligonucleotide
<400> 197
   ggtctgcgct tcgcagccgg gatgaagctg gtttccgtcg ccctgatgta cctgggttcg 60
   ctcgccttcc taggcgctga caccgctcgg ttggatgtcg cgtcggagtt tcgaaagaa 119
<210> 198
   <211> 4
   <212> DNA
   <213> Artificial Organism
<220>
   <223> Synthetic oligonucleotide
<400> 198
   gtga 4
<210> 199
   <211> 146
   <212> DNA
   <213> Artificial Organism
<220>
   <223> Synthetic oligonucleotide
<400> 199
   gtccgggcag cgccttcccc cttgctggta cctggcaggc aaggggaact gaccgttggt 60
   cccgaaggtc tagaagtgaa tgggagcagg gacaggcctg ggcgtcacct gaacgcacgc 120
   gaatcgggtc tgcttgtgtt ttccag 146
<210> 200
   <211> 59
   <212> DNA
   <213> Artificial Organism
<220>
   <223> Synthetic oligonucleotide
<400> 200
   gtggaataag tgggctctga gtcgtgggaa gagggaactg cggatgtcca gcagctacc 59
<210> 201
   <211> 40
   <212> DNA
   <213> Artificial Organism
<220>
   <223> Synthetic oligonucleotide
<400> 201
   ccaccgggct cgctgacgtg aaggccgggc ctgcccagac 40
<210> 202
   <211> 51
   <212> DNA
   <213> Artificial Organism
<220>
   <223> Synthetic oligonucleotide
<400> 202
   ccttattcgg ccccaggaca tgaagggtgc ctctcgaagc cccgaagaca g 51
<210> 203
   <211> 202
   <212> DNA
   <213> Artificial Organism
<220>
   <223> Synthetic oligonucleotide
<400> 203
   gtaactacgc cctgtgctgt ccagggacgg gagggaagga aggtgtgcgg gaggagttct 60
   ctgtctccac tcccctggcc cgggggatcg tcggggctgg accgcagctc agatggcgcg 120
   agcagtttcc agctccctct ggctctagaa tggctcccgt tcccggtgtt ggggccaaag 180
   ctctgcttga tggggtctca ag 202
<210> 204
   <211> 31
   <212> DNA
   <213> Artificial Organism
<220>
   <223> Synthetic oligonucleotide
<400> 204
   ttgcctttct tccccctccc cccgcccgca g 31
<210> 205
   <211> 3
   <212> DNA
   <213> Artificial Organism
<220>
   <223> Synthetic oligonucleotide
<400> 205
   cag 3
<210> 206
   <211> 81
   <212> DNA
   <213> Artificial Organism
<220>
   <223> Synthetic oligonucleotide
<400> 206
   tccggatgcc gcccgcatcc gagtcaagcg ctaccgccag agcatgaaca acttccaggg 60
   cctccggagc tttggctgcc g 81
<210> 207
   <211> 161
   <212> DNA
   <213> Artificial Organism
<220>
   <223> Synthetic oligonucleotide
<400> 207
   cttcgggacg tgcacggtgc agaagctggc acaccagatc taccagttca cagataagga     60
   caaggacaac gtcgccccca ggagcaagat cagcccccag ggctacggcc gccggcgccg    120
   gcgctccctg cccgaggccg gcccgggtcg gactctggtg t                         161
<210> 208
   <211> 76
   <212> DNA
   <213> Artificial Organism
<220>
   <223> Synthetic oligonucleotide
<400> 208
   cttctaagcc acaagcacac ggggctccag cccccccgag tggaagtgct ccccactttc     60
   tttaggattt aggcgc                                                     76
<210> 209
   <211> 121
   <212> DNA
   <213> Artificial Organism
<220>
   <223> Synthetic oligonucleotide
<400> 209
   ccatggtaca aggaatagtc gcgcaagcat cccgctggtg cctcccggga cgaaggactt     60
   cccgagcggt gtggggaccg ggctctgaca gccctgcgga gaccctgagt ccgggaggca    120
   c                                                                    121
<210> 210
   <211> 629
   <212> DNA
   <213> Artificial Organism
<220>
   <223> Synthetic oligonucleotide
<400> 210
   cgtccggcgg cgagctctgg ctttgcaagg gcccctcctt ctgggggctt cgcttcctta 60
   gccttgctca ggtgcaagtg ccccaggggg cggggtgcag aagaatccga gtgtttgcca 120
   ggcttaagga gaggagaaac tgagaaatga atgctgagac ccccggagca ggggtctgag 180
   ccacagccgt gctcgcccac aaactgattt ctcacggcgt gtcaccccac cagggcgcaa 240
   gcctcactat tacttgaact ttccaaaacc taaagaggaa aagtgcaatg cgtgttgtac 300
   atacagaggt aactatcaat atttaagttt gttgctgtca agattttttt tgtaacttca 360
   aatatagaga tatttttgta cgttatatat tgtattaagg gcattttaaa agcaattata 420
   ttgtcctccc ctattttaag acgtgaatgt ctcagcgagg tgtaaagttg ttcgccgcgt 480
   ggaatgtgag tgtgtttgtg tgcatgaaag agaaagactg attacctcct gtgtggaaga 540
   aggaaacacc gagtctctgt ataatctatt tacataaaat gggtgatatg cgaacagcaa 600
   accaataaac tgtctcaatg ctgattcat 629
<210> 211
   <211> 1615
   <212> DNA
   <213> Homo sapiens
<400> 211
   acacatcagg ggcttgctct tgcaaaacca aaccacaaga cagacttgca aaagaaggca 60
   tgcacagctc agcactgctc tgttgcctgg tcctcctgac tggggtgagg gccagcccag 120
   gccagggcac ccagtctgag aacagctgca cccacttccc aggcaacctg cctaacatgc 180
   ttcgagatct ccgagatgcc ttcagcagac aaatgaagga tcagctggac aacttgttgt 240
   taaaggagtc cttgctggag gactttaagg gttacctggg ttgccaagcc ttgtctgaga 300
   tgatccagtt ttacctggag gaggtgatgc cccaagctga gaaccaagac ccagacatca 360
   aggcgcatgt gaactccctg ggggagaacc tgaagaccct caggctgagg ctacggcgct 420
   gtcatcgatt tcttccctgt gaaaacaaga gcaaggccgt ggagcaggtg aagaatgcct 480
   ttaataagct ccaagagaaa ggcatctaca aagccatgag tgagtttgac atcttcatca 540
   actacataga agcctacatg acaatgaaga tacgaaactg agacatcagg gtggcgactc 600
   tatagactct aggacataaa ttagaggtct ccaaaatcgg atctggggct ctgggatagc 660
   tgacccagcc ccttgagaaa ccttattgta cctctctcat agaatattta ttacctctga 720
   tacctcaacc cccatttcta tttatttact gagcttctct gtgaacgatt tagaaagaag 780
   cccaatatta taattttttt caatatttat tattttcacc tgtttttaag ctgtttccat 840
   agggtgacac actatggtat ttgagtgttt taagataaat tataagttac ataagggagg 900
   aaaaaaaatg ttctttgggg agccaacaga agcttccatt ccaagcctga ccacgctttc     960
   tagctgttga gctgttttcc ctgacctccc tctaatttat cttgtctctg ggcttggggc    1020
   ttcctaactg ctacaaatac tcttaggaag agaaaccagg gagccccttt gatgattaat    1080
   tcaccttcca gtgtctcgga gggattcccc taacctcatt ccccaaccac ttcattcttg    1140
   aaagctgtgg ccagcttgtt atttataaca acctaaattt ggttctaggc cgggcgcggt    1200
   ggctcacgcc tgtaatccca gcactttggg aggctgaggc gggtggatca cttgaggtca    1260
   ggagttccta accagcctgg tcaacatggt gaaaccccgt ctctactaaa aatacaaaaa    1320
   ttagccgggc atggtggcgc gcacctgtaa tcccagctac ttgggaggct gaggcaagag    1380
   aattgcttga acccaggaga tggaagttgc agtgagctga tatcatgccc ctgtactcca    1440
   gcctgggtga cagagcaaga ctctgtctca aaaaataaaa ataaaaataa atttggttct    1500
   aatagaactc agttttaact agaatttatt caattcctct gggaatgtta cattgtttgt    1560
   ctgtcttcat agcagatttt aattttgaat aaataaatgt atcttattca catca         1615
<210> 212
   <211> 1486
   <212> DNA
   <213> Homo sapiens
<400> 212
   caaagggctc agtcccttcg gggaggcttc tggtgaagga ggatcgctag aaccaagctg 60
   tcctcttaag ctagttgcag cagcccctcc tcccagccac ctccgccaat ctctcactca 120
   cctttggctc ctgcccttag ggttacctgg gttgccaagc cttgtctgag atgatccagt 180
   tttacctgga ggaggtgatg ccccaagctg agaaccaaga cccagacatc aaggcgcatg 240
   tgaactccct gggggagaac ctgaagaccc tcaggctgag gctacggcgc tgtcatcgat 300
   ttcttccctg tgaaaacaag agcaaggccg tggagcaggt gaagaatgcc tttaataagc 360
   tccaagagaa aggcatctac aaagccatga gtgagtttga catcttcatc aactacatag 420
   aagcctacat gacaatgaag atacgaaact gagacatcag ggtggcgact ctatagactc 480
   taggacataa attagaggtc tccaaaatcg gatctggggc tctgggatag ctgacccagc 540
   cccttgagaa accttattgt acctctctca tagaatattt attacctctg atacctcaac 600
   ccccatttct atttatttac tgagcttctc tgtgaacgat ttagaaagaa gcccaatatt 660
   ataatttttt tcaatattta ttattttcac ctgtttttaa gctgtttcca tagggtgaca 720
   cactatggta tttgagtgtt ttaagataaa ttataagtta cataagggag gaaaaaaaat 780
   gttctttggg gagccaacag aagcttccat tccaagcctg accacgcttt ctagctgttg 840
   agctgttttc cctgacctcc ctctaattta tcttgtctct gggcttgggg cttcctaact 900
   gctacaaata ctcttaggaa gagaaaccag ggagcccctt tgatgattaa ttcaccttcc 960
   agtgtctcgg agggattccc ctaacctcat tccccaacca cttcattctt gaaagctgtg 1020
   gccagcttgt tatttataac aacctaaatt tggttctagg ccgggcgcgg tggctcacgc 1080
   ctgtaatccc agcactttgg gaggctgagg cgggtggatc acttgaggtc aggagttcct 1140
   aaccagcctg gtcaacatgg tgaaaccccg tctctactaa aaatacaaaa attagccggg 1200
   catggtggcg cgcacctgta atcccagcta cttgggaggc tgaggcaaga gaattgcttg 1260
   aacccaggag atggaagttg cagtgagctg atatcatgcc cctgtactcc agcctgggtg 1320
   acagagcaag actctgtctc aaaaaataaa aataaaaata aatttggttc taatagaact 1380
   cagttttaac tagaatttat tcaattcctc tgggaatgtt acattgtttg tctgtcttca 1440
   tagcagattt taattttgaa taaataaatg tatcttattc acatca 1486
<210> 213
   <211> 1564
   <212> DNA
   <213> Homo sapiens
<400> 213
   acacatcagg ggcttgctct tgcaaaacca aaccacaaga cagacttgca aaagaaggca 60
   tgcacagctc agcactgctc tgttgcctgg tcctcctgac tggggtgagg gccagcccag 120
   gccagggcac ccagtctgag aacagctgca cccacttccc aggcaacctg cctaacatgc 180
   ttcgagatct ccgagatgcc ttcagcagag tgaagacttt ctttcaaatg aaggatcagc 240
   tggacaactt gttgttaaag gagtccttgc tggaggactt taagggttac ctgggttgcc 300
   aagccttgtc tgagatgatc cagttttacc tggaggaggt gatgccccaa gctgagaacc     360
   aagacccaga catcaaggcg catgtgaact ccctggggga gaacctgaag accctcaggc     420
   tgaggctacg gcgctgtctc caagagaaag gcatctacaa agccatgagt gagtttgaca 480
   tcttcatcaa ctacatagaa gcctacatga caatgaagat acgaaactga gacatcaggg 540
   tggcgactct atagactcta ggacataaat tagaggtctc caaaatcgga tctggggctc 600
   tgggatagct gacccagccc cttgagaaac cttattgtac ctctctcata gaatatttat 660
   tacctctgat acctcaaccc ccatttctat ttatttactg agcttctctg tgaacgattt 720
   agaaagaagc ccaatattat aatttttttc aatatttatt attttcacct gtttttaagc 780
   tgtttccata gggtgacaca ctatggtatt tgagtgtttt aagataaatt ataagttaca 840
   taagggagga aaaaaaatgt tctttgggga gccaacagaa gcttccattc caagcctgac 900
   cacgctttct agctgttgag ctgttttccc tgacctccct ctaatttatc ttgtctctgg 960
   gcttggggct tcctaactgc tacaaatact cttaggaaga gaaaccaggg agcccctttg 1020
   atgattaatt caccttccag tgtctcggag ggattcccct aacctcattc cccaaccact 1080
   tcattcttga aagctgtggc cagcttgtta tttataacaa cctaaatttg gttctaggcc 1140
   gggcgcggtg gctcacgcct gtaatcccag cactttggga ggctgaggcg ggtggatcac 1200
   ttgaggtcag gagttcctaa ccagcctggt caacatggtg aaaccccgtc tctactaaaa 1260
   atacaaaaat tagccgggca tggtggcgcg cacctgtaat cccagctact tgggaggctg 1320
   aggcaagaga attgcttgaa cccaggagat ggaagttgca gtgagctgat atcatgcccc 1380
   tgtactccag cctgggtgac agagcaagac tctgtctcaa aaaataaaaa taaaaataaa 1440
   tttggttcta atagaactca gttttaacta gaatttattc aattcctctg ggaatgttac 1500
   attgtttgtc tgtcttcata gcagatttta attttgaata aataaatgta tcttattcac 1560
   atca 1564
<210> 214
   <211> 1458
   <212> DNA
   <213> Homo sapiens
<400> 214
   aggagatact gtagagttct gggggaggaa ggaatcccaa gacctgggtt gtcatccaag 60
   ccttgcaaac atcttggagt gagtcctgga gaaatacatt taactcccag ggccatggaa 120
   gcagggctca gttctctctg ggagctgtga ggcaaggcat ttggataaat ctggcctcct 180
   catgatgcca ccagcttgtc ccctaagtgt gatggacatg gagctggaag ccaggatcac 240
   caacactttc tcttttcttc cacagcatcg atttcttccc tgtgaaaaca agagcaaggc 300
   cgtggagcag gtgaagaatg cctttaataa gctccaagag aaaggcatct acaaagccat 360
   gagtgagttt gacatcttca tcaactacat agaagcctac atgacaatga agatacgaaa 420
   ctgagacatc agggtggcga ctctatagac tctaggacat aaattagagg tctccaaaat 480
   cggatctggg gctctgggat agctgaccca gccccttgag aaaccttatt gtacctctct 540
   catagaatat ttattacctc tgatacctca acccccattt ctatttattt actgagcttc 600
   tctgtgaacg atttagaaag aagcccaata ttataatttt tttcaatatt tattattttc 660
   acctgttttt aagctgtttc catagggtga cacactatgg tatttgagtg ttttaagata 720
   aattataagt tacataaggg aggaaaaaaa atgttctttg gggagccaac agaagcttcc 780
   attccaagcc tgaccacgct ttctagctgt tgagctgttt tccctgacct ccctctaatt 840
   tatcttgtct ctgggcttgg ggcttcctaa ctgctacaaa tactcttagg aagagaaacc 900
   agggagcccc tttgatgatt aattcacctt ccagtgtctc ggagggattc ccctaacctc 960
   attccccaac cacttcattc ttgaaagctg tggccagctt gttatttata acaacctaaa 1020
   tttggttcta ggccgggcgc ggtggctcac gcctgtaatc ccagcacttt gggaggctga 1080
   ggcgggtgga tcacttgagg tcaggagttc ctaaccagcc tggtcaacat ggtgaaaccc 1140
   cgtctctact aaaaatacaa aaattagccg ggcatggtgg cgcgcacctg taatcccagc 1200
   tacttgggag gctgaggcaa gagaattgct tgaacccagg agatggaagt tgcagtgagc 1260
   tgatatcatg cccctgtact ccagcctggg tgacagagca agactctgtc tcaaaaaata 1320
   aaaataaaaa taaatttggt tctaatagaa ctcagtttta actagaattt attcaattcc 1380
   tctgggaatg ttacattgtt tgtctgtctt catagcagat tttaattttg aataaataaa 1440
   tgtatcttat tcacatca 1458
<210> 215
   <211> 93
   <212> PRT
   <213> Homo sapiens
<400> 215
   Met Ile Gln Phe Tyr Leu Glu Glu Val Met Pro Gln Ala Glu Asn Gln
   1 5 10 15
   Asp Pro Asp Ile Lys Ala His Val Asn Ser Leu Gly Glu Asn Leu Lys
   20 25 30
   Thr Leu Arg Leu Arg Leu Arg Arg Cys His Arg Phe Leu Pro Cys Glu
   35 40 45
   Asn Lys Ser Lys Ala Val Glu Gln Val Lys Asn Ala Phe Asn Lys Leu
   50 55 60
   Gln Glu Lys Gly Ile Tyr Lys Ala Met Ser Glu Phe Asp Ile Phe Ile
   65 70 75 80
   Asn Tyr Ile Glu Ala Tyr Met Thr Met Lys Ile Arg Asn
   85 90
<210> 216
   <211> 80
   <212> PRT
   <213> Homo sapiens
<400> 216
   Met Met Pro Pro Ala Cys Pro Leu Ser Val Met Asp Met Glu Leu Glu
   1 5 10 15
   Ala Arg Ile Thr Asn Thr Phe Ser Phe Leu Pro Gln His Arg Phe Leu
   20 25 30
   Pro Cys Glu Asn Lys Ser Lys Ala Val Glu Gln Val Lys Asn Ala Phe
   35 40 45
   Asn Lys Leu Gln Glu Lys Gly Ile Tyr Lys Ala Met Ser Glu Phe Asp
   50 55 60
   Ile Phe Ile Asn Tyr Ile Glu Ala Tyr Met Thr Met Lys Ile Arg Asn
   65 70 75 80
<210> 217
   <211> 173
   <212> PRT
   <213> Homo sapiens
<400> 217
   Met His Ser Ser Ala Leu Leu Cys Cys Leu Val Leu Leu Thr Gly Val
   1               5                   10                  15
   Arg Ala Ser Pro Gly Gln Gly Thr Gln Ser Glu Asn Ser Cys Thr His
               20                  25                  30
   Phe Pro Gly Asn Leu Pro Asn Met Leu Arg Asp Leu Arg Asp Ala Phe
           35                  40                  45
   Ser Arg Gln Met Lys Asp Gln Leu Asp Asn Leu Leu Leu Lys Glu Ser
       50                  55                  60
   Leu Leu Glu Asp Phe Lys Gly Tyr Leu Gly Cys Gln Ala Leu Ser Glu
   65                  70                  75                  80
   Met Ile Gln Phe Tyr Leu Glu Glu Val Met Pro Gln Ala Glu Asn Gln
                   85                  90                  95
   Asp Pro Asp Ile Lys Ala His Val Asn Ser Leu Gly Glu Asn Leu Lys
               100                 105                 110
   Thr Leu Arg Leu Arg Leu Arg Arg Cys His Arg Phe Leu Pro Cys Glu
           115                 120                 125
   Asn Lys Ser Lys Ala Val Glu Gln Val Lys Asn Ala Phe Asn Lys Leu
       130                 135                 140
   Gln Glu Lys Gly Ile Tyr Lys Ala Met Ser Glu Phe Asp Ile Phe Ile
   145                 150                 155                 160
   Asn Tyr Ile Glu Ala Tyr Met Thr Met Lys Ile Arg Asn
                   165                 170
<210> 218
   <211> 156
   <212> PRT
   <213> Homo sapiens
<400> 218
   Met His Ser Ser Ala Leu Leu Cys Cys Leu Val Leu Leu Thr Gly Val
   1               5                   10                  15
   Arg Ala Ser Pro Gly Gln Gly Thr Gln Ser Glu Asn Ser Cys Thr His
   20 25 30
   Phe Pro Gly Asn Leu Pro Asn Met Leu Arg Asp Leu Arg Asp Ala Phe
   35 40 45
   Ser Arg Val Lys Thr Phe Phe Gln Met Lys Asp Gln Leu Asp Asn Leu
   50 55 60
   Leu Leu Lys Glu Ser Leu Leu Glu Asp Phe Lys Gly Tyr Leu Gly Cys
   65 70 75 80
   Gln Ala Leu Ser Glu Met Ile Gln Phe Tyr Leu Glu Glu Val Met Pro
   85 90 95
   Gln Ala Glu Asn Gln Asp Pro Asp Ile Lys Ala His Val Asn Ser Leu
   100 105 110
   Gly Glu Asn Leu Lys Thr Leu Arg Leu Arg Leu Arg Arg Cys Leu Gln
   115 120 125
   Glu Lys Gly Ile Tyr Lys Ala Met Ser Glu Phe Asp Ile Phe Ile Asn
   130 135 140
   Tyr Ile Glu Ala Tyr Met Thr Met Lys Ile Arg Asn
   145 150 155
<210> 219
   <211> 79
   <212> PRT
   <213> Homo sapiens
<400> 219
   Met Pro Pro Ala Cys Pro Leu Ser Val Met Asp Met Glu Leu Glu Ala
   1 5 10 15
   Arg Ile Thr Asn Thr Phe Ser Phe Leu Pro Gln His Arg Phe Leu Pro
   20 25 30
   Cys Glu Asn Lys Ser Lys Ala Val Glu Gln Val Lys Asn Ala Phe Asn
   35 40 45
   Lys Leu Gln Glu Lys Gly Ile Tyr Lys Ala Met Ser Glu Phe Asp Ile
   50 55 60
   Phe Ile Asn Tyr Ile Glu Ala Tyr Met Thr Met Lys Ile Arg Asn
   65 70 75
<210> 220
   <211> 209
   <212> DNA
   <213> Artificial Organism
<220>
   <223> Synthetic oligonucleotide
<400> 220
   acacatcagg ggcttgctct tgcaaaacca aaccacaaga cagacttgca aaagaaggca 60
   tgcacagctc agcactgctc tgttgcctgg tcctcctgac tggggtgagg gccagcccag 120
   gccagggcac ccagtctgag aacagctgca cccacttccc aggcaacctg cctaacatgc 180
   ttcgagatct ccgagatgcc ttcagcaga 209
<210> 221
   <211> 15
   <212> DNA
   <213> Artificial Organism
<220>
   <223> Synthetic oligonucleotide
<400> 221
   gtgaagactt tcttt 15
<210> 222
   <211> 60
   <212> DNA
   <213> Artificial Organism
<220>
   <223> Synthetic oligonucleotide
<400> 222
   caaatgaagg atcagctgga caacttgttg ttaaaggagt ccttgctgga ggactttaag 60
<210> 223
   <211> 140
   <212> DNA
   <213> Artificial Organism
<220>
   <223> Synthetic oligonucleotide
<400> 223
   caaagggctc agtcccttcg gggaggcttc tggtgaagga ggatcgctag aaccaagctg 60
   tcctcttaag ctagttgcag cagcccctcc tcccagccac ctccgccaat ctctcactca 120
   cctttggctc ctgcccttag 140
<210> 224
   <211> 153
   <212> DNA
   <213> Artificial Organism
<220>
   <223> Synthetic oligonucleotide
<400> 224
   ggttacctgg gttgccaagc cttgtctgag atgatccagt tttacctgga ggaggtgatg 60
   ccccaagctg agaaccaaga cccagacatc aaggcgcatg tgaactccct gggggagaac 120
   ctgaagaccc tcaggctgag gctacggcgc tgt 153
<210> 225
   <211> 265
   <212> DNA
   <213> Artificial Organism
<220>
   <223> Synthetic oligonucleotide
<400> 225
   aggagatact gtagagttct gggggaggaa ggaatcccaa gacctgggtt gtcatccaag 60
   ccttgcaaac atcttggagt gagtcctgga gaaatacatt taactcccag ggccatggaa 120
   gcagggctca gttctctctg ggagctgtga ggcaaggcat ttggataaat ctggcctcct 180
   catgatgcca ccagcttgtc ccctaagtgt gatggacatg gagctggaag ccaggatcac 240
   caacactttc tcttttcttc cacag 265
<210> 226
   <211> 66
   <212> DNA
   <213> Artificial Organism
<220>
   <223> Synthetic oligonucleotide
<400> 226
   catcgatttc ttccctgtga aaacaagagc aaggccgtgg agcaggtgaa gaatgccttt 60
   aataag 66
<210> 227
   <211> 458
   <212> DNA
   <213> Artificial Organism
<220>
   <223> Synthetic oligonucleotide
<400> 227
   ctccaagaga aaggcatcta caaagccatg agtgagtttg acatcttcat caactacata 60
   gaagcctaca tgacaatgaa gatacgaaac tgagacatca gggtggcgac tctatagact 120
   ctaggacata aattagaggt ctccaaaatc ggatctgggg ctctgggata gctgacccag 180
   ccccttgaga aaccttattg tacctctctc atagaatatt tattacctct gatacctcaa 240
   cccccatttc tatttattta ctgagcttct ctgtgaacga tttagaaaga agcccaatat 300
   tataattttt ttcaatattt attattttca cctgttttta agctgtttcc atagggtgac 360
   acactatggt atttgagtgt tttaagataa attataagtt acataaggga ggaaaaaaaa 420
   tgttctttgg ggagccaaca gaagcttcca ttccaagc 458
<210> 228
   <211> 25
   <212> DNA
   <213> Artificial Organism
<220>
   <223> Synthetic oligonucleotide
<400> 228
   ctgaccacgc tttctagctg ttgag 25
<210> 229
   <211> 213
   <212> DNA
   <213> Artificial Organism
<220>
   <223> Synthetic oligonucleotide
<400> 229
   ctgttttccc tgacctccct ctaatttatc ttgtctctgg gcttggggct tcctaactgc 60
   tacaaatact cttaggaaga gaaaccaggg agcccctttg atgattaatt caccttccag 120
   tgtctcggag ggattcccct aacctcattc cccaaccact tcattcttga aagctgtggc 180
   cagcttgtta tttataacaa cctaaatttg gtt 213
<210> 230
   <211> 4
   <212> DNA
   <213> Artificial Organism
<220>
   <223> Synthetic oligonucleotide
<400> 230
   ctag 4
<210> 231
   <211> 14
   <212> DNA
   <213> Artificial Organism
<220>
   <223> Synthetic oligonucleotide
<400> 231
   gccgggcgcg gtgg 14
<210> 232
   <211> 214
   <212> DNA
   <213> Artificial Organism
<220>
   <223> Synthetic oligonucleotide
<400> 232
   ctcacgcctg taatcccagc actttgggag gctgaggcgg gtggatcact tgaggtcagg 60
   agttcctaac cagcctggtc aacatggtga aaccccgtct ctactaaaaa tacaaaaatt 120
   agccgggcat ggtggcgcgc acctgtaatc ccagctactt gggaggctga ggcaagagaa 180
   ttgcttgaac ccaggagatg gaagttgcag tgag 214
<210> 233
   <211> 82
   <212> DNA
   <213> Artificial Organism
<220>
   <223> Synthetic oligonucleotide
<400> 233
   ctgatatcat gcccctgtac tccagcctgg gtgacagagc aagactctgt ctcaaaaaat 60
   aaaaataaaa ataaatttgg tt 82
<210> 234
   <211> 117
   <212> DNA
   <213> Artificial Organism
<220>
   <223> Synthetic oligonucleotide
<400> 234
   ctaatagaac tcagttttaa ctagaattta ttcaattcct ctgggaatgt tacattgttt 60
   gtctgtcttc atagcagatt ttaattttga ataaataaat gtatcttatt cacatca 117
<210> 235
   <211> 1849
   <212> DNA
   <213> Homo sapiens
<400> 235
   ggctggagct cagcccagca gtggaatcca ggagcccaga ggtggccggg tgctgacgtg 60
   atggccacag caggaaatcc ctggggctgg ttcctggggt acctcatcct tggtgtcgca 120
   ggatctctcg tctctggtag ctgcagccaa atcataaacg gcgaggactg cagcccgcac     180
   tcgcagccct ggcaggcggc actggtcatg gaaaacgaat tgttctgctc gggcgtcctg     240
   gtgcatccgc agtgggtgct gtcagccgca cactgtttcc agaactccta caccatcggg     300
   ctgggcctgc acagtcttga ggccgaccaa gagccaggga gccagatggt ggaggccagc     360
   ctctccgtac ggcacccaga gtacaacaga cccttgctcg ctaacgacct catgctcatc     420
   aagttggacg aatccgtgtc cgagtctgac accatccgga gcatcagcat tgcttcgcag     480
   tgccctaccg cggggaactc ttgcctcgtt tctggctggg gtctgctggc gaacgatgct     540
   gtgattgcca tccagtccca gactgtggga ggctgggagt gtgagaagct ttcccaaccc     600
   tggcagggtt gtaccatttc ggcaacttcc agtgcaagga cgtcctgctg catcctcact     660
   gggtgctcac tactgctcac tgcatcaccc ggtgtggaga tacgacggga ttcagctggg     720
   tgcagccaca tgataaagga aggaccggag cttggtgtaa cccctgatcc ctcctagatg     780
   gggctaggtg gggctagcct agatggggct aagtcctagc gacttccacc tggacttgtc     840
   tggtgtaaca tccctcatcc acaggtctgg aagcccatgt ggaaattctg tcactttctc     900
   agcatgcctg tcccagttat gcattattct gtaattggtg aaataccgga agggtaggtt     960
   cagagaccca cgggacccat tgtgagatgg gccttggcca caactcctga cctccaccag    1020
   gcgccacact gattgttgga ctacagtggt gttttgggtc tcttgtaatt agtgtcagtt    1080
   aagaatcagt gtccaataat ctccaaaaca tctggtaact gactcaagtt tatgaattga    1140
   acgatgggcc aggattcttt gcttttatga ttcaaattat acttctgttc actggaccca    1200
   agaagtcttc tgattacata gaaataattt agtcttttcc aatctatttc tcttccagga    1260
   acactgtgat caactagcca gcaccatagt tctccgaagt cagactatca tgattactgt    1320
   gttgactgtg ctgtctattg tactaaccat gccgatgttt aggtgaaatt agcgtcactt    1380
   ggcctcaacc atcttggtat ccagttatcc tcactgaatt gagatttcct gcttcagtgt    1440
   cagccattcc cacataattt ctgacctaca gaggtgaggg atcatatagc tcttcaagga    1500
   tgctggtact cccctcacaa attcatttct cctgttgtag tgaaaggtgc gccctctgga    1560
   gcctcccagg gtgggtgtgc aggtcacaat gatgaatgta tgatcgtgtt cccattaccc    1620
   aaagccttta aatccctcat gctcagtaca ccagggcagg tctagcattt cttcatttag    1680
   tgtatgctgt ccattcatgc aaccacctca ggactcctgg attctctgcc tagttgagct    1740
   cctgcatgct gcctccttgg ggaggtgagg gagagggccc atggttcaat gggatctgtg    1800
   cagttgtaac acattaggtg cttaataaac agaagctgtg atgttaacc                1849
<210> 236
   <211> 238
   <212> PRT
   <213> Homo sapiens
<400> 236
   Met Ala Thr Ala Gly Asn Pro Trp Gly Trp Phe Leu Gly Tyr Leu Ile
   1               5                   10                  15
   Leu Gly Val Ala Gly Ser Leu Val Ser Gly Ser Cys Ser Gln Ile Ile
               20                  25                  30
   Asn Gly Glu Asp Cys Ser Pro His Ser Gln Pro Trp Gln Ala Ala Leu
           35                  40                  45
   Val Met Glu Asn Glu Leu Phe Cys Ser Gly Val Leu Val His Pro Gln
       50                  55                  60
   Trp Val Leu Ser Ala Ala His Cys Phe Gln Asn Ser Tyr Thr Ile Gly
   65                  70                  75                  80
   Leu Gly Leu His Ser Leu Glu Ala Asp Gln Glu Pro Gly Ser Gln Met
                   85                  90                  95
   Val Glu Ala Ser Leu Ser Val Arg His Pro Glu Tyr Asn Arg Pro Leu
               100                 105                 110
   Leu Ala Asn Asp Leu Met Leu Ile Lys Leu Asp Glu Ser Val Ser Glu
           115                 120                 125
   Ser Asp Thr Ile Arg Ser Ile Ser Ile Ala Ser Gln Cys Pro Thr Ala
       130                 135                 140
   Gly Asn Ser Cys Leu Val Ser Gly Trp Gly Leu Leu Ala Asn Asp Ala
   145                 150                 155                 160
   Val Ile Ala Ile Gln Ser Gln Thr Val Gly Gly Trp Glu Cys Glu Lys
                   165                 170                 175
   Leu Ser Gln Pro Trp Gln Gly Cys Thr Ile Ser Ala Thr Ser Ser Ala
               180                 185                 190
   Arg Thr Ser Cys Cys Ile Leu Thr Gly Cys Ser Leu Leu Leu Thr Ala
           195                 200                 205
   Ser Pro Gly Val Glu Ile Arg Arg Asp Ser Ala Gly Cys Ser His Met
       210                 215                 220
   Ile Lys Glu Gly Pro Glu Leu Gly Val Thr Pro Asp Pro Ser
   225                 230                 235
<210> 237
   <211> 49
   <212> DNA
   <213> Artificial Organism
<220>
   <223> Synthetic oligonucleotide
<400> 237
   ggctggagct cagcccagca gtggaatcca ggagcccaga ggtggccgg 49
<210> 238
   <211> 72
   <212> DNA
   <213> Artificial Organism
<220>
   <223> Synthetic oligonucleotide
<400> 238
   gtgctgacgt gatggccaca gcaggaaatc cctggggctg gttcctgggg tacctcatcc 60
   ttggtgtcgc ag 72
<210> 239
   <211> 163
   <212> DNA
   <213> Artificial Organism
<220>
   <223> Synthetic oligonucleotide
<400> 239
   gatctctcgt ctctggtagc tgcagccaaa tcataaacgg cgaggactgc agcccgcact 60
   cgcagccctg gcaggcggca ctggtcatgg aaaacgaatt gttctgctcg ggcgtcctgg 120
   tgcatccgca gtgggtgctg tcagccgcac actgtttcca gaa 163
<210> 240
   <211> 251
   <212> DNA
   <213> Artificial Organism
<220>
   <223> Synthetic oligonucleotide
<400> 240
   ctcctacacc atcgggctgg gcctgcacag tcttgaggcc gaccaagagc cagggagcca 60
   gatggtggag gccagcctct ccgtacggca cccagagtac aacagaccct tgctcgctaa 120
   cgacctcatg ctcatcaagt tggacgaatc cgtgtccgag tctgacacca tccggagcat 180
   cagcattgct tcgcagtgcc ctaccgcggg gaactcttgc ctcgtttctg gctggggtct 240
   gctggcgaac g 251
<210> 241
   <211> 156
   <212> DNA
   <213> Artificial Organism
<220>
   <223> Synthetic oligonucleotide
<400> 241
   atgctgtgat tgccatccag tcccagactg tgggaggctg ggagtgtgag aagctttccc 60
   aaccctggca gggttgtacc atttcggcaa cttccagtgc aaggacgtcc tgctgcatcc 120
   tcactgggtg ctcactactg ctcactgcat cacccg 156
<210> 242
   <211> 567
   <212> DNA
   <213> Artificial Organism
<220>
   <223> Synthetic oligonucleotide
<400> 242
   gtgtggagat acgacgggat tcagctgggt gcagccacat gataaaggaa ggaccggagc 60
   ttggtgtaac ccctgatccc tcctagatgg ggctaggtgg ggctagccta gatggggcta 120
   agtcctagcg acttccacct ggacttgtct ggtgtaacat ccctcatcca caggtctgga 180
   agcccatgtg gaaattctgt cactttctca gcatgcctgt cccagttatg cattattctg 240
   taattggtga aataccggaa gggtaggttc agagacccac gggacccatt gtgagatggg 300
   ccttggccac aactcctgac ctccaccagg cgccacactg attgttggac tacagtggtg 360
   ttttgggtct cttgtaatta gtgtcagtta agaatcagtg tccaataatc tccaaaacat 420
   ctggtaactg actcaagttt atgaattgaa cgatgggcca ggattctttg cttttatgat 480
   tcaaattata cttctgttca ctggacccaa gaagtcttct gattacatag aaataattta 540
   gtcttttcca atctatttct cttccag 567
<210> 243
   <211> 591
   <212> DNA
   <213> Artificial Organism
<220>
   <223> Synthetic oligonucleotide
<400> 243
   gaacactgtg atcaactagc cagcaccata gttctccgaa gtcagactat catgattact     60
   gtgttgactg tgctgtctat tgtactaacc atgccgatgt ttaggtgaaa ttagcgtcac    120
   ttggcctcaa ccatcttggt atccagttat cctcactgaa ttgagatttc ctgcttcagt    180
   gtcagccatt cccacataat ttctgaccta cagaggtgag ggatcatata gctcttcaag    240
   gatgctggta ctcccctcac aaattcattt ctcctgttgt agtgaaaggt gcgccctctg    300
   gagcctccca gggtgggtgt gcaggtcaca atgatgaatg tatgatcgtg ttcccattac    360
   ccaaagcctt taaatccctc atgctcagta caccagggca ggtctagcat ttcttcattt    420
   agtgtatgct gtccattcat gcaaccacct caggactcct ggattctctg cctagttgag    480
   ctcctgcatg ctgcctcctt ggggaggtga gggagagggc ccatggttca atgggatctg    540
   tgcagttgta acacattagg tgcttaataa acagaagctg tgatgttaac c             591
<210> 244
   <211> 755
   <212> DNA
   <213> Homo sapiens
<400> 244
   cgctccccag tagaggaccc ggaaccagaa ctggaatccg cccttaccgc ttgctgccaa     60
   aacagtgggg gctgaactga cctctcccct ttgggagaga aaaactgtct gggagcttga    120
   caaaggcatg caggagagaa caggagcagc cacagccagg agggagagcc ttccccaagc    180
   aaacaatcca gagcagctgt gcaaacaacg gtgcataaat gaggcctcct ggaccatgaa    240
   gcgagtcctg agctgcgtcc cggagcccac ggtggtcatg gctgccagag cgctctgcat    300
   gctggggctg gtcctggcct tgctgtcctc cagctctgct gaggagtacg tgggcctgtc    360
   ccagcaaggg ctctggcagc tgacagggct ttgtctggga cagctgcaaa ccagtgtgcc    420
   gtgccagcca aggacagggt ggactgcggc tacccccatg tcacccccaa ggagtgcaac    480
   aaccggggct gctgctttga ctccaggatc cctggagtgc cttggtgttt caagcccctg    540
   caggaagcag aatgcacctt ctgaggcacc tccagctgcc cccggccggg ggatgcgagg    600
   ctcggagcac ccttgcccgg ctgtgattgc tgccaggcac tgttcatctc agcttttctg    660
   tccctttgct cccggcaagc gcttctgctg aaagttcata tctggagcct gatgtcttaa    720
   cgaataaagg tcccatgctc cacccgagga cagtt                                755
<210> 245
   <211> 137
   <212> PRT
   <213> Homo sapiens
<400> 245
   Met Ala Ala Arg Ala Leu Cys Met Leu Gly Leu Val Leu Ala Leu Leu
   1               5                   10                  15
   Ser Ser Ser Ser Ala Glu Glu Tyr Val Gly Leu Ser Gln Gln Gly Leu
               20                  25                  30
   Trp Gln Leu Thr Gly Leu Cys Leu Gly Gln Leu Gln Thr Ser Val Pro
           35                  40                  45
   Cys Gln Pro Arg Thr Gly Trp Thr Ala Ala Thr Pro Met Ser Pro Pro
       50                  55                  60
   Arg Ser Ala Thr Thr Gly Ala Ala Ala Leu Thr Pro Gly Ser Leu Glu
   65                  70                  75                  80
   Cys Leu Gly Val Ser Ser Pro Cys Arg Lys Gln Asn Ala Pro Ser Glu
                   85                  90                  95
   Ala Pro Pro Ala Ala Pro Gly Arg Gly Met Arg Gly Ser Glu His Pro
               100                 105                 110
   Cys Pro Ala Val Ile Ala Ala Arg His Cys Ser Ser Gln Leu Phe Cys
           115                 120                 125
   Pro Phe Ala Pro Gly Lys Arg Phe Cys
       130                 135
<210> 246
   <211> 359
   <212> DNA
   <213> Artificial Organism
<220>
   <223> Synthetic oligonucleotide
<400> 246
   cgctccccag tagaggaccc ggaaccagaa ctggaatccg cccttaccgc ttgctgccaa 60
   aacagtgggg gctgaactga cctctcccct ttgggagaga aaaactgtct gggagcttga 120
   caaaggcatg caggagagaa caggagcagc cacagccagg agggagagcc ttccccaagc 180
   aaacaatcca gagcagctgt gcaaacaacg gtgcataaat gaggcctcct ggaccatgaa 240
   gcgagtcctg agctgcgtcc cggagcccac ggtggtcatg gctgccagag cgctctgcat 300
   gctggggctg gtcctggcct tgctgtcctc cagctctgct gaggagtacg tgggcctgt 359
<210> 247
   <211> 44
   <212> DNA
   <213> Artificial Organism
<220>
   <223> Synthetic oligonucleotide
<400> 247
   cccagcaagg gctctggcag ctgacagggc tttgtctggg acag 44
<210> 248
   <211> 97
   <212> DNA
   <213> Artificial Organism
<220>
   <223> Synthetic oligonucleotide
<400> 248
   ctgcaaacca gtgtgccgtg ccagccaagg acagggtgga ctgcggctac ccccatgtca 60
   cccccaagga gtgcaacaac cggggctgct gctttga 97
<210> 249
   <211> 50
   <212> DNA
   <213> Artificial Organism
<220>
   <223> Synthetic oligonucleotide
<400> 249
   ctccaggatc cctggagtgc cttggtgttt caagcccctg caggaagcag 50
<210> 250
   <211> 19
   <212> DNA
   <213> Artificial Organism
<220>
   <223> Synthetic oligonucleotide
<400> 250
   aatgcacctt ctgaggcac 19
<210> 251
   <211> 584
   <212> DNA
   <213> Artificial Organism
<220>
   <223> Synthetic oligonucleotide
<400> 251
   ctccagctgc ccccggccgg gggatgcgag gctcggagca cccttgcccg gctgtgattg 60
   ctgccaggca ctgttcatct cagcttttct gtccctttgc tcccggcaag cgcttctgct 120
   gaaagttcat atctggagcc tgatgtctta acgaataaag gtcccatgct ccacccgagg 180
   acagttcttc gtgcctgaga ctttctgagg ttgtgcttta tttctgctgc gtcgtgggag 240
   agggcgggag ggtgtcaggg gagagtctgc ccaggcctca agggcaggaa aagactccct 300
   aaggagctgc agtgcatgca aggatatttt gaatccagac tggcacccac gtcacaggaa 360
   agcctaggaa cactgtaagt gccgcttcct cgggaaagca gaaaaaatac atttcaggta 420
   gaagttttca aaaatcacaa gtctttcttg gtgaagacag caagccaata aaactgtctt 480
   ccaaagtggt cctttatttc acaaccactc tcgctactgt tcaatacttg tactattcct 540
   gggttttgtt tctttgtaca gtaaacatta tgaacaaaca ggca 584
<210> 252
   <211> 2664
   <212> DNA
   <213> Homo sapiens
<400> 252
   gactcccacc ttgggcctct gccagcctgg cactgatagc cacaggcaga gctgagacaa      60
   aagagagggg ccctggggag tatcagcagc agccaatccc ggaagacatc tatgtcaggt     120
   ggtttctgga aatcgaaagt agactctttt ctgaagcatt tcctgggatc agcctgacca     180
   cgctccatac tgggagaggc ttctgggtca aaggaccagt ctgcagaggg atcctgtggc     240
   tggaagcgag gaggctccac acggccgttg cagctaccgc agccaggatc tgggcatcca     300
   ggcacggcca tgacccctcc gaggctcttc tgggtgtggc tgctggttgc aggaacccaa     360
   ggcgtgaacg atggtgacat gcggctggcc gatgggggcg ccaccaacca gggccgcgtg     420
   gagatcttct acagaggcca gtggggcact gtgtgtgaca acctgtggga cctgactgat     480
   gccagcgtcg tctgccgggc cctgggcttc gagaacgcca cccaggctct gggcagagct     540
   gccttcgggc aaggatcagg ccccatcatg ctggacgagg tccagtgcac gggaaccgag     600
   gcctcactgg ccgactgcaa gtccctgggc tggctgaaga gcaactgcag gcacgagaga     660
   gacgctggtg tggtctgcac caatggagca cccacaccct ggacctctcc agggagctct     720
   cggaggccct tggccagatc tttgacagcc agcggggctg cgacctgtcc atcagcgtga     780
   atgtgcaggg cgaggacgcc ctgggcttct gtggccacac ggtcatcctg actgccaacc     840
   tggaggccca ggccctgtgg aaggagccgg gcagcaatgt caccatgagt gtggatgctg     900
   agtgtgtgcc catggtcagg gaccttctca ggtgagccgt ctcgtcctca ggctctgggg     960
   ccgccagggg ctttgtcctc ctcccctctt caaaagcaca gccccccaca cacacacctg    1020
   cacatactca tgcatgcaca tgtacacacg cagtcacaca tgcactcacg cagttgcaca    1080
   cacacgcatg ctcactccca cactgtgtgc actcaggtgg ctgtgttgga cagttgggcc    1140
   cagggctccc ctgctgtcct gtggggccgg catctgctct ccttctttct ccccaggtac    1200
   ttctactccc gaaggattga catcaccctg tcgtcagtca agtgcttcca caagctggcc    1260
   tctgcctatg gggccaggca gctgcagggc tactgcgcaa gcctctttgc catcctcctc    1320
   ccccaggacc cctcgttcca gatgcccctg gacctgtatg cctatgcagt ggccacaggg    1380
   gacgccctgc tggagaagct ctgcctacag ttcctggcct ggaacttcga ggccttgacg    1440
   caggccgagg cctggcccag tgtccccaca gacctgctcc aactgctgct gcccaggagc    1500
   gacctggcgg tgcccagcga gctggcccta ctgaaggccg tggacacctg gagctggggg    1560
   gagcgtgcct cccatgagga ggtggagggc ttggtggaga agatccgctt ccccatgatg    1620
   ctccctgagg agctctttga gctgcagttc aacctgtccc tgtactggag ccacgaggcc 1680
   ctgttccaga agaagactct gcaggccctg gaattccaca ctgtgccctt ccagttgctg 1740
   gcccggtaca aaggcctgaa cctcaccgag gatacctaca agccccggat ttacacctcg 1800
   cccacctgga gtgcctttgt gacagacagt tcctggagtg cacggaagtc acaactggtc 1860
   tatcagtcca gacgggggcc tttggtcaaa tattcttctg attacttcca agccccctct 1920
   gactacagat actaccccta ccagtccttc cagactccac aacaccccag cttcctcttc 1980
   caggacaaga gggtgtcctg gtccctggtc tacctcccca ccatccagag ctgctggaac 2040
   tacggcttct cctgctcctc ggacgagctc cctgtcctgg gcctcaccaa gtctggcggc 2100
   tcagatcgca ccattgccta cgaaaacaaa gccctgatgc tctgcgaagg gctcttcgtg 2160
   gcagacgtca ccgatttcga gggctggaag gctgcgattc ccagtgccct ggacaccaac 2220
   agctcgaaga gcacctcctc cttcccctgc ccggcagggc acttcaacgg cttccgcacg 2280
   gtcatccgcc ccttctacct gaccaactcc tcaggtgtgg actagacggc gtggcccaag 2340
   ggtggtgaga accggagaac cccaggacgc cctcactgca ggctcccctc ctcggcttcc 2400
   ttcctctctg caatgacctt caacaaccgg ccaccagatg tcgccctact cacctgagcg 2460
   ctcagcttca agaaattact ggaaggcttc cactagggtc caccaggagt tctcccacca 2520
   cctcaccagt ttccaggtgg taagcaccag gacgccctcg aggttgctct gggatccccc 2580
   cacagcccct ggtcagtctg cccttgtcac tggtctgagg tcattaaaat tacattgagg 2640
   ttcctacatt tgtttgtaca gaaa 2664
<210> 253
   <211> 2153
   <212> DNA
   <213> Homo sapiens
<400> 253
   gactcccacc ttgggcctct gccagcctgg cactgatagc cacaggcaga gctgagacaa 60
   aagagagggg ccctggggag tatcagcagc agccaatccc ggaagacatc tatgtcaggt 120
   ggtttctgga aatcgaaagt agactctttt ctgaagcatt tcctgggatc agcctgacca 180
   cgctccatac tgggagaggc ttctgggtca aaggaccagt ctgcagaggg atcctgtggc 240
   tggaagcgag gaggctccac acggccgttg cagctaccgc agccaggatc tgggcatcca 300
   ggcacggcca tgacccctcc gaggctcttc tgggtgtggc tgctggttgc aggaacccaa 360
   ggcgtgaacg atggtgacat gcggctggcc gatgggggcg ccaccaacca gggccgcgtg 420
   gagatcttct acagaggcca gtggggcact gtgtgtgaca acctgtggga cctgactgat 480
   gccagcgtcg tctgccgggc cctgggcttc gagaacgcca cccaggctct gggcagagct 540
   gccttcgggc aaggatcagg ccccatcatg ctggacgagg tccagtgcac gggaaccgag 600
   gcctcactgg ccgactgcaa gtccctgggc tggctgaaga gcaactgcag gcacgagaga 660
   gacgctggtg tggtctgcac caatggtact tctactcccg aaggattgac atcaccctgt 720
   cgtcagtcaa gtgcttccac aagctggcct ctgcctatgg ggccaggcag ctgcagggct 780
   actgcgcaag cctctttgcc atcctcctcc cccaggaccc ctcgttccag atgcccctgg 840
   acctgtatgc ctatgcagtg gccacagggg acgccctgct ggagaagctc tgcctacagt 900
   tcctggcctg gaacttcgag gccttgacgc aggccgaggc ctggcccagt gtccccacag 960
   acctgctcca actgctgctg cccaggagcg acctggcggt gcccagcgag ctggccctac 1020
   tgaaggccgt ggacacctgg agctgggggg agcgtgcctc ccatgaggag gtggagggct 1080
   tggtggagaa gatccgcttc cccatgatgc tccctgagga gctctttgag ctgcagttca 1140
   acctgtccct gtactggagc cacgaggccc tgttccagaa gaagactctg caggccctgg 1200
   aattccacac tgtgcccttc cagttgctgg cccggtacaa aggcctgaac ctcaccgagg 1260
   atacctacaa gccccggatt tacacctcgc ccacctggag tgcctttgtg acagacagtt 1320
   cctggagtgc acggaagtca caactggtct atcagtccag acgggggcct ttggtcaaat 1380
   attcttctga ttacttccaa gccccctctg actacagata ctacccctac cagtccttcc 1440
   agactccaca acaccccagc ttcctcttcc aggacaagag ggtgtcctgg tccctggtct 1500
   acctccccac catccagagc tgctggaact acggcttctc ctgctcctcg gacgagctcc 1560
   ctgtcctggg cctcaccaag tctggcggct cagatcgcac cattgcctac gaaaacaaag 1620
   ccctgatgct ctgcgaaggg ctcttcgtgg cagacgtcac cgatttcgag ggctggaagg 1680
   ctgcgattcc cagtgccctg gacaccaaca gctcgaagag cacctcctcc ttcccctgcc 1740
   cggcagggca cttcaacggc ttccgcacgg tcatccgccc cttctacctg accaactcct 1800
   caggtgtgga ctagacggcg tggcccaagg gtggtgagaa ccggagaacc ccaggacgcc 1860
   ctcactgcag gctcccctcc tcggcttcct tcctctctgc aatgaccttc aacaaccggc 1920
   caccagatgt cgccctactc acctgagcgc tcagcttcaa gaaattactg gaaggcttcc 1980
   actagggtcc accaggagtt ctcccaccac ctcaccagtt tccaggtggt aagcaccagg 2040
   acgccctcga ggttgctctg ggatcccccc acagcccctg gtcagtctgc ccttgtcact 2100
   ggtctgaggt cattaaaatt acattgaggt tcctacattt gtttgtacag aaa 2153
<210> 254
   <211> 156
   <212> PRT
   <213> Homo sapiens
<400> 254
   Met Thr Pro Pro Arg Leu Phe Trp Val Trp Leu Leu Val Ala Gly Thr
   1               5                   10                  15
   Gln Gly Val Asn Asp Gly Asp Met Arg Leu Ala Asp Gly Gly Ala Thr
               20                  25                  30
   Asn Gln Gly Arg Val Glu Ile Phe Tyr Arg Gly Gln Trp Gly Thr Val
           35                  40                  45
   Cys Asp Asn Leu Trp Asp Leu Thr Asp Ala Ser Val Val Cys Arg Ala
       50                  55                  60
   Leu Gly Phe Glu Asn Ala Thr Gln Ala Leu Gly Arg Ala Ala Phe Gly
   65                  70                  75                  80
   Gln Gly Ser Gly Pro Ile Met Leu Asp Glu Val Gln Cys Thr Gly Thr
                   85                  90                  95
   Glu Ala Ser Leu Ala Asp Cys Lys Ser Leu Gly Trp Leu Lys Ser Asn
               100                 105                 110
   Cys Arg His Glu Arg Asp Ala Gly Val Val Cys Thr Asn Gly Ala Pro
           115                 120                 125
   Thr Pro Trp Thr Ser Pro Gly Ser Ser Arg Arg Pro Leu Ala Arg Ser
       130                 135                 140
   Leu Thr Ala Ser Gly Ala Ala Thr Cys Pro Ser Ala
   145                 150                 155
<210> 255
   <211> 205
   <212> PRT
   <213> Homo sapiens

<400> 255
   Met Thr Pro Pro Arg Leu Phe Trp Val Trp Leu Leu Val Ala Gly Thr
   1               5                   10                  15
   Gln Gly Val Asn Asp Gly Asp Met Arg Leu Ala Asp Gly Gly Ala Thr
               20                  25                  30
   Asn Gln Gly Arg Val Glu Ile Phe Tyr Arg Gly Gln Trp Gly Thr Val
           35                  40                  45
   Cys Asp Asn Leu Trp Asp Leu Thr Asp Ala Ser Val Val Cys Arg Ala
       50                  55                  60
   Leu Gly Phe Glu Asn Ala Thr Gln Ala Leu Gly Arg Ala Ala Phe Gly
   65                  70                  75                  80
   Gln Gly Ser Gly Pro Ile Met Leu Asp Glu Val Gln Cys Thr Gly Thr
                   85                  90                  95
   Glu Ala Ser Leu Ala Asp Cys Lys Ser Leu Gly Trp Leu Lys Ser Asn
               100                 105                 110
   Cys Arg His Glu Arg Asp Ala Gly Val Val Cys Thr Asn Gly Thr Ser
           115                 120                 125
   Thr Pro Glu Gly Leu Thr Ser Pro Cys Arg Gln Ser Ser Ala Ser Thr
       130                 135                 140
   Ser Trp Pro Leu Pro Met Gly Pro Gly Ser Cys Arg Ala Thr Ala Gln
   145                 150                 155                 160
   Ala Ser Leu Pro Ser Ser Ser Pro Arg Thr Pro Arg Ser Arg Cys Pro
                   165                 170                 175
   Trp Thr Cys Met Pro Met Gln Trp Pro Gln Gly Thr Pro Cys Trp Arg
               180                 185                 190
   Ser Ser Ala Tyr Ser Ser Trp Pro Gly Thr Ser Arg Pro
           195                 200                 205
<210> 256
   <211> 233
   <212> DNA
   <213> Artificial Organism
<220>
   <223> Synthetic oligonucleotide
<400> 256
   gactcccacc ttgggcctct gccagcctgg cactgatagc cacaggcaga gctgagacaa      60
   aagagagggg ccctggggag tatcagcagc agccaatccc ggaagacatc tatgtcaggt     120
   ggtttctgga aatcgaaagt agactctttt ctgaagcatt tcctgggatc agcctgacca     180
   cgctccatac tgggagaggc ttctgggtca aaggaccagt ctgcagaggg atc            233
<210> 257
   <211> 6
   <212> DNA
   <213> Artificial Organism
<220>
   <223> Synthetic oligonucleotide
<400> 257
   ctgtgg 6
<210> 258
   <211> 23
   <212> DNA
   <213> Artificial Organism
<220>
   <223> Synthetic oligonucleotide
<400> 258
   ctggaagcga ggaggctcca cac 23
<210> 259
   <211> 24
   <212> DNA
   <213> Artificial Organism
<220>
   <223> Synthetic oligonucleotide
<400> 259
   ggccgttgca gctaccgcag ccag 24
<210> 260
   <211> 19
   <212> DNA
   <213> Artificial Organism
<220>
   <223> Synthetic oligonucleotide
<400> 260
   gatctgggca tccaggcac 19
<210> 261
   <211> 56
   <212> DNA
   <213> Artificial Organism
<220>
   <223> Synthetic oligonucleotide
<400> 261
   ggccatgacc cctccgaggc tcttctgggt gtggctgctg gttgcaggaa cccaag 56
<210> 262
   <211> 47
   <212> DNA
   <213> Artificial Organism
<220>
   <223> Synthetic oligonucleotide
<400> 262
   gcgtgaacga tggtgacatg cggctggccg atgggggcgc caccaac 47
<210> 263
   <211> 40
   <212> DNA
   <213> Artificial Organism
<220>
   <223> Synthetic oligonucleotide
<400> 263
   cagggccgcg tggagatctt ctacagaggc cagtggggca 40
<210> 264
   <211> 6
   <212> DNA
   <213> Artificial Organism
<220>
   <223> Synthetic oligonucleotide
<400> 264
   ctgtgt 6
<210> 265
   <211> 35
   <212> DNA
   <213> Artificial Organism
<220>
   <223> Synthetic oligonucleotide
<400> 265
   gtgacaacct gtgggacctg actgatgcca gcgtc 35
<210> 266
   <211> 64
   <212> DNA
   <213> Artificial Organism
<220>
   <223> Synthetic oligonucleotide
<400> 266
   gtctgccggg ccctgggctt cgagaacgcc acccaggctc tgggcagagc tgccttcggg 60
   caag 64
<210> 267
   <211> 71
   <212> DNA
   <213> Artificial Organism
<220>
   <223> Synthetic oligonucleotide
<400> 267
   gatcaggccc catcatgctg gacgaggtcc agtgcacggg aaccgaggcc tcactggccg 60
   actgcaagtc c 71
<210> 268
   <211> 61
   <212> DNA
   <213> Artificial Organism
<220>
   <223> Synthetic oligonucleotide
<400> 268
   ctgggctggc tgaagagcaa ctgcaggcac gagagagacg ctggtgtggt ctgcaccaat 60
   g 61
<210> 269
   <211> 19
   <212> DNA
   <213> Artificial Organism
<220>
   <223> Synthetic oligonucleotide
<400> 269
   gagcacccac accctggac 19
<210> 270
   <211> 48
   <212> DNA
   <213> Artificial Organism
<220>
   <223> Synthetic oligonucleotide
<400> 270
   ctctccaggg agctctcgga ggcccttggc cagatctttg acagccag 48
<210> 271
   <211> 23
   <212> DNA
   <213> Artificial Organism
<220>
   <223> Synthetic oligonucleotide
<400> 271
   cggggctgcg acctgtccat cag 23
<210> 272
   <211> 79
   <212> DNA
   <213> Artificial Organism
<220>
   <223> Synthetic oligonucleotide
<400> 272
   cgtgaatgtg cagggcgagg acgccctggg cttctgtggc cacacggtca tcctgactgc 60
   caacctggag gcccaggcc 79
<210> 273
   <211> 77
   <212> DNA
   <213> Artificial Organism
<220>
   <223> Synthetic oligonucleotide
<400> 273
   ctgtggaagg agccgggcag caatgtcacc atgagtgtgg atgctgagtg tgtgcccatg 60
   gtcagggacc ttctcag 77
<210> 274
   <211> 265
   <212> DNA
   <213> Artificial Organism
<220>
   <223> Synthetic oligonucleotide
<400> 274
   gtgagccgtc tcgtcctcag gctctggggc cgccaggggc tttgtcctcc tcccctcttc 60
   aaaagcacag ccccccacac acacacctgc acatactcat gcatgcacat gtacacacgc 120
   agtcacacat gcactcacgc agttgcacac acacgcatgc tcactcccac actgtgtgca 180
   ctcaggtggc tgtgttggac agttgggccc agggctcccc tgctgtcctg tggggccggc 240
   atctgctctc cttctttctc cccag 265
<210> 275
   <211> 76
   <212> DNA
   <213> Artificial Organism
<220>
   <223> Synthetic oligonucleotide
<400> 275
   gtacttctac tcccgaagga ttgacatcac cctgtcgtca gtcaagtgct tccacaagct 60
   ggcctctgcc tatggg 76
<210> 276
   <211> 81
   <212> DNA
   <213> Artificial Organism
<220>
   <223> Synthetic oligonucleotide
<400> 276
   gccaggcagc tgcagggcta ctgcgcaagc ctctttgcca tcctcctccc ccaggacccc 60
   tcgttccaga tgcccctgga c 81
<210> 277
   <211> 33
   <212> DNA
   <213> Artificial Organism
<220>
   <223> Synthetic oligonucleotide
<400> 277
   ctgtatgcct atgcagtggc cacaggggac gcc 33
<210> 278
   <211> 12
   <212> DNA
   <213> Artificial Organism
<220>
   <223> Synthetic oligonucleotide
<400> 278
   ctgctggaga ag 12
<210> 279
   <211> 133
   <212> DNA
   <213> Artificial Organism
<220>
   <223> Synthetic oligonucleotide
<400> 279
   ctctgcctac agttcctggc ctggaacttc gaggccttga cgcaggccga ggcctggccc 60
   agtgtcccca cagacctgct ccaactgctg ctgcccagga gcgacctggc ggtgcccagc 120
   gagctggccc tac 133
<210> 280
   <211> 255
   <212> DNA
   <213> Artificial Organism
<220>
   <223> Synthetic oligonucleotide
<400> 280
   tgaaggccgt ggacacctgg agctgggggg agcgtgcctc ccatgaggag gtggagggct 60
   tggtggagaa gatccgcttc cccatgatgc tccctgagga gctctttgag ctgcagttca 120
   acctgtccct gtactggagc cacgaggccc tgttccagaa gaagactctg caggccctgg 180
   aattccacac tgtgcccttc cagttgctgg cccggtacaa aggcctgaac ctcaccgagg 240
   atacctacaa gcccc 255
<210> 281
   <211> 10
   <212> DNA
   <213> Artificial Organism
<220>
   <223> Synthetic oligonucleotide
<400> 281
   ggatttacac 10
<210> 282
   <211> 9
   <212> DNA
   <213> Artificial Organism
<220>
   <223> Synthetic oligonucleotide
<400> 282
   ctcgcccac 9
<210> 283
   <211> 38
   <212> DNA
   <213> Artificial Organism
<220>
   <223> Synthetic oligonucleotide
<400> 283
   ctggagtgcc tttgtgacag acagttcctg gagtgcac 38
<210> 284
   <211> 117
   <212> DNA
   <213> Artificial Organism
<220>
   <223> Synthetic oligonucleotide
<400> 284
   ggaagtcaca actggtctat cagtccagac gggggccttt ggtcaaatat tcttctgatt 60
   acttccaagc cccctctgac tacagatact acccctacca gtccttccag actccac 117
<210> 285
   <211> 16
   <212> DNA
   <213> Artificial Organism
<220>
   <223> Synthetic oligonucleotide
<400> 285
   aacaccccag cttcct 16
<210> 286
   <211> 49
   <212> DNA
   <213> Artificial Organism
<220>
   <223> Synthetic oligonucleotide
<400> 286
   cttccaggac aagagggtgt cctggtccct ggtctacctc cccaccatc 49
<210> 287
   <211> 9
   <212> DNA
   <213> Artificial Organism
<220>
   <223> Synthetic oligonucleotide
<400> 287
   cagagctgc 9
<210> 288
   <211> 17
   <212> DNA
   <213> Artificial Organism
<220>
   <223> Synthetic oligonucleotide
<400> 288
   tggaactacg gcttctc 17
<210> 289
   <211> 20
   <212> DNA
   <213> Artificial Organism
<220>
   <223> Synthetic oligonucleotide
<400> 289
   ctgctcctcg gacgagctcc 20
<210> 290
   <211> 95
   <212> DNA
   <213> Artificial Organism
<220>
   <223> Synthetic oligonucleotide
<400> 290
   ctgtcctggg cctcaccaag tctggcggct cagatcgcac cattgcctac gaaaacaaag 60
   ccctgatgct ctgcgaaggg ctcttcgtgg cagac 95
<210> 291
   <211> 5
   <212> DNA
   <213> Artificial Organism
<220>
   <223> Synthetic oligonucleotide
<400> 291
   gtcac 5
<210> 292
   <211> 45
   <212> DNA
   <213> Artificial Organism
<220>
   <223> Synthetic oligonucleotide
<400> 292
   cgatttcgag ggctggaagg ctgcgattcc cagtgccctg gacac 45
<210> 293
   <211> 18
   <212> DNA
   <213> Artificial Organism
<220>
   <223> Synthetic oligonucleotide
<400> 293
   caacagctcg aagagcac 18
<210> 294
   <211> 10
   <212> DNA
   <213> Artificial Organism
<220>
   <223> Synthetic oligonucleotide
<400> 294
   ctcctccttc 10
<210> 295
   <211> 2
   <212> DNA
   <213> Artificial Organism
<220>
   <223> Synthetic oligonucleotide
<400> 295
   cc 2
<210> 296
   <211> 57
   <212> DNA
   <213> Artificial Organism
<220>
   <223> Synthetic oligonucleotide
<400> 296
   ctgcccggca gggcacttca acggcttccg cacggtcatc cgccccttct acctgac 57
<210> 297
   <211> 6
   <212> DNA
   <213> Artificial Organism
<220>
   <223> Synthetic oligonucleotide
<400> 297
   caactc 6
<210> 298
   <211> 78
   <212> DNA
   <213> Artificial Organism
<220>
   <223> Synthetic oligonucleotide
<400> 298
   ctcaggtgtg gactagacgg cgtggcccaa gggtggtgag aaccggagaa ccccaggacg 60
   ccctcactgc aggctccc 78
<210> 299
   <211> 8
   <212> DNA
   <213> Artificial Organism
<220>
   <223> Synthetic oligonucleotide
<400> 299
   ctcctcgg 8
<210> 300
   <211> 5
   <212> DNA
   <213> Artificial Organism
<220>
   <223> Synthetic oligonucleotide
<400> 300
   cttcc 5
<210> 301
   <211> 153
   <212> DNA
   <213> Artificial Organism
<220>
   <223> Synthetic oligonucleotide
<400> 301
   ttcctctctg caatgacctt caacaaccgg ccaccagatg tcgccctact cacctgagcg 60
   ctcagcttca agaaattact ggaaggcttc cactagggtc caccaggagt tctcccacca 120
   cctcaccagt ttccaggtgg taagcaccag gac 153
<210> 302
   <211> 30
   <212> DNA
   <213> Artificial Organism
<220>
   <223> Synthetic oligonucleotide
<400> 302
   gccctcgagg ttgctctggg atccccccac 30
<210> 303
   <211> 19
   <212> DNA
   <213> Artificial Organism
<220>
   <223> Synthetic oligonucleotide
<400> 303
   agcccctggt cagtctgcc 19
<210> 304
   <211> 62
   <212> DNA
   <213> Artificial Organism
<220>
   <223> Synthetic oligonucleotide
<400> 304
   cttgtcactg gtctgaggtc attaaaatta cattgaggtt cctacatttg tttgtacaga 60
   aa 62
<210> 305
   <211> 2225
   <212> DNA
   <213> Homo sapiens
<400> 305
   ggggagggat aggacagaca gacaaagaaa ggggtgcggc agcactgcca ggggaagagg 60
   gtgatccgac ccggggaagg tcgctgggca gggcgagttg ggaaagcggc agcccccgcc     120
   gcccccgcag ccccttctcc tcctttctcc cacgtcctat ctgcctctcg ctggaggcca     180
   ggccgtgcag catcgaagac aggaggaact ggagcctcat tggccggccc ggggcgccgg 240
   cctcgggctt aaataggagc tccgggctct ggctgggacc cgaccgctgc cggccgcgct 300
   cccgctgctc ctgccgggtg atggaaaacc ccagcccggc cgccgccctg ggcaaggccc 360
   tctgcgctct cctcctggcc actctcggcg ccgccggcca gcctcttggg ggagagtcca 420
   tctgttccgc cagagccccg gccaaataca gcatcacctt cacgggcaag tggagccaga 480
   cggccttccc caagcagtac cccctgttcc gcccccctgc gcagtggtct tcgctgctgg 540
   gtaagtacag ccccctcctc gcctcctgct ccaccaggag ggctgcggcg ccggagcccc 600
   ccgcacagct gcacggcggc gcttcccagt ctggggcgtg ctgacgtcgg gagactctaa 660
   caccagaatg tttgccgagc gcctgcggtg tgcggttccg gggatgcggt gggggcaggg 720
   ggcgctcacg gggagggcgc aggtgggtcg cggcaggggc agccacaggg tgtcacggtc 780
   cgacgtgact gggggccatg gttccctgcg ggcggcgggg agggatttcc tggggcgccc 840
   cctggaatcc aagcccgacc gcagtgtctg accatggtcc gcggcgcggc cgggtggtcc 900
   tgcaggggcc gcgcatagct ccgactacag catgtggagg aagaaccagt acgtcagtaa 960
   cgggctgcgc gactttgcgg agcgcggcga ggcctgggcg ctgatgaagg agatcgaggc 1020
   ggcgggggag gcgctgcaga gcgtgcacgc ggtgttttcg gcgcccgccg tccccagcgg 1080
   caccgggcag acgtcggcgg agctggaggt gcagcgcagg cactcgctgg tctcgtttgt 1140
   ggtgcgcatc gtgcccagcc ccgactggtt cgtgggcgtg gacagcctgg acctgtgcga 1200
   cggggaccgt tggcgggaac aggcggcgct ggacctgtac ccctacgacg ccgggacgga 1260
   cagcggcttc accttctcct cccccaactt cgccaccatc ccgcaggaca cggtgaccga 1320
   gataacgtcc tcctctccca gccacccggc caactccttc tactacccgc ggctgaaggc 1380
   cctgcctccc atcgccaggg tgacactggt gcggctgcga cagagcccca gggccttcat 1440
   ccctcccgcc ccagtcctgc ccagcaggga caatgagatt gtagacagcg cctcagttcc 1500
   agaaacgccg ctggactgcg aggtctccct gtggtcgtcc tggggactgt gcggaggcca 1560
   ctgtgggagg ctcgggacca agagcaggac tcgctacgtc cgggtccagc ccgccaacaa 1620
   cgggagcccc tgccccgagc tcgaagaaga ggctgagtgc gtccctgata actgcgtcta   1680
   agaccagagc cccgcagccc ctggggcccc ccggagccat ggggtgtcgg gggctcctgt   1740
   gcaggctcat gctgcaggcg gccgagggca cagggggttt cgcgctgctc ctgaccgcgg   1800
   tgaggccgcg ccgaccatct ctgcactgaa gggccctctg gtggccggca cgggcattgg   1860
   gaaacagcct cctcctttcc caaccttgct tcttaggggc ccccgtgtcc cgtctgctct   1920
   cagcctcctc ctcctgcagg ataaagtcat ccccaaggct ccagctactc taaattatgt   1980
   ctccttataa gttattgctg ctccaggaga ttgtccttca tcgtccaggg gcctggctcc   2040
   cacgtggttg cagatacctc agacctggtg ctctaggctg tgctgagccc actctcccga   2100
   gggcgcatcc aagcgggggc cacttgagaa gtgaataaat ggggcggttt cggaagcgtc   2160
   agtgtttcca tgttatggat ctctctgcgt ttgaataaag actatctctg ttgctcacaa   2220
   atgtc                                                               2225
<210> 306
   <211> 1799
   <212> DNA
   <213> Homo sapiens
<400> 306
   ggggagggat aggacagaca gacaaagaaa ggggtgcggc agcactgcca ggggaagagg      60
   gtgatccgac ccggggaagg tcgctgggca gggcgagttg ggaaagcggc agcccccgcc     120
   gcccccgcag ccccttctcc tcctttctcc cacgtcctat ctgcctctcg ctggaggcca     180
   ggccgtgcag catcgaagac aggaggaact ggagcctcat tggccggccc ggggcgccgg     240
   cctcgggctt aaataggagc tccgggctct ggctgggacc cgaccgctgc cggccgcgct     300
   cccgctgctc ctgccgggtg atggaaaacc ccagcccggc cgccgccctg ggcaaggccc     360
   tctgcgctct cctcctggcc actctcggcg ccgccggcca gcctcttggg ggagagtcca     420
   tctgttccgc cagagccccg gccaaataca gcatcacctt cacgggcaag tggagccaga     480
   cggccttccc caagcagtac cccctgttcc gcccccctgc gcagtggtct tcgctgctgg     540
   gggccgcgca tagctccgac tacagcatgt ggaggaagaa ccagtacgtc agtaacgggc     600
   tgcgcgactt tgcggagcgc ggcgaggcct gggcgctgat gaaggagatc gaggcggcgg     660
   gggaggcgct gcagagcgtg cacgcggtgt tttcggcgcc cgccgtcccc agcggcaccg     720
   ggcagacctt tctacagcag gggtgtccgc cctctcccgg ggttcccacg gggttcccag     780
   gggcctctta ctctgccacg atgtgggagt tccaccacca cagggacttg agcggcagct     840
   ccggctctta cgtagaaacg cgcaactcca gtccctaggt tgtgtccgag gttgctatgg     900
   tgccatccca tcttgccgct cactctgcga ctgtgcggag aaacgcaagt gcccccgaag     960
   ggtgggcgtg gcctctgatg aatgcacacg ttggtgggag gtggcttccg tttgtacgaa    1020
   gcgcctcttc acgcgagcgt tcacctcggt ctcccctttg cttggtccag ttccagaaac    1080
   gccgctggac tgcgaggtct ccctgtggtc gtcctgggga ctgtgcggag gccactgtgg    1140
   gaggctcggg accaagagca ggactcgcta cgtccgggtc cagcccgcca acaacgggag    1200
   cccctgcccc gagctcgaag aagaggctga gtgcgtccct gataactgcg tctaagacca    1260
   gagccccgca gcccctgggg ccccccggag ccatggggtg tcgggggctc ctgtgcaggc    1320
   tcatgctgca ggcggccgag ggcacagggg gtttcgcgct gctcctgacc gcggtgaggc    1380
   cgcgccgacc atctctgcac tgaagggccc tctggtggcc ggcacgggca ttgggaaaca    1440
   gcctcctcct ttcccaacct tgcttcttag gggcccccgt gtcccgtctg ctctcagcct    1500
   cctcctcctg caggataaag tcatccccaa ggctccagct actctaaatt atgtctcctt    1560
   ataagttatt gctgctccag gagattgtcc ttcatcgtcc aggggcctgg ctcccacgtg    1620
   gttgcagata cctcagacct ggtgctctag gctgtgctga gcccactctc ccgagggcgc    1680
   atccaagcgg gggccacttg agaagtgaat aaatggggcg gtttcggaag cgtcagtgtt    1740
   tccatgttat ggatctctct gcgtttgaat aaagactatc tctgttgctc acaaatgtc     1799
<210> 307
   <211> 185
   <212> PRT
   <213> Homo sapiens
<400> 307
   Met Glu Asn Pro Ser Pro Ala Ala Ala Leu Gly Lys Ala Leu Cys Ala
   1               5                   10                  15
   Leu Leu Leu Ala Thr Leu Gly Ala Ala Gly Gln Pro Leu Gly Gly Glu
               20                  25                  30
   Ser Ile Cys Ser Ala Arg Ala Pro Ala Lys Tyr Ser Ile Thr Phe Thr
           35                  40                  45
   Gly Lys Trp Ser Gln Thr Ala Phe Pro Lys Gln Tyr Pro Leu Phe Arg
       50                  55                  60
   Pro Pro Ala Gln Trp Ser Ser Leu Leu Gly Ala Ala His Ser Ser Asp
   65                  70                  75                  80
   Tyr Ser Met Trp Arg Lys Asn Gln Tyr Val Ser Asn Gly Leu Arg Asp
                   85                  90                  95
   Phe Ala Glu Arg Gly Glu Ala Trp Ala Leu Met Lys Glu Ile Glu Ala
               100                 105                 110
   Ala Gly Glu Ala Leu Gln Ser Val His Ala Val Phe Ser Ala Pro Ala
           115                 120                 125
   Val Pro Ser Gly Thr Gly Gln Thr Phe Leu Gln Gln Gly Cys Pro Pro
       130                 135                 140
   Ser Pro Gly Val Pro Thr Gly Phe Pro Gly Ala Ser Tyr Ser Ala Thr
   145                 150                 155                 160
   Met Trp Glu Phe His His His Arg Asp Leu Ser Gly Ser Ser Gly Ser
                   165                 170                 175
   Tyr Val Glu Thr Arg Asn Ser Ser Pro
               180                 185
<210> 308
   <211> 107
   <212> PRT
   <213> Homo sapiens
<400> 308
<210> 309
   <211> 79
   <212> DNA
   <213> Artificial Organism
<220>
   <223> Synthetic oligonucleotide
<400> 309
   ggggagggat aggacagaca gacaaagaaa ggggtgcggc agcactgcca ggggaagagg 60
   gtgatccgac ccggggaag 79
<210> 310
   <211> 74
   <212> DNA
   <213> Artificial Organism
<220>
   <223> Synthetic oligonucleotide
<400> 310
   gtcgctgggc agggcgagtt gggaaagcgg cagcccccgc cgcccccgca gccccttctc 60
   ctcctttctc ccac 74
<210> 311
   <211> 28
   <212> DNA
   <213> Artificial Organism
<220>
   <223> Synthetic oligonucleotide
<400> 311
   gtcctatctg cctctcgctg gaggccag 28
<210> 312
   <211> 78
   <212> DNA
   <213> Artificial Organism
<220>
   <223> Synthetic oligonucleotide
<400> 312
   gccgtgcagc atcgaagaca ggaggaactg gagcctcatt ggccggcccg gggcgccggc 60
   ctcgggctta aataggag 78
<210> 313
   <211> 27
   <212> DNA
   <213> Artificial Organism
<220>
   <223> Synthetic oligonucleotide
<400> 313
   ctccgggctc tggctgggac ccgaccg 27
<210> 314
   <211> 31
   <212> DNA
   <213> Artificial Organism
<220>
   <223> Synthetic oligonucleotide
<400> 314
   ctgccggccg cgctcccgct gctcctgccg g 31
<210> 315
   <211> 84
   <212> DNA
   <213> Artificial Organism
<220>
   <223> Synthetic oligonucleotide
<400> 315
   gtgatggaaa accccagccc ggccgccgcc ctgggcaagg ccctctgcgc tctcctcctg 60
   gccactctcg gcgccgccgg ccag 84
<210> 316
   <211> 34
   <212> DNA
   <213> Artificial Organism
<220>
   <223> Synthetic oligonucleotide
<400> 316
   cctcttgggg gagagtccat ctgttccgcc agag 34
<210> 317
   <211> 22
   <212> DNA
   <213> Artificial Organism
<220>
   <223> Synthetic oligonucleotide
<400> 317
   ccccggccaa atacagcatc ac 22
<210> 318
   <211> 79
   <212> DNA
   <213> Artificial Organism
<220>
   <223> Synthetic oligonucleotide
<400> 318
   cttcacgggc aagtggagcc agacggcctt ccccaagcag taccccctgt tccgcccccc 60
   tgcgcagtgg tcttcgctg 79
<210> 319
   <211> 4
   <212> DNA
   <213> Artificial Organism
<220>
   <223> Synthetic oligonucleotide
<400> 319
   ctgg 4
<210> 320
   <211> 365
   <212> DNA
   <213> Artificial Organism
<220>
   <223> Synthetic oligonucleotide
<400> 320
   gtaagtacag ccccctcctc gcctcctgct ccaccaggag ggctgcggcg ccggagcccc 60
   ccgcacagct gcacggcggc gcttcccagt ctggggcgtg ctgacgtcgg gagactctaa 120
   caccagaatg tttgccgagc gcctgcggtg tgcggttccg gggatgcggt gggggcaggg 180
   ggcgctcacg gggagggcgc aggtgggtcg cggcaggggc agccacaggg tgtcacggtc 240
   cgacgtgact gggggccatg gttccctgcg ggcggcgggg agggatttcc tggggcgccc 300
   cctggaatcc aagcccgacc gcagtgtctg accatggtcc gcggcgcggc cgggtggtcc 360
   tgcag 365
<210> 321
   <211> 62
   <212> DNA
   <213> Artificial Organism
<220>
   <223> Synthetic oligonucleotide
<400> 321
   gggccgcgca tagctccgac tacagcatgt ggaggaagaa ccagtacgtc agtaacgggc 60
   tg 62
<210> 322
   <211> 118
   <212> DNA
   <213> Artificial Organism
<220>
   <223> Synthetic oligonucleotide
<400> 322
   cgcgactttg cggagcgcgg cgaggcctgg gcgctgatga aggagatcga ggcggcgggg 60
   gaggcgctgc agagcgtgca cgcggtgttt tcggcgcccg ccgtccccag cggcaccg 118
<210> 323
   <211> 7
   <212> DNA
   <213> Artificial Organism
<220>
   <223> Synthetic oligonucleotide
<400> 323
   ggcagac 7
<210> 324
   <211> 37
   <212> DNA
   <213> Artificial Organism
<220>
   <223> Synthetic oligonucleotide
<400> 324
   gtcggcggag ctggaggtgc agcgcaggca ctcgctg 37
<210> 325
   <211> 143
   <212> DNA
   <213> Artificial Organism
<220>
   <223> Synthetic oligonucleotide
<400> 325
   gtctcgtttg tggtgcgcat cgtgcccagc cccgactggt tcgtgggcgt ggacagcctg 60
   gacctgtgcg acggggaccg ttggcgggaa caggcggcgc tggacctgta cccctacgac 120
   gccgggacgg acagcggctt cac 143
<210> 326
   <211> 49
   <212> DNA
   <213> Artificial Organism
<220>
   <223> Synthetic oligonucleotide
<400> 326
   cttctcctcc cccaacttcg ccaccatccc gcaggacacg gtgaccgag 49
<210> 327
   <211> 175
   <212> DNA
   <213> Artificial Organism
<220>
   <223> Synthetic oligonucleotide
<400> 327
   ataacgtcct cctctcccag ccacccggcc aactccttct actacccgcg gctgaaggcc 60
   ctgcctccca tcgccagggt gacactggtg cggctgcgac agagccccag ggccttcatc 120
   cctcccgccc cagtcctgcc cagcagggac aatgagattg tagacagcgc ctcag 175
<210> 328
   <211> 343
   <212> DNA
   <213> Artificial Organism
<220>
   <223> Synthetic oligonucleotide
<400> 328
   ctttctacag caggggtgtc cgccctctcc cggggttccc acggggttcc caggggcctc 60
   ttactctgcc acgatgtggg agttccacca ccacagggac ttgagcggca gctccggctc 120
   ttacgtagaa acgcgcaact ccagtcccta ggttgtgtcc gaggttgcta tggtgccatc 180
   ccatcttgcc gctcactctg cgactgtgcg gagaaacgca agtgcccccg aagggtgggc 240
   gtggcctctg atgaatgcac acgttggtgg gaggtggctt ccgtttgtac gaagcgcctc 300
   ttcacgcgag cgttcacctc ggtctcccct ttgcttggtc cag 343
<210> 329
   <211> 52
   <212> DNA
   <213> Artificial Organism
<220>
   <223> Synthetic oligonucleotide
<400> 329
   ttccagaaac gccgctggac tgcgaggtct ccctgtggtc gtcctgggga ct 52
<210> 330
   <211> 100
   <212> DNA
   <213> Artificial Organism
<220>
   <223> Synthetic oligonucleotide
<400> 330
   gtgcggaggc cactgtggga ggctcgggac caagagcagg actcgctacg tccgggtcca 60
   gcccgccaac aacgggagcc cctgccccga gctcgaagaa 100
<210> 331
   <211> 38
   <212> DNA
   <213> Artificial Organism
<220>
   <223> Synthetic oligonucleotide
<400> 331
   gaggctgagt gcgtccctga taactgcgtc taagacca 38
<210> 332
   <211> 113
   <212> DNA
   <213> Artificial Organism
<220>
   <223> Synthetic oligonucleotide
<400> 332
   gagccccgca gcccctgggg ccccccggag ccatggggtg tcgggggctc ctgtgcaggc 60
   tcatgctgca ggcggccgag ggcacagggg gtttcgcgct gctcctgacc gcg 113
<210> 333
   <211> 5
   <212> DNA
   <213> Artificial Organism
<220>
   <223> Synthetic oligonucleotide
<400> 333
   gtgag 5
<210> 334
   <211> 27
   <212> DNA
   <213> Artificial Organism
<220>
   <223> Synthetic oligonucleotide
<400> 334
   gccgcgccga ccatctctgc actgaag 27
<210> 335
   <211> 36
   <212> DNA
   <213> Artificial Organism
<220>
   <223> Synthetic oligonucleotide
<400> 335
   ggccctctgg tggccggcac gggcattggg aaacag 36
<210> 336
   <211> 7
   <212> DNA
   <213> Artificial Organism
<220>
   <223> Synthetic oligonucleotide
<400> 336
   cctcctc 7
<210> 337
   <211> 22
   <212> DNA
   <213> Artificial Organism
<220>
   <223> Synthetic oligonucleotide
<400> 337
   ctttcccaac cttgcttctt ag 22
<210> 338
   <211> 68
   <212> DNA
   <213> Artificial Organism
<220>
   <223> Synthetic oligonucleotide
<400> 338
   gggcccccgt gtcccgtctg ctctcagcct cctcctcctg caggataaag tcatccccaa 60
   ggctccag 68
<210> 339
   <211> 261
   <212> DNA
   <213> Artificial Organism
<220>
   <223> Synthetic oligonucleotide
<400> 339
   ctactctaaa ttatgtctcc ttataagtta ttgctgctcc aggagattgt ccttcatcgt 60
   ccaggggcct ggctcccacg tggttgcaga tacctcagac ctggtgctct aggctgtgct 120
   gagcccactc tcccgagggc gcatccaagc gggggccact tgagaagtga ataaatgggg 180
   cggtttcgga agcgtcagtg tttccatgtt atggatctct ctgcgtttga ataaagacta 240
   tctctgttgc tcacaaatgt c 261
<210> 340
   <211> 1282
   <212> DNA
   <213> Homo sapiens
<400> 340
   gtttcctgct tatgcaatag tagctgggag aggccgaaag aattctggtg gggccacacc 60
   cactggtgaa agaataaata gtgaggtttg gcattggcca tcagagtcac tcctgccttc 120
   accatgaagt ccagcggcct cttccccttc ctggtgctgc ttgccctggg aactctggca 180
   ccttgggctg tggaaggctc tggaaagtcc ttcaaagctg gagtctgtcc tcctaagaaa 240
   tctgcccagt gccttagata caagaaacct gagtgccaga gtgactggca gtgtccaggg 300
   aagaagagat gttgtcctga cacttgtggc atcaaatgcc tggatcctgt tgacacccca 360
   aacccaacaa ggaggaagcc tgggaagtgc ccagtgactt atggccaatg tttgatgctt 420
   aaccccccca atttctgtga gatggatggc cagtgcaagc gtgacttgaa gtgttgcatg 480
   ggcatgtgtg ggaaatcctg cgtttcccct gtgaaaggta agcaggggat gagggcacac 540
   tgagctccct ccagccctct cagcctcaac cctctggagg cccaggcata tgggcagggg 600
   gactcctgaa ccctactcca agcacagcct ctgtctgact cccttgtcct tcaagagaac 660
   tgttctccag gtctcagggc caggatttcc ataggatcgc ctgtggcttt gattctattc 720
   tagtgtctct gggtgggggt cctgggcaag tgtctttctg agtctcagtt tctttatcgg 780
   taaaatgtac ataatgagat tgaaagtgct ctgcaaagca ctatgtgcac taagaattta 840
   ttattcaggt tgtttccatc atgttttctg aggtgaaatc acaaaggatc agtggagttt 900
   gaggattatc tagttcaatg ctttgagttt agagttttac ggtgaaaatg agacttgtct 960
   cctgacacta agtctctctc aactatagcg ctatcttgct attttctcta tctcagaagg 1020
   atccttgggc aggaggaagg atgtggatat gatttggctg gtttctatgc tgaagctctt 1080
   atctgatttt ctctcacagc ttgattcctg ccatatggag gaggctctgg agtcctgctc 1140
   tgtgtggtcc aggtcctttc caccctgaga cttggctcca ccactgatat cctcctttgg 1200
   ggaaaggctt ggcacacagc aggctttcaa gaagtgccag ttgatcaatg aataaataaa 1260
   cgagcctatt tctctttgca ca 1282
<210> 341
   <211> 696
   <212> DNA
   <213> Homo sapiens
<400> 341
   gtttcctgct tatgcaatag tagctgggag aggccgaaag aattctggtg gggccacacc     60
   cactggtgaa agaataaata gtgaggtttg gcattggcca tcagagtcac tcctgccttc    120
   accatgaagt ccagcggcct cttccccttc ctggtgctgc ttgccctggg aactctggca    180
   ccttgggctg tggaaggctc tggaaagtcc ttcaaagctg gagtctgtcc tcctaagaaa    240
   tctgcccagt gccttagata caagaaacct gagtgccaga gtgactggca gtgtccaggg    300
   aagaagagat gttgtcctga cacttgtggc atcaaatgcc tggatcctgt tgacacccca    360
   aacccaagag gaagcctggg aagtgcccag tgacttatgg ccaatgtttg atgcttaacc    420
   cccccaattt ctgtgagatg gatggccagt gcaagcgtga cttgaagtgt tgcatgggca    480
   tgtgtgggaa atcctgcgtt tcccctgtga aagcttgatt cctgccatat ggaggaggct    540
   ctggagtcct gctctgtgtg gtccaggtcc tttccaccct gagacttggc tccaccactg    600
   atatcctcct ttggggaaag gcttggcaca cagcaggctt tcaagaagtg ccagttgatc    660
   aatgaataaa taaacgagcc tatttctctt tgcaca                              696
<210> 342
   <211> 695
   <212> DNA
   <213> Homo sapiens
<400> 342
   gtttcctgct tatgcaatag tagctgggag aggccgaaag aattctggtg gggccacacc     60
   cactggtgaa agaataaata gtgaggtttg gcattggcca tcagagtcac tcctgccttc    120
   accatgaagt ccagcggcct cttccccttc ctggtgctgc ttgccctggg aactctggca    180
   ccttgggctg tggaaggctc tggaaagtcc ttcaaagctg gagtctgtcc tcctaagaaa    240
   tctgcccagt gccttagata caagaaacct gagtgccaga gtgactggca gtgtccaggg    300
   aagaagagat gttgtcctga cacttgtggc atcaaatgcc tggatcctgt tgacacccca    360
   aacccaacaa ggaggaagcc tgggaagtgc ccagtgactt atggccaatg tttgatgctt    420
   aaccccccca atttctgtga gatggatggc cagtgcaagc gtgacttgaa gtgttgcatg    480
   ggcatgtgtg ggaaatcctg cgtttcccct gtgaaaggct gcttcagtcc ttcaataagc    540
   ccatcacact ttttcaccat gtcatctatc agcacttttt ctgcagtgtt acgaacatca    600
   gcttcatcac tgtcagcctg cgttttgcct gcaacccatc aaatgaggtc aggagaggag    660
   ttttccactt ttggcttcat gttggtgctc aaaac                               695
<210> 343
   <211> 885
   <212> DNA
   <213> Homo sapiens
<400> 343
   gtttcctgct tatgcaatag tagctgggag aggccgaaag aattctggtg gggccacacc      60
   cactggtgaa agaataaata gtgaggtttg gcattggcca tcagagtcac tcctgccttc     120
   accatgaagt ccagcggcct cttccccttc ctggtgctgc ttgccctggg aactctggca     180
   ccttgggctg tggaaggctc tggaaagtcc ttcaaagctg gagtctgtcc tcctaagaaa     240
   tctgcccagt gccttagata caagaaacct gagtgccaga gtgactggca gtgtccaggg     300
   aagaagagat gttgtcctga cacttgtggc atcaaatgcc tggatcctgt tgacacccca     360
   aacccaacaa ggaggaagcc tgggaagtgc ccagtgactt atggccaatg tttgatgctt     420
   aaccccccca atttctgtga gatggatggc cagtgcaagc gtgacttgaa gtgttgcatg     480
   ggcatgtgtg ggaaatcctg cgtttcccct gtgaaaggtg aaaagagaca tcacaagcaa     540
   ttgagggacc aggaagtgga tcctctagag atgaggaggc attctgctgg atgactttta     600
   aaaatgtttt ctccagagtc atctctctca ttaacaatgt tttttgtctt agaaatttct     660
   tgttgatttt taaacttaca tgatttcttg ttttggtatg aatacaggct gcttcagtcc     720
   ttcaataagc ccatcacact ttttcaccat gtcatctatc agcacttttt ctgcagtgtt     780
   acgaacatca gcttcatcac tgtcagcctg cgttttgcct gcaacccatc aaatgaggtc     840
   aggagaggag ttttccactt ttggcttcat gttggtgctc aaaac                       885
<210> 344
   <211> 550
   <212> DNA
   <213> Homo sapiens

<400> 344
   gtttcctgct tatgcaatag tagctgggag aggccgaaag aattctggtg gggccacacc      60
   cactggtgaa agaataaata gtgaggtttg gcattggcca tcagagtcac tcctgccttc     120
   accatgaagt ccagcggcct cttccccttc ctggtgctgc ttgccctggg aactctggca     180
   ccttgggctg tggaaggctc tggaaagtcc ttcaaagctg gagtctgtcc tcctaagaaa     240
   tctgcccagt gccttagata caagaaacct gagtgccaga gtgactggca gtgtccaggg     300
   aagaagagat gttgtcctga cacttgtggc atcaaatgcc tggatcctgt tgacacccca     360
   aacccaactt gattcctgcc atatggagga ggctctggag tcctgctctg tgtggtccag     420
   gtcctttcca ccctgagact tggctccacc actgatatcc tcctttgggg aaaggcttgg     480
   cacacagcag gctttcaaga agtgccagtt gatcaatgaa taaataaacg agcctatttc     540
   tctttgcaca                                                            550
<210> 345
   <211> 139
   <212> PRT
   <213> Homo sapiens
<400> 345
   Met Lys Ser Ser Gly Leu Phe Pro Phe Leu Val Leu Leu Ala Leu Gly
   1               5                   10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Thr Arg Arg Lys Pro Gly Lys Cys Pro Val Thr Tyr Gly Gln Cys
                   85                  90                  95
   Leu Met Leu Asn Pro Pro Asn Phe Cys Glu Met Asp Gly Gln Cys Lys
               100                 105                 110
   Arg Asp Leu Lys Cys Cys Met Gly Met Cys Gly Lys Ser Cys Val Ser
           115                 120                 125
   Pro Val Lys Gly Lys Gln Gly Met Arg Ala His
       130                 135
<210> 346
   <211> 190
   <212> PRT
   <213> Homo sapiens
<400> 346
   Met Lys Ser Ser Gly Leu Phe Pro Phe Leu Val Leu Leu Ala Leu Gly
   1               5                   10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Thr Arg Arg Lys Pro Gly Lys Cys Pro Val Thr Tyr Gly Gln Cys
                   85                  90                  95
   Leu Met Leu Asn Pro Pro Asn Phe Cys Glu Met Asp Gly Gln Cys Lys
               100                 105                 110
   Arg Asp Leu Lys Cys Cys Met Gly Met Cys Gly Lys Ser Cys Val Ser
           115                 120                 125
   Pro Val Lys Gly Cys Phe Ser Pro Ser Ile Ser Pro Ser His Phe Phe
       130                 135                 140
   Thr Met Ser Ser Ile Ser Thr Phe Ser Ala Val Leu Arg Thr Ser Ala
   145                 150                 155                 160
   Ser Ser Leu Ser Ala Cys Val Leu Pro Ala Thr His Gln Met Arg Ser
                   165                 170                 175
   Gly Glu Glu Phe Ser Thr Phe Gly Phe Met Leu Val Leu Lys
               180                 185                 190
<210> 347
   <211> 156
   <212> PRT
   <213> Homo sapiens
<400> 347
   Met Lys Ser Ser Gly Leu Phe Pro Phe Leu Val Leu Leu Ala Leu Gly
   1               5                   10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Thr Arg Arg Lys Pro Gly Lys Cys Pro Val Thr Tyr Gly Gln Cys
                   85                  90                  95
   Leu Met Leu Asn Pro Pro Asn Phe Cys Glu Met Asp Gly Gln Cys Lys
               100                 105                 110
   Arg Asp Leu Lys Cys Cys Met Gly Met Cys Gly Lys Ser Cys Val Ser
           115                 120                 125
   Pro Val Lys Gly Glu Lys Arg His His Lys Gln Leu Arg Asp Gln Glu
       130                 135                 140
   Val Asp Pro Leu Glu Met Arg Arg His Ser Ala Gly
   145                 150                 155
<210> 348
   <211> 89
   <212> PRT
   <213> Homo sapiens
<400> 348
   Met Lys Ser Ser Gly Leu Phe Pro Phe Leu Val Leu Leu Ala Leu Gly
   1               5                   10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Arg Gly Ser Leu Gly Ser Ala Gln
                   85
<210> 349
   <211> 82
   <212> PRT
   <213> Homo sapiens
<400> 349
   Met Lys Ser Ser Gly Leu Phe Pro Phe Leu Val Leu Leu Ala Leu Gly
   1               5                   10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Thr
<210> 350
   <211> 208
   <212> DNA
   <213> Artificial Organism
<220>
   <223> Synthetic oligonucleotide
<400> 350
   gtttcctgct tatgcaatag tagctgggag aggccgaaag aattctggtg gggccacacc 60
   cactggtgaa agaataaata gtgaggtttg gcattggcca tcagagtcac tcctgccttc 120
   accatgaagt ccagcggcct cttccccttc ctggtgctgc ttgccctggg aactctggca 180
   ccttgggctg tggaaggctc tggaaagt 208
<210> 351
   <211> 37
   <212> DNA
   <213> Artificial Organism
<220>
   <223> Synthetic oligonucleotide
<400> 351
   ccttcaaagc tggagtctgt cctcctaaga aatctgc 37
<210> 352
   <211> 122
   <212> DNA
   <213> Artificial Organism
<220>
   <223> Synthetic oligonucleotide
<400> 352
   ccagtgcctt agatacaaga aacctgagtg ccagagtgac tggcagtgtc cagggaagaa 60
   gagatgttgt cctgacactt gtggcatcaa atgcctggat cctgttgaca ccccaaaccc 120
   aa 122
<210> 353
   <211> 4
   <212> DNA
   <213> Artificial Organism
<220>
   <223> Synthetic oligonucleotide
<400> 353
   caag 4
<210> 354
   <211> 146
   <212> DNA
   <213> Artificial Organism
<220>
   <223> Synthetic oligonucleotide
<400> 354
   gaggaagcct gggaagtgcc cagtgactta tggccaatgt ttgatgctta acccccccaa 60
   tttctgtgag atggatggcc agtgcaagcg tgacttgaag tgttgcatgg gcatgtgtgg 120
   gaaatcctgc gtttcccctg tgaaag 146
<210> 355
   <211> 582
   <212> DNA
   <213> Artificial Organism
<220>
   <223> Synthetic oligonucleotide
<400> 355
   gtaagcaggg gatgagggca cactgagctc cctccagccc tctcagcctc aaccctctgg 60
   aggcccaggc atatgggcag ggggactcct gaaccctact ccaagcacag cctctgtctg 120
   actcccttgt ccttcaagag aactgttctc caggtctcag ggccaggatt tccataggat 180
   cgcctgtggc tttgattcta ttctagtgtc tctgggtggg ggtcctgggc aagtgtcttt 240
   ctgagtctca gtttctttat cggtaaaatg tacataatga gattgaaagt gctctgcaaa 300
   gcactatgtg cactaagaat ttattattca ggttgtttcc atcatgtttt ctgaggtgaa 360
   atcacaaagg atcagtggag tttgaggatt atctagttca atgctttgag tttagagttt 420
   tacggtgaaa atgagacttg tctcctgaca ctaagtctct ctcaactata gcgctatctt 480
   gctattttct ctatctcaga aggatccttg ggcaggagga aggatgtgga tatgatttgg 540
   ctggtttcta tgctgaagct cttatctgat tttctctcac ag 582
<210> 356
   <211> 183
   <212> DNA
   <213> Artificial Organism
<220>
   <223> Synthetic oligonucleotide
<400> 356
   cttgattcct gccatatgga ggaggctctg gagtcctgct ctgtgtggtc caggtccttt 60
   ccaccctgag acttggctcc accactgata tcctcctttg gggaaaggct tggcacacag 120
   caggctttca agaagtgcca gttgatcaat gaataaataa acgagcctat ttctctttgc 180
   aca 183
<210> 357
   <211> 190
   <212> DNA
   <213> Artificial Organism
<220>
   <223> Synthetic oligonucleotide
<400> 357
   gtgaaaagag acatcacaag caattgaggg accaggaagt ggatcctcta gagatgagga      60
   ggcattctgc tggatgactt ttaaaaatgt tttctccaga gtcatctctc tcattaacaa     120
   tgttttttgt cttagaaatt tcttgttgat ttttaaactt acatgatttc ttgttttggt     180
   atgaatacag                                                            190
<210> 358
   <211> 178
   <212> DNA
   <213> Artificial Organism
<220>
   <223> Synthetic oligonucleotide
<400> 358
   gctgcttcag tccttcaata agcccatcac actttttcac catgtcatct atcagcactt      60
   tttctgcagt gttacgaaca tcagcttcat cactgtcagc ctgcgttttg cctgcaaccc     120
   atcaaatgag gtcaggagag gagttttcca cttttggctt catgttggtg ctcaaaac       178
<210> 359
   <211> 1338
   <212> PRT
   <213> Homo sapiens
<400> 359
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
   210 215 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545 550 555 560
   Val Asn Leu Glu Lys Met Pro Thr Glu Gly Glu Asp Leu Lys Leu Ser
   565 570 575
   Cys Thr Val Asn Lys Phe Leu Tyr Arg Asp Val Thr Trp Ile Leu Leu
   580 585 590
   Arg Thr Val Asn Asn Arg Thr Met His Tyr Ser Ile Ser Lys Gln Lys
   595 600 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Asp Gln Glu Ala Pro Tyr Leu Leu Arg Asn Leu Ser Asp His Thr Val
   660 665 670
   Ala Ile Ser Ser Ser Thr Thr Leu Asp Cys His Ala Asn Gly Val Pro
   675 680 685
   Glu Pro Gln Ile Thr Trp Phe Lys Asn Asn His Lys Ile Gln Gln Glu
   690 695 700
   Pro Gly Ile Ile Leu Gly Pro Gly Ser Ser Thr Leu Phe Ile Glu Arg
   705 710 715 720
   Val Thr Glu Glu Asp Glu Gly Val Tyr His Cys Lys Ala Thr Asn Gln
   725 730 735
   Lys Gly Ser Val Glu Ser Ser Ala Tyr Leu Thr Val Gln Gly Thr Ser
   740 745 750
   Asp Lys Ser Asn Leu Glu Leu Ile Thr Leu Thr Cys Thr Cys Val Ala
   755 760 765
   Ala Thr Leu Phe Trp Leu Leu Leu Thr Leu Leu Ile Arg Lys Met Lys
   770 775 780
   Arg Ser Ser Ser Glu Ile Lys Thr Asp Tyr Leu Ser Ile Ile Met Asp
   785 790 795 800
   Pro Asp Glu Val Pro Leu Asp Glu Gln Cys Glu Arg Leu Pro Tyr Asp
   805 810 815
   Ala Ser Lys Trp Glu Phe Ala Arg Glu Arg Leu Lys Leu Gly Lys Ser
   820 825 830
   Leu Gly Arg Gly Ala Phe Gly Lys Val Val Gln Ala Ser Ala Phe Gly
   835 840 845
   Ile Lys Lys Ser Pro Thr Cys Arg Thr Val Ala Val Lys Met Leu Lys
   850 855 860
   Glu Gly Ala Thr Ala Ser Glu Tyr Lys Ala Leu Met Thr Glu Leu Lys
   865 870 875 880
   Ile Leu Thr His Ile Gly His His Leu Asn Val Val Asn Leu Leu Gly
   885 890 895
   Ala Cys Thr Lys Gln Gly Gly Pro Leu Met Val Ile Val Glu Tyr Cys
   900 905 910
   Lys Tyr Gly Asn Leu Ser Asn Tyr Leu Lys Ser Lys Arg Asp Leu Phe
   915 920 925
   Phe Leu Asn Lys Asp Ala Ala Leu His Met Glu Pro Lys Lys Glu Lys
   930 935 940
   Met Glu Pro Gly Leu Glu Gln Gly Lys Lys Pro Arg Leu Asp Ser Val
   945 950 955 960
   Thr Ser Ser Glu Ser Phe Ala Ser Ser Gly Phe Gln Glu Asp Lys Ser
   965 970 975
   Leu Ser Asp Val Glu Glu Glu Glu Asp Ser Asp Gly Phe Tyr Lys Glu
   980 985 990
   Pro Ile Thr Met Glu Asp Leu Ile Ser Tyr Ser Phe Gln Val Ala Arg
   995 1000 1005
   Gly Met Glu Phe Leu Ser Ser Arg Lys Cys Ile His Arg Asp Leu
   1010 1015 1020
   Ala Ala Arg Asn Ile Leu Leu Ser Glu Asn Asn Val Val Lys Ile
   1025 1030 1035
   Cys Asp Phe Gly Leu Ala Arg Asp Ile Tyr Lys Asn Pro Asp Tyr
   1040 1045 1050
   Val Arg Lys Gly Asp Thr Arg Leu Pro Leu Lys Trp Met Ala Pro
   1055 1060 1065
   Glu Ser Ile Phe Asp Lys Ile Tyr Ser Thr Lys Ser Asp Val Trp
   1070 1075 1080
   Ser Tyr Gly Val Leu Leu Trp Glu Ile Phe Ser Leu Gly Gly Ser
   1085 1090 1095
   Pro Tyr Pro Gly Val Gln Met Asp Glu Asp Phe Cys Ser Arg Leu
   1100 1105 1110
   Arg Glu Gly Met Arg Met Arg Ala Pro Glu Tyr Ser Thr Pro Glu
   1115 1120 1125
   Ile Tyr Gln Ile Met Leu Asp Cys Trp His Arg Asp Pro Lys Glu
   1130 1135 1140
   Arg Pro Arg Phe Ala Glu Leu Val Glu Lys Leu Gly Asp Leu Leu
   1145 1150 1155
   Gln Ala Asn Val Gln Gln Asp Gly Lys Asp Tyr Ile Pro Ile Asn
   1160 1165 1170
   Ala Ile Leu Thr Gly Asn Ser Gly Phe Thr Tyr Ser Thr Pro Ala
   1175 1180 1185
   Phe Ser Glu Asp Phe Phe Lys Glu Ser Ile Ser Ala Pro Lys Phe
   1190 1195 1200
   Asn Ser Gly Ser Ser Asp Asp Val Arg Tyr Val Asn Ala Phe Lys
   1205 1210 1215
   Phe Met Ser Leu Glu Arg Ile Lys Thr Phe Glu Glu Leu Leu Pro
   1220 1225 1230
   Asn Ala Thr Ser Met Phe Asp Asp Tyr Gln Gly Asp Ser Ser Thr
   1235 1240 1245
   Leu Leu Ala Ser Pro Met Leu Lys Arg Phe Thr Trp Thr Asp Ser
   1250 1255 1260
   Lys Pro Lys Ala Ser Leu Lys Ile Asp Leu Arg Val Thr Ser Lys
   1265 1270 1275
   Ser Lys Glu Ser Gly Leu Ser Asp Val Ser Arg Pro Ser Phe Cys
   1280 1285 1290
   His Ser Ser Cys Gly His Val Ser Glu Gly Lys Arg Arg Phe Thr
   1295 1300 1305
   Tyr Asp His Ala Glu Leu Glu Arg Lys Ile Ala Cys Cys Ser Pro
   1310 1315 1320
   Pro Pro Asp Tyr Asn Ser Val Val Leu Tyr Ser Thr Pro Pro Ile
   1325 1330 1335
<210> 360
   <211> 687
   <212> PRT
   <213> Homo sapiens
<400> 360
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545 550 555 560
   Val Asn Leu Glu Lys Met Pro Thr Glu Gly Glu Asp Leu Lys Leu Ser
   565 570 575
   Cys Thr Val Asn Lys Phe Leu Tyr Arg Asp Val Thr Trp Ile Leu Leu
   580 585 590
   Arg Thr Val Asn Asn Arg Thr Met His Tyr Ser Ile Ser Lys Gln Lys
   595 600 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Gly Glu His Cys Asn Lys Lys Ala Val Phe Ser Arg Ile Ser Lys Phe
   660 665 670
   Lys Ser Thr Arg Asn Asp Cys Thr Thr Gln Ser Asn Val Lys His
   675 680 685
<210> 361
   <211> 21
   <212> DNA
   <213> Homo sapiens
<400> 361
   gcatagttca gcgttgccaa c 21
<210> 362
   <211> 20
   <212> DNA
   <213> Homo sapiens
<400> 362
   ccatggccaa gctgtattca 20
<210> 363
   <211> 127
   <212> DNA
   <213> Homo sapiens
<400> 363
   gcatagttca gcgttgccaa ctcatgctat gctttctaat cattgtagat gtctgtgttc      60
   tctaaataag tcagttttct gttggccaag agttacatta tcatgaatga atacagcttg     120
   gccatgg                                                               127
<210> 364
   <211> 2277
   <212> DNA
   <213> Homo sapiens
<400> 364
   gactgcgcgg cggcaacagc agacatgtcg ggggtccggg gcctgtcgcg gctgctgagc      60
   gctcggcgcc tggcgctggc caaggcgtgg ccaacagtgt tgcaaacagg aacccgaggt     120
   tttcacttca ctgttgatgg gaacaagagg gcatctgcta aagtttcaga ttccatttct     180
   gctcagtatc cagtagtgga tcatgaattt gatgcagtgg tggtaggcgc tggaggggca     240
   ggcttgcgag ctgcatttgg cctttctgag gcagggttta atacagcatg tgttaccaag     300
   ctgtttccta ccaggtcaca cactgttgca gcgcagggag gaatcaatgc tgctctgggg     360
   aacatggagg aggacaactg gaggtggcat ttctacgaca ccgtgaaggg ctccgactgg     420
   ctgggggacc aggatgccat ccactacatg acggagcagg cccccgccgc cgtggtcgag     480
   ctagaaaatt atggcatgcc gtttagcaga actgaagatg ggaagattta tcagcgtgca     540
   tttggtggac agagcctcaa gtttggaaag ggcgggcagg cccatcggtg ctgctgtgtg     600
   gctgatcgga ctggccactc gctattgcac accttatatg gacggtctct gcgatatgat     660
   accagctatt ttgtggagta ttttgccttg gatctcctga tggagaacgg ggagtgccgt     720
   ggtgtcatcg cactgtgcat agaggacggg tccatccatc gcataagagc aaagaacact     780
   gttgttgcca caggaggcta cgggcgcacc tacttcagct gcacgtctgc ccacaccagc     840
   actggcgacg gcacggccat gatcaccagg gcaggccttc cttgccagga cctagagttt     900
   gttcagttcc accccacagg catatatggt gctggttgtc tcattacgga aggatgtcgt     960
   ggagagggag gcattctcat taacagtcaa ggcgaaaggt ttatggagcg atacgcccct    1020
   gtcgcgaagg acctggcgtc tagagatgtg gtgtctcggt cgatgactct ggagatccga    1080
   gaaggaagag gctgtggccc tgagaaagat cacgtctacc tgcagctgca ccacctacct    1140
   ccagagcagc tggccacgcg cctgcctggc atttcagaga cagccatgat cttcgctggc    1200
   gtggacgtca cgaaggagcc gatccctgtc ctccccaccg tgcattataa catgggcggc    1260
   attcccacca actacaaggg gcaggtcctg aggcacgtga atggccagga tcagattgtg    1320
   cccggcctgt acgcctgtgg ggaggccgcc tgtgcctcgg tacatggtgc caaccgcctc    1380
   ggggcaaact cgctcttgga cctggttgtc tttggtcggg catgtgccct gagcatcgaa    1440
   gagtcatgca ggcctggaga taaagtccct ccaattaaac caaacgctgg ggaagaatct 1500
   gtcatgaatc ttgacaaatt gagatttgct gatggaagca taagaacatc ggaactgcga 1560
   ctcagcatgc agaagtcaat gcaaaatcat gctgccgtgt tccgtgtggg aagcgtgttg 1620
   caagaaggtt gtgggaaaat cagcaagctc tatggagacc taaagcacct gaagacgttc 1680
   gaccggggaa tggtctggaa cacagacctg gtggagaccc tggagctgca gaacctgatg 1740
   ctgtgtgcgc tgcagaccat ctacggagca gaggcgcgga aggagtcacg gggcgcgcat 1800
   gccagggaag actacaaggt gcggattgat gagtacgatt actccaagcc catccagggg 1860
   caacagaaga agccctttga ggagcactgg aggaagcaca ccctgtcctt tgtggacgtt 1920
   ggcactggga aggtcactct ggaatataga cccgtaatcg acaaaacttt gaacgaggct 1980
   gactgtgcca ccatcccgcc agccattcgc tcctactgat gagacaagat gtggtgatga 2040
   cagaatcagc ttttgtaatt atgtataata gctcatgcat gtgtccatgt cataactgtc 2100
   ttcatacgct tctgcactct ggggaagaag gagtacattg aagggagatt ggcacctagt 2160
   ggctgggagc ttgccaggaa cccagtggcc agggagcgtg gcacttacct ttgtcccttg 2220
   cttcattctt gtgagatgat aaaactgggc acagctctta aataaaatat aaatgag 2277
<210> 365
   <211> 86
   <212> DNA
   <213> Artificial Organism
<220>
   <223> Synthetic oligonucleotide
<400> 365
   tgggaacaag agggcatctg ctaaagtttc agattccatt tctgctcagt atccagtagt 60
   ggatcatgaa tttgatgcag tggtgg 86
<210> 366
   <211> 2201
   <212> DNA
   <213> Homo sapiens
<400> 366
   cgggatttgg gtcgcggttc ttgtttgtgg atcgctgtga tcgtcacttg acaatgcaga      60
   tcttcgtgaa gactctgact ggtaagacca tcaccctcga ggttgagccc agtgacacca     120
   tcgagaatgt caaggcaaag atccaagata aggaaggcat ccctcctgac cagcagaggc     180
   tgatctttgc tggaaaacag ctggaagatg ggcgcaccct gtctgactac aacatccaga     240
   aagagtccac cctgcacctg gtgctccgtc tcagaggtgg gatgcaaatc ttcgtgaaga     300
   cactcactgg caagaccatc acccttgagg tggagcccag tgacaccatc gagaacgtca     360
   aagcaaagat ccaggacaag gaaggcattc ctcctgacca gcagaggttg atctttgccg     420
   gaaagcagct ggaagatggg cgcaccctgt ctgactacaa catccagaaa gagtctaccc 480
   tgcacctggt gctccgtctc agaggtggga tgcagatctt cgtgaagacc ctgactggta 540
   agaccatcac cctcgaggtg gagcccagtg acaccatcga gaatgtcaag gcaaagatcc 600
   aagataagga aggcattcct cctgatcagc agaggttgat ctttgccgga aaacagctgg 660
   aagatggtcg taccctgtct gactacaaca tccagaaaga gtccaccttg cacctggtac 720
   tccgtctcag aggtgggatg caaatcttcg tgaagacact cactggcaag accatcaccc 780
   ttgaggtcga gcccagtgac actatcgaga acgtcaaagc aaagatccaa gacaaggaag 840
   gcattcctcc tgaccagcag aggttgatct ttgccggaaa gcagctggaa gatgggcgca 900
   ccctgtctga ctacaacatc cagaaagagt ctaccctgca cctggtgctc cgtctcagag 960
   gtgggatgca gatcttcgtg aagaccctga ctggtaagac catcaccctc gaagtggagc 1020
   cgagtgacac cattgagaat gtcaaggcaa agatccaaga caaggaaggc atccctcctg 1080
   accagcagag gttgatcttt gccggaaaac agctggaaga tggtcgtacc ctgtctgact 1140
   acaacatcca gaaagagtcc accttgcacc tggtgctccg tctcagaggt gggatgcaga 1200
   tcttcgtgaa gaccctgact ggtaagacca tcactctcga ggtggagccg agtgacacca 1260
   ttgagaatgt caaggcaaag atccaagaca aggaaggcat ccctcctgat cagcagaggt 1320
   tgatctttgc tgggaaacag ctggaagatg gacgcaccct gtctgactac aacatccaga 1380
   aagagtccac cctgcacctg gtgctccgtc ttagaggtgg gatgcagatc ttcgtgaaga 1440
   ccctgactgg taagaccatc actctcgaag tggagccgag tgacaccatt gagaatgtca 1500
   aggcaaagat ccaagacaag gaaggcatcc ctcctgacca gcagaggttg atctttgctg 1560
   ggaaacagct ggaagatgga cgcaccctgt ctgactacaa catccagaaa gagtccaccc 1620
   tgcacctggt gctccgtctt agaggtggga tgcagatctt cgtgaagacc ctgactggta 1680
   agaccatcac tctcgaagtg gagccgagtg acaccattga gaatgtcaag gcaaagatcc 1740
   aagacaagga aggcatccct cctgaccagc agaggttgat ctttgctggg aaacagctgg 1800
   aagatggacg caccctgtct gactacaaca tccagaaaga gtccaccctg cacctggtgc 1860
   tccgtctcag aggtgggatg cagatcttcg tgaagaccct gactggtaag accatcaccc 1920
   tcgaggtgga gcccagtgac accatcgaga atgtcaaggc aaagatccaa gataaggaag 1980
   gcatccctcc tgatcagcag aggttgatct ttgctgggaa acagctggaa gatggacgca 2040
   ccctgtctga ctacaacatc cagaaagagt ccactctgca cttggtcctg cgcttgaggg 2100
   ggggtgtcta agtttcccct tttaaggttt caacaaattt cattgcactt tcctttcaat 2160
   aaagttgttg cattcccaaa aaaaaaaaaa aaaaaaaaaa a 2201
<210> 367
   <211> 133
   <212> DNA
   <213> Artificial Organism
<220>
   <223> Synthetic oligonucleotide
<400> 367
   atttgggtcg cggttcttgt ttgtggatcg ctgtgatcgt cacttgacaa tgcagatctt 60
   cgtgaagact ctgactggta agaccatcac cctcgaggtt gagcccagtg acaccatcga 120
   gaatgtcaag gca 133
<210> 368
   <211> 101
   <212> DNA
   <213> Artificial Organism
<220>
   <223> Synthetic oligonucleotide
<400> 368
   tggcaagaag aaggtctggt tagaccccaa tgagaccaat gaaatcgcca atgccaactc 60
   ccgtcagcag atccggaagc tcatcaaaga tgggctgatc a 101
<210> 369
   <211> 748
   <212> DNA
   <213> Homo sapiens
<400> 369
   gcagataatg ggaggagccg ggcccgagcg agctctttcc tttcgctgct gcggccgcag      60
   ccatgagtat gctcaggctt cagaagaggc tcgcctctag tgtcctccgc tgtggcaaga     120
   agaaggtctg gttagacccc aatgagacca atgaaatcgc caatgccaac tcccgtcagc     180
   agatccggaa gctcatcaaa gatgggctga tcatccgcaa gcctgtgacg gtccattccc     240
   gggctcgatg ccggaaaaac accttggccc gccggaaggg caggcacatg ggcataggta     300
   agcggaaggg tacagccaat gcccgaatgc cagagaaggt cacatggatg aggagaatga     360
   ggattttgcg ccggctgctc agaagatacc gtgaatctaa gaagatcgat cgccacatgt     420
   atcacagcct gtacctgaag gtgaagggga atgtgttcaa aaacaagcgg attctcatgg     480
   aacacatcca caagctgaag gcagacaagg cccgcaagaa gctcctggct gaccaggctg     540
   aggcccgcag gtctaagacc aaggaagcac gcaagcgccg tgaagagcgc ctccaggcca     600
   agaaggagga gatcatcaag actttatcca aggaggaaga gaccaagaaa taaaacctcc     660
   cactttgtct gtacatactg gcctctgtga ttacatagat cagccattaa aataaaacaa     720
   gccttaatct gcaaaaaaaa aaaaaaaa                                         748
<210> 370
   <211> 122
   <212> DNA
   <213> Artificial Organism
<220>
   <223> Synthetic oligonucleotide
<400> 370
   cggtttgctg cggtaatcat gaggataaga gagccacgaa ccacggcact gattttcagt      60
   tctgggaaaa tggtgtgcac aggagccaag agtgaagaac agtccagact ggcagcaaga     120
   aa                                                                    122
<210> 371
   <211> 1867
   <212> DNA
   <213> Homo sapiens
<400> 371
   ggttcgctgt ggcgggcgcc tgggccgccg gctgtttaac ttcgcttccg ctggcccata 60
   gtgatctttg cagtgaccca gcagcatcac tgtttcttgg cgtgtgaaga taacccaagg 120
   aattgaggaa gttgctgaga agagtgtgct ggagatgctc taggaaaaaa ttgaatagtg 180
   agacgagttc cagcgcaagg gtttctggtt tgccaagaag aaagtgaaca tcatggatca 240
   gaacaacagc ctgccacctt acgctcaggg cttggcctcc cctcagggtg ccatgactcc 300
   cggaatccct atctttagtc caatgatgcc ttatggcact ggactgaccc cacagcctat 360
   tcagaacacc aatagtctgt ctattttgga agagcaacaa aggcagcagc agcaacaaca 420
   acagcagcag cagcagcagc agcagcaaca gcaacagcag cagcagcagc agcagcagca 480
   gcagcagcag cagcagcagc agcagcagca gcaacaggca gtggcagctg cagccgttca 540
   gcagtcaacg tcccagcagg caacacaggg aacctcaggc caggcaccac agctcttcca 600
   ctcacagact ctcacaactg cacccttgcc gggcaccact ccactgtatc cctcccccat 660
   gactcccatg acccccatca ctcctgccac gccagcttcg gagagttctg ggattgtacc 720
   gcagctgcaa aatattgtat ccacagtgaa tcttggttgt aaacttgacc taaagaccat 780
   tgcacttcgt gcccgaaacg ccgaatataa tcccaagcgg tttgctgcgg taatcatgag 840
   gataagagag ccacgaacca cggcactgat tttcagttct gggaaaatgg tgtgcacagg 900
   agccaagagt gaagaacagt ccagactggc agcaagaaaa tatgctagag ttgtacagaa 960
   gttgggtttt ccagctaagt tcttggactt caagattcag aatatggtgg ggagctgtga 1020
   tgtgaagttt cctataaggt tagaaggcct tgtgctcacc caccaacaat ttagtagtta 1080
   tgagccagag ttatttcctg gtttaatcta cagaatgatc aaacccagaa ttgttctcct 1140
   tatttttgtt tctggaaaag ttgtattaac aggtgctaaa gtcagagcag aaatttatga 1200
   agcatttgaa aacatctacc ctattctaaa gggattcagg aagacgacgt aatggctctc 1260
   atgtaccctt gcctccccca cccccttctt tttttttttt taaacaaatc agtttgtttt 1320
   ggtaccttta aatggtggtg ttgtgagaag atggatgttg agttgcaggg tgtggcacca   1380
   ggtgatgccc ttctgtaagt gcccaccgcg ggatgccggg aaggggcatt atttgtgcac   1440
   tgagaacacc gcgcagcgtg actgtgagtt gctcataccg tgctgctatc tgggcagcgc   1500
   tgcccattta tttatatgta gattttaaac actgctgttg acaagttggt ttgagggaga   1560
   aaactttaag tgttaaagcc acctctataa ttgattggac tttttaattt taatgttttt   1620
   ccccatgaac cacagttttt atatttctac cagaaaagta aaaatctttt ttaaaagtgt   1680
   tgtttttcta atttataact cctaggggtt atttctgtgc cagacacatt ccacctctcc   1740
   agtattgcag gacagaatat atgtgttaat gaaaatgaat ggctgtacat atttttttct   1800
   ttcttcagag tactctgtac aataaatgca gtttataaaa gtgttaaaaa aaaaaaaaaa   1860
   aaaaaaa                                                             1867
<210> 372
   <211> 177
   <212> PRT
   <213> Homo sapiens
<400> 372
   Met Glu Ile Cys Arg Gly Leu Arg Ser His Leu Ile Thr Leu Leu Leu
   1               5                   10                  15
   Phe Leu Phe His Ser Glu Thr Ile Cys Arg Pro Ser Gly Arg Lys Ser
               20                  25                  30
   Ser Lys Met Gln Ala Phe Arg Ile Trp Asp Val Asn Gln Lys Thr Phe
           35                  40                  45
   Tyr Leu Arg Asn Asn Gln Leu Val Ala Gly Tyr Leu Gln Gly Pro Asn
       50                  55                  60
   Val Asn Leu Glu Glu Lys Ile Asp Val Val Pro Ile Glu Pro His Ala
   65                  70                  75                  80
   Leu Phe Leu Gly Ile His Gly Gly Lys Met Cys Leu Ser Cys Val Lys
                   85                  90                  95
   Ser Gly Asp Glu Thr Arg Leu Gln Leu Glu Ala Val Asn Ile Thr Asp
               100                 105                 110
   Leu Ser Glu Asn Arg Lys Gln Asp Lys Arg Phe Ala Phe Ile Arg Ser
           115                 120                 125
   Asp Ser Gly Pro Thr Thr Ser Phe Glu Ser Ala Ala Cys Pro Gly Trp
       130                 135                 140
   Phe Leu Cys Thr Ala Met Glu Ala Asp Gln Pro Val Ser Leu Thr Asn
   145                 150                 155                 160
   Met Pro Asp Glu Gly Val Met Val Thr Lys Phe Tyr Phe Gln Glu Asp
                   165                 170                 175
   Glu
<210> 373
   <211> 159
   <212> PRT
   <213> Homo sapiens
<400> 373
   Met Ala Leu Glu Thr Ile Cys Arg Pro Ser Gly Arg Lys Ser Ser Lys
   1 5 10 15
   Met Gln Ala Phe Arg Ile Trp Asp Val Asn Gln Lys Thr Phe Tyr Leu
   20 25 30
   Arg Asn Asn Gln Leu Val Ala Gly Tyr Leu Gln Gly Pro Asn Val Asn
   35 40 45
   Leu Glu Glu Lys Ile Asp Val Val Pro Ile Glu Pro His Ala Leu Phe
   50 55 60
   Leu Gly Ile His Gly Gly Lys Met Cys Leu Ser Cys Val Lys Ser Gly
   65 70 75 80
   Asp Glu Thr Arg Leu Gln Leu Glu Ala Val Asn Ile Thr Asp Leu Ser
   85 90 95
   Glu Asn Arg Lys Gln Asp Lys Arg Phe Ala Phe Ile Arg Ser Asp Ser
   100 105 110
   Gly Pro Thr Thr Ser Phe Glu Ser Ala Ala Cys Pro Gly Trp Phe Leu
   115 120 125
   Cys Thr Ala Met Glu Ala Asp Gln Pro Val Ser Leu Thr Asn Met Pro
   130 135 140
   Asp Glu Gly Val Met Val Thr Lys Phe Tyr Phe Gln Glu Asp Glu
   145 150 155
<210> 374
   <211> 180
   <212> PRT
   <213> Homo sapiens
<400> 374
   Met Ala Leu Ala Asp Leu Tyr Glu Glu Gly Gly Gly Gly Gly Gly Glu
   1 5 10 15
   Gly Glu Asp Asn Ala Asp Ser Lys Glu Thr Ile Cys Arg Pro Ser Gly
   20 25 30
   Arg Lys Ser Ser Lys Met Gln Ala Phe Arg Ile Trp Asp Val Asn Gln
   35 40 45
   Lys Thr Phe Tyr Leu Arg Asn Asn Gln Leu Val Ala Gly Tyr Leu Gln
   50 55 60
   Gly Pro Asn Val Asn Leu Glu Glu Lys Ile Asp Val Val Pro Ile Glu
   65 70 75 80
   Pro His Ala Leu Phe Leu Gly Ile His Gly Gly Lys Met Cys Leu Ser
   85 90 95
   Cys Val Lys Ser Gly Asp Glu Thr Arg Leu Gln Leu Glu Ala Val Asn
   100 105 110
   Ile Thr Asp Leu Ser Glu Asn Arg Lys Gln Asp Lys Arg Phe Ala Phe
   115 120 125
   Ile Arg Ser Asp Ser Gly Pro Thr Thr Ser Phe Glu Ser Ala Ala Cys
   130 135 140
   Pro Gly Trp Phe Leu Cys Thr Ala Met Glu Ala Asp Gln Pro Val Ser
   145 150 155 160
   Leu Thr Asn Met Pro Asp Glu Gly Val Met Val Thr Lys Phe Tyr Phe
   165 170 175
   Gln Glu Asp Glu
   180
<210> 375
   <211> 143
   <212> PRT
   <213> Homo sapiens
<400> 375
   Met Gln Ala Phe Arg Ile Trp Asp Val Asn Gln Lys Thr Phe Tyr Leu
   1 5 10 15
   Arg Asn Asn Gln Leu Val Ala Gly Tyr Leu Gln Gly Pro Asn Val Asn
   20 25 30
   Leu Glu Glu Lys Ile Asp Val Val Pro Ile Glu Pro His Ala Leu Phe
   35 40 45
   Leu Gly Ile His Gly Gly Lys Met Cys Leu Ser Cys Val Lys Ser Gly
   50 55 60
   Asp Glu Thr Arg Leu Gln Leu Glu Ala Val Asn Ile Thr Asp Leu Ser
   65 70 75 80
   Glu Asn Arg Lys Gln Asp Lys Arg Phe Ala Phe Ile Arg Ser Asp Ser
   85 90 95
   Gly Pro Thr Thr Ser Phe Glu Ser Ala Ala Cys Pro Gly Trp Phe Leu
   100 105 110
   Cys Thr Ala Met Glu Ala Asp Gln Pro Val Ser Leu Thr Asn Met Pro
   115 120 125
   Asp Glu Gly Val Met Val Thr Lys Phe Tyr Phe Gln Glu Asp Glu
   130 135 140
<210> 376
   <211> 139
   <212> DNA
   <213> Homo sapiens
<400> 376
   tgctgactca aaggggggat tataaaacta atcatcaaag ccaagaaggc aagagcaagc 60
   atgtaccgct gaaaacacaa gataactgca taagtaatga ctttcagtgc agattcatag 120
   ctaacccata aactgctgg 139
<210> 377
   <211> 19
   <212> DNA
   <213> Homo sapiens
<400> 377
   tgctgactca aagggggga 19
<210> 378
   <211> 24
   <212> DNA
   <213> Homo sapiens
<400> 378
   ccagcagttt atgggttagc tatg 24
<210> 379
   <211> 1331
   <212> DNA
   <213> Homo sapiens
<400> 379
   tcttgctgcg cctccgcctc ctcctctgct ccgccaccgg cttcctcctc ctgagcagtc      60
   agcccgcgcg ccggccggct ccgttatggc gacccgcagc cctggcgtcg tgattagtga     120
   tgatgaacca ggttatgacc ttgatttatt ttgcatacct aatcattatg ctgaggattt     180
   ggaaagggtg tttattcctc atggactaat tatggacagg actgaacgtc ttgctcgaga     240
   tgtgatgaag gagatgggag gccatcacat tgtagccctc tgtgtgctca aggggggcta     300
   taaattcttt gctgacctgc tggattacat caaagcactg aatagaaata gtgatagatc     360
   cattcctatg actgtagatt ttatcagact gaagagctat tgtaatgacc agtcaacagg     420
   ggacataaaa gtaattggtg gagatgatct ctcaacttta actggaaaga atgtcttgat     480
   tgtggaagat ataattgaca ctggcaaaac aatgcagact ttgctttcct tggtcaggca     540
   gtataatcca aagatggtca aggtcgcaag cttgctggtg aaaaggaccc cacgaagtgt     600
   tggatataag ccagactttg ttggatttga aattccagac aagtttgttg taggatatgc     660
   ccttgactat aatgaatact tcagggattt gaatcatgtt tgtgtcatta gtgaaactgg     720
   aaaagcaaaa tacaaagcct aagatgagag ttcaagttga gtttggaaac atctggagtc     780
   ctattgacat cgccagtaaa attatcaatg ttctagttct gtggccatct gcttagtaga     840
   gctttttgca tgtatcttct aagaatttta tctgttttgt actttagaaa tgtcagttgc     900
   tgcattccta aactgtttat ttgcactatg agcctataga ctatcagttc cctttgggcg     960
   gattgttgtt taacttgtaa atgaaaaaat tctcttaaac cacagcacta ttgagtgaaa    1020
   cattgaactc atatctgtaa gaaataaaga gaagatatat tagtttttta attggtattt    1080
   taatttttat atatgcagga aagaatagaa gtgattgaat attgttaatt ataccaccgt    1140
   gtgttagaaa agtaagaagc agtcaatttt cacatcaaag acagcatcta agaagttttg    1200
   ttctgtcctg gaattatttt agtagtgttt cagtaatgtt gactgtattt tccaacttgt    1260
   tcaaattatt accagtgaat ctttgtcagc agttcccttt taaatgcaaa tcaataaatt    1320
   cccaaaaatt t                                                         1331
<210> 380
   <211> 94
   <212> DNA
   <213> Artificial Organism
<220>
   <223> Synthetic oligonucleotide
<400> 380
   tgacactggc aaaacaatgc agactttgct ttccttggtc aggcagtata atccaaagat 60
   ggtcaaggtc gcaagcttgc tggtgaaaag gacc 94
<210> 381
   <211> 1536
   <212> DNA
   <213> Homo sapiens

<400> 381
   gtgacgcgag gctctgcgga gaccaggagt cagactgtag gacgacctcg ggtcccacgt 60
   gtccccggta ctcgccggcc ggagcccccg gcttcccggg gccgggggac cttagcggca 120
   cccacacaca gcctactttc caagcggagc catgtctggt aacggcaatg cggctgcaac 180
   ggcggaagaa aacagcccaa agatgagagt gattcgcgtg ggtacccgca agagccagct 240
   tgctcgcata cagacggaca gtgtggtggc aacattgaaa gcctcgtacc ctggcctgca 300
   gtttgaaatc attgctatgt ccaccacagg ggacaagatt cttgatactg cactctctaa 360
   gattggagag aaaagcctgt ttaccaagga gcttgaacat gccctggaga agaatgaagt 420
   ggacctggtt gttcactcct tgaaggacct gcccactgtg cttcctcctg gcttcaccat 480
   cggagccatc tgcaagcggg aaaaccctca tgatgctgtt gtctttcacc caaaatttgt 540
   tgggaagacc ctagaaaccc tgccagagaa gagtgtggtg ggaaccagct ccctgcgaag 600
   agcagcccag ctgcagagaa agttcccgca tctggagttc aggagtattc ggggaaacct 660
   caacacccgg cttcggaagc tggacgagca gcaggagttc agtgccatca tcctggcaac 720
   agctggcctg cagcgcatgg gctggcacaa ccgggtgggg cagatcctgc accctgagga 780
   atgcatgtat gctgtgggcc agggggcctt gggcgtggaa gtgcgagcca aggaccagga 840
   catcttggat ctggtgggtg tgctgcacga tcccgagact ctgcttcgct gcatcgctga 900
   aagggccttc ctgaggcacc tggaaggagg ctgcagtgtg ccagtagccg tgcatacagc 960
   tatgaaggat gggcaactgt acctgactgg aggagtctgg agtctagacg gctcagatag 1020
   catacaagag accatgcagg ctaccatcca tgtccctgcc cagcatgaag atggccctga 1080
   ggatgaccca cagttggtag gcatcactgc tcgtaacatt ccacgagggc cccagttggc 1140
   tgcccagaac ttgggcatca gcctggccaa cttgttgctg agcaaaggag ccaaaaacat 1200
   cctggatgtt gcacggcagc ttaacgatgc ccattaactg gtttgtgggg cacagatgcc 1260
   tgggttgctg ctgtccagtg cctacatccc gggcctcagt gccccattct cactgctatc 1320
   tggggagtga ttaccccggg agactgaact gcagggttca agccttccag ggatttgcct 1380
   caccttgggg ccttgatgac tgccttgcct cctcagtatg tgggggcttc atctctttag 1440
   agaagtccaa gcaacagcct ttgaatgtaa ccaatcctac taataaacca gttctgaagg 1500
   taaaaaaaaa aaaaaaaaaa aaaaaaaaaa aaaaaa 1536
<210> 382
   <211> 91
   <212> DNA
   <213> Artificial Organism
<220>
   <223> Synthetic oligonucleotide
<400> 382
   tgagagtgat tcgcgtgggt acccgcaaga gccagcttgc tcgcatacag acggacagtg 60
   tggtggcaac attgaaagcc tcgtaccctg g 91
<210> 383
   <211> 103
   <212> DNA
   <213> Homo sapiens
<400> 383
   aggaccaaat gtcaatttag aagatagatg tggtacccat tgagcctcat gctctgttct 60
   tgggaatcca tggagggaag atgtgcctgt cctgtgtcaa gtc 103
<210> 384
   <211> 28
   <212> DNA
   <213> Homo sapiens
<400> 384
   aggaccaaat gtcaatttag aagataga 28
<210> 385
   <211> 22
   <212> DNA
   <213> Homo sapiens
<400> 385
   gacttgacac aggacaggca ca 22
<210> 386
   <211> 101
   <212> DNA
   <213> Homo sapiens
<400> 386
   caaccaacta gttgctggat acttgcaagg accaaatgtc aatttagaag gtgagtggtt 60
   gccaggaaag ccaatgtatg tgggcatcac gtcactttgc c 101
<210> 387
   <211> 25
   <212> DNA
   <213> Homo sapiens
<400> 387
   caaccaacta gttgctggat acttg 25
<210> 388
   <211> 19
   <212> DNA
   <213> Homo sapiens
<400> 388
   ggcaaagtga cgtgatgcc 19
<210> 389
   <211> 108
   <212> DNA
   <213> Homo sapiens
<400> 389
   gagcgagaac agaaagcagg acaagcgctt cgccttcatc cgctcagaca gtggccccac 60
   caccagtttt gagtctgccg cctgccccgg ttggttcctc tgcacagc 108
<210> 390
   <211> 21
   <212> DNA
   <213> Homo sapiens
<400> 390
   gagcgagaac agaaagcagg a 21
<210> 391
   <211> 20
   <212> DNA
   <213> Homo sapiens
<400> 391
   gctgtgcaga ggaaccaacc 20
<210> 392
   <211> 221
   <212> PRT
   <213> Homo sapiens
<400> 392
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65 70 75 80
   Leu Arg Cys Thr Gly Cys Cys Gly Asp Glu Asn Leu His Cys Val Pro
   85 90 95
   Val Glu Thr Ala Asn Val Thr Met Gln Leu Leu Lys Ile Arg Ser Gly
   100 105 110
   Asp Arg Pro Ser Tyr Val Glu Leu Thr Phe Ser Gln His Val Arg Cys
   115 120 125
   Glu Cys Arg His Ser Pro Gly Arg Gln Ser Pro Asp Met Pro Gly Asp
   130 135 140
   Phe Arg Ala Asp Ala Pro Ser Phe Leu Pro Pro Arg Arg Ser Leu Pro
   145 150 155 160
   Met Leu Phe Arg Met Glu Trp Gly Cys Ala Leu Thr Gly Ser Gln Ser
   165 170 175
   Ala Val Trp Pro Ser Ser Pro Val Pro Glu Glu Ile Pro Arg Met His
   180 185 190
   Pro Gly Arg Asn Gly Lys Lys Gln Gln Arg Lys Pro Leu Arg Glu Lys
   195 200 205
   Met Lys Pro Glu Arg Cys Gly Asp Ala Val Pro Arg Arg
   210 215 220
<210> 393
   <211> 101
   <212> DNA
   <213> Homo sapiens
<400> 393
   tcgtgtccga gtaccccagc gaggtggagc acatgttcag cccatcctgt gtctccctgc 60
   tgcgctgcac cggctgctgc ggcgatgaga atctgcactg t 101
<210> 394
   <211> 19
   <212> DNA
   <213> Homo sapiens
<400> 394
   tcgtgtccga gtaccccag 19
<210> 395
   <211> 21
   <212> DNA
   <213> Homo sapiens
<400> 395
   acagtgcaga ttctcatcgc c 21
<210> 396
   <211> 98
   <212> DNA
   <213> Homo sapiens
<400> 396
   tggtttggct ggggctcggg gctattctcg ggcctgccag cctctgtcct agcatggggt 60
   tccccagcca ccttgtcctg acgcttggct tattgcag 98
<210> 397
   <211> 17
   <212> DNA
   <213> Homo sapiens
<400> 397
   tggtttggct ggggctc 17
<210> 398
   <211> 21
   <212> DNA
   <213> Homo sapiens
<400> 398
   ctgcaataag ccaagcgtca g 21
<210> 399
   <211> 109
   <212> DNA
   <213> Homo sapiens
<400> 399
   cttgtcctga cgcttggctt attgcaggag gagacccaag ggcaggggga agaggaggag 60
   agagaagcag agacccacag actgccacct gtgcggcgat gctgttccc 109
<210> 400
   <211> 21
   <212> DNA
   <213> Homo sapiens
<400> 400
   cttgtcctga cgcttggctt a 21
<210> 401
   <211> 16
   <212> DNA
   <213> Homo sapiens
<400> 401
   gggaacagca tcgccg 16
<210> 402
   <211> 101
   <212> DNA
   <213> Artificial Organism
<220>
   <223> Synthetic oligonucleotide
<400> 402
   ctggcaagca gctggaagat ggacgtactt tgtctgacta caatattcaa aaggagtcta      60
   ctcttcatct tgtgttgaga cttcgtggtg gtgctaagaa a                         101
<210> 403
   <211> 541
   <212> DNA
   <213> Homo sapiens
<400> 403
   cttttcgatc cgccatctgc ggtggagccg ccaccaaaat gcagattttc gtgaaaaccc      60
   ttacggggaa gaccatcacc ctcgaggttg aaccctcgga tacgatagaa aatgtaaagg     120
   ccaagatcca ggataaggaa ggaattcctc ctgatcagca gagactgatc tttgctggca     180
   agcagctgga agatggacgt actttgtctg actacaatat tcaaaaggag tctactcttc     240
   atcttgtgtt gagacttcgt ggtggtgcta agaaaaggaa gaagaagtct tacaccactc     300
   ccaagaagaa taagcacaag agaaagaagg ttaagctggc tgtcctgaaa tattataagg     360
   tggatgagaa tggcaaaatt agtcgccttc gtcgagagtg cccttctgat gaatgtggtg     420
   ctggggtgtt tatggcaagt cactttgaca gacattattg tggcaaatgt tgtctgactt     480
   actgtttcaa caaaccagaa gacaagtaac tgtatgagtt aataaaagac atgaactaac     540
   a                                                                     541
<210> 404
   <211> 111
   <212> DNA
   <213> Artificial Organism
<220>
   <223> Synthetic oligonucleotide
<400> 404
   gaggccgtca ccaagaacat tcacgagtcc tgcatgagcc agataggctg gaaccgcatc      60
   atcgtggaga agcccttcgg gagggacctg cagagctctg accggctgtc c              111
<210> 405
   <211> 2395
   <212> DNA
   <213> Homo sapiens
<400> 405
   agaggcaggg gctggcctgg gatgcgcgcg cacctgccct cgccccgccc cgcccgcacg    60
   aggggtggtg gccgaggccc cgccccgcac gcctcgcctg aggcgggtcc gctcagccca   120
   ggcgcccgcc cccgcccccg ccgattaaat gggccggcgg ggctcagccc ccggaaacgg   180
   tcgtacactt cggggctgcg agcgcggagg gcgacgacga cgaagcgcag acagcgtcat   240
   ggcagagcag gtggccctga gccggaccca ggtgtgcggg atcctgcggg aagagctttt   300
   ccagggcgat gccttccatc agtcggatac acacatattc atcatcatgg gtgcatcggg   360
   tgacctggcc aagaagaaga tctaccccac catctggtgg ctgttccggg atggccttct   420
   gcccgaaaac accttcatcg tgggctatgc ccgttcccgc ctcacagtgg ctgacatccg   480
   caaacagagt gagcccttct tcaaggccac cccagaggag aagctcaagc tggaggactt   540
   ctttgcccgc aactcctatg tggctggcca gtacgatgat gcagcctcct accagcgcct   600
   caacagccac atgaatgccc tccacctggg gtcacaggcc aaccgcctct tctacctggc   660
   cttgcccccg accgtctacg aggccgtcac caagaacatt cacgagtcct gcatgagcca   720
   gataggctgg aaccgcatca tcgtggagaa gcccttcggg agggacctgc agagctctga   780
   ccggctgtcc aaccacatct cctccctgtt ccgtgaggac cagatctacc gcatcgacca   840
   ctacctgggc aaggagatgg tgcagaacct catggtgctg agatttgcca acaggatctt   900
   cggccccatc tggaaccggg acaacatcgc ctgcgttatc ctcaccttca aggagccctt   960
   tggcactgag ggtcgcgggg gctatttcga tgaatttggg atcatccggg acgtgatgca  1020
   gaaccaccta ctgcagatgc tgtgtctggt ggccatggag aagcccgcct ccaccaactc  1080
   agatgacgtc cgtgatgaga aggtcaaggt gttgaaatgc atctcagagg tgcaggccaa  1140
   caatgtggtc ctgggccagt acgtggggaa ccccgatgga gagggcgagg ccaccaaagg  1200
   gtacctggac gaccccacgg tgccccgcgg gtccaccacc gccacttttg cagccgtcgt  1260
   cctctatgtg gagaatgaga ggtgggatgg ggtgcccttc atcctgcgct gcggcaaggc  1320
   cctgaacgag cgcaaggccg aggtgaggct gcagttccat gatgtggccg gcgacatctt  1380
   ccaccagcag tgcaagcgca acgagctggt gatccgcgtg cagcccaacg aggccgtgta  1440
   caccaagatg atgaccaaga agccgggcat gttcttcaac cccgaggagt cggagctgga  1500
   cctgacctac ggcaacagat acaagaacgt gaagctccct gacgcctacg agcgcctcat  1560
   cctggacgtc ttctgcggga gccagatgca cttcgtgcgc agcgacgagc tccgtgaggc  1620
   ctggcgtatt ttcaccccac tgctgcacca gattgagctg gagaagccca agcccatccc  1680
   ctatatttat ggcagccgag gccccacgga ggcagacgag ctgatgaaga gagtgggttt 1740
   ccagtatgag ggcacctaca agtgggtgaa cccccacaag ctctgagccc tgggcaccca 1800
   cctccacccc cgccacggcc accctccttc ccgccgcccg accccgagtc gggaggactc 1860
   cgggaccatt gacctcagct gcacattcct ggccccgggc tctggccacc ctggcccgcc 1920
   cctcgctgct gctactaccc gagcccagct acattcctca gctgccaagc actcgagacc 1980
   atcctggccc ctccagaccc tgcctgagcc caggagctga gtcacctcct ccactcactc 2040
   cagcccaaca gaaggaagga ggagggcgcc cattcgtctg tcccagagct tattggccac 2100
   tgggtctcac tcctgagtgg ggccagggtg ggagggaggg acaaggggga ggaaaggggc 2160
   gagcacccac gtgagagaat ctgcctgtgg ccttgcccgc cagcctcagt gccacttgac 2220
   attccttgtc accagcaaca tctcgagccc cctggatgtc ccctgtccca ccaactctgc 2280
   actccatggc caccccgtgc cacccgtagg cagcctctct gctataagaa aagcagacgc 2340
   agcagctggg acccctccca acctcaatgc cctgccatta aatccgcaaa cagcc 2395
<210> 406
   <211> 381
   <212> PRT
   <213> Homo sapiens
<400> 406
<210> 407
   <211> 113
   <212> DNA
   <213> Artificial Organism
<220>
   <223> Synthetic oligonucleotide
<400> 407
   cccactgccc cctccttagc ccaatgcctg ctctcctcct ccccctaccc tgccactgca 60
   tgaccctctc cctctgtggt cccactgcaa tgcaccaagg aggacagaaa cca 113
<210> 408
   <211> 17
   <212> DNA
   <213> Homo sapiens
<400> 408
   cccactgccc cctcctt 17
<210> 409
   <211> 21
   <212> DNA
   <213> Homo sapiens
<400> 409
   tggtttctgt cctccttggt g 21
<210> 410
   <211> 94
   <212> DNA
   <213> Artificial Organism
<220>
   <223> Synthetic oligonucleotide
<400> 410
   gagaatggct gccgcgagac tctgagtgcc acctctgcag tccgtgacca cccaggccgg 60
   gaacagcagc gagcaggcca taccacaggc aatg 94
<210> 411
   <211> 16
   <212> DNA
   <213> Homo sapiens
<400> 411
   gagaatggct gccgcg 16
<210> 412
   <211> 20
   <212> DNA
   <213> Homo sapiens
<400> 412
   cattgcctgt ggtatggcct 20
<210> 413
   <211> 185
   <212> PRT
   <213> Homo sapiens
<400> 413
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Arg Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 414
   <211> 162
   <212> DNA
   <213> Artificial Organism
<220>
   <223> Synthetic oligonucleotide
<400> 414
   tggtttccgt cgccctgatg tacctgggtt cgctcgcctt cctaggcgct gacaccgctc 60
   ggttggatgt cgcgtcggag tttcgaaaga agtgagtccg ggcagcgcct tcccccttgc 120
   tggtacctgg caggcaaggg gaactgaccg ttggtcccga ag 162
<210> 415
   <211> 20
   <212> DNA
   <213> Homo sapiens
<400> 415
   tggtttccgt cgccctgatg 20
<210> 416
   <211> 22
   <212> DNA
   <213> Homo sapiens
<400> 416
   cttcgggacc aacggtcagt tc 22
<210> 417
   <211> 115
   <212> DNA
   <213> Artificial Organism
<220>
   <223> Synthetic oligonucleotide
<400> 417
   acgggaggga aggaaggtgt gcgggaggag ttctctgtct ccactcccct ggcccggggg 60
   atcgtcgggg ctggaccgca gctcagatgg cgcgagcagt ttccagctcc ctctg 115
<210> 418
   <211> 19
   <212> DNA
   <213> Homo sapiens
<400> 418
   acgggaggga aggaaggtg 19
<210> 419
   <211> 20
   <212> DNA
   <213> Homo sapiens
<400> 419
   cagagggagc tggaaactgc 20
<210> 420
   <211> 101
   <212> DNA
   <213> Artificial Organism
<220>
   <223> Synthetic oligonucleotide
<400> 420
   tgcacggtgc agaagctggc acaccagatc taccagttca cagataagga caaggacaac      60
   gtcgccccca ggagcaagat cagcccccag ggctacggcc g                         101
<210> 421
   <211> 18
   <212> DNA
   <213> Homo sapiens
<400> 421
   tgcacggtgc agaagctg 18
<210> 422
   <211> 15
   <212> DNA
   <213> Homo sapiens
<400> 422
   cggccgtagc cctgg 15
<210> 423
   <211> 178
   <212> PRT
   <213> Homo sapiens
<400> 423
   Met His Ser Ser Ala Leu Leu Cys Cys Leu Val Leu Leu Thr Gly Val
   1               5                   10                  15
   Arg Ala Ser Pro Gly Gln Gly Thr Gln Ser Glu Asn Ser Cys Thr His
               20                  25                  30
   Phe Pro Gly Asn Leu Pro Asn Met Leu Arg Asp Leu Arg Asp Ala Phe
           35                  40                  45
   Ser Arg Val Lys Thr Phe Phe Gln Met Lys Asp Gln Leu Asp Asn Leu
       50                  55                  60
   Leu Leu Lys Glu Ser Leu Leu Glu Asp Phe Lys Gly Tyr Leu Gly Cys
   65                  70                  75                  80
   Gln Ala Leu Ser Glu Met Ile Gln Phe Tyr Leu Glu Glu Val Met Pro
   85 90 95
   Gln Ala Glu Asn Gln Asp Pro Asp Ile Lys Ala His Val Asn Ser Leu
   100 105 110
   Gly Glu Asn Leu Lys Thr Leu Arg Leu Arg Leu Arg Arg Cys His Arg
   115 120 125
   Phe Leu Pro Cys Glu Asn Lys Ser Lys Ala Val Glu Gln Val Lys Asn
   130 135 140
   Ala Phe Asn Lys Leu Gln Glu Lys Gly Ile Tyr Lys Ala Met Ser Glu
   145 150 155 160
   Phe Asp Ile Phe Ile Asn Tyr Ile Glu Ala Tyr Met Thr Met Lys Ile
   165 170 175
   Arg Asn
<210> 424
   <211> 91
   <212> DNA
   <213> Artificial Organism
<220>
   <223> Synthetic oligonucleotide
<400> 424
   tctggtgaag gaggatcgct agaaccaagc tgtcctctta agctagttgc agcagcccct 60
   cctcccagcc acctccgcca atctctcact c 91
<210> 425
   <211> 20
   <212> DNA
   <213> Homo sapiens
<400> 425
   tctggtgaag gaggatcgct 20
<210> 426
   <211> 21
   <212> DNA
   <213> Homo sapiens
<400> 426
   gagtgagaga ttggcggagg t 21
<210> 427
   <211> 136
   <212> DNA
   <213> Artificial Organism
<220>
   <223> Synthetic oligonucleotide
<400> 427
   aagaaggcat gcacagctca gcactgctct gttgcctggt cctcctgact ggggtgaggg      60
   ccagcccagg ccagggcacc cagtctgaga acagctgcac ccacttccca ggcaacctgc     120
   ctaacatgct tcgaga                                                     136
<210> 428
   <211> 21
   <212> DNA
   <213> Homo sapiens
<400> 428
   aagaaggcat gcacagctca g 21
<210> 429
   <211> 21
   <212> DNA
   <213> Homo sapiens
<400> 429
   tctcgaagca tgttaggcag g 21
<210> 430
   <211> 254
   <212> PRT
   <213> Homo sapiens
<400> 430
   Met Ala Thr Ala Gly Asn Pro Trp Gly Trp Phe Leu Gly Tyr Leu Ile
   1               5                   10                  15
   Leu Gly Val Ala Gly Ser Leu Val Ser Gly Ser Cys Ser Gln Ile Ile
               20                  25                  30
   Asn Gly Glu Asp Cys Ser Pro His Ser Gln Pro Trp Gln Ala Ala Leu
           35                  40                  45
   Val Met Glu Asn Glu Leu Phe Cys Ser Gly Val Leu Val His Pro Gln
       50                  55                  60
   Trp Val Leu Ser Ala Ala His Cys Phe Gln Asn Ser Tyr Thr Ile Gly
   65                  70                  75                  80
   Leu Gly Leu His Ser Leu Glu Ala Asp Gln Glu Pro Gly Ser Gln Met
                   85                  90                  95
   Val Glu Ala Ser Leu Ser Val Arg His Pro Glu Tyr Asn Arg Pro Leu
   100 105 110
   Leu Ala Asn Asp Leu Met Leu Ile Lys Leu Asp Glu Ser Val Ser Glu
   115 120 125
   Ser Asp Thr Ile Arg Ser Ile Ser Ile Ala Ser Gln Cys Pro Thr Ala
   130 135 140
   Gly Asn Ser Cys Leu Val Ser Gly Trp Gly Leu Leu Ala Asn Gly Arg
   145 150 155 160
   Met Pro Thr Val Leu Gln Cys Val Asn Val Ser Val Val Ser Glu Glu
   165 170 175
   Val Cys Ser Lys Leu Tyr Asp Pro Leu Tyr His Pro Ser Met Phe Cys
   180 185 190
   Ala Gly Gly Gly Gln Asp Gln Lys Asp Ser Cys Asn Gly Asp Ser Gly
   195 200 205
   Gly Pro Leu Ile Cys Asn Gly Tyr Leu Gln Gly Leu Val Ser Phe Gly
   210 215 220
   Lys Ala Pro Cys Gly Gln Val Gly Val Pro Gly Val Tyr Thr Asn Leu
   225 230 235 240
   Cys Lys Phe Thr Glu Trp Ile Glu Lys Thr Val Gln Ala Ser
   245 250
<210> 431
   <211> 101
   <212> DNA
   <213> Artificial Organism
<220>
   <223> Synthetic oligonucleotide
<400> 431
   ctgctggcga acgatgctgt gattgccatc cagtcccaga ctgtgggagg ctgggagtgt 60
   gagaagcttt cccaaccctg gcagggttgt accatttcgg c 101
<210> 432
   <211> 18
   <212> DNA
   <213> Homo sapiens
<400> 432
   ctgctggcga acgatgct 18
<210> 433
   <211> 20
   <212> DNA
   <213> Homo sapiens
<400> 433
   gccgaaatgg tacaaccctg 20
<210> 434
   <211> 101
   <212> DNA
   <213> Artificial Organism
<220>
   <223> Synthetic oligonucleotide
<400> 434
   aatgcctacc gtgctgcagt gcgtgaacgt gtcggtggtg tctgaggagg tctgcagtaa 60
   gctctatgac ccgctgtacc accccagcat gttctgcgcc g 101
<210> 435
   <211> 19
   <212> DNA
   <213> Homo sapiens
<400> 435
   aatgcctacc gtgctgcag 19
<210> 436
   <211> 17
   <212> DNA
   <213> Homo sapiens
<400> 436
   cggcgcagaa catgctg 17
<210> 437
   <211> 118
   <212> DNA
   <213> Artificial Organism
<220>
   <223> Synthetic oligonucleotide
<400> 437
   ctgggtgcag ccacatgata aaggaaggac cggagcttgg tgtaacccct gatccctcct 60
   agatggggct aggtggggct agcctagatg gggctaagtc ctagcgactt ccacctgg 118
<210> 438
   <211> 20
   <212> DNA
   <213> Homo sapiens
<400> 438
   ctgggtgcag ccacatgata 20
<210> 439
   <211> 20
   <212> DNA
   <213> Homo sapiens
<400> 439
   ccaggtggaa gtcgctagga 20
<210> 440
   <211> 80
   <212> PRT
   <213> Homo sapiens
<400> 440
   Met Ala Ala Arg Ala Leu Cys Met Leu Gly Leu Val Leu Ala Leu Leu
   1 5 10 15
   Ser Ser Ser Ser Ala Glu Glu Tyr Val Gly Leu Ser Ala Asn Gln Cys
   20 25 30
   Ala Val Pro Ala Lys Asp Arg Val Asp Cys Gly Tyr Pro His Val Thr
   35 40 45
   Pro Lys Glu Cys Asn Asn Arg Gly Cys Cys Phe Asp Ser Arg Ile Pro
   50 55 60
   Gly Val Pro Trp Cys Phe Lys Pro Leu Gln Glu Ala Glu Cys Thr Phe
   65 70 75 80
<210> 441
   <211> 585
   <212> PRT
   <213> Homo sapiens
<400> 441
   Met Thr Pro Pro Arg Leu Phe Trp Val Trp Leu Leu Val Ala Gly Thr
   1               5                   10                  15
   Gln Gly Val Asn Asp Gly Asp Met Arg Leu Ala Asp Gly Gly Ala Thr
               20                  25                  30
   Asn Gln Gly Arg Val Glu Ile Phe Tyr Arg Gly Gln Trp Gly Thr Val
           35                  40                  45
   Cys Asp Asn Leu Trp Asp Leu Thr Asp Ala Ser Val Val Cys Arg Ala
   50 55 60
   Leu Gly Phe Glu Asn Ala Thr Gln Ala Leu Gly Arg Ala Ala Phe Gly
   65 70 75 80
   Gln Gly Ser Gly Pro Ile Met Leu Asp Glu Val Gln Cys Thr Gly Thr
   85 90 95
   Glu Ala Ser Leu Ala Asp Cys Lys Ser Leu Gly Trp Leu Lys Ser Asn
   100 105 110
   Cys Arg His Glu Arg Asp Ala Gly Val Val Cys Thr Asn Glu Thr Arg
   115 120 125
   Ser Thr His Thr Leu Asp Leu Ser Arg Glu Leu Ser Glu Ala Leu Gly
   130 135 140
   Gln Ile Phe Asp Ser Gln Arg Gly Cys Asp Leu Ser Ile Ser Val Asn
   145 150 155 160
   Val Gln Gly Glu Asp Ala Leu Gly Phe Cys Gly His Thr Val Ile Leu
   165 170 175
   Thr Ala Asn Leu Glu Ala Gln Ala Leu Trp Lys Glu Pro Gly Ser Asn
   180 185 190
   Val Thr Met Ser Val Asp Ala Glu Cys Val Pro Met Val Arg Asp Leu
   195 200 205
   Leu Arg Tyr Phe Tyr Ser Arg Arg Ile Asp Ile Thr Leu Ser Ser Val
   210 215 220
   Lys Cys Phe His Lys Leu Ala Ser Ala Tyr Gly Ala Arg Gln Leu Gln
   225 230 235 240
   Gly Tyr Cys Ala Ser Leu Phe Ala Ile Leu Leu Pro Gln Asp Pro Ser
   245 250 255
   Phe Gln Met Pro Leu Asp Leu Tyr Ala Tyr Ala Val Ala Thr Gly Asp
   260 265 270
   Ala Leu Leu Glu Lys Leu Cys Leu Gln Phe Leu Ala Trp Asn Phe Glu
   275 280 285
   Ala Leu Thr Gln Ala Glu Ala Trp Pro Ser Val Pro Thr Asp Leu Leu
   290 295 300
   Gln Leu Leu Leu Pro Arg Ser Asp Leu Ala Val Pro Ser Glu Leu Ala
   305 310 315 320
   Leu Leu Lys Ala Val Asp Thr Trp Ser Trp Gly Glu Arg Ala Ser His
   325 330 335
   Glu Glu Val Glu Gly Leu Val Glu Lys Ile Arg Phe Pro Met Met Leu
   340 345 350
   Pro Glu Glu Leu Phe Glu Leu Gln Phe Asn Leu Ser Leu Tyr Trp Ser
   355 360 365
   His Glu Ala Leu Phe Gln Lys Lys Thr Leu Gln Ala Leu Glu Phe His
   370 375 380
   Thr Val Pro Phe Gln Leu Leu Ala Arg Tyr Lys Gly Leu Asn Leu Thr
   385 390 395 400
   Glu Asp Thr Tyr Lys Pro Arg Ile Tyr Thr Ser Pro Thr Trp Ser Ala
   405 410 415
   Phe Val Thr Asp Ser Ser Trp Ser Ala Arg Lys Ser Gln Leu Val Tyr
   420 425 430
   Gln Ser Arg Arg Gly Pro Leu Val Lys Tyr Ser Ser Asp Tyr Phe Gln
   435 440 445
   Ala Pro Ser Asp Tyr Arg Tyr Tyr Pro Tyr Gln Ser Phe Gln Thr Pro
   450 455 460
   Gln His Pro Ser Phe Leu Phe Gln Asp Lys Arg Val Ser Trp Ser Leu
   465 470 475 480
   Val Tyr Leu Pro Thr Ile Gln Ser Cys Trp Asn Tyr Gly Phe Ser Cys
   485 490 495
   Ser Ser Asp Glu Leu Pro Val Leu Gly Leu Thr Lys Ser Gly Gly Ser
   500 505 510
   Asp Arg Thr Ile Ala Tyr Glu Asn Lys Ala Leu Met Leu Cys Glu Gly
   515 520 525
   Leu Phe Val Ala Asp Val Thr Asp Phe Glu Gly Trp Lys Ala Ala Ile
   530 535 540
   Pro Ser Ala Leu Asp Thr Asn Ser Ser Lys Ser Thr Ser Ser Phe Pro
   545 550 555 560
   Cys Pro Ala Gly His Phe Asn Gly Phe Arg Thr Val Ile Arg Pro Phe
   565 570 575
   Tyr Leu Thr Asn Ser Ser Gly Val Asp
   580 585
<210> 442
   <211> 331
   <212> PRT
   <213> Homo sapiens
<400> 442
<210> 443
   <211> 132
   <212> PRT
   <213> Homo sapiens
<400> 443
   Met Lys Ser Ser Gly Leu Phe Pro Phe Leu Val Leu Leu Ala Leu Gly
   1               5                   10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Thr Arg Arg Lys Pro Gly Lys Cys Pro Val Thr Tyr Gly Gln Cys
                   85                  90                  95
   Leu Met Leu Asn Pro Pro Asn Phe Cys Glu Met Asp Gly Gln Cys Lys
               100                 105                 110
   Arg Asp Leu Lys Cys Cys Met Gly Met Cys Gly Lys Ser Cys Val Ser
           115                 120                 125
   Pro Val Lys Ala
       130
<210> 444
   <211> 101
   <212> DNA
   <213> Artificial Organism
<220>
   <223> Synthetic oligonucleotide
<400> 444
   accccaaacc caacttgatt cctgccatat ggaggaggct ctggagtcct gctctgtgtg 60
   gtccaggtcc tttccaccct gagacttggc tccaccactg a 101
<210> 445
   <211> 21
   <212> DNA
   <213> Homo sapiens
<400> 445
   accccaaacc caacttgatt c 21
<210> 446
   <211> 21
   <212> DNA
   <213> Homo sapiens
<400> 446
   tcagtggtgg agccaagtct c 21
<210> 447
   <211> 101
   <212> DNA
   <213> Artificial Organism
<220>
   <223> Synthetic oligonucleotide
<400> 447
   aagaaatctg cccagtgcct tagatacaag aaacctgagt gccagagtga ctggcagtgt 60
   ccagggaaga agagatgttg tcctgacact tgtggcatca a 101
<210> 448
   <211> 20
   <212> DNA
   <213> Homo sapiens
<400> 448
   aagaaatctg cccagtgcct 20
<210> 449
   <211> 21
   <212> DNA
   <213> Homo sapiens
<400> 449
   ttgatgccac aagtgtcagg a 21
<210> 450
   <211> 116
   <212> DNA
   <213> Artificial Organism
<220>
   <223> Synthetic oligonucleotide
<400> 450
   tgcaccacca actgcttagc acccctggcc aaggtcatcc atgacaactt tggtatcgtg 60
   gaaggactca tgaccacagt ccatgccatc actgccaccc agaagactgt ggatgg 116
<210> 451
   <211> 1261
   <212> DNA
   <213> Homo sapiens
<400> 451
   cttttgcgtc gccagccgag ccacatcgct cagacaccat ggggaaggtg aaggtcggag 60
   tcaacggatt tggtcgtatt gggcgcctgg tcaccagggc tgcttttaac tctggtaaag 120
   tggatattgt tgccatcaat gaccccttca ttgacctcaa ctacatggtt tacatgttcc 180
   aatatgattc cacccatggc aaattccatg gcaccgtcaa ggctgagaac gggaagcttg 240
   tcatcaatgg aaatcccatc accatcttcc aggagcgaga tccctccaaa atcaagtggg 300
   gcgatgctgg cgctgagtac gtcgtggagt ccactggcgt cttcaccacc atggagaagg 360
   ctggggctca tttgcagggg ggagccaaaa gggtcatcat ctctgccccc tctgctgatg 420
   cccccatgtt cgtcatgggt gtgaaccatg agaagtatga caacagcctc aagatcatca 480
   gcaatgcctc ctgcaccacc aactgcttag cacccctggc caaggtcatc catgacaact 540
   ttggtatcgt ggaaggactc atgaccacag tccatgccat cactgccacc cagaagactg 600
   tggatggccc ctccgggaaa ctgtggcgtg atggccgcgg ggctctccag aacatcatcc 660
   ctgcctctac tggcgctgcc aaggctgtgg gcaaggtcat ccctgagctg aacgggaagc 720
   tcactggcat ggccttccgt gtccccactg ccaacgtgtc agtggtggac ctgacctgcc 780
   gtctagaaaa acctgccaaa tatgatgaca tcaagaaggt ggtgaagcag gcgtcggagg 840
   gccccctcaa gggcatcctg ggctacactg agcaccaggt ggtctcctct gacttcaaca 900
   gcgacaccca ctcctccacc tttgacgctg gggctggcat tgccctcaac gaccactttg 960
   tcaagctcat ttcctggtat gacaacgaat ttggctacag caacagggtg gtggacctca 1020
   tggcccacat ggcctccaag gagtaagacc cctggaccac cagccccagc aagagcacaa 1080
   gaggaagaga gagaccctca ctgctgggga gtccctgcca cactcagtcc cccaccacac 1140
   tgaatctccc ctcctcacag ttgccatgta gaccccttga agaggggagg ggcctaggga 1200
   gccgcacctt gtcatgtacc atcaataaag taccctgtgc tcaaccaaaa aaaaaaaaaa 1260
   a 1261
<210> 452
   <211> 101
   <212> DNA
   <213> Artificial Organism
<220>
   <223> Synthetic oligonucleotide
<400> 452
   ctcctgaacc ctactccaag cacagcctct gtctgactcc cttgtccttc aagagaactg 60
   ttctccaggt ctcagggcca ggatttccat aggatcgcct g 101
<210> 453
   <211> 22
   <212> DNA
   <213> Homo sapiens
<400> 453
   ctcctgaacc ctactccaag ca 22
<210> 454
   <211> 21
   <212> DNA
   <213> Homo sapiens
<400> 454
   caggcgatcc tatggaaatc c 21
<210> 455
   <211> 113
   <212> DNA
   <213> Artificial Organism
<220>
   <223> Synthetic oligonucleotide
<400> 455
   caagcaattg agggaccagg aagtggatcc tctagagatg aggaggcatt ctgctggatg 60
   acttttaaaa atgttttctc cagagtcatc tctctcatta acaatgtttt ttg 113

<210> 456
   <211> 20
   <212> DNA
   <213> Homo sapiens
<400> 456
   caagcaattg agggaccagg 20
<210> 457
   <211> 29
   <212> DNA
   <213> Homo sapiens
<400> 457
   caaaaaacat tgttaatgag agagatgac 29
<210> 458
   <211> 50
   <212> DNA
   <213> Homo sapiens
<400> 458
   aactctggca ccttgggctg tggaaggctc tggaaagtcc ttcaaagctg 50
<210> 459
   <211> 4
   <212> PRT
   <213> Artificial Organism
<220>
   <223> Synthetic peptide
<400> 459
   Met Thr Ala Pro
   1
<210> 460
   <211> 682
   <212> PRT
   <213> Artificial Organism
<220>
   <223> Synthetic peptide
<400> 460
   Asp Gln Glu Ala Pro Tyr Leu Leu Arg Asn Leu Ser Asp His Thr Val
   1 5 10 15
   Ala Ile Ser Ser Ser Thr Thr Leu Asp Cys His Ala Asn Gly Val Pro
   20 25 30
   Glu Pro Gln Ile Thr Trp Phe Lys Asn Asn His Lys Ile Gln Gln Glu
   35 40 45
   Pro Gly Ile Ile Leu Gly Pro Gly Ser Ser Thr Leu Phe Ile Glu Arg
   50 55 60
   Val Thr Glu Glu Asp Glu Gly Val Tyr His Cys Lys Ala Thr Asn Gln
   65 70 75 80
   Lys Gly Ser Val Glu Ser Ser Ala Tyr Leu Thr Val Gln Gly Thr Ser
   85 90 95
   Asp Lys Ser Asn Leu Glu Leu Ile Thr Leu Thr Cys Thr Cys Val Ala
   100 105 110
   Ala Thr Leu Phe Trp Leu Leu Leu Thr Leu Phe Ile Arg Lys Met Lys
   115 120 125
   Arg Ser Ser Ser Glu Ile Lys Thr Asp Tyr Leu Ser Ile Ile Met Asp
   130 135 140
   Pro Asp Glu Val Pro Leu Asp Glu Gln Cys Glu Arg Leu Pro Tyr Asp
   145 150 155 160
   Ala Ser Lys Trp Glu Phe Ala Arg Glu Arg Leu Lys Leu Gly Lys Ser
   165 170 175
   Leu Gly Arg Gly Ala Phe Gly Lys Val Val Gln Ala Ser Ala Phe Gly
   180 185 190
   Ile Lys Lys Ser Pro Thr Cys Arg Thr Val Ala Val Lys Met Leu Lys
   195 200 205
   Glu Gly Ala Thr Ala Ser Glu Tyr Lys Ala Leu Met Thr Glu Leu Lys
   210 215 220
   Ile Leu Thr His Ile Gly His His Leu Asn Val Val Asn Leu Leu Gly
   225 230 235 240
   Ala Cys Thr Lys Gln Gly Gly Pro Leu Met Val Ile Val Glu Tyr Cys
   245 250 255
   Lys Tyr Gly Asn Leu Ser Asn Tyr Leu Lys Ser Lys Arg Asp Leu Phe
   260 265 270
   Phe Leu Asn Lys Asp Ala Ala Leu His Met Glu Pro Lys Lys Glu Lys
   275 280 285
   Met Glu Pro Gly Leu Glu Gln Gly Lys Lys Pro Arg Leu Asp Ser Val
   290 295 300
   Thr Ser Ser Glu Ser Phe Ala Ser Ser Gly Phe Gln Glu Asp Lys Ser
   305 310 315 320
   Leu Ser Asp Val Glu Glu Glu Glu Asp Ser Asp Gly Phe Tyr Lys Glu
   325 330 335
   Pro Ile Thr Met Glu Asp Leu Ile Ser Tyr Ser Phe Gln Val Ala Arg
   340 345 350
   Gly Met Glu Phe Leu Ser Ser Arg Lys Cys Ile His Arg Asp Leu Ala
   355 360 365
   Ala Arg Asn Ile Leu Leu Ser Glu Asn Asn Val Val Lys Ile Cys Asp
   370 375 380
   Phe Gly Leu Ala Arg Asp Ile Tyr Lys Asn Pro Asp Tyr Val Arg Lys
   385 390 395 400
   Gly Asp Thr Arg Leu Pro Leu Lys Trp Met Ala Pro Glu Ser Ile Phe
   405 410 415
   Asp Lys Ile Tyr Ser Thr Lys Ser Asp Val Trp Ser Tyr Gly Val Leu
   420 425 430
   Leu Trp Glu Ile Phe Ser Leu Gly Gly Ser Pro Tyr Pro Gly Val Gln
   435 440 445
   Met Asp Glu Asp Phe Cys Ser Arg Leu Arg Glu Gly Met Arg Met Arg
   450 455 460
   Ala Pro Glu Tyr Ser Thr Pro Glu Ile Tyr Gln Ile Met Leu Asp Cys
   465 470 475 480
   Trp His Arg Asp Pro Lys Glu Arg Pro Arg Phe Ala Glu Leu Val Glu
   485 490 495
   Lys Leu Gly Asp Leu Leu Gln Ala Asn Val Gln Gln Asp Gly Lys Asp
   500 505 510
   Tyr Ile Pro Ile Asn Ala Ile Leu Thr Gly Asn Ser Gly Phe Thr Tyr
   515 520 525
   Ser Thr Pro Ala Phe Ser Glu Asp Phe Phe Lys Glu Ser Ile Ser Ala
   530 535 540
   Pro Lys Phe Asn Ser Gly Ser Ser Asp Asp Val Arg Tyr Val Asn Ala
   545 550 555 560
   Phe Lys Phe Met Ser Leu Glu Arg Ile Lys Thr Phe Glu Glu Leu Leu
   565 570 575
   Pro Asn Ala Thr Ser Met Phe Asp Asp Tyr Gln Gly Asp Ser Ser Thr
   580 585 590
   Leu Leu Ala Ser Pro Met Leu Lys Arg Phe Thr Trp Thr Asp Ser Lys
   595 600 605
   Pro Lys Ala Ser Leu Lys Ile Asp Leu Arg Val Thr Ser Lys Ser Lys
   610 615 620
   Glu Ser Gly Leu Ser Asp Val Ser Arg Pro Ser Phe Cys His Ser Ser
   625 630 635 640
   Cys Gly His Val Ser Glu Gly Lys Arg Arg Phe Thr Tyr Asp His Ala
   645 650 655
   Glu Leu Glu Arg Lys Ile Ala Cys Cys Ser Pro Pro Pro Asp Tyr Asn
   660 665 670
   Ser Val Val Leu Tyr Ser Thr Pro Pro Ile
   675 680
<210> 461
   <211> 682
   <212> PRT
   <213> Artificial Organism
<220>
   <223> Synthetic peptide
<400> 461
   Asp Gln Glu Ala Pro Tyr Leu Leu Arg Asn Leu Ser Asp His Thr Val
   1               5                   10                  15
   Ala Ile Ser Ser Ser Thr Thr Leu Asp Cys His Ala Asn Gly Val Pro
               20                  25                  30
   Glu Pro Gln Ile Thr Trp Phe Lys Asn Asn His Lys Ile Gln Gln Glu
           35                  40                  45
   Pro Gly Ile Ile Leu Gly Pro Gly Ser Ser Thr Leu Phe Ile Glu Arg
       50                  55                  60
   Val Thr Glu Glu Asp Glu Gly Val Tyr His Cys Lys Ala Thr Asn Gln
   65                  70                  75                      80
   Lys Gly Ser Val Glu Ser Ser Ala Tyr Leu Thr Val Gln Gly Thr Ser
                   85                  90                  95
   Asp Lys Ser Asn Leu Glu Leu Ile Thr Leu Thr Cys Thr Cys Val Ala
               100                 105                 110
   Ala Thr Leu Phe Trp Leu Leu Leu Thr Leu Phe Ile Arg Lys Met Lys
           115                 120                 125
   Arg Ser Ser Ser Glu Ile Lys Thr Asp Tyr Leu Ser Ile Ile Met Asp
       130                 135                 140
   Pro Asp Glu Val Pro Leu Asp Glu Gln Cys Glu Arg Leu Pro Tyr Asp
   145                 150                 155                 160
   Ala Ser Lys Trp Glu Phe Ala Arg Glu Arg Leu Lys Leu Gly Lys Ser
                   165                 170                 175
   Leu Gly Arg Gly Ala Phe Gly Lys Val Val Gln Ala Ser Ala Phe Gly
               180                 185                 190
   Ile Lys Lys Ser Pro Thr Cys Arg Thr Val Ala Val Lys Met Leu Lys
           195                 200                 205
   Glu Gly Ala Thr Ala Ser Glu Tyr Lys Ala Leu Met Thr Glu Leu Lys
       210                 215                 220
   Ile Leu Thr His Ile Gly His His Leu Asn Val Val Asn Leu Leu Gly
   225                 230                 235                 240
   Ala Cys Thr Lys Gln Gly Gly Pro Leu Met Val Ile Val Glu Tyr Cys
                   245                 250                 255
   Lys Tyr Gly Asn Leu Ser Asn Tyr Leu Lys Ser Lys Arg Asp Leu Phe
               260                 265                 270
   Phe Leu Asn Lys Asp Ala Ala Leu His Met Glu Pro Lys Lys Glu Lys
           275                 280                 285
   Met Glu Pro Gly Leu Glu Gln Gly Lys Lys Pro Arg Leu Asp Ser Val
       290                 295                 300
   Thr Ser Ser Glu Ser Phe Ala Ser Ser Gly Phe Gln Glu Asp Lys Ser
   305                 310                 315                 320
   Leu Ser Asp Val Glu Glu Glu Glu Asp Ser Asp Gly Phe Tyr Lys Glu
                   325                 330                 335
   Pro Ile Thr Met Glu Asp Leu Ile Ser Tyr Ser Phe Gln Val Ala Arg
   340 345 350
   Gly Met Glu Phe Leu Ser Ser Arg Lys Cys Ile His Arg Asp Leu Ala
   355 360 365
   Ala Arg Asn Ile Leu Leu Ser Glu Asn Asn Val Val Lys Ile Cys Asp
   370 375 380
   Phe Gly Leu Ala Arg Asp Ile Tyr Lys Asn Pro Asp Tyr Val Arg Lys
   385 390 395 400
   Gly Asp Thr Arg Leu Pro Leu Lys Trp Met Ala Pro Glu Ser Ile Phe
   405 410 415
   Asp Lys Ile Tyr Ser Thr Lys Ser Asp Val Trp Ser Tyr Gly Val Leu
   420 425 430
   Leu Trp Glu Ile Phe Ser Leu Gly Gly Ser Pro Tyr Pro Gly Val Gln
   435 440 445
   Met Asp Glu Asp Phe Cys Ser Arg Leu Arg Glu Gly Met Arg Met Arg
   450 455 460
   Ala Pro Glu Tyr Ser Thr Pro Glu Ile Tyr Gln Ile Met Leu Asp Cys
   465 470 475 480
   Trp His Arg Asp Pro Lys Glu Arg Pro Arg Phe Ala Glu Leu Val Glu
   485 490 495
   Lys Leu Gly Asp Leu Leu Gln Ala Asn Val Gln Gln Asp Gly Lys Asp
   500 505 510
   Tyr Ile Pro Ile Asn Ala Ile Leu Thr Gly Asn Ser Gly Phe Thr Tyr
   515 520 525
   Ser Thr Pro Ala Phe Ser Glu Asp Phe Phe Lys Glu Ser Ile Ser Ala
   530 535 540
   Pro Lys Phe Asn Ser Gly Ser Ser Asp Asp Val Arg Tyr Val Asn Ala
   545 550 555 560
   Phe Lys Phe Met Ser Leu Glu Arg Ile Lys Thr Phe Glu Glu Leu Leu
   565 570 575
   Pro Asn Ala Thr Ser Met Phe Asp Asp Tyr Gln Gly Asp Ser Ser Thr
   580 585 590
   Leu Leu Ala Ser Pro Met Leu Lys Arg Phe Thr Trp Thr Asp Ser Lys
   595 600 605
   Pro Lys Ala Ser Leu Lys Ile Asp Leu Arg Val Thr Ser Lys Ser Lys
   610 615 620
   Glu Ser Gly Leu Ser Asp Val Ser Arg Pro Ser Phe Cys His Ser Ser
   625 630 635 640
   Cys Gly His Val Ser Glu Gly Lys Arg Arg Phe Thr Tyr Asp His Ala
   645 650 655
   Glu Leu Glu Arg Lys Ile Ala Cys Cys Ser Pro Pro Pro Asp Tyr Asn
   660 665 670
   Ser Val Val Leu Tyr Ser Thr Pro Pro Ile
   675 680
<210> 462
   <211> 28
   <212> PRT
   <213> Artificial Organism
<220>
   <223> Synthetic peptide
<400> 462
   Glu Leu Tyr Thr Ser Thr Ser Pro Ser Ser Ser Ser Ser Ser Pro Leu
   1 5 10 15
   Ser Ser Ser Ser Ser Ser Ser Ser Ser Ser Ser Ser
   20 25
<210> 463
   <211> 77
   <212> PRT
   <213> Artificial Organism
<220>
   <223> Synthetic peptide
<400> 463
   Asp Gln Glu Ala Pro Tyr Leu Leu Arg Asn Leu Ser Asp His Thr Val
   1 5 10 15
   Ala Ile Ser Ser Ser Thr Thr Leu Asp Cys His Ala Asn Gly Val Pro
   20 25 30
   Glu Pro Gln Ile Thr Trp Phe Lys Asn Asn His Lys Ile Gln Gln Glu
   35 40 45
   Pro Glu Leu Tyr Thr Ser Thr Ser Pro Ser Ser Ser Ser Ser Ser Pro
   50 55 60
   Leu Ser Ser Ser Ser Ser Ser Ser Ser Ser Ser Ser Ser
   65 70 75
<210> 464
   <211> 12
   <212> PRT
   <213> Artificial Organism
<220>
   <223> Synthetic peptide
<400> 464
   Ser Ala Asn Thr Ala Val Asn Lys Lys Thr Glu Ile
   1 5 10
<210> 465
   <211> 62
   <212> PRT
   <213> Artificial Organism
<220>
   <223> Synthetic peptide
<400> 465
   Asp Gln Glu Ala Pro Tyr Leu Leu Arg Asn Leu Ser Asp His Thr Val
   1 5 10 15
   Ala Ile Ser Ser Ser Thr Thr Leu Asp Cys His Ala Asn Gly Val Pro
   20 25 30
   Glu Pro Gln Ile Thr Trp Phe Lys Asn Asn His Lys Ile Gln Gln Glu
   35 40 45
   Pro Gly Ser Ala Asn Thr Ala Val Asn Lys Lys Thr Glu Ile
   50 55 60
<210> 466
   <211> 30
   <212> PRT
   <213> Artificial Organism
<220>
   <223> Synthetic peptide
<400> 466
   Lys Arg Leu Phe Phe Leu Pro Phe Ile Ile Ser His Leu Ser Ser Ala
   1 5 10 15
   Pro Leu Ser Leu Asn Ser Pro Val Thr Cys Phe Gln Tyr Val
   20 25 30
<210> 467
   <211> 80
   <212> PRT
   <213> Artificial Organism
<220>
   <223> Synthetic peptide
<400> 467
   Asp Gln Glu Ala Pro Tyr Leu Leu Arg Asn Leu Ser Asp His Thr Val
   1 5 10 15
   Ala Ile Ser Ser Ser Thr Thr Leu Asp Cys His Ala Asn Gly Val Pro
   20 25 30
   Glu Pro Gln Ile Thr Trp Phe Lys Asn Asn His Lys Ile Gln Gln Glu
   35 40 45
   Pro Gly Lys Arg Leu Phe Phe Leu Pro Phe Ile Ile Ser His Leu Ser
   50 55 60
   Ser Ala Pro Leu Ser Leu Asn Ser Pro Val Thr Cys Phe Gln Tyr Val
   65 70 75 80
<210> 468
   <211> 30
   <212> PRT
   <213> Artificial Organism
<220>
   <223> Synthetic peptide
<400> 468
   Tyr Leu Asp Ile Arg Thr Glu Glu Gln Ile Phe Ser Phe Ile Gln Lys
   1               5                   10                  15
   Thr Gln Thr Leu Lys Leu Thr Val Ser Cys Lys Ala Ala Phe
               20                  25                  30
<210> 469
   <211> 36
   <212> PRT
   <213> Artificial Organism
<220>
   <223> Synthetic peptide
<400> 469
   Gly Lys His Ser Ser Ala Leu Pro Thr His Ala Met Leu Ser Asn His
   1               5                   10                  15
   Cys Arg Cys Leu Cys Ser Leu Asn Lys Ser Val Phe Cys Trp Pro Arg
               20                  25                  30
   Val Thr Leu Ser
           35
<210> 470
   <211> 43
   <212> PRT
   <213> Artificial Organism
<220>
   <223> Synthetic peptide
<400> 470
   Val Asn Leu Asn Thr Ala Ile Leu Ser Ile Leu Ser Leu Gln Ile Ser
   1               5                   10                  15
   Ile Met Lys Phe Tyr Ser Phe Tyr Leu Ser Gly Ile Ile Ser Leu Gln
               20                  25                  30
   Thr Pro Gly Leu Leu Ser Gly Leu Ser Cys Asn
           35                  40
<210> 471
   <211> 54
   <212> PRT
   <213> Artificial Organism
<220>
   <223> Synthetic peptide
<400> 471
   Gly Lys Thr Ser Ile Phe Tyr Ile Leu Phe Ala Phe Ala Leu Gln Met
   1 5 10 15
   Ser His Lys Ser Thr Leu Ile His Trp Lys Gly Cys Phe Pro Ser Glu
   20 25 30
   Tyr Glu Arg Asn Gly Leu Gly Lys Arg Phe His Pro Ser Cys Arg His
   35 40 45
   Phe Arg Gly Cys Gln Phe
   50
<210> 472
   <211> 24
   <212> PRT
   <213> Artificial Organism
<220>
   <223> Synthetic peptide
<400> 472
   Leu Pro Pro Ala Asn Ser Ser Phe Met Leu Pro Pro Thr Ser Phe Ser
   1 5 10 15
   Ser Asn Tyr Phe His Phe Leu Pro
   20
<210> 473
   <211> 14
   <212> PRT
   <213> Artificial Organism
<220>
   <223> Synthetic peptide
<400> 473
   Glu Leu Ser Asn Phe Glu Cys Leu His Pro Cys Ser Gln Glu
   1 5 10
<210> 474
   <211> 30
   <212> PRT
   <213> Artificial Organism
<220>
   <223> Synthetic peptide
<400> 474
   Gly Glu Trp Leu Pro Gly Lys Pro Met Tyr Val Gly Ile Thr Ser Leu
   1 5 10 15
   Cys Pro Ser Val Cys Ser Ser Met Ala Cys Leu His Lys Pro
   20 25 30
<210> 475
   <211> 30
   <212> PRT
   <213> Artificial Organism
<220>
   <223> Synthetic peptide
<400> 475
   Gly Glu Trp Leu Pro Gly Lys Pro Met Tyr Val Gly Ile Thr Ser Leu
   1 5 10 15
   Cys Pro Ser Val Cys Ser Ser Met Ala Cys Leu His Lys Pro
   20 25 30
<210> 476
   <211> 30
   <212> PRT
   <213> Artificial Organism
<220>
   <223> Synthetic peptide
<400> 476
   Gly Glu Trp Leu Pro Gly Lys Pro Met Tyr Val Gly Ile Thr Ser Leu
   1 5 10 15
   Cys Pro Ser Val Cys Ser Ser Met Ala Cys Leu His Lys Pro
   20 25 30
<210> 477
   <211> 3
   <212> PRT
   <213> Artificial Organism
<220>
   <223> Synthetic peptide
<400> 477
   Gly Gly Leu
   1
<210> 478
   <211> 6
   <212> PRT
   <213> Artificial Organism
<220>
   <223> Synthetic peptide
<400> 478
   Asp Arg Cys Gly Thr His
   1               5
<210> 479
   <211> 6
   <212> PRT
   <213> Artificial Organism
<220>
   <223> Synthetic peptide
<400> 479
   Asp Arg Cys Gly Thr His
   1               5
<210> 480
   <211> 63
   <212> PRT
   <213> Artificial Organism
<220>
   <223> Synthetic peptide
<400> 480
   Ser His Leu Val Leu Thr Leu Gly Leu Leu Gln Glu Glu Thr Gln Gly
   1               5                   10                  15
   Gln Gly Glu Glu Glu Glu Arg Glu Ala Glu Thr His Arg Leu Pro Pro
               20                  25                  30
   Val Arg Arg Cys Cys Ser Pro Glu Val Thr His Pro Leu Glu Glu Arg
           35                  40                  45
   Asp Pro Ala Pro Gly Ser Cys Ile Tyr Tyr Arg His Thr Leu Gln
       50                  55                  60
<210> 481
   <211> 22
   <212> PRT
   <213> Artificial Organism
<220>
   <223> Synthetic peptide
<400> 481
   Ser Glu Pro Thr Ala Pro Ser Leu Ala Gln Cys Leu Leu Ser Ser Ser
   1               5                   10                  15
   Pro Tyr Pro Ala Thr Ala
               20
<210> 482
   <211> 229
   <212> PRT
   <213> Artificial Organism
<220>
   <223> Synthetic peptide
<400> 482
   Val Arg Ala Ala Ser Ser Pro Pro Ala Cys Leu Pro Thr Gln Ala Pro
   1               5                   10                  15
   Val Pro Thr His Gly Glu Pro His Thr Gln His Pro Ser Gln Pro Asp
   20 25 30
   Thr His Thr His Thr His Thr His Thr Ala Pro Lys Pro Ala Arg His
   35 40 45
   Lys His Thr Ala Pro Gln Pro Ala Gly His Thr His Thr His Thr His
   50 55 60
   Asn Thr Pro Ala Gly Arg Thr His Thr His Thr Val Pro Gln Leu Ala
   65 70 75 80
   Gly His Thr His Thr Gln His Pro Ile Gln Thr His Thr His Thr Gln
   85 90 95
   Tyr Pro Ser Gln Leu Glu Thr His Thr His Thr Ala Leu His Pro Asp
   100 105 110
   Thr Tyr Pro His Ser Thr Pro Ala Ser Gln Thr His Thr His Thr His
   115 120 125
   Thr His Thr His Thr Gln His Thr His Ser Thr Pro Ala Gly His Thr
   130 135 140
   His Thr His Thr His Pro Val His Lys Gly Pro Arg Lys Leu Arg Ala
   145 150 155 160
   Leu Gln Pro Cys Thr Arg Pro Trp Ala Pro Arg Phe Arg Cys Thr Arg
   165 170 175
   Trp Ala Cys Thr Leu Thr His Pro Tyr Thr Leu Thr Leu Thr His Met
   180 185 190
   Leu Thr His Leu Phe Ile Leu Thr Tyr Met Leu Met Leu Ile His Thr
   195 200 205
   Gln Ser Arg Pro Pro Ala Leu Lys Ser Pro His Ser Pro Ile Phe Ala
   210 215 220
   Phe Cys Pro Pro Thr
   225
<210> 483
   <211> 22
   <212> PRT
   <213> Artificial Organism
<220>
   <223> Synthetic peptide
<400> 483
   Ser Glu Pro Thr Ala Pro Ser Leu Ala Gln Cys Leu Leu Ser Ser Ser
   1               5                   10                  15
   Pro Tyr Pro Ala Thr Ala
               20
<210> 484
   <211> 5
   <212> PRT
   <213> Artificial Organism
<220>
   <223> Synthetic peptide
<400> 484
   Leu Ala Pro Val Cys
   1               5
<210> 485
   <211> 73
   <212> PRT
   <213> Artificial Organism
<220>
   <223> Synthetic peptide
<400> 485
   Val Arg His Pro Gly Pro His Arg Ala Gln Glu His Thr His Thr Cys
   1               5                   10                  15
   Ser Ser Leu Gln Leu Pro Pro Leu Ser Gln Leu Thr Pro Pro Ser Gly
               20                  25                  30
   Pro Ser Trp Leu Pro Glu Val Arg Arg Gly Glu Ser Arg Leu Cys Ile
           35                  40                  45
   Ala Pro Thr Gln Gly Thr Leu Gly Leu Arg Leu Arg Pro Gly Arg Cys
       50                  55                  60
   Gln Ala Tyr Ser Ser Cys Asn Lys His
   65                  70
<210> 486
   <211> 229
   <212> PRT
   <213> Artificial Organism
<220>
   <223> Synthetic peptide
<400> 486
   Val Arg Ala Ala Ser Ser Pro Pro Ala Cys Leu Pro Thr Gln Ala Pro
   1               5                   10                  15
   Val Pro Thr His Gly Glu Pro His Thr Gln His Pro Ser Gln Pro Asp
               20                  25                  30
   Thr His Thr His Thr His Thr His Thr Ala Pro Lys Pro Ala Arg His
           35                  40                  45
   Lys His Thr Ala Pro Gln Pro Ala Gly His Thr His Thr His Thr His
       50                  55                  60
   Asn Thr Pro Ala Gly Arg Thr His Thr His Thr Val Pro Gln Leu Ala
   65                  70                  75                  80
   Gly His Thr His Thr Gln His Pro Ile Gln Thr His Thr His Thr Gln
                   85                  90                  95
   Tyr Pro Ser Gln Leu Glu Thr His Thr His Thr Ala Leu His Pro Asp
               100                 105                 110
   Thr Tyr Pro His Ser Thr Pro Ala Ser Gln Thr His Thr His Thr His
           115                 120                 125
   Thr His Thr His Thr Gln His Thr His Ser Thr Pro Ala Gly His Thr
       130                 135                 140
   His Thr His Thr His Pro Val His Lys Gly Pro Arg Lys Leu Arg Ala
   145                 150                 155                 160
   Leu Gln Pro Cys Thr Arg Pro Trp Ala Pro Arg Phe Arg Cys Thr Arg
                   165                 170                 175
   Trp Ala Cys Thr Leu Thr His Pro Tyr Thr Leu Thr Leu Thr His Met
               180                 185                 190
   Leu Thr His Leu Phe Ile Leu Thr Tyr Met Leu Met Leu Ile His Thr
           195                 200                 205
   Gln Ser Arg Pro Pro Ala Leu Lys Ser Pro His Ser Pro Ile Phe Ala
       210                 215                 220
   Phe Cys Pro Pro Thr
   225
<210> 487
   <211> 24
   <212> PRT
   <213> Artificial Organism
<220>
   <223> Synthetic peptide
<400> 487
   Arg Thr Ser Pro Leu Glu Leu Gly Ala Ser Pro Thr His Val Ser Ser
   1               5                   10                  15
   Thr Leu Gly Pro Leu Pro Pro Gln
               20
<210> 488
   <211> 18
   <212> PRT
   <213> Artificial Organism
<220>
   <223> Synthetic peptide
<400> 488
   Arg Arg Gln Glu Asn Gly Cys Arg Glu Thr Leu Ser Ala Thr Ser Ala
   1               5                   10                  15
   Cys Pro
<210> 489
   <211> 24
   <212> PRT
   <213> Artificial Organism
<220>
   <223> Synthetic peptide
<400> 489
   Lys Lys Thr Pro Ser Phe Lys Val Leu Gln Tyr Gly Gln Thr Trp Trp
   1               5                   10                  15
   Leu Thr Pro Ala Ile Pro Ala Pro
               20
<210> 490
   <211> 56
   <212> PRT
   <213> Artificial Organism
<220>
   <223> Synthetic peptide
<400> 490
   Cys Leu Ser Ser Pro Ser Pro Arg Pro Gln Gln Ser Gly Cys Arg Pro
   1               5                   10                  15
   His Pro Ser Gln Ala Leu Pro Pro Glu His Glu Gln Leu Pro Gly Pro
               20                  25                  30
   Pro Glu Leu Trp Leu Pro Leu Arg Asp Val His Gly Ala Glu Ala Gly
           35                  40                  45
   Thr Pro Asp Leu Pro Val His Arg
       50                  55
<210> 491
   <211> 123
   <212> PRT
   <213> Artificial Organism
<220>
   <223> Synthetic peptide
<400> 491
   Gln Met Lys Asp Gln Leu Asp Asn Leu Leu Leu Lys Glu Ser Leu Leu
   1               5                   10                  15
   Glu Asp Phe Lys Gly Tyr Leu Gly Cys Gln Ala Leu Ser Glu Met Ile
               20                  25                  30
   Gln Phe Tyr Leu Glu Glu Val Met Pro Gln Ala Glu Asn Gln Asp Pro
           35                  40                  45
   Asp Ile Lys Ala His Val Asn Ser Leu Gly Glu Asn Leu Lys Thr Leu
       50                  55                  60
   Arg Leu Arg Leu Arg Arg Cys His Arg Phe Leu Pro Cys Glu Asn Lys
   65                  70                  75                  80
   Ser Lys Ala Val Glu Gln Val Lys Asn Ala Phe Asn Lys Leu Gln Glu
                   85                  90                  95
   Lys Gly Ile Tyr Lys Ala Met Ser Glu Phe Asp Ile Phe Ile Asn Tyr
               100                 105                 110
   Ile Glu Ala Tyr Met Thr Met Lys Ile Arg Asn
           115                 120
<210> 492
   <211> 101
   <212> PRT
   <213> Artificial Organism
<220>
   <223> Synthetic peptide
<400> 492
   Gln Met Lys Asp Gln Leu Asp Asn Leu Leu Leu Lys Glu Ser Leu Leu
   1               5                   10                  15
   Glu Asp Phe Lys Gly Tyr Leu Gly Cys Gln Ala Leu Ser Glu Met Ile
               20                  25                  30
   Gln Phe Tyr Leu Glu Glu Val Met Pro Gln Ala Glu Asn Gln Asp Pro
           35                  40                  45
   Asp Ile Lys Ala His Val Asn Ser Leu Gly Glu Asn Leu Lys Thr Leu
       50                  55                  60
   Arg Leu Arg Leu Arg Arg Cys Leu Gln Glu Lys Gly Ile Tyr Lys Ala
   65                  70                  75                  80
   Met Ser Glu Phe Asp Ile Phe Ile Asn Tyr Ile Glu Ala Tyr Met Thr
                   85                  90                  95
   Met Lys Ile Arg Asn
               100
<210> 493
   <211> 80
   <212> PRT
   <213> Artificial Organism
<220>
   <223> Synthetic peptide
<400> 493
   Asp Ala Val Ile Ala Ile Gln Ser Gln Thr Val Gly Gly Trp Glu Cys
   1               5                   10                  15
   Glu Lys Leu Ser Gln Pro Trp Gln Gly Cys Thr Ile Ser Ala Thr Ser
               20                  25                  30
   Ser Ala Arg Thr Ser Cys Cys Ile Leu Thr Gly Cys Ser Leu Leu Leu
           35                  40                  45
   Thr Ala Ser Pro Gly Val Glu Ile Arg Arg Asp Ser Ala Gly Cys Ser
       50                  55                  60
   His Met Ile Lys Glu Gly Pro Glu Leu Gly Val Thr Pro Asp Pro Ser
   65                  70                  75                  80
<210> 494
   <211> 49
   <212> PRT
   <213> Artificial Organism
<220>
   <223> Synthetic peptide
<400> 494
   Phe Leu Gln Gln Gly Cys Pro Pro Ser Pro Gly Val Pro Thr Gly Phe
   1               5                   10                  15
   Pro Gly Ala Ser Tyr Ser Ala Thr Met Trp Glu Phe His His His Arg
               20                  25                  30
   Asp Leu Ser Gly Ser Ser Gly Ser Tyr Val Glu Thr Arg Asn Ser Ser
           35                  40                  45
   Pro
<210> 495
   <211> 8
   <212> PRT
   <213> Artificial Organism
<220>
   <223> Synthetic peptide
<400> 495
   Gly Lys Gln Gly Met Arg Ala His
   1               5
<210> 496
   <211> 59
   <212> PRT
   <213> Artificial Organism
<220>
   <223> Synthetic peptide
<400> 496
   Gly Cys Phe Ser Pro Ser Ile Ser Pro Ser His Phe Phe Thr Met Ser
   1               5                   10                  15
   Ser Ile Ser Thr Phe Ser Ala Val Leu Arg Thr Ser Ala Ser Ser Leu
               20                  25                  30
   Ser Ala Cys Val Leu Pro Ala Thr His Gln Met Arg Ser Gly Glu Glu
           35                  40                  45
   Phe Ser Thr Phe Gly Phe Met Leu Val Leu Lys
                       50                  55
<210> 497
   <211> 25
   <212> PRT
   <213> Artificial Organism
<220>
   <223> Synthetic peptide
<400> 497
   Gly Glu Lys Arg His His Lys Gln Leu Arg Asp Gln Glu Val Asp Pro
   1               5                   10                  15
   Leu Glu Met Arg Arg His Ser Ala Gly
               20                  25
<210> 498
   <211> 8
   <212> PRT
   <213> Artificial Organism
<220>
   <223> Synthetic peptide
<400> 498
   Arg Gly Ser Leu Gly Ser Ala Gln
   1               5
<210> 499
   <211> 31
   <212> PRT
   <213> Artificial Organism
<220>
   <223> Synthetic peptide
<400> 499
   Gly Ala Pro Thr Pro Trp Thr Ser Pro Gly Ser Ser Arg Arg Pro Leu
   1               5                   10                  15
   Ala Arg Ser Leu Thr Ala Ser Gly Ala Ala Thr Cys Pro Ser Ala
               20                  25                  30
<210> 500
   <211> 80
   <212> PRT
   <213> Artificial Organism
<220>
   <223> Synthetic peptide
<400> 500
   Gly Thr Ser Thr Pro Glu Gly Leu Thr Ser Pro Cys Arg Gln Ser Ser
   1               5                   10                  15
   Ala Ser Thr Ser Trp Pro Leu Pro Met Gly Pro Gly Ser Cys Arg Ala
               20                  25                  30
   Thr Ala Gln Ala Ser Leu Pro Ser Ser Ser Pro Arg Thr Pro Arg Ser
           35                  40                  45
   Arg Cys Pro Trp Thr Cys Met Pro Met Gln Trp Pro Gln Gly Thr Pro
       50                  55                  60
   Cys Trp Arg Ser Ser Ala Tyr Ser Ser Trp Pro Gly Thr Ser Arg Pro
   65                  70                  75                  80
<210> 501
   <211> 109
   <212> PRT
   <213> Artificial Organism
<220>
   <223> Synthetic peptide
<400> 501
   Gln Gln Gly Leu Trp Gln Leu Thr Gly Leu Cys Leu Gly Gln Leu Gln
   1 5 10 15
   Thr Ser Val Pro Cys Gln Pro Arg Thr Gly Trp Thr Ala Ala Thr Pro
   20 25 30
   Met Ser Pro Pro Arg Ser Ala Thr Thr Gly Ala Ala Ala Leu Thr Pro
   35 40 45
   Gly Ser Leu Glu Cys Leu Gly Val Ser Ser Pro Cys Arg Lys Gln Asn
   50 55 60
   Ala Pro Ser Glu Ala Pro Pro Ala Ala Pro Gly Arg Gly Met Arg Gly
   65 70 75 80
   Ser Glu His Pro Cys Pro Ala Val Ile Ala Ala Arg His Cys Ser Ser
   85 90 95
   Gln Leu Phe Cys Pro Phe Ala Pro Gly Lys Arg Phe Cys
   100 105
<210> 502
   <211> 97
   <212> DNA
   <213> Homo sapiens
<400> 502
   gatctagttc aggttcaaaa ttaaaagatc ctgaactgag tttaaaaggc acccagcaca 60
   tcatgcaagc aggccagaca ctgcatctcc aatgcag 97
<210> 503
   <211> 118
   <212> DNA
   <213> Homo sapiens
<400> 503
   ataaagcatt catcactgtg aaacatcgaa aacagcaggt gcttgaaacc gtagctggca 60
   agcggtctta ccggctctct atgaaagtga aggcatttcc ctcgccggaa gttgtatg 118
<210> 504
   <211> 115
   <212> DNA
   <213> Homo sapiens
<400> 504
   gtgtgacttt tgttccaata atgaagagtc ctttatcctg gatgctgaca gcaacatggg 60
   aaacagaatt gagagcatca ctcagcgcat ggcaataata gaaggaaaga ataag 115
<210> 505
   <211> 85
   <212> DNA
   <213> Homo sapiens
<400> 505
   cttccaccag ctaacagttc tttcatgttg ccacctacaa gcttctcttc caactacttc 60
   catttccttc cgtgactcta aacgg 85
<210> 506
   <211> 109
   <212> DNA
   <213> Homo sapiens
<400> 506
   atggctagca ccttggttgt ggctgactct agaatttctg gaatctacat ttgcatagct 60
   tccaataaag ttgggactgt gggaagaaac ataagctttt atatcacag 109
<210> 507
   <211> 65
   <212> DNA
   <213> Homo sapiens
<400> 507
   aattgtcaaa ctttgagtgc cttcatcctt gctctcagga atagaattct acctcatcgg 60
   atctc 65
<210> 508
   <211> 107
   <212> DNA
   <213> Homo sapiens
<400> 508
   gaacctcgga caagtctaat ctggagctga tcactctaac atgcacctgt gtggctgcga 60
   ctctcttctg gctcctatta accctcttta tccgaaaaat gaaaagg 107
<210> 509
   <211> 105
   <212> DNA
   <213> Homo sapiens
<400> 509
   gcaaatcact tggaagaggg gcttttggaa aagtggttca agcatcagca tttggcatta 60
   agaaatcacc tacgtgccgg actgtggctg tgaaaatgct gaaag 105
<210> 510
   <211> 114
   <212> DNA
   <213> Homo sapiens
<400> 510
   agggggccac ggccagcgag tacaaagctc tgatgactga gctaaaaatc ttgacccaca 60
   ttggccacca tctgaacgtg gttaacctgc tgggagcctg caccaagcaa ggag 114
<210> 511
   <211> 89
   <212> DNA
   <213> Homo sapiens
<400> 511
   ggcctctgat ggtgattgtt gaatactgca aatatggaaa tctctccaac tacctcaaga 60
   gcaaacgtga cttatttttt ctcaacaag 89
<210> 512
   <211> 33
   <212> DNA
   <213> Homo sapiens
<400> 512
   attacaaaca gttacggttc taatgctttc cag 33
<210> 513
   <211> 98
   <212> DNA
   <213> Homo sapiens
<400> 513
   attctgacgg tttctacaag gagcccatca ctatggaaga tctgatttct tacagttttc 60
   aagtggccag aggcatggag ttcctgtctt ccagaaag 98
<210> 514
   <211> 112
   <212> DNA
   <213> Homo sapiens
<400> 514
   actcgacttc ctctgaaatg gatggctcct gaatctatct ttgacaaaat ctacagcacc 60
   aagagcgacg tgtggtctta cggagtattg ctgtgggaaa tcttctcctt ag 112
<210> 515
   <211> 100
   <212> DNA
   <213> Homo sapiens
<400> 515
   gtgggtctcc atacccagga gtacaaatgg atgaggactt ttgcagtcgc ctgagggaag 60
   gcatgaggat gagagctcct gagtactcta ctcctgaaat 100
<210> 516
   <211> 106
   <212> DNA
   <213> Homo sapiens
<400> 516
   ctatcagatc atgctggact gctggcacag agacccaaaa gaaaggccaa gatttgcaga 60
   acttgtggaa aaactaggtg atttgcttca agcaaatgta caacag 106
<210> 517
   <211> 86
   <212> DNA
   <213> Homo sapiens
<400> 517
   gatggtaaag actacatccc aatcaatgcc atactgacag gaaatagtgg gtttacatac 60
   tcaactcctg ccttctctga ggactt 86
<210> 518
   <211> 15
   <212> DNA
   <213> Homo sapiens
<400> 518
   cttcaaggaa agtat 15
<210> 519
   <211> 42
   <212> DNA
   <213> Homo sapiens

<400> 519
   ttcagctccg aagtttaatt caggaagctc tgatgatgtc ag 42
<210> 520
   <211> 85
   <212> DNA
   <213> Homo sapiens
<400> 520
   atatgtaaat gctttcaagt tcatgagcct ggaaagaatc aaaacctttg aagaactttt 60
   accgaatgcc acctccatgt ttgat 85
<210> 521
   <211> 95
   <212> DNA
   <213> Homo sapiens
<400> 521
   gactaccagg gcgacagcag cactctgttg gcctctccca tgctgaagcg cttcacctgg 60
   actgacagca aacccaaggc ctcgctcaag attga 95
<210> 522
   <211> 92
   <212> DNA
   <213> Homo sapiens
<400> 522
   cttgagagta accagtaaaa gtaaggagtc ggggctgtct gatgtcagca ggcccagttt 60
   ctgccattcc agctgtgggc acgtcagcga ag 92
<210> 523
   <211> 83
   <212> DNA
   <213> Homo sapiens
<400> 523
   gcaagcgcag gttcacctac gaccacgctg agctggaaag gaaaatcgcg tgctgctccc 60
   cgcccccaga ctacaactcg gtg 83
<210> 524
   <211> 17
   <212> DNA
   <213> Homo sapiens
<400> 524
   ttagttgtga aagaaag 17
<210> 525
   <211> 32
   <212> DNA
   <213> Homo sapiens
<400> 525
   ctgagaagaa tgaaaatgca gtcctgagga ga 32
<210> 526
   <211> 43
   <212> DNA
   <213> Homo sapiens
<400> 526
   ggagttttct ccatatcaaa acgagggctg atggaggaaa aag 43
<210> 527
   <211> 36
   <212> DNA
   <213> Homo sapiens
<400> 527
   ttaagcactt taagctcctt gagtaaaaag gtggta 36
<210> 528
   <211> 51
   <212> DNA
   <213> Homo sapiens
<400> 528
   gtggattgct gcttcttggg gagaagagta tgcttccttt tatccatgta a 51
<210> 529
   <211> 77
   <212> DNA
   <213> Homo sapiens
<400> 529
   tttaactgta gaacctgagc tctaagtaac cgaagaatgt atgcctctgt tcttatgtgc 60
   cacatccttg tttaaag 77
<210> 530
   <211> 1131
   <212> DNA
   <213> Homo sapiens
<400> 530
   gctctctgta tgaagagatg ggaccgtcat cagcacattc cctagtgagc ctactggctc 60
   ctggcagcgg cttttgtgga agactcacta gccagaagag aggagtggga cagtcctctc 120
   caccaagatc taaatccaaa caaaagcagg ctagagccag aagagaggac aaatctttgt 180
   tcttcctctt ctttacatac gcaaaccacc tgtgacagct ggcaatttta taaatcaggt 240
   aactggaagg aggttaaaca cagaaaaaag aagacctcag tcaattctct actttttttt 300
   ttttttccaa atcagataat agcccagcaa atagtgataa caaataaaac cttagctatt 360
   catgtcttga tttcaataat taattcttaa tcattaagag accataataa atactccttt 420
   tcaagagaaa agcaaaacca ttagaattgt tactcagctc cttcaaactc aggtttgtag 480
   catacatgag tccatccatc agtcaaagaa tggttccatc tggagtctta atgtagaaag 540
   aaaaatggag acttgtaata atgagctagt tacaaagtgc ttgttcatta aaatagcact 600
   gaaaattgaa acatgaatta actgataata ttccaatcat ttgccattta tgacaaaaat 660
   ggttggcact aacaaagaac gagcacttcc tttcagagtt tctgagataa tgtacgtgga 720
   acagtctggg tggaatgggg ctgaaaccat gtgcaagtct gtgtcttgtc agtccaagaa 780
   gtgacaccga gatgttaatt ttagggaccc gtgccttgtt tcctagccca caagaatgca 840
   aacatcaaac agatactcgc tagcctcatt taaattgatt aaaggaggag tgcatctttg 900
   gccgacagtg gtgtaactgt atgtgtgtgt gtgtgtgtgt gtgtgtgtgt gggtgtatgt 960
   gtgttttgtg cataactatt taaggaaact ggaattttaa agttactttt atacaaacca 1020
   agaatatatg ctacagatat aagacagaca tggtttggtc ctatatttct agtcatgatg 1080
   aatgtatttt gtataccatc ttcatataat aaacttccaa aaacacatat a 1131
<210> 531
   <211> 1338
   <212> PRT
   <213> Homo sapiens
<400> 531
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
   210 215 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545 550 555 560
   Val Asn Leu Glu Lys Met Pro Thr Glu Gly Glu Asp Leu Lys Leu Ser
   565 570 575
   Cys Thr Val Asn Lys Phe Leu Tyr Arg Asp Val Thr Trp Ile Leu Leu
   580 585 590
   Arg Thr Val Asn Asn Arg Thr Met His Tyr Ser Ile Ser Lys Gln Lys
   595 600 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Asp Gln Glu Ala Pro Tyr Leu Leu Arg Asn Leu Ser Asp His Thr Val
   660 665 670
   Ala Ile Ser Ser Ser Thr Thr Leu Asp Cys His Ala Asn Gly Val Pro
   675 680 685
   Glu Pro Gln Ile Thr Trp Phe Lys Asn Asn His Lys Ile Gln Gln Glu
   690 695 700
   Pro Gly Ile Ile Leu Gly Pro Gly Ser Ser Thr Leu Phe Ile Glu Arg
   705 710 715 720
   Val Thr Glu Glu Asp Glu Gly Val Tyr His Cys Lys Ala Thr Asn Gln
   725 730 735
   Lys Gly Ser Val Glu Ser Ser Ala Tyr Leu Thr Val Gln Gly Thr Ser
   740 745 750
   Asp Lys Ser Asn Leu Glu Leu Ile Thr Leu Thr Cys Thr Cys Val Ala
   755 760 765
   Ala Thr Leu Phe Trp Leu Leu Leu Thr Leu Leu Ile Arg Lys Met Lys
   770 775 780
   Arg Ser Ser Ser Glu Ile Lys Thr Asp Tyr Leu Ser Ile Ile Met Asp
   785 790 795 800
   Pro Asp Glu Val Pro Leu Asp Glu Gln Cys Glu Arg Leu Pro Tyr Asp
   805 810 815
   Ala Ser Lys Trp Glu Phe Ala Arg Glu Arg Leu Lys Leu Gly Lys Ser
   820 825 830
   Leu Gly Arg Gly Ala Phe Gly Lys Val Val Gln Ala Ser Ala Phe Gly
   835 840 845
   Ile Lys Lys Ser Pro Thr Cys Arg Thr Val Ala Val Lys Met Leu Lys
   850 855 860
   Glu Gly Ala Thr Ala Ser Glu Tyr Lys Ala Leu Met Thr Glu Leu Lys
   865 870 875 880
   Ile Leu Thr His Ile Gly His His Leu Asn Val Val Asn Leu Leu Gly
   885 890 895
   Ala Cys Thr Lys Gln Gly Gly Pro Leu Met Val Ile Val Glu Tyr Cys
   900 905 910
   Lys Tyr Gly Asn Leu Ser Asn Tyr Leu Lys Ser Lys Arg Asp Leu Phe
   915 920 925
   Phe Leu Asn Lys Asp Ala Ala Leu His Met Glu Pro Lys Lys Glu Lys
   930 935 940
   Met Glu Pro Gly Leu Glu Gln Gly Lys Lys Pro Arg Leu Asp Ser Val
   945 950 955 960
   Thr Ser Ser Glu Ser Phe Ala Ser Ser Gly Phe Gln Glu Asp Lys Ser
   965 970 975
   Leu Ser Asp Val Glu Glu Glu Glu Asp Ser Asp Gly Phe Tyr Lys Glu
   980 985 990
   Pro Ile Thr Met Glu Asp Leu Ile Ser Tyr Ser Phe Gln Val Ala Arg
   995 1000 1005
   Gly Met Glu Phe Leu Ser Ser Arg Lys Cys Ile His Arg Asp Leu
   1010 1015 1020
   Ala Ala Arg Asn Ile Leu Leu Ser Glu Asn Asn Val Val Lys Ile
   1025 1030 1035
   Cys Asp Phe Gly Leu Ala Arg Asp Ile Tyr Lys Asn Pro Asp Tyr
   1040 1045 1050
   Val Arg Lys Gly Asp Thr Arg Leu Pro Leu Lys Trp Met Ala Pro
   1055 1060 1065
   Glu Ser Ile Phe Asp Lys Ile Tyr Ser Thr Lys Ser Asp Val Trp
   1070 1075 1080
   Ser Tyr Gly Val Leu Leu Trp Glu Ile Phe Ser Leu Gly Gly Ser
   1085 1090 1095
   Pro Tyr Pro Gly Val Gln Met Asp Glu Asp Phe Cys Ser Arg Leu
   1100 1105 1110
   Arg Glu Gly Met Arg Met Arg Ala Pro Glu Tyr Ser Thr Pro Glu
   1115 1120 1125
   Ile Tyr Gln Ile Met Leu Asp Cys Trp His Arg Asp Pro Lys Glu
   1130 1135 1140
   Arg Pro Arg Phe Ala Glu Leu Val Glu Lys Leu Gly Asp Leu Leu
   1145 1150 1155
   Gln Ala Asn Val Gln Gln Asp Gly Lys Asp Tyr Ile Pro Ile Asn
   1160 1165 1170
   Ala Ile Leu Thr Gly Asn Ser Gly Phe Thr Tyr Ser Thr Pro Ala
   1175 1180 1185
   Phe Ser Glu Asp Phe Phe Lys Glu Ser Ile Ser Ala Pro Lys Phe
   1190 1195 1200
   Asn Ser Gly Ser Ser Asp Asp Val Arg Tyr Val Asn Ala Phe Lys
   1205 1210 1215
   Phe Met Ser Leu Glu Arg Ile Lys Thr Phe Glu Glu Leu Leu Pro
   1220 1225 1230
   Asn Ala Thr Ser Met Phe Asp Asp Tyr Gln Gly Asp Ser Ser Thr
   1235 1240 1245
   Leu Leu Ala Ser Pro Met Leu Lys Arg Phe Thr Trp Thr Asp Ser
   1250 1255 1260
   Lys Pro Lys Ala Ser Leu Lys Ile Asp Leu Arg Val Thr Ser Lys
   1265 1270 1275
   Ser Lys Glu Ser Gly Leu Ser Asp Val Ser Arg Pro Ser Phe Cys
   1280 1285 1290
   His Ser Ser Cys Gly His Val Ser Glu Gly Lys Arg Arg Phe Thr
   1295 1300 1305
   Tyr Asp His Ala Glu Leu Glu Arg Lys Ile Ala Cys Cys Ser Pro
   1310 1315 1320
   Pro Pro Asp Tyr Asn Ser Val Val Leu Tyr Ser Thr Pro Pro Ile
   1325 1330 1335
<210> 532
   <211> 159
   <212> PRT
   <213> Homo sapiens
<400> 532
   Met Ala Leu Glu Thr Ile Cys Arg Pro Ser Gly Arg Lys Ser Ser Lys
   1               5                   10                  15
   Met Gln Ala Phe Arg Ile Trp Asp Val Asn Gln Lys Thr Phe Tyr Leu
               20                  25                  30
   Arg Asn Asn Gln Leu Val Ala Gly Tyr Leu Gln Gly Pro Asn Val Asn
           35                  40                  45
   Leu Glu Glu Lys Ile Asp Val Val Pro Ile Glu Pro His Ala Leu Phe
       50                  55                  60
   Leu Gly Ile His Gly Gly Lys Met Cys Leu Ser Cys Val Lys Ser Gly
   65                  70                  75                  80
   Asp Glu Thr Arg Leu Gln Leu Glu Ala Val Asn Ile Thr Asp Leu Ser
                   85                  90                  95
   Glu Asn Arg Lys Gln Asp Lys Arg Phe Ala Phe Ile Arg Ser Asp Ser
               100                 105                 110
   Gly Pro Thr Thr Ser Phe Glu Ser Ala Ala Cys Pro Gly Trp Phe Leu
           115                 120                 125
   Cys Thr Ala Met Glu Ala Asp Gln Pro Val Ser Leu Thr Asn Met Pro
       130                 135                 140
   Asp Glu Gly Val Met Val Thr Lys Phe Tyr Phe Gln Glu Asp Glu
   145                 150                 155
<210> 533
   <211> 180
   <212> PRT
   <213> Homo sapiens
<400> 533
   Met Ala Leu Ala Asp Leu Tyr Glu Glu Gly Gly Gly Gly Gly Gly Glu
   1               5                   10                  15
   Gly Glu Asp Asn Ala Asp Ser Lys Glu Thr Ile Cys Arg Pro Ser Gly
               20                  25                  30
   Arg Lys Ser Ser Lys Met Gln Ala Phe Arg Ile Trp Asp Val Asn Gln
           35                  40                  45
   Lys Thr Phe Tyr Leu Arg Asn Asn Gln Leu Val Ala Gly Tyr Leu Gln
       50                  55                  60
   Gly Pro Asn Val Asn Leu Glu Glu Lys Ile Asp Val Val Pro Ile Glu
   65                  70                  75                  80
   Pro His Ala Leu Phe Leu Gly Ile His Gly Gly Lys Met Cys Leu Ser
                   85                  90                  95
   Cys Val Lys Ser Gly Asp Glu Thr Arg Leu Gln Leu Glu Ala Val Asn
               100                 105                 110
   Ile Thr Asp Leu Ser Glu Asn Arg Lys Gln Asp Lys Arg Phe Ala Phe
           115                 120                 125
   Ile Arg Ser Asp Ser Gly Pro Thr Thr Ser Phe Glu Ser Ala Ala Cys
       130                 135                 140
   Pro Gly Trp Phe Leu Cys Thr Ala Met Glu Ala Asp Gln Pro Val Ser
   145                 150                 155                 160
   Leu Thr Asn Met Pro Asp Glu Gly Val Met Val Thr Lys Phe Tyr Phe
                   165                 170                 175
   Gln Glu Asp Glu
               180
<210> 534
   <211> 177
   <212> PRT
   <213> Homo sapiens
<400> 534
   Met Glu Ile Cys Arg Gly Leu Arg Ser His Leu Ile Thr Leu Leu Leu
   1               5                   10                  15
   Phe Leu Phe His Ser Glu Thr Ile Cys Arg Pro Ser Gly Arg Lys Ser
               20                  25                  30
   Ser Lys Met Gln Ala Phe Arg Ile Trp Asp Val Asn Gln Lys Thr Phe
           35                  40                  45
   Tyr Leu Arg Asn Asn Gln Leu Val Ala Gly Tyr Leu Gln Gly Pro Asn
       50                  55                  60
   Val Asn Leu Glu Glu Lys Ile Asp Val Val Pro Ile Glu Pro His Ala
   65                  70                  75                  80
   Leu Phe Leu Gly Ile His Gly Gly Lys Met Cys Leu Ser Cys Val Lys
                   85                  90                  95
   Ser Gly Asp Glu Thr Arg Leu Gln Leu Glu Ala Val Asn Ile Thr Asp
               100                 105                 110
   Leu Ser Glu Asn Arg Lys Gln Asp Lys Arg Phe Ala Phe Ile Arg Ser
           115                 120                 125
   Asp Ser Gly Pro Thr Thr Ser Phe Glu Ser Ala Ala Cys Pro Gly Trp
       130                 135                 140
   Phe Leu Cys Thr Ala Met Glu Ala Asp Gln Pro Val Ser Leu Thr Asn
   145                 150                 155                 160
   Met Pro Asp Glu Gly Val Met Val Thr Lys Phe Tyr Phe Gln Glu Asp
                   165                 170                 175
   Glu
<210> 535
   <211> 143
   <212> PRT
   <213> Homo sapiens
<400> 535
   Met Gln Ala Phe Arg Ile Trp Asp Val Asn Gln Lys Thr Phe Tyr Leu
   1 5 10 15
   Arg Asn Asn Gln Leu Val Ala Gly Tyr Leu Gln Gly Pro Asn Val Asn
   20 25 30
   Leu Glu Glu Lys Ile Asp Val Val Pro Ile Glu Pro His Ala Leu Phe
   35 40 45
   Leu Gly Ile His Gly Gly Lys Met Cys Leu Ser Cys Val Lys Ser Gly
   50 55 60
   Asp Glu Thr Arg Leu Gln Leu Glu Ala Val Asn Ile Thr Asp Leu Ser
   65 70 75 80
   Glu Asn Arg Lys Gln Asp Lys Arg Phe Ala Phe Ile Arg Ser Asp Ser
   85 90 95
   Gly Pro Thr Thr Ser Phe Glu Ser Ala Ala Cys Pro Gly Trp Phe Leu
   100 105 110
   Cys Thr Ala Met Glu Ala Asp Gln Pro Val Ser Leu Thr Asn Met Pro
   115 120 125
   Asp Glu Gly Val Met Val Thr Lys Phe Tyr Phe Gln Glu Asp Glu
   130 135 140
<210> 536
   <211> 221
   <212> PRT
   <213> Homo sapiens
<400> 536
   Met Pro Val Met Arg Leu Phe Pro Cys Phe Leu Gln Leu Leu Ala Gly
   1 5 10 15
   Leu Ala Leu Pro Ala Val Pro Pro Gln Gln Trp Ala Leu Ser Ala Gly
   20 25 30
   Asn Gly Ser Ser Glu Val Glu Val Val Pro Phe Gln Glu Val Trp Gly
   35 40 45
   Arg Ser Tyr Cys Arg Ala Leu Glu Arg Leu Val Asp Val Val Ser Glu
   50 55 60
   Tyr Pro Ser Glu Val Glu His Met Phe Ser Pro Ser Cys Val Ser Leu
   65 70 75 80
   Leu Arg Cys Thr Gly Cys Cys Gly Asp Glu Asn Leu His Cys Val Pro
   85 90 95
   Val Glu Thr Ala Asn Val Thr Met Gln Leu Leu Lys Ile Arg Ser Gly
   100 105 110
   Asp Arg Pro Ser Tyr Val Glu Leu Thr Phe Ser Gln His Val Arg Cys
   115 120 125
   Glu Cys Arg His Ser Pro Gly Arg Gln Ser Pro Asp Met Pro Gly Asp
   130 135 140
   Phe Arg Ala Asp Ala Pro Ser Phe Leu Pro Pro Arg Arg Ser Leu Pro
   145 150 155 160
   Met Leu Phe Arg Met Glu Trp Gly Cys Ala Leu Thr Gly Ser Gln Ser
   165 170 175
   Ala Val Trp Pro Ser Ser Pro Val Pro Glu Glu Ile Pro Arg Met His
   180 185 190
   Pro Gly Arg Asn Gly Lys Lys Gln Gln Arg Lys Pro Leu Arg Glu Lys
   195 200 205
   Met Lys Pro Glu Arg Cys Gly Asp Ala Val Pro Arg Arg
   210 215 220
<210> 537
   <211> 221
   <212> PRT
   <213> Homo sapiens
<400> 537
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg His Ser Pro Gly Arg Gln Ser Pro Asp Met Pro Gly Asp
       130                 135                 140
   Phe Arg Ala Asp Ala Pro Ser Phe Leu Pro Pro Arg Arg Ser Leu Pro
   145                 150                 155                 160
   Met Leu Phe Arg Met Glu Trp Gly Cys Ala Leu Thr Gly Ser Gln Ser
                   165                 170                 175
   Ala Val Trp Pro Ser Ser Pro Val Pro Glu Glu Ile Pro Arg Met His
               180                 185                 190
   Pro Gly Arg Asn Gly Lys Lys Gln Gln Arg Lys Pro Leu Arg Glu Lys
           195                 200                 205
   Met Lys Pro Glu Arg Cys Gly Asp Ala Val Pro Arg Arg
       210                 215                 220
<210> 538
   <211> 170
   <212> PRT
   <213> Homo sapiens
<400> 538
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg Arg Arg Pro
       130                 135                 140
   Lys Gly Arg Gly Lys Arg Arg Arg Glu Lys Gln Arg Pro Thr Asp Cys
   145                 150                 155                 160
   His Leu Cys Gly Asp Ala Val Pro Arg Arg
                   165                 170
<210> 539
   <211> 381
   <212> PRT
   <213> Homo sapiens
<400> 539
<210> 540
   <211> 381
   <212> PRT
   <213> Homo sapiens
<400> 540
<210> 541
   <211> 185
   <212> PRT
   <213> Homo sapiens
<400> 541
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Arg Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 542
   <211> 160
   <212> PRT
   <213> Homo sapiens
<400> 542
   Ser Pro Gly Gln Gly Thr Gln Ser Glu Asn Ser Cys Thr His Phe Pro
   1               5                   10                  15
   Gly Asn Leu Pro Asn Met Leu Arg Asp Leu Arg Asp Ala Phe Ser Arg
               20                  25                  30
   Val Lys Thr Phe Phe Gln Met Lys Asp Gln Leu Asp Asn Leu Leu Leu
           35                  40                  45
   Lys Glu Ser Leu Leu Glu Asp Phe Lys Gly Tyr Leu Gly Cys Gln Ala
       50                  55                  60
   Leu Ser Glu Met Ile Gln Phe Tyr Leu Glu Glu Val Met Pro Gln Ala
   65                  70                  75                  80
   Glu Asn Gln Asp Pro Asp Ile Lys Ala His Val Asn Ser Leu Gly Glu
                   85                  90                  95
   Asn Leu Lys Thr Leu Arg Leu Arg Leu Arg Arg Cys His Arg Phe Leu
               100                 105                 110
   Pro Cys Glu Asn Lys Ser Lys Ala Val Glu Gln Val Lys Asn Ala Phe
           115                 120                 125
   Asn Lys Leu Gln Glu Lys Gly Ile Tyr Lys Ala Met Ser Glu Phe Asp
       130                 135                 140
   Ile Phe Ile Asn Tyr Ile Glu Ala Tyr Met Thr Met Lys Ile Arg Asn
   145                 150                 155                 160
<210> 543
   <211> 178
   <212> PRT
   <213> Homo sapiens
<400> 543
   Met His Ser Ser Ala Leu Leu Cys Cys Leu Val Leu Leu Thr Gly Val
   1               5                   10                  15
   Arg Ala Ser Pro Gly Gln Gly Thr Gln Ser Glu Asn Ser Cys Thr His
               20                  25                  30
   Phe Pro Gly Asn Leu Pro Asn Met Leu Arg Asp Leu Arg Asp Ala Phe
           35                  40                  45
   Ser Arg Val Lys Thr Phe Phe Gln Met Lys Asp Gln Leu Asp Asn Leu
       50                  55                  60
   Leu Leu Lys Glu Ser Leu Leu Glu Asp Phe Lys Gly Tyr Leu Gly Cys
   65 70 75 80
   Gln Ala Leu Ser Glu Met Ile Gln Phe Tyr Leu Glu Glu Val Met Pro
   85 90 95
   Gln Ala Glu Asn Gln Asp Pro Asp Ile Lys Ala His Val Asn Ser Leu
   100 105 110
   Gly Glu Asn Leu Lys Thr Leu Arg Leu Arg Leu Arg Arg Cys His Arg
   115 120 125
   Phe Leu Pro Cys Glu Asn Lys Ser Lys Ala Val Glu Gln Val Lys Asn
   130 135 140
   Ala Phe Asn Lys Leu Gln Glu Lys Gly Ile Tyr Lys Ala Met Ser Glu
   145 150 155 160
   Phe Asp Ile Phe Ile Asn Tyr Ile Glu Ala Tyr Met Thr Met Lys Ile
   165 170 175
   Arg Asn
<210> 544
   <211> 178
   <212> PRT
   <213> Homo sapiens
<400> 544
   Met His Ser Ser Ala Leu Leu Cys Cys Leu Val Leu Leu Thr Gly Val
   1 5 10 15
   Arg Ala Ser Pro Gly Gln Gly Thr Gln Ser Glu Asn Ser Cys Thr His
   20 25 30
   Phe Pro Gly Asn Leu Pro Asn Met Leu Arg Asp Leu Arg Asp Ala Phe
   35 40 45
   Ser Arg Val Lys Thr Phe Phe Gln Met Lys Asp Gln Leu Asp Asn Leu
   50 55 60
   Leu Leu Lys Glu Ser Leu Leu Glu Asp Phe Lys Gly Tyr Leu Gly Cys
   65 70 75 80
   Gln Ala Leu Ser Glu Met Ile Gln Phe Tyr Leu Glu Glu Val Met Pro
   85 90 95
   Gln Ala Glu Asn Gln Asp Pro Asp Ile Lys Ala His Val Asn Ser Leu
   100 105 110
   Gly Glu Asn Leu Lys Thr Leu Arg Leu Arg Leu Arg Arg Cys His Arg
   115 120 125
   Phe Leu Pro Cys Glu Asn Lys Ser Lys Ala Val Glu Gln Val Lys Asn
   130 135 140
   Ala Phe Asn Lys Leu Gln Glu Lys Gly Ile Tyr Lys Ala Met Ser Glu
   145 150 155 160
   Phe Asp Ile Phe Ile Asn Tyr Ile Glu Ala Tyr Met Thr Met Lys Ile
   165 170 175
   Arg Asn
<210> 545
   <211> 178
   <212> PRT
   <213> Homo sapiens
<400> 545
   Met His Ser Ser Ala Leu Leu Cys Cys Leu Val Leu Leu Thr Gly Val
   1               5                   10                  15
   Arg Ala Ser Pro Gly Gln Gly Thr Gln Ser Glu Asn Ser Cys Thr His
               20                  25                  30
   Phe Pro Gly Asn Leu Pro Asn Met Leu Arg Asp Leu Arg Asp Ala Phe
           35                  40                  45
   Ser Arg Val Lys Thr Phe Phe Gln Met Lys Asp Gln Leu Asp Asn Leu
       50                  55                  60
   Leu Leu Lys Glu Ser Leu Leu Glu Asp Phe Lys Gly Tyr Leu Gly Cys
   65                  70                  75                  80
   Gln Ala Leu Ser Glu Met Ile Gln Phe Tyr Leu Glu Glu Val Met Pro
                   85                  90                  95
   Gln Ala Glu Asn Gln Asp Pro Asp Ile Lys Ala His Val Asn Ser Leu
               100                 105                 110
   Gly Glu Asn Leu Lys Thr Leu Arg Leu Arg Leu Arg Arg Cys Leu Arg
           115                 120                 125
   Phe Leu Pro Cys Glu Asn Lys Ser Lys Ala Val Glu Gln Val Lys Asn
       130                 135                 140
   Ala Phe Asn Lys Leu Gln Glu Lys Gly Ile Tyr Lys Ala Met Ser Glu
   145                 150                 155                 160
   Phe Asp Ile Phe Ile Asn Tyr Ile Glu Ala Tyr Met Thr Met Lys Ile
                   165                 170                 175
   Arg Asn
<210> 546
   <211> 55
   <212> PRT
   <213> Homo sapiens
<400> 546
   Met His Ser Ser Ala Leu Leu Cys Cys Leu Val Leu Leu Thr Gly Val
   1 5 10 15
   Arg Ala Ser Pro Gly Gln Gly Thr Gln Ser Glu Asn Ser Cys Thr His
   20 25 30
   Phe Pro Gly Asn Leu Pro Asn Met Leu Arg Asp Leu Arg Asp Ala Phe
   35 40 45
   Ser Arg Val Lys Thr Phe Phe
   50 55
<210> 547
   <211> 159
   <212> PRT
   <213> Homo sapiens
<400> 547
   Met Ala Thr Ala Gly Asn Pro Trp Gly Trp Phe Leu Gly Tyr Leu Ile
   1 5 10 15
   Leu Gly Val Ala Gly Ser Leu Val Ser Gly Ser Cys Ser Gln Ile Ile
   20 25 30
   Asn Gly Glu Asp Cys Ser Pro His Ser Gln Pro Trp Gln Ala Ala Leu
   35 40 45
   Val Met Glu Asn Glu Leu Phe Cys Ser Gly Val Leu Val His Pro Gln
   50 55 60
   Trp Val Leu Ser Ala Ala His Cys Phe Gln Asn Ser Tyr Thr Ile Gly
   65 70 75 80
   Leu Gly Leu His Ser Leu Glu Ala Asp Gln Glu Pro Gly Ser Gln Met
   85 90 95
   Val Glu Ala Ser Leu Ser Val Arg His Pro Glu Tyr Asn Arg Pro Leu
   100 105 110
   Leu Ala Asn Asp Leu Met Leu Ile Lys Leu Asp Glu Ser Val Ser Glu
   115 120 125
   Ser Asp Thr Ile Arg Ser Ile Ser Ile Ala Ser Gln Cys Pro Thr Ala
   130 135 140
   Gly Asn Ser Cys Leu Val Ser Gly Trp Gly Leu Leu Ala Asn Gly
   145 150 155
<210> 548
   <211> 195
   <212> PRT
   <213> Homo sapiens
<400> 548
   Met Ala Thr Ala Gly Asn Pro Trp Gly Trp Phe Leu Gly Tyr Leu Ile
   1               5                   10                  15
   Leu Gly Val Ala Gly Ser Leu Val Ser Gly Ser Cys Ser Gln Ile Ile
               20                  25                  30
   Asn Gly Glu Asp Cys Ser Pro His Ser Gln Pro Trp Gln Ala Ala Leu
           35                  40                  45
   Val Met Glu Asn Glu Leu Phe Cys Ser Gly Val Leu Val His Pro Gln
       50                  55                  60
   Trp Val Leu Ser Ala Ala His Cys Phe Gln Asn Ser Tyr Thr Ile Gly
   65                  70                  75                  80
   Leu Gly Leu His Ser Leu Glu Ala Asp Gln Glu Pro Gly Ser Gln Met
                   85                  90                  95
   Val Glu Ala Ser Leu Ser Val Arg His Pro Glu Tyr Asn Arg Pro Leu
               100                 105                 110
   Leu Ala Asn Asp Leu Met Leu Ile Lys Leu Asp Glu Ser Val Ser Glu
           115                 120                 125
   Ser Asp Thr Ile Arg Ser Ile Ser Ile Ala Ser Gln Cys Pro Thr Ala
       130                 135                 140
   Gly Asn Ser Cys Leu Val Ser Gly Trp Gly Leu Leu Ala Asn Gly Glu
   145                 150                 155                 160
   Leu Thr Gly Val Cys Leu Pro Ser Ser Arg Arg Ser Ser Ala Gln Ser
                   165                 170                 175
   Arg Gly Leu Thr Gln Ser Ser Ala Ser Gln Ala Glu Cys Leu Pro Cys
               180                 185                 190
   Cys Ser Ala
           195
<210> 549
   <211> 331
   <212> PRT
   <213> Homo sapiens
<400> 549
   Met Glu Asn Pro Ser Pro Ala Ala Ala Leu Gly Lys Ala Leu Cys Ala
   1               5                   10                  15
   Leu Leu Leu Ala Thr Leu Gly Ala Ala Gly Gln Pro Leu Gly Gly Glu
               20                  25                  30
   Ser Ile Cys Ser Ala Arg Ala Pro Ala Lys Tyr Ser Ile Thr Phe Thr
           35                  40                  45
   Gly Lys Trp Ser Gln Thr Ala Phe Pro Lys Gln Tyr Pro Leu Phe Arg
       50                  55                  60
   Pro Pro Ala Gln Trp Ser Ser Leu Leu Gly Ala Ala His Ser Ser Asp
   65                  70                  75                  80
   Tyr Ser Met Trp Arg Lys Asn Gln Tyr Val Ser Asn Gly Leu Arg Asp
                   85                  90                  95
   Phe Ala Glu Arg Gly Glu Ala Trp Ala Leu Met Lys Glu Ile Glu Ala
               100                 105                 110
   Ala Gly Glu Ala Leu Gln Ser Val His Ala Val Phe Ser Ala Pro Ala
           115                 120                 125
   Val Pro Ser Gly Thr Gly Gln Thr Ser Ala Glu Leu Glu Val Gln Arg
       130                 135                 140
   Arg His Ser Leu Val Ser Phe Val Val Arg Ile Val Pro Ser Pro Asp
   145                 150                 155                 160
   Trp Phe Val Gly Val Asp Ser Leu Asp Leu Cys Asp Gly Asp Arg Trp
                   165                 170                 175
   Arg Glu Gln Ala Ala Leu Asp Leu Tyr Pro Tyr Asp Ala Gly Thr Asp
               180                 185                 190
   Ser Gly Phe Thr Phe Ser Ser Pro Asn Phe Ala Thr Ile Pro Gln Asp
           195                 200                 205
   Thr Val Thr Glu Ile Thr Ser Ser Ser Pro Ser His Pro Ala Asn Ser
       210                 215                 220
   Phe Tyr Tyr Pro Arg Leu Lys Ala Leu Pro Pro Ile Ala Arg Val Thr
   225                 230                 235                 240
   Leu Val Arg Leu Arg Gln Ser Pro Arg Ala Phe Ile Pro Pro Ala Pro
                   245                 250                 255
   Val Leu Pro Ser Arg Asp Asn Glu Ile Val Asp Ser Ala Ser Val Pro
               260                 265                 270
   Glu Thr Pro Leu Asp Cys Glu Val Ser Leu Trp Ser Ser Trp Gly Leu
           275                 280                 285
   Cys Gly Gly His Cys Gly Arg Leu Gly Thr Lys Ser Arg Thr Arg Tyr
       290                 295                 300
   Val Arg Val Gln Pro Ala Asn Asn Gly Ser Pro Cys Pro Glu Leu Glu
   305                 310                 315                 320
   Glu Glu Ala Glu Cys Val Pro Asp Asn Cys Val
                   325                 330
<210> 550
   <211> 132
   <212> PRT
   <213> Homo sapiens
<400> 550
   Met Lys Ser Ser Gly Leu Phe Pro Phe Leu Val Leu Leu Ala Leu Gly
   1               5                   10                  15
   Thr Leu Ala Pro Trp Ala Val Glu Gly Ser Gly Lys Ser Phe Lys Ala
               20                  25                  30
   Gly Val Cys Pro Pro Lys Lys Ser Ala Gln Cys Leu Arg Tyr Lys Lys
           35                  40                  45
   Pro Glu Cys Gln Ser Asp Trp Gln Cys Pro Gly Lys Lys Arg Cys Cys
       50                  55                  60
   Pro Asp Thr Cys Gly Ile Lys Cys Leu Asp Pro Val Asp Thr Pro Asn
   65                  70                  75                  80
   Pro Thr Arg Arg Lys Pro Gly Lys Cys Pro Val Thr Tyr Gly Gln Cys
                   85                  90                  95
   Leu Met Leu Asn Pro Pro Asn Phe Cys Glu Met Asp Gly Gln Cys Lys
               100                 105                 110
   Arg Asp Leu Lys Cys Cys Met Gly Met Cys Gly Lys Ser Cys Val Ser
           115                 120                 125
   Pro Val Lys Ala
       130

<210> 551
   <211> 585
   <212> PRT
   <213> Homo sapiens
<400> 551
   Met Thr Pro Pro Arg Leu Phe Trp Val Trp Leu Leu Val Ala Gly Thr
   1               5                   10                  15
   Gln Gly Val Asn Asp Gly Asp Met Arg Leu Ala Asp Gly Gly Ala Thr
               20                  25                  30
   Asn Gln Gly Arg Val Glu Ile Phe Tyr Arg Gly Gln Trp Gly Thr Val
           35                  40                  45
   Cys Asp Asn Leu Trp Asp Leu Thr Asp Ala Ser Val Val Cys Arg Ala
       50                  55                  60
   Leu Gly Phe Glu Asn Ala Thr Gln Ala Leu Gly Arg Ala Ala Phe Gly
   65                  70                  75                  80
   Gln Gly Ser Gly Pro Ile Met Leu Asp Glu Val Gln Cys Thr Gly Thr
                   85                  90                  95
   Glu Ala Ser Leu Ala Asp Cys Lys Ser Leu Gly Trp Leu Lys Ser Asn
               100                 105                 110
   Cys Arg His Glu Arg Asp Ala Gly Val Val Cys Thr Asn Glu Thr Arg
           115                 120                 125
   Ser Thr His Thr Leu Asp Leu Ser Arg Glu Leu Ser Glu Ala Leu Gly
       130                 135                 140
   Gln Ile Phe Asp Ser Gln Arg Gly Cys Asp Leu Ser Ile Ser Val Asn
   145 150 155 160
   Val Gln Gly Glu Asp Ala Leu Gly Phe Cys Gly His Thr Val Ile Leu
   165 170 175
   Thr Ala Asn Leu Glu Ala Gln Ala Leu Trp Lys Glu Pro Gly Ser Asn
   180 185 190
   Val Thr Met Ser Val Asp Ala Glu Cys Val Pro Met Val Arg Asp Leu
   195 200 205
   Leu Arg Tyr Phe Tyr Ser Arg Arg Ile Asp Ile Thr Leu Ser Ser Val
   210 215 220
   Lys Cys Phe His Lys Leu Ala Ser Ala Tyr Gly Ala Arg Gln Leu Gln
   225 230 235 240
   Gly Tyr Cys Ala Ser Leu Phe Ala Ile Leu Leu Pro Gln Asp Pro Ser
   245 250 255
   Phe Gln Met Pro Leu Asp Leu Tyr Ala Tyr Ala Val Ala Thr Gly Asp
   260 265 270
   Ala Leu Leu Glu Lys Leu Cys Leu Gln Phe Leu Ala Trp Asn Phe Glu
   275 280 285
   Ala Leu Thr Gln Ala Glu Ala Trp Pro Ser Val Pro Thr Asp Leu Leu
   290 295 300
   Gln Leu Leu Leu Pro Arg Ser Asp Leu Ala Val Pro Ser Glu Leu Ala
   305 310 315 320
   Leu Leu Lys Ala Val Asp Thr Trp Ser Trp Gly Glu Arg Ala Ser His
   325 330 335
   Glu Glu Val Glu Gly Leu Val Glu Lys Ile Arg Phe Pro Met Met Leu
   340 345 350
   Pro Glu Glu Leu Phe Glu Leu Gln Phe Asn Leu Ser Leu Tyr Trp Ser
   355 360 365
   His Glu Ala Leu Phe Gln Lys Lys Thr Leu Gln Ala Leu Glu Phe His
   370 375 380
   Thr Val Pro Phe Gln Leu Leu Ala Arg Tyr Lys Gly Leu Asn Leu Thr
   385 390 395 400
   Glu Asp Thr Tyr Lys Pro Arg Ile Tyr Thr Ser Pro Thr Trp Ser Ala
   405 410 415
   Phe Val Thr Asp Ser Ser Trp Ser Ala Arg Lys Ser Gln Leu Val Tyr
   420 425 430
   Gln Ser Arg Arg Gly Pro Leu Val Lys Tyr Ser Ser Asp Tyr Phe Gln
   435 440 445
   Ala Pro Ser Asp Tyr Arg Tyr Tyr Pro Tyr Gln Ser Phe Gln Thr Pro
   450 455 460
   Gln His Pro Ser Phe Leu Phe Gln Asp Lys Arg Val Ser Trp Ser Leu
   465 470 475 480
   Val Tyr Leu Pro Thr Ile Gln Ser Cys Trp Asn Tyr Gly Phe Ser Cys
   485 490 495
   Ser Ser Asp Glu Leu Pro Val Leu Gly Leu Thr Lys Ser Gly Gly Ser
   500 505 510
   Asp Arg Thr Ile Ala Tyr Glu Asn Lys Ala Leu Met Leu Cys Glu Gly
   515 520 525
   Leu Phe Val Ala Asp Val Thr Asp Phe Glu Gly Trp Lys Ala Ala Ile
   530 535 540
   Pro Ser Ala Leu Asp Thr Asn Ser Ser Lys Ser Thr Ser Ser Phe Pro
   545 550 555 560
   Cys Pro Ala Gly His Phe Asn Gly Phe Arg Thr Val Ile Arg Pro Phe
   565 570 575
   Tyr Leu Thr Asn Ser Ser Gly Val Asp
   580 585
<210> 552
   <211> 24
   <212> PRT
   <213> Homo sapiens
<400> 552
   Leu Pro Pro Ala Asp Ser Ser Phe Met Leu Pro Pro Thr Ser Phe Ser
   1 5 10 15
   Ser Asn Tyr Phe His Phe Leu Pro
   20
<210> 553
   <211> 14
   <212> PRT
   <213> Homo sapiens
<400> 553
   Glu Leu Ser Asn Phe Glu Cys Leu His Pro Cys Ser Gln Glu
   1 5 10
<210> 554
   <211> 101
   <212> PRT
   <213> Homo sapiens
<400> 554
   Met Gln Glu Arg Thr Gly Ala Ala Thr Ala Arg Arg Glu Ser Leu Pro
   1 5 10 15
   Gln Ala Asn Asn Pro Glu Gln Leu Cys Lys Gln Arg Cys Ile Asn Glu
   20 25 30
   Ala Ser Trp Thr Met Lys Arg Val Leu Ser Cys Val Pro Glu Pro Thr
   35 40 45
   Val Val Met Ala Ala Arg Ala Leu Cys Met Leu Gly Leu Val Leu Ala
   50 55 60
   Leu Leu Ser Ser Ser Ser Ala Glu Glu Tyr Val Gly Leu Ser Ala Asn
   65 70 75 80
   Gln Cys Ala Val Pro Ala Lys Asp Arg Val Asp Cys Gly Tyr Pro His
   85 90 95
   Val Thr Pro Lys Glu
   100
<210> 555
   <211> 130
   <212> PRT
   <213> Homo sapiens
<400> 555
   Met Gln Glu Arg Thr Gly Ala Ala Thr Ala Arg Arg Glu Ser Leu Pro
   1 5 10 15
   Gln Ala Asn Asn Pro Glu Gln Leu Cys Lys Gln Arg Cys Ile Asn Glu
   20 25 30
   Ala Ser Trp Thr Met Lys Arg Val Leu Ser Cys Val Pro Glu Pro Thr
   35 40 45
   Val Val Met Ala Ala Arg Ala Leu Cys Met Leu Gly Leu Val Leu Ala
   50 55 60
   Leu Leu Ser Ser Ser Ser Ala Glu Glu Tyr Val Gly Leu Ser Ala Asn
   65 70 75 80
   Gln Cys Ala Val Pro Ala Lys Asp Arg Val Asp Cys Gly Tyr Pro His
   85 90 95
   Val Thr Pro Lys Glu Cys Asn Asn Arg Gly Cys Cys Phe Asp Ser Arg
   100 105 110
   Ile Pro Gly Val Pro Trp Cys Phe Lys Pro Leu Gln Glu Ala Glu Cys
   115 120 125
   Thr Phe
   130
<210> 556
   <211> 20
   <212> DNA
   <213> Artificial Organism
<220>
   <223> Synthetic oligonucleotide
<400> 556
   tgggaacaag agggcatctg 20
<210> 557
   <211> 22
   <212> DNA
   <213> Artificial Organism
<220>
   <223> Synthetic oligonucleotide
<400> 557
   ccaccactgc atcaaattca tg 22
<210> 558
   <211> 19
   <212> DNA
   <213> Artificial Organism
<220>
   <223> Synthetic oligonucleotide
<400> 558
   tgagagtgat tcgcgtggg 19
<210> 559
   <211> 21
   <212> DNA
   <213> Artificial Organism
<220>
   <223> Synthetic oligonucleotide
<400> 559
   ccagggtacg aggctttcaa t 21
<210> 560
   <211> 21
   <212> DNA
   <213> Artificial Organism
<220>
   <223> Synthetic oligonucleotide
<400> 560
   tgacactggc aaaacaatgc a 21
<210> 561
   <211> 21
   <212> DNA
   <213> Artificial Organism
<220>
   <223> Synthetic oligonucleotide
<400> 561
   ggtccttttc accagcaagc t 21
<210> 562
   <211> 20
   <212> DNA
   <213> Artificial Organism
<220>
   <223> Synthetic oligonucleotide
<400> 562
   tgcaccacca actgcttagc 20
<210> 563
   <211> 19
   <212> DNA
   <213> Artificial Organism
<220>
   <223> Synthetic oligonucleotide
<400> 563
   ccatcacgcc acagtttcc 19
<210> 564
   <211> 20
   <212> DNA
   <213> Artificial Organism
<220>
   <223> Synthetic oligonucleotide
<400> 564
   gaggccgtca ccaagaacat 20
<210> 565
   <211> 19
   <212> DNA
   <213> Artificial Organism
<220>
   <223> Synthetic oligonucleotide
<400> 565
   ggacagccgg tcagagctc 19
<210> 566
   <211> 20
   <212> DNA
   <213> Artificial Organism
<220>
   <223> Synthetic oligonucleotide
<400> 566
   ctggcaagca gctggaagat 20
<210> 567
   <211> 23
   <212> DNA
   <213> Artificial Organism
<220>
   <223> Synthetic oligonucleotide
<400> 567
   tttcttagca ccaccacgaa gtc 23
<210> 568
   <211> 19
   <212> DNA
   <213> Artificial Organism
<220>
   <223> Synthetic oligonucleotide
<400> 568
   atttgggtcg cggttcttg 19
<210> 569
   <211> 21
   <212> DNA
   <213> Artificial Organism
<220>
   <223> Synthetic oligonucleotide
<400> 569
   tgccttgaca ttctcgatgg t 21
<210> 570
   <211> 23
   <212> DNA
   <213> Artificial Organism
<220>
   <223> Synthetic oligonucleotide
<400> 570
   tggcaagaag aaggtctggt tag 23
<210> 571
   <211> 22
   <212> DNA
   <213> Artificial Organism
<220>
   <223> Synthetic oligonucleotide
<400> 571
   tgatcagccc atctttgatg ag 22
<210> 572
   <211> 20
   <212> DNA
   <213> Artificial Organism
<220>
   <223> Synthetic oligonucleotide
<400> 572
   cggtttgctg cggtaatcat 20
<210> 573
   <211> 21
   <212> DNA
   <213> Artificial Organism
<220>
   <223> Synthetic oligonucleotide
<400> 573
   tttcttgctg ccagtctgga c 21
<210> 574
   <211> 1338
   <212> PRT
   <213> Homo sapiens
<400> 574
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545 550 555 560
   Val Asn Leu Glu Lys Met Pro Thr Glu Gly Glu Asp Leu Lys Leu Ser
   565 570 575
   Cys Thr Val Asn Lys Phe Leu Tyr Arg Asp Val Thr Trp Ile Leu Leu
   580 585 590
   Arg Thr Val Asn Asn Arg Thr Met His Tyr Ser Ile Ser Lys Gln Lys
   595 600 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Asp Gln Glu Ala Pro Tyr Leu Leu Arg Asn Leu Ser Asp His Thr Val
   660 665 670
   Ala Ile Ser Ser Ser Thr Thr Leu Asp Cys His Ala Asn Gly Val Pro
   675 680 685
   Glu Pro Gln Ile Thr Trp Phe Lys Asn Asn His Lys Ile Gln Gln Glu
   690 695 700
   Pro Gly Ile Ile Leu Gly Pro Gly Ser Ser Thr Leu Phe Ile Glu Arg
   705 710 715 720
   Val Thr Glu Glu Asp Glu Gly Val Tyr His Cys Lys Ala Thr Asn Gln
   725 730 735
   Lys Gly Ser Val Glu Ser Ser Ala Tyr Leu Thr Val Gln Gly Thr Ser
   740 745 750
   Asp Lys Ser Asn Leu Glu Leu Ile Thr Leu Thr Cys Thr Cys Val Ala
           755                 760                 765
   Ala Thr Leu Phe Trp Leu Leu Leu Thr Leu Phe Ile Arg Lys Met Lys
       770                 775                 780
   Arg Ser Ser Ser Glu Ile Lys Thr Asp Tyr Leu Ser Ile Ile Met Asp
   785                 790                 795                 800
   Pro Asp Glu Val Pro Leu Asp Glu Gln Cys Glu Arg Leu Pro Tyr Asp
                   805                 810                 815
   Ala Ser Lys Trp Glu Phe Ala Arg Glu Arg Leu Lys Leu Gly Lys Ser
               820                 825                 830
   Leu Gly Arg Gly Ala Phe Gly Lys Val Val Gln Ala Ser Ala Phe Gly
           835                 840                 845
   Ile Lys Lys Ser Pro Thr Cys Arg Thr Val Ala Val Lys Met Leu Lys
       850                 855                 860
   Glu Gly Ala Thr Ala Ser Glu Tyr Lys Ala Leu Met Thr Glu Leu Lys
   865                 870                 875                 880
   Ile Leu Thr His Ile Gly His His Leu Asn Val Val Asn Leu Leu Gly
                   885                 890                 895
   Ala Cys Thr Lys Gln Gly Gly Pro Leu Met Val Ile Val Glu Tyr Cys
               900                 905                 910
   Lys Tyr Gly Asn Leu Ser Asn Tyr Leu Lys Ser Lys Arg Asp Leu Phe
           915                 920                 925
   Phe Leu Asn Lys Asp Ala Ala Leu His Met Glu Pro Lys Lys Glu Lys
       930                 935                 940
   Met Glu Pro Gly Leu Glu Gln Gly Lys Lys Pro Arg Leu Asp Ser Val
   945                 950                 955                 960
   Thr Ser Ser Glu Ser Phe Ala Ser Ser Gly Phe Gln Glu Asp Lys Ser
                   965                 970                 975
   Leu Ser Asp Val Glu Glu Glu Glu Asp Ser Asp Gly Phe Tyr Lys Glu
               980                 985                 990
   Pro Ile Thr Met Glu Asp Leu Ile  Ser Tyr Ser Phe Gln  Val Ala Arg
           995                 1000                 1005
   Gly Met  Glu Phe Leu Ser Ser  Arg Lys Cys Ile His  Arg Asp Leu
       1010                 1015                 1020
   Ala Ala  Arg Asn Ile Leu Leu  Ser Glu Asn Asn Val  Val Lys Ile
       1025                 1030                 1035
   Cys Asp  Phe Gly Leu Ala Arg  Asp Ile Tyr Lys Asn  Pro Asp Tyr
       1040                 1045                 1050
   Val Arg  Lys Gly Asp Thr Arg  Leu Pro Leu Lys Trp  Met Ala Pro
       1055                 1060                 1065
   Glu Ser  Ile Phe Asp Lys Ile  Tyr Ser Thr Lys Ser  Asp Val Trp
       1070                 1075                 1080
   Ser Tyr  Gly Val Leu Leu Trp  Glu Ile Phe Ser Leu  Gly Gly Ser
       1085                 1090                 1095
   Pro Tyr  Pro Gly Val Gln Met  Asp Glu Asp Phe Cys  Ser Arg Leu
       1100                 1105                 1110
   Arg Glu  Gly Met Arg Met Arg  Ala Pro Glu Tyr Ser  Thr Pro Glu
       1115                 1120                 1125
   Ile Tyr  Gln Ile Met Leu Asp  Cys Trp His Arg Asp  Pro Lys Glu
       1130                 1135                 1140
   Arg Pro  Arg Phe Ala Glu Leu  Val Glu Lys Leu Gly  Asp Leu Leu
       1145                 1150                 1155
   Gln Ala  Asn Val Gln Gln Asp  Gly Lys Asp Tyr Ile  Pro Ile Asn
       1160                 1165                 1170
   Ala Ile  Leu Thr Gly Asn Ser  Gly Phe Thr Tyr Ser  Thr Pro Ala
       1175                 1180                 1185
   Phe Ser  Glu Asp Phe Phe Lys  Glu Ser Ile Ser Ala  Pro Lys Phe
       1190                 1195                 1200
   Asn Ser  Gly Ser Ser Asp Asp  Val Arg Tyr Val Asn  Ala Phe Lys
       1205                 1210                 1215
   Phe Met  Ser Leu Glu Arg Ile  Lys Thr Phe Glu Glu  Leu Leu Pro
       1220                 1225                 1230
   Asn Ala  Thr Ser Met Phe Asp  Asp Tyr Gln Gly Asp  Ser Ser Thr
       1235                 1240                 1245
   Leu Leu  Ala Ser Pro Met Leu  Lys Arg Phe Thr Trp  Thr Asp Ser
   1250 1255 1260
   Lys Pro Lys Ala Ser Leu Lys Ile Asp Leu Arg Val Thr Ser Lys
   1265 1270 1275
   Ser Lys Glu Ser Gly Leu Ser Asp Val Ser Arg Pro Ser Phe Cys
   1280 1285 1290
   His Ser Ser Cys Gly His Val Ser Glu Gly Lys Arg Arg Phe Thr
   1295 1300 1305
   Tyr Asp His Ala Glu Leu Glu Arg Lys Ile Ala Cys Cys Ser Pro
   1310 1315 1320
   Pro Pro Asp Tyr Asn Ser Val Val Leu Tyr Ser Thr Pro Pro Ile
   1325 1330 1335
<210> 575
   <211> 1338
   <212> PRT
   <213> Homo sapiens
<400> 575
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
   165 170 175
   Leu Ile Pro Asp Gly Lys Arg Ile Ile Trp Asp Ser Arg Lys Gly Phe
   180 185 190
   Ile Ile Ser Asn Ala Thr Tyr Lys Glu Ile Gly Leu Leu Thr Cys Glu
   195 200 205
   Ala Thr Val Asn Gly His Leu Tyr Lys Thr Asn Tyr Leu Thr His Arg
   210 215 220
   Gln Thr Asn Thr Ile Ile Asp Val Gln Ile Ser Thr Pro Arg Pro Val
   225 230 235 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Met Ala Ser Thr Leu Val Val Ala Asp Ser Arg
   515 520 525
   Ile Ser Gly Ile Tyr Ile Cys Ile Ala Ser Asn Lys Val Gly Thr Val
   530 535 540
   Gly Arg Asn Ile Ser Phe Tyr Ile Thr Asp Val Pro Asn Gly Phe His
   545 550 555 560
   Val Asn Leu Glu Lys Met Pro Thr Glu Gly Glu Asp Leu Lys Leu Ser
   565 570 575
   Cys Thr Val Asn Lys Phe Leu Tyr Arg Asp Val Thr Trp Ile Leu Leu
   580 585 590
   Arg Thr Val Asn Asn Arg Thr Met His Tyr Ser Ile Ser Lys Gln Lys
   595 600 605
   Met Ala Ile Thr Lys Glu His Ser Ile Thr Leu Asn Leu Thr Ile Met
   610 615 620
   Asn Val Ser Leu Gln Asp Ser Gly Thr Tyr Ala Cys Arg Ala Arg Asn
   625 630 635 640
   Val Tyr Thr Gly Glu Glu Ile Leu Gln Lys Lys Glu Ile Thr Ile Arg
   645 650 655
   Asp Gln Glu Ala Pro Tyr Leu Leu Arg Asn Leu Ser Asp His Thr Val
   660 665 670
   Ala Ile Ser Ser Ser Thr Thr Leu Asp Cys His Ala Asn Gly Val Pro
   675 680 685
   Glu Pro Gln Ile Thr Trp Phe Lys Asn Asn His Lys Ile Gln Gln Glu
   690 695 700
   Pro Gly Ile Ile Leu Gly Pro Gly Ser Ser Thr Leu Phe Ile Glu Arg
   705 710 715 720
   Val Thr Glu Glu Asp Glu Gly Val Tyr His Cys Lys Ala Thr Asn Gln
   725 730 735
   Lys Gly Ser Val Glu Ser Ser Ala Tyr Leu Thr Val Gln Gly Thr Ser
   740 745 750
   Asp Lys Ser Asn Leu Glu Leu Ile Thr Leu Thr Cys Thr Cys Val Ala
   755 760 765
   Ala Thr Leu Phe Trp Leu Leu Leu Thr Leu Phe Ile Arg Lys Met Lys
   770 775 780
   Arg Ser Ser Ser Glu Ile Lys Thr Asp Tyr Leu Ser Ile Ile Met Asp
   785 790 795 800
   Pro Asp Glu Val Pro Leu Asp Glu Gln Cys Glu Arg Leu Pro Tyr Asp
   805 810 815
   Ala Ser Lys Trp Glu Phe Ala Arg Glu Arg Leu Lys Leu Gly Lys Ser
   820 825 830
   Leu Gly Arg Gly Ala Phe Gly Lys Val Val Gln Ala Ser Ala Phe Gly
   835 840 845
   Ile Lys Lys Ser Pro Thr Cys Arg Thr Val Ala Val Lys Met Leu Lys
   850 855 860
   Glu Gly Ala Thr Ala Ser Glu Tyr Lys Ala Leu Met Thr Glu Leu Lys
   865 870 875 880
   Ile Leu Thr His Ile Gly His His Leu Asn Val Val Asn Leu Leu Gly
   885 890 895
   Ala Cys Thr Lys Gln Gly Gly Pro Leu Met Val Ile Val Glu Tyr Cys
   900 905 910
   Lys Tyr Gly Asn Leu Ser Asn Tyr Leu Lys Ser Lys Arg Asp Leu Phe
   915 920 925
   Phe Leu Asn Lys Asp Ala Ala Leu His Met Glu Pro Lys Lys Glu Lys
   930 935 940
   Met Glu Pro Gly Leu Glu Gln Gly Lys Lys Pro Arg Leu Asp Ser Val
   945 950 955 960
   Thr Ser Ser Glu Ser Phe Ala Ser Ser Gly Phe Gln Glu Asp Lys Ser
   965 970 975
   Leu Ser Asp Val Glu Glu Glu Glu Asp Ser Asp Gly Phe Tyr Lys Glu
   980 985 990
   Pro Ile Thr Met Glu Asp Leu Ile Ser Tyr Ser Phe Gln Val Ala Arg
   995 1000 1005
   Gly Met Glu Phe Leu Ser Ser Arg Lys Cys Ile His Arg Asp Leu
   1010 1015 1020
   Ala Ala Arg Asn Ile Leu Leu Ser Glu Asn Asn Val Val Lys Ile
   1025 1030 1035
   Cys Asp Phe Gly Leu Ala Arg Asp Ile Tyr Lys Asn Pro Asp Tyr
   1040 1045 1050
   Val Arg Lys Gly Asp Thr Arg Leu Pro Leu Lys Trp Met Ala Pro
   1055 1060 1065
   Glu Ser Ile Phe Asp Lys Ile Tyr Ser Thr Lys Ser Asp Val Trp
   1070 1075 1080
   Ser Tyr Gly Val Leu Leu Trp Glu Ile Phe Ser Leu Gly Gly Ser
   1085 1090 1095
   Pro Tyr Pro Gly Val Gln Met Asp Glu Asp Phe Cys Ser Arg Leu
   1100 1105 1110
   Arg Glu Gly Met Arg Met Arg Ala Pro Glu Tyr Ser Thr Pro Glu
   1115 1120 1125
   Ile Tyr Gln Ile Met Leu Asp Cys Trp His Arg Asp Pro Lys Glu
   1130 1135 1140
   Arg Pro Arg Phe Ala Glu Leu Val Glu Lys Leu Gly Asp Leu Leu
   1145 1150 1155
   Gln Ala Asn Val Gln Gln Asp Gly Lys Asp Tyr Ile Pro Ile Asn
   1160 1165 1170
   Ala Ile Leu Thr Gly Asn Ser Gly Phe Thr Tyr Ser Thr Pro Ala
   1175 1180 1185
   Phe Ser Glu Asp Phe Phe Lys Glu Ser Ile Ser Ala Pro Lys Phe
   1190 1195 1200
   Asn Ser Gly Ser Ser Asp Asp Val Arg Tyr Val Asn Ala Phe Lys
   1205 1210 1215
   Phe Met Ser Leu Glu Arg Ile Lys Thr Phe Glu Glu Leu Leu Pro
   1220 1225 1230
   Asn Ala Thr Ser Met Phe Asp Asp Tyr Gln Gly Asp Ser Ser Thr
   1235 1240 1245
   Leu Leu Ala Ser Pro Met Leu Lys Arg Phe Thr Trp Thr Asp Ser
   1250 1255 1260
   Lys Pro Lys Ala Ser Leu Lys Ile Asp Leu Arg Val Thr Ser Lys
   1265 1270 1275
   Ser Lys Glu Ser Gly Leu Ser Asp Val Ser Arg Pro Ser Phe Cys
   1280 1285 1290
   His Ser Ser Cys Gly His Val Ser Glu Gly Lys Arg Arg Phe Thr
   1295 1300 1305
   Tyr Asp His Ala Glu Leu Glu Arg Lys Ile Ala Cys Cys Ser Pro
   1310 1315 1320
   Pro Pro Asp Tyr Asn Ser Val Val Leu Tyr Ser Thr Pro Pro Ile
   1325 1330 1335
<210> 576
   <211> 6
   <212> PRT
   <213> Homo sapiens
<400> 576
   Met Pro Leu Pro Phe Gln
   1 5
<210> 577
   <211> 44
   <212> PRT
   <213> Homo sapiens
<400> 577
   Leu Trp Ala Ala Cys Pro Ala Gln Ala Cys Ser Gly Asn Ala Gly Gln
   1 5 10 15
   Glu Arg Gly Gly Leu Gln Ser Ala Ala Gly Leu Pro Ser Gln Pro Ser
   20 25 30
   Cys Phe Leu Gln Thr Gly Val Gly Leu Ala Asn Gln
   35 40
<210> 578
   <211> 21
   <212> PRT
   <213> Homo sapiens
<400> 578
   Met Glu Ile Cys Arg Gly Leu Arg Ser His Leu Ile Thr Leu Leu Leu
   1 5 10 15
   Phe Leu Phe His Ser
   20
<210> 579
   <211> 34
   <212> PRT
   <213> Homo sapiens
<400> 579
   Met Glu Ile Cys Arg Gly Leu Arg Ser His Leu Ile Thr Leu Leu Leu
   1 5 10 15
   Phe Leu Phe His Ser Glu Thr Ile Cys Arg Pro Ser Gly Arg Lys Ser
   20 25 30
   Ser Lys
<210> 580
   <211> 3
   <212> PRT
   <213> Homo sapiens
<400> 580
   Met Ala Leu
   1
<210> 581
   <211> 16
   <212> PRT
   <213> Homo sapiens
<400> 581
   Met Ala Leu Glu Thr Ile Cys Arg Pro Ser Gly Arg Lys Ser Ser Lys
   1 5 10 15
<210> 582
   <211> 12
   <212> PRT
   <213> Homo sapiens
<400> 582
   Met His Gln Ala Gly Tyr Pro Gly Cys Arg Gly Ala
   1 5 10
<210> 583
   <211> 49
   <212> PRT
   <213> Homo sapiens
<400> 583
   Met Asp Glu Met Gly Gln Val Gly Leu Val Gly Ser Cys Met Cys Leu
   1 5 10 15
   Gly Val Leu Cys Trp Pro Leu Pro Lys Arg Thr Ser Pro Leu Glu Leu
   20 25 30
   Gly Ala Ser Pro Thr His Val Ser Ser Thr Leu Gly Pro Leu Pro Pro
   35 40 45
   Gln
<210> 584
   <211> 28
   <212> PRT
   <213> Homo sapiens
<400> 584
   Met Met Pro Pro Ala Cys Pro Leu Ser Val Met Asp Met Glu Leu Glu
   1 5 10 15
   Ala Arg Ile Thr Asn Thr Phe Ser Phe Leu Pro Gln
   20 25
<210> 585
   <211> 29
   <212> PRT
   <213> Homo sapiens
<400> 585
   Met Met Pro Pro Ala Cys Pro Leu Ser Val Met Asp Met Glu Leu Glu
   1               5                   10                  15
   Ala Arg Ile Thr Asn Thr Phe Ser Phe Leu Pro Gln His
               20                  25
<210> 586
   <211> 18
   <212> PRT
   <213> Homo sapiens
<400> 586
   Met His Ser Ser Ala Leu Leu Cys Cys Leu Val Leu Leu Thr Gly Val
   1               5                   10                  15
   Arg Ala
<210> 587
   <211> 27
   <212> PRT
   <213> Homo sapiens
<400> 587
   Met Pro Pro Ala Cys Pro Leu Ser Val Met Asp Met Glu Leu Glu Ala
   1               5                   10                  15
   Arg Ile Thr Asn Thr Phe Ser Phe Leu Pro Gln
               20                  25
<210> 588
   <211> 28
   <212> PRT
   <213> Homo sapiens
<400> 588
   Met Pro Pro Ala Cys Pro Leu Ser Val Met Asp Met Glu Leu Glu Ala
   1               5                   10                  15
   Arg Ile Thr Asn Thr Phe Ser Phe Leu Pro Gln His
               20                  25
<210> 589
   <211> 156
   <212> PRT
   <213> Homo sapiens
<400> 589
   Met Thr Pro Pro Arg Leu Phe Trp Val Trp Leu Leu Val Ala Gly Thr
   1 5 10 15
   Gln Gly Val Asn Asp Gly Asp Met Arg Leu Ala Asp Gly Gly Ala Thr
   20 25 30
   Asn Gln Gly Arg Val Glu Ile Phe Tyr Arg Gly Gln Trp Gly Thr Val
   35 40 45
   Cys Asp Asn Leu Trp Asp Leu Thr Asp Ala Ser Val Val Cys Arg Ala
   50 55 60
   Leu Gly Phe Glu Asn Ala Thr Gln Ala Leu Gly Arg Ala Ala Phe Gly
   65 70 75 80
   Gln Gly Ser Gly Pro Ile Met Leu Asp Glu Val Gln Cys Thr Gly Thr
   85 90 95
   Glu Ala Ser Leu Ala Asp Cys Lys Ser Leu Gly Trp Leu Lys Ser Asn
   100 105 110
   Cys Arg His Glu Arg Asp Ala Gly Val Val Cys Thr Asn Gly Ala Pro
   115 120 125
   Thr Pro Trp Thr Ser Pro Gly Ser Ser Arg Arg Pro Leu Ala Arg Ser
   130 135 140
   Leu Thr Ala Ser Gly Ala Ala Thr Cys Pro Ser Ala
   145 150 155
<210> 590
   <211> 205
   <212> PRT
   <213> Homo sapiens
<400> 590
   Met Thr Pro Pro Arg Leu Phe Trp Val Trp Leu Leu Val Ala Gly Thr
   1 5 10 15
   Gln Gly Val Asn Asp Gly Asp Met Arg Leu Ala Asp Gly Gly Ala Thr
   20 25 30
   Asn Gln Gly Arg Val Glu Ile Phe Tyr Arg Gly Gln Trp Gly Thr Val
   35 40 45
   Cys Asp Asn Leu Trp Asp Leu Thr Asp Ala Ser Val Val Cys Arg Ala
   50 55 60
   Leu Gly Phe Glu Asn Ala Thr Gln Ala Leu Gly Arg Ala Ala Phe Gly
   65 70 75 80
   Gln Gly Ser Gly Pro Ile Met Leu Asp Glu Val Gln Cys Thr Gly Thr
   85 90 95
   Glu Ala Ser Leu Ala Asp Cys Lys Ser Leu Gly Trp Leu Lys Ser Asn
   100 105 110
   Cys Arg His Glu Arg Asp Ala Gly Val Val Cys Thr Asn Gly Thr Ser
   115 120 125
   Thr Pro Glu Gly Leu Thr Ser Pro Cys Arg Gln Ser Ser Ala Ser Thr
   130 135 140
   Ser Trp Pro Leu Pro Met Gly Pro Gly Ser Cys Arg Ala Thr Ala Gln
   145 150 155 160
   Ala Ser Leu Pro Ser Ser Ser Pro Arg Thr Pro Arg Ser Arg Cys Pro
   165 170 175
   Trp Thr Cys Met Pro Met Gln Trp Pro Gln Gly Thr Pro Cys Trp Arg
   180 185 190
   Ser Ser Ala Tyr Ser Ser Trp Pro Gly Thr Ser Arg Pro
   195 200 205
<210> 591
   <211> 82
   <212> PRT
   <213> Homo sapiens
<400> 591
   Met Ala Ala Arg Ala Leu Cys Met Leu Gly Leu Val Leu Ala Leu Leu
   1 5 10 15
   Ser Ser Ser Ser Ala Glu Glu Tyr Val Gly Leu Ser Gln Gln Gly Leu
   20 25 30
   Trp Gln Leu Thr Gly Leu Cys Leu Gly Gln Leu Gln Thr Ser Val Pro
   35 40 45
   Cys Gln Pro Arg Thr Gly Trp Thr Ala Ala Thr Pro Met Ser Pro Pro
   50 55 60
   Arg Ser Ala Thr Thr Gly Ala Ala Ala Leu Thr Pro Gly Ser Leu Glu
   65 70 75 80
   Cys Leu
<210> 592
   <211> 137
   <212> PRT
   <213> Homo sapiens
<400> 592
   Met Ala Ala Arg Ala Leu Cys Met Leu Gly Leu Val Leu Ala Leu Leu
   1               5                   10                  15
   Ser Ser Ser Ser Ala Glu Glu Tyr Val Gly Leu Ser Gln Gln Gly Leu
               20                  25                  30
   Trp Gln Leu Thr Gly Leu Cys Leu Gly Gln Leu Gln Thr Ser Val Pro
           35                  40                  45
   Cys Gln Pro Arg Thr Gly Trp Thr Ala Ala Thr Pro Met Ser Pro Pro
       50                  55                  60
   Arg Ser Ala Thr Thr Gly Ala Ala Ala Leu Thr Pro Gly Ser Leu Glu
   65                  70                  75                  80
   Cys Leu Gly Val Ser Ser Pro Cys Arg Lys Gln Asn Ala Pro Ser Glu
                   85                  90                  95
   Ala Pro Pro Ala Ala Pro Gly Arg Gly Met Arg Gly Ser Glu His Pro
               100                 105                 110
   Cys Pro Ala Val Ile Ala Ala Arg His Cys Ser Ser Gln Leu Phe Cys
           115                 120                 125
   Pro Phe Ala Pro Gly Lys Arg Phe Cys
       130                 135